# FASCICULE DE BREVET EUROPEEN

(11) **EP 0 516 647 B1**
(45) Date de publication et mention de la délivrance du brevet: **30.12.1998**
(21) Numéro de dépôt: 91903408.2
(22) Date de dépôt: 30.01.1991
(51) Int. Cl.: C12N 15/52, C07K 14/00, C12P 19/42, C12N 9/00, C12N 1/21

(54) **POLYPEPTIDES IMPLIQUES DANS LA BIOSYNTHESE DES COBALAMINES ET/OU DES COBAMIDES, SEQUENCES D'ADN CODANT POUR CES POLYPEPTIDES, PROCEDE DE PREPARATION, ET LEUR UTILISATION**
POLYPEPTIDE BETEILIGT AN DER KOBALAMIN- UND/ODER KOBAMID-BIOSYNTHESE, FÜR DIESE KODIERENDE DNS-SEQUENCEN, UND IHRE HERSTELLUNG UND VEWENDUNG.
POLYPEPTIDES INVOLVED IN THE BIOSYNTHESIS OF COBALAMINES AND/OR COBAMIDES, DNA SEQUENCES CODING FOR THESE POLYPEPTIDES, AND THEIR PREPARATION AND USE

(30) Priorité: 31.01.1990 FR 9001137
(43) Date de publication de la demande: 09.12.1992
(73) Titulaire: RHONE-POULENC BIOCHIMIE, 92160 Antony Cedex (FR)
(72) Inventeur: BLANCHE, Francis, F-75019 Paris (FR); CAMERON, Béatrice, F-75005 Paris (FR); CROUZET, Joel, F-75012 Paris (FR); DEBUSSCHE, Laurent, F-75013 Paris (FR); LEVY-SCHIL, Sophie, F-75007 Paris (FR); THIBAUT, Denis, F-75013 Paris (FR)
(74) Mandataire: Savina, Jacques
(86) Numéro de dépôt international: FR9100054
(87) Numéro de publication internationale: WO9111518

(56) Documents cités:
- WO-A-87/01391
- Journal of Bacteriology, volume 171, no. 1, janvier 1989, American Society for Microbiology, (Washington, DC, US) B, Cameron et al.: "Cloning and analysis of genes involved in coenzyme B12 biosynthesis in Pseudomonas denitrificans", pages 547-557
- Journal of Bacteriology, volume 171, no. 8, août 1989, American Society for Microbiology, (Washington, DC, US), F. Blanche et al.: "Purification and characterization of S-adenosyl-L-methionine: Uroporphyrinogen III methyltransferase from Pseudomonas denitrificans", pages 4222-4231
- Journal of Bacteriology, volume 169, no. 7, juillet 1987, American Society for Microbiology, (Washington, DC, US) R.M. Jeter et al.: "Cobalamin (vitamin B12) biosynthetic genes of Salmonella typhimurium", pages 3189-3198
- Journal of Bacteriology, volume 167, no. 2, août 1986, American Society for Microbiology, (Washington, DC, US), R.N. Brey et al.: "Cloning of multiple genes involved with cobalamin (vitamin B12) biosynthesis in bacillus megaterium", pages 623-630
- Journal of Bacteriology, volume 172, no. 10, octobre 1990, American Society for Microbiology, (Washington, DC, US) J. Crouzet et al.: "Nucleotide sequence of a Pseudomonas denitrificans 5,4-kilobase DNA fragment containing five cob genes and indentification of structural genes encoding S-adenosyl-L-methionine: Uroporphyrinogen III methyltransferase and cobyrinic acid a,c-diamide synthase", pages 5968-5979
- Journal of Bacteriology, volume 172, no. 10, octobre 1990, American Society for Microbiology, (Washington, DC, US), J. Crouzet et al.: "Genetic and sequence analysis of an 8,7-kilobase Pseudomonas denitrificans fragment carrying eight genes involved in transformation of precorrin-2 to cobyrinic acid", pages 5980- 5990

## Description

La présente invention concerne de nouveaux polypeptides impliqués dans la biosynthèse des cobalamines et/ou des cobamides, et en particulier du coenzyme B₁₂. Elle concerne également le matériel génétique responsable de l'expression de ces polypeptides, ainsi qu'un procédé permettant leur préparation. Elle concerne enfin un procédé d'amplification de la production de cobalamines, et plus particulièrement du coenzyme B₁₂, par les techniques d'ADN recombinant.

La vitamine B₁₂ fait partie des vitamines du groupe B. Il s'agit d'une vitamine hydrosoluble qui a été identifiée comme étant le facteur permettant de traiter des malades souffrant d'anémie pernicieuse. Elle est généralement prescrite pour stimuler l'hématopoïèse chez les sujets fatigués, mais elle est aussi utilisée dans de nombreux autres cas qui comprennent les troubles hépatiques, des déficiences nerveuses ou comme stimulant de l'appétit, principe tonifiant, ainsi qu'en dermatologie (Beck, 1982, Fraser et al., 1983). Dans les élevages industriels d'animaux non ruminants, l'alimentation étant essentiellement à base de protéines d'origines végétales, il est nécessaire d'incorporer aux rations alimentaires de la vitamine B₁₂ à des quantités de 10 à 15 mg par tonne d'aliments (Barrère et al., 1981).

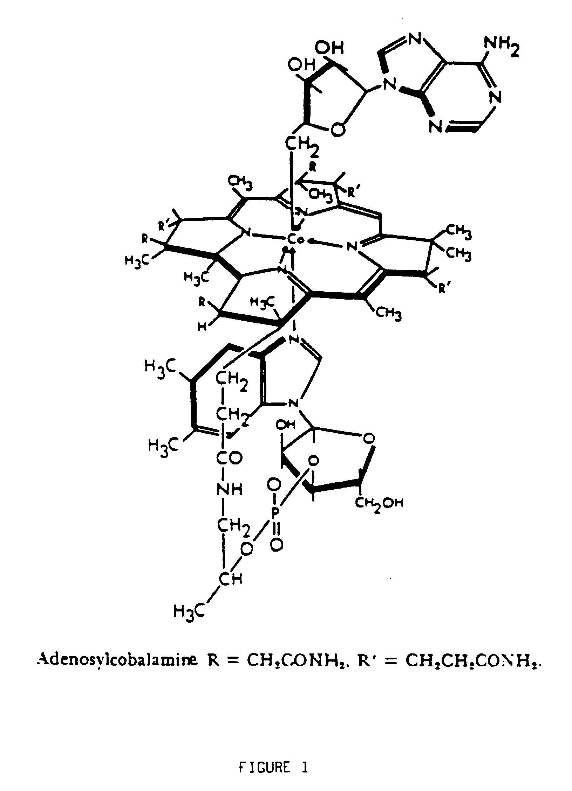

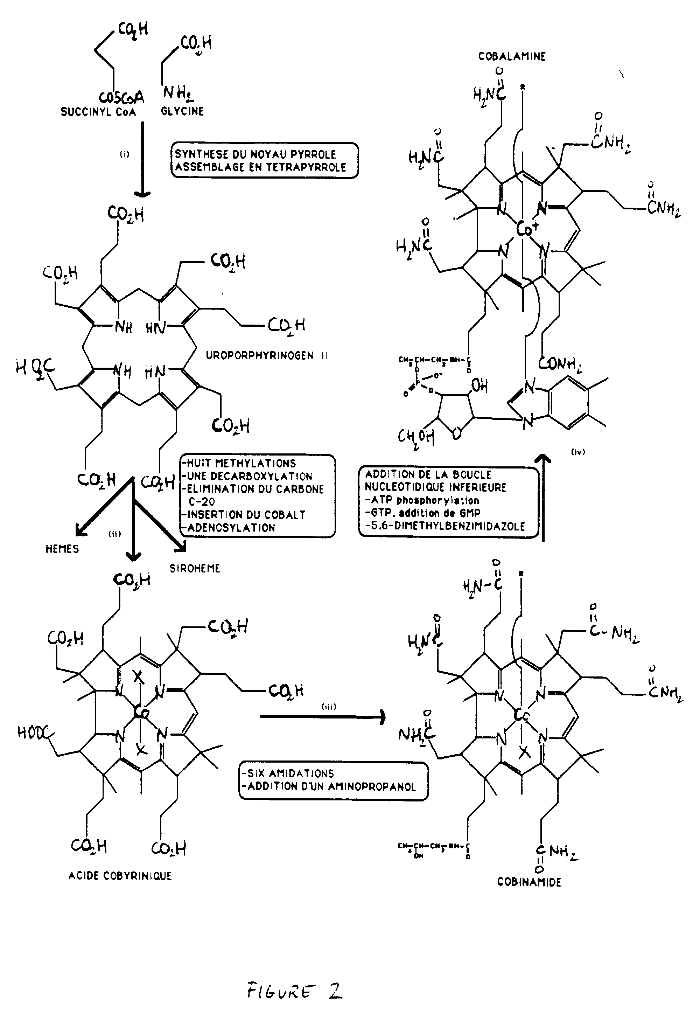

La vitamine B₁₂ fait partie d'une classe de molécules appelées cobalamines, dont la structure est présentée à la figure 1. Les cobamides diffèrent des cobalamines par la base du nucléotide inférieur qui n'est plus le 5,6-diméthylbenzimidazole, mais une autre base, par exemple, le 5-hydroxybenzimidazole, pour la vitamine B₁₂-facteur III synthétisée entre autre par Clostridium thermoaceticum et Methanosarcina barkeri (Iron et al., 1984). Ces similitudes structurales expliquent que les voies métaboliques de biosynthèse des cobalamines et des cobamides soient communes pour la majeure partie. Les cobalamines sont synthétisées presque exclusivement par des bactéries, selon un processus complexe et encore très mal connu, qui peut se diviser en quatre étapes (figure 2) :
i) synthèse de l'uroporphyrinogène III (ou uro'gen III), puis
ii) transformation de l'uro'gen III en acide cobyrinique suivie de
iii) transformation de celui-ci en cobinamide et
iv) construction de la boucle nucléotidique inférieure avec incorporation de la base particulière (5,6-diméthylbenzimidazole dans le cas des cobalamines).

Pour le coenzyme B₁₂, il est probable que l'addition du groupement 5'-déoxy-5'-adénosyl se produit peu après que le noyau corrine soit synthétisé (Huennekens et al., 1892).

Dans le cas des cobamides, seule l'étape de synthèse et d'incorporation de la base inférieure est différente.

La première partie de la biosynthèse des cobalamines est très bien connue puisqu'elle est commune à celle des hèmes ainsi qu'à celle des chlorophylles (Battersby et al., 1980). Elle fait intervenir successivement la δ-aminolevulinate synthase (EC 2.3.137), la δ-aminolévulinate déhydrase (EC 4.2.1.24), la porphobilinogène déaminase (EC 4.3.1.8) et l'uro'gen III cosynthase (EC 4.2.1.75) qui transforment le succinyl CoA et la glycine en uro'gen III. Toutefois la première étape se fait chez certains organismes [par exemple E.coli (Avissar et al., 1989) et chez les bactéries méthanogènes (Kannangara et al., 1989), par exemple] par la conversion grâce à un complexe multienzymatique de l'acide glutamique en acide δ-aminolévulinique.

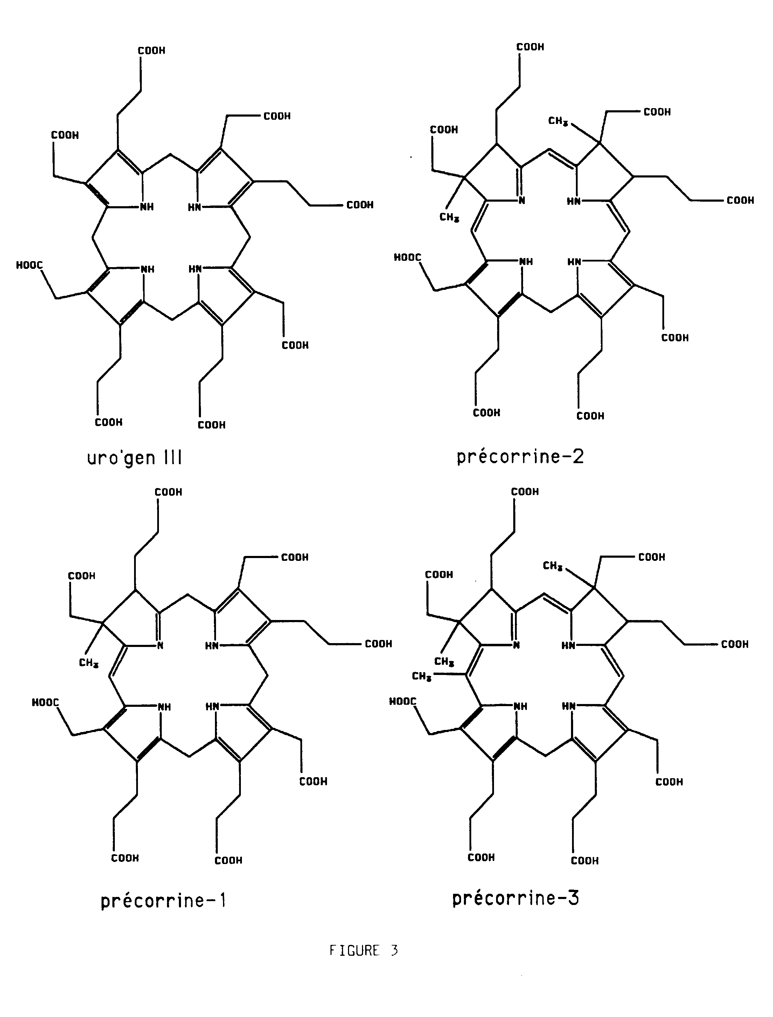

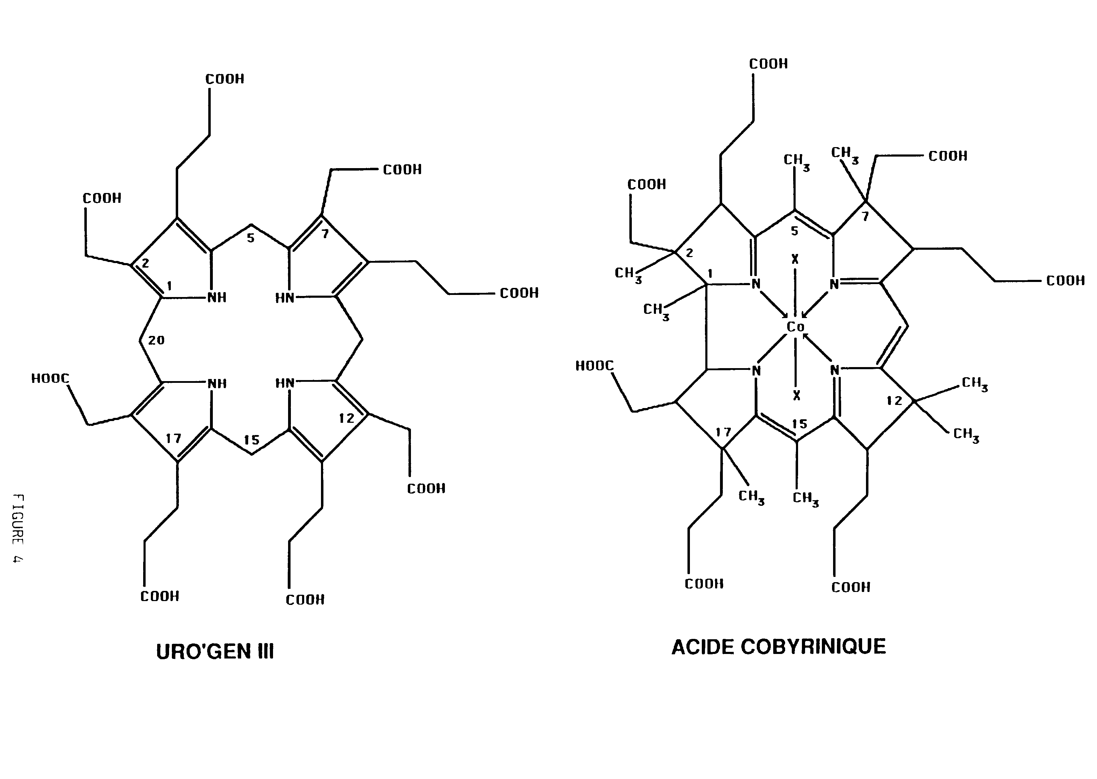

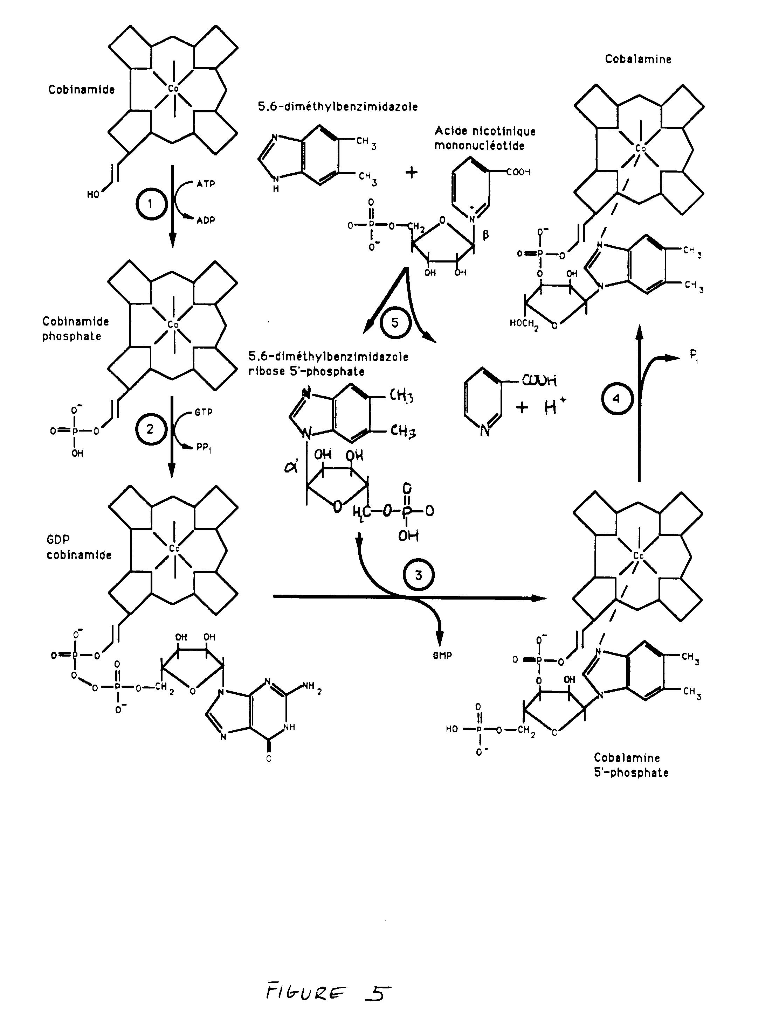

Entre l'uro'gen III et l'acide cobyrinique, seuls trois dérivés d'intermédiaires ont été purifiés à ce jour ; il s'agit des Facteurs FI, FII et FIII qui sont des produits d'oxydation respectivement des trois intermédiaires précorrine-1, précorrine-2 et précorrine-3 qui correspondent aux dérivés mono-, di- et triméthylés de l'uro'gen III (figure 3) ; ces intermédiaires sont obtenus par des transferts successifs de groupements méthyl à partir du SAM (S-adénosyl-L-méthionine) sur l'uro'gen III aux positions C2, C7 et C20 respectivement. Les autres réactions qui ont lieu pour donner l'acide cobyrinique sont, outre cinq autres transferts de groupements méthyl à partir du SAM en C17, C12, C1, C15 et C5, l'élimination du carbone en C20, la décarboxylation en C12 et l'insertion d'un atome de cobalt (figure 4). Ces étapes de biosynthèse ont été déduites à partir d'expériences effectuées in vitro sur des extraits acellulaires de Propionibacterium shermanii ou de Clostridium tetanomorphum. Dans ces extraits, l'acide cobyrinique est obtenu par transformation de l'uro'gen III, après incubation dans des conditions appropriées en anaérobiose (Batterby et al., 1982). Aucun intermédiaire entre le précorrine-3 et l'acide cobyrinique, pouvant être transformé en corrinoïdes par incubations ultérieures avec des extraits de bactéries productrices de cobalamines, n'a été isolé à ce jour chez ces microorganismes. La difficulté d'isoler et d'identifier ces intermédiaires est liée à
i) leur grande instabilité
ii) leur sensibilité à l'oxygène et
iii) leur faible niveau d'accumulation in vivo.
Dans cette partie de la voie, seule une enzyme de Pseudomonas denitrificans a été purifiée et étudiée ; il s'agit de la SAM:uro'gen III méthyltransférase (Blanche et al., 1989) appelée SUMT.

Entre l'acide cobyrinique et le cobinamide, les réactions suivantes sont effectuées :
i) addition du groupement 5'-désoxyadénosyl (s'il s'agit du coenzyme B₁₂ qui doit être synthétisé),
ii) amidation de six des sept fonctions carboxyliques par addition de groupements amines,
iii) amidation de la dernière fonction carboxylique (chaîne d'acide propionique du noyau pyrrole D) par addition du R-1-amino-2-propanol (figure 2).

Il n'a pas été élucidé s'il existait réellement un ordre dans les amidations (Herbert et al., 1970). Enfin, aucun dosage d'activité dans cette partie de la voie n'a été décrit sauf en ce qui concerne l'addition du groupement 5'-désoxyadénosyl (Huennekens et al., 1982).

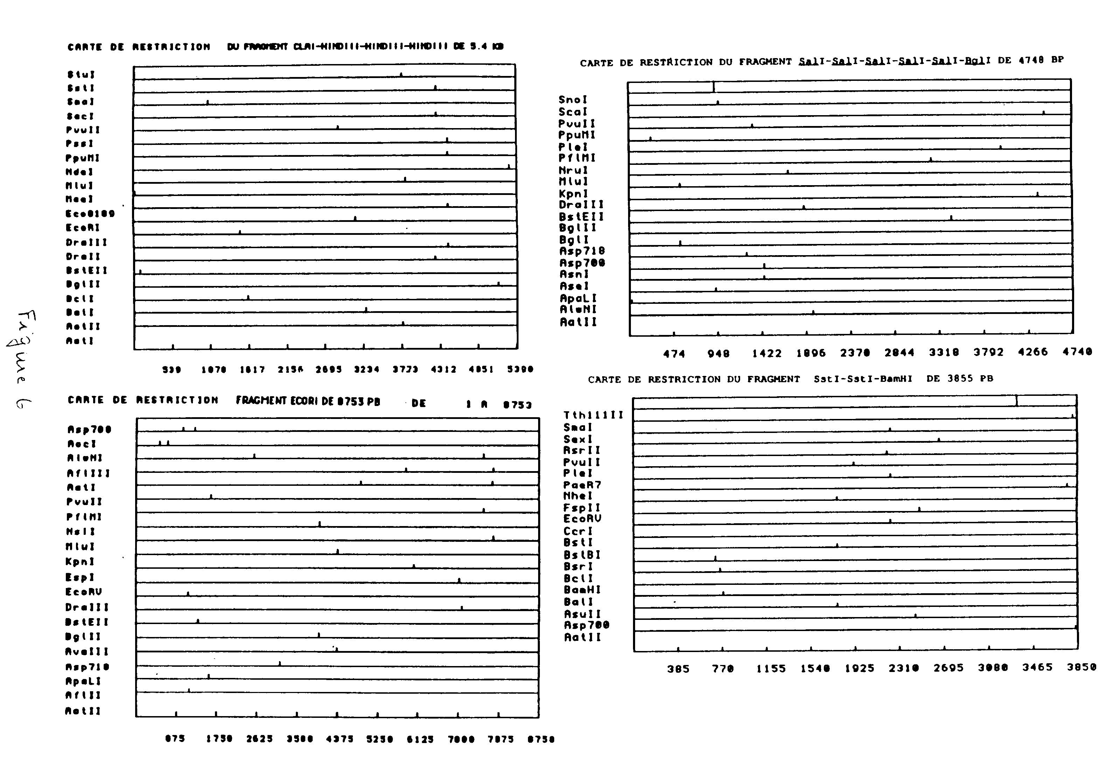

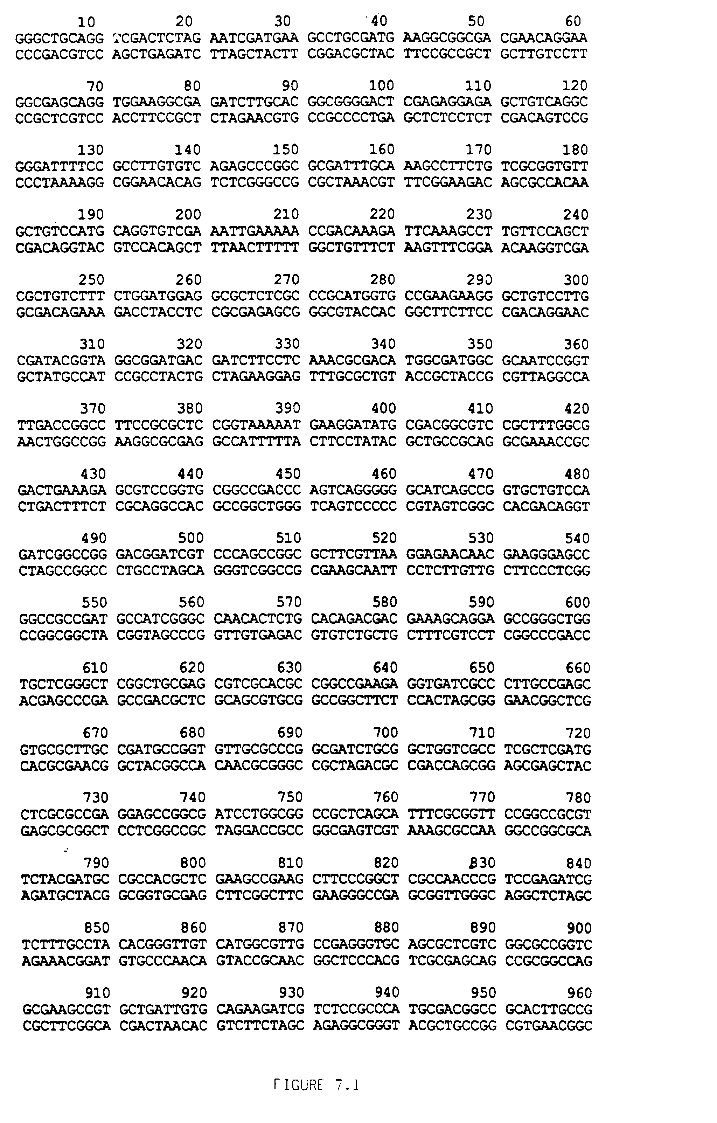

La dernière étape de la biosynthèse d'une cobalamine, par exemple le coenzyme B₁₂ comprend quatre phases successives décrites sur la figure 5 (Huennekens et al., 1982), à savoir :
i) la phosphorylation du groupement hydroxyl du résidu aminopropanol du cobinamide pour donner le cobinamide phosphate, puis
ii) l'addition d'une guanosine diphosphate par réaction avec la guanosine 5'-triphosphate ; le composé obtenu est le GDP-cobinamide (Friedmann, 1975) qui
iii) réagit avec le α-ribazole 5'-phosphate, lui-même synthétisé à partir de la riboflavine, pour donner l'adénosylcobalamine 5'-phosphate (Friedmann et al, 1968) qui
iv) par déphosphorylation conduit au coenzyme B₁₂ (Schneider et Friedmann, 1972).

Parmi les bactéries capables de produire des cobalamines, on peut citer notamment :
Agrobacterium tumefaciens,
Agrobacterium radiobacter
Bacillus megaterium
Clostridium sticklandii
Clostridium tetanomorphum
Clostridium thermoaceticum
Corynebacterium XG
Eubacterium limosum
Methanobacterium arbophilicum
Methanobacterium ivanovii
Methanobacterium ruminantium
Methanobacterium thermoautotrophicum
Methanosarcina barkeri
Propionobacterium shermanii
Protaminobacter ruber
Pseudomonas denitrificans
Pseudomonas putida
Rhizobium melitoti
Rhodopseudomonas sphaeroides
Salmonella typhimurium
Spirulina platensis
Streptomyces antibioticus
Streptomyces aureofaciens
Streptomyces griseus
Streptomyces olivaceus

Au niveau industriel, en raison de la grande complexité des mécanismes de biosynthèse, la production des cobalamines, et en particulier de la vitamine B₁₂, est exclusivement microbiologique. Elle est réalisée par des cultures en grands volumes des bactéries Pseudomonas denitrificans, Propionobacterium shermanii et Propionibacterium freudenreichii (Florent, 1986). Les souches utilisées pour la production industrielle sont issues de souches sauvages; elles peuvent avoir subi de nombreux cycles de mutation au hasard puis de sélection de clones améliorés pour la production de cobalamines (Florent, 1986). Les mutations sont obtenues par mutagénèse avec des agents mutagènes ou par traitements physiques tels que des traitements aux rayons ultra-violets (Barrère et al., 1981). Par cette méthode empirique, des mutations au hasard sont obtenues et améliorent la production de cobalamines. Par exemple, il est décrit qu'à partir de la souche originale de Pseudomonas denitrificans initialement isolée par Miller et Rosenblum (1960, brevet US 2 938 822) la production de ce microorganisme a été graduellement augmentée en dix ans, par les techniques citées ci-dessus de 0,6 mg/l à 60 mg/l (Florent, 1986). Pour les bactéries du genre Propionibacterium, [Propionobacterium shermanii (ATCC 13673) et freudenreichii (ATCC 6207)] les mêmes valeurs de production semblent être décrites dans la littérature ; par exemple une production de 65 mg/l a été décrite (brevet Européen 87920). Toutefois, aucun crible n'a encore été décrit permettant de sélectionner ou de repérer facilement soit des mutants surproducteurs de cobalamines soit des mutants nettement améliorés dans leur production de cobalamines.

Au niveau génétique, peu de travaux ont été effectués à ce jour. Le clonage de gènes cob (codant pour des enzymes impliquées dans le procédé de biosynthèse) a été décrit chez Bacillus megaterium (Brey et al., 1986). Onze groupes de complémentation ont été identifiés par complémentation de mutants cob de Bacillus megaterium avec des plasmides portant différents fragments d'ADN de Bacillus megaterium. Ces gènes sont groupés sur le même locus, porté par un fragment de 12 kb.

Des études ont également été menées sur les gènes cob de Salmonella typhimurium. Sans que le clonage de ceux-ci ait été décrit, il a été montré que presque tous les gènes de biosynthèse des cobalamines sont regroupés entre les minutes 40 et 42 du chromosome (Jeter et Roth, 1987). Seul le locus cysG, qui doit permettre la transformation de l'uro'gen III en précorrine-2 ne fait pas partie de ce groupe de gènes. Toutefois, l'activité codée par ce locus, ainsi que ses propriétés biochimiques n'ont pas été décrites.

En outre, des phénotypes ont été associés aux mutations cob. Chez Salmonella typhimurium et chez Bacillus megaterium, les mutants cob ne montrent plus de croissance sur milieu minimum avec de l'éthanolamine comme source de carbone ou comme source d'azote (Roof et Roth, 1988). Ceci est dû au fait qu'une enzyme du catabolisme de l'éthanolamine, l'éthanolamine ammonia-lyase (EC 4.3.1.7), a pour cofacteur le coenzyme B₁₂; les mutants cob ne synthétisant plus de coenzyme B₁₂, ils ne peuvent plus croître avec l'éthanolamine comme source de carbone et/ou comme source d'azote. Des mutants metE de Salmonella typhimurium n'ont plus qu'une homocystéine-méthyl-transférase (EC 2.1.1.13) méthylcobalamine dépendante. Les mutants cob de Salmonella typhimurium metE sont auxotrophes pour la méthionine (Jeter et al., 1984).

Chez Pseudomonas denitrificans et Agrobacterium tumefaciens des phénotypes associés à une déficience totale en synthèse de cobalamines n'ont pas été décrits à ce jour.

Enfin, des travaux sur Pseudomonas denitrificans (Cameron et al., 1989) ont abouti au clonage de fragments d'ADN portant des gènes cob de cette bactérie. Ceux-ci sont répartis en quatre groupes de complémentation portés par au moins 30 kb d'ADN. Au moins quatorze groupes de complémentation ont été identifiés par complémentation hétérologue de mutants cob d'Agrobacterium tumefaciens et de Pseudomonas putida avec des fragments d'ADN de Pseudomonas denitrificans portant des gènes cob.

Cependant, jusqu'à maintenant, aucun de ces gènes n'a été purifié, et aucune séquence nucléotidique n'a été décrite. L'article de F. BLANCHE et al (J. of Bact. (1989), 171 p 4222-4231 fait état des 2 enzymes, SUM et Cob I alamin adenosyltransferase, impliquée dans la biosynthèse des cobalamines. Enfin, aucune amélioration de production de cobalamines par les techniques d'ADN recombinant n'a pu être obtenue. L'amplification de gènes cob de Bacillus megaterium ne procure pas, chez la souche à partir de laquelle ils ont été clonés, une amélioration de production de cobalamines (Brey et al., 1986). Chez Salmonella typhimurium, des études physiologiques ont été menées afin de déterminer des conditions dans lesquelles une forte transcription des gènes cob étudiés était observée (Escalante et Roth, 1987). Dans ces conditions, il n'y a pas d'amélioration de la production de cobalamines, bien que des gènes de la voie de biosynthèse soient plus exprimés que dans les conditions standard de culture.

La présente invention résulte de l'identification précise de séquences d'ADN codant pour des polypeptides impliqués dans la biosynthèse des cobalamines et/ou des cobamides. Un objet de l'invention concerne donc les séquences d'ADN codant pour les polypeptides impliqués dans la biosynthèse des cobalamines et/ou des cobamides. Plus particulièrement, l'invention a pour objet les gènes cobA, cobB, cobC, cobD, cobE, cobF, cobG, cobH, cobI, cobJ, cobK, cobL, cobM, cobN, cobO, cobP, cobQ, cobS, cobT, cobU, cobV, cobW, cobX et corA, toute séquence d'ADN homologue de ces gènes résultant de la dégénérescence du code génétique, ainsi que les séquences d'ADN, de toute origine (naturelle,synthétique, recombinante) qui hybrident et/ou qui présentent des homologies significatives avec ces séquences ou avec des fragments de celles-ci et qui codent pour des polypeptides impliqués dans la biosynthèse des cobalamines et/ou des cobamides. L'invention a aussi pour objet les gènes contenant ces sequences d'ADN.

Les séquences d'ADN selon la présente invention ont été isolées à partir de différentes souches : une souche industrielle, Pseudomonas denitrificans SC510, dérivée de la souche MB580 (brevet US 3 018 225), par complémentation de mutants cob de A.tumefaciens et P.putida; et de Methanobacterium ivanovii. Les clones obtenus ont pu être analysés précisément, notamment par cartographie à l'aide d'insertions d'un dérivé du transposon Tn5. Ces études génétiques ont permis de localiser les gènes cob ou cor sur la carte de restriction et de réaliser leur séquençage. Une analyse des phases ouvertes a ensuite permis de mettre en évidence les régions codantes de ces fragments d'ADN.

La présente invention a aussi pour objet l'utilisation de ces séquences nucléotidiques pour cloner les gènes cob d'autres bactéries. En effet il est connu que pour des protéines catalysant les mêmes activités, les séquences sont conservées avec, comme divergence, la divergence évolutive (Wein-Hsiung et al., 1985). Il est montré dans la présente invention qu'il y a une homologie significative entre les sequences nucléotidiques de gènes de differents microorganismes codant pour des polypeptides impliqués dans la biosynthèse des cobalamines et/ou des cobamides. Les differences qui apparaissent résultent de la dégénérescence évolutive, et de la dégénérescence du code génétique qui est liée au pourcentage en GC du génome du microorganisme étudié (Wein-Hsiung et al., 1985).

Selon la présente invention, une sonde peut être faite avec une ou plusieurs des séquences d'ADN de Pseudomonas denitrificans notamment, ou avec des fragments de celles-ci, ou avec des séquences analogues présentant un degré de dégénérescence spécifique, au niveau de l'usage des codons et du pourcentage en GC de l'ADN de la bactérie que l'on veut étudier. Dans ces conditions, il est possible de détecter un signal d'hybridation spécifique entre la sonde et des fragments d'ADN génomique de la bactérie étudiée ; ce signal d'hybridation spécifique correspond à l'hybridation de la sonde avec les gènes cob isofonctionnels de la bactérie. Les gènes cob ainsi que leurs produits peuvent ensuite être isolés, purifiés et caractérisés. l'invention fournit ainsi un moyen permettant, par hybridation, d'accéder aux sequences nucléotidiques et aux polypeptides impliqués dans la biosynthèse des cobalamines et/ou des cobamides de tout microorganisme.

La présente invention a également pour objet un ADN recombinant contenant au moins une séquence d'ADN codant pour un polypeptide impliqué dans la biosynthèse des cobalamines et/ou des cobamides, et notamment, un ADN recombinant dans lequel la ou lesdites séquences sont placées sous le contrôle de signaux d'expression.

A cet égard, on peut en particulier positionner en 5' de la séquence d'ADN des régions promotrices. De telles régions peuvent être homologues ou hétérologues de la séquence d'ADN. En particulier, des promoteurs bactériens forts, tels que le promoteur de l'opéron tryptophane Ptrp ou de l'opéron lactose Plac de E.coli, le promoteur gauche ou droit du bactériophage lambda, les promoteurs forts de phages de bactéries, telles que les corynebactéries, les promoteurs fonctionnels chez les bactéries gram-négatives, tel que le promoteur Ptac de E.coli, le promoteur PxvlS des gènes du catabolisme du xylène du plasmide TOL, le promoteur de l'amylase de Bacillus subtilis Pamy, pourront être utilisés. On peut citer également les promoteurs dérivés de gènes glycolytiques de levures, tels que les promoteurs des gènes codant pour le phosphoglycérate kinase, la glycéraldéhyde-3-phosphate déshydrogénase, la lactase ou l'énolase, qui pourront être utilisés lorsque l'ADN recombinant sera introduit dans un hôte eucaryote. Un site de fixation des ribosomes sera également positionné en 5' de la séquence d'ADN et il pourra être homologue ou hétérologue, tel le site de fixation des ribosomes du gène cII du bactériophage lambda.

Des signaux nécessaires à la terminaison de la transcription pourront être placés en 3' de la séquence d'ADN.

L'ADN recombinant selon la présente invention peut ensuite être introduit directement dans une cellule hôte compatible avec les signaux d'expression choisis ou être cloné sur un vecteur plasmidique pour permettre d'introduire de manière stable la séquence d'ADN en question dans la cellule hôte.

Un autre objet de l'invention concerne les plasmides ainsi obtenus contenant une séquence d'ADN codant pour un polypeptide impliqué dans la biosynthèse des cobalamines et/ou des cobamides. Plus précisément, ces plasmides contiennent aussi un système de réplication fonctionnel et un marqueur de sélection.

L'invention a aussi pour objet les cellules hôtes dans lesquelles une ou plusieurs sequences d'ADN telles que définies précedemment, ou un plasmide tel que défini ci-avant, à été introduit.

Un autre objet de l'invention concerne un procédé de production de polypeptides impliqués dans la biosynthèse des cobalamines et/ou des cobamides. Selon ce procédé,ntrointroduit dans une cellule hôte une séquence d'ADN telle que décrite précédemment, on cultive cette cellule recombinante dans des conditions d'expression de ladite séquence, puis on récupère les polypeptides produits.

Les cellules hôtes qui pourront être utilisées dans ce but sont aussi bien des procaryotes que des eucaryotes, des cellules animales ou des cellules végétales. Préférentiellement, elles seront choisies parmi les bactéries et, en particulier, les bactéries du genre E.coli, P.denitrificans, A.tumefaciens ou R.melitoti.

Une autre utilisation des séquences d'ADN selon la présente invention réside dans un procédé d'amplification de la production de cobalamines et/ou de cobamides ou de leurs précurseurs de biosynthèse, par les techniques d'ADN recombinant. En effet, si la limitation du flux métabolique de la biosynthèse des cobalamines et/ou des cobamides ou de leurs précurseurs est due à une limitation dans l'activité d'une enzyme dans la voie de biosynthèse, l'augmentation de cette activité par augmentation de l'expression de cette même enzyme à l'aide des techniques d'ADN recombinant (amplification génique, substitution des signaux de transcription-traduction par des signaux plus efficaces ...) conduira à une augmentation de la biosynthèse des cobalamines et/ou des cobamides. Il est possible également que la limitation de la production de cobalamines et/ou de cobamides résulte d'une régulation biochimique. Dans ce cas, le ou les gènes cob correspondant à l'enzyme régulée pourront être mutagénisés in vitro spécifiquement afin d'obtenir des gènes mutés dont les produits auront perdu les régulations allant à l'encontre d'une amélioration de la production.

Le procédé selon la présente invention consiste à introduire dans un microorganisme producteur de cobalamines et/ou de cobamides, ou seulement potentiellement producteur de ces composés (c'est-à-dire déficient dans une ou plusieurs des étapes de la biosynthèse), une séquence d'ADN telle que définie plus haut, puis à cultiver ce microorganisme dans des conditions d'expression de ladite séquence et de synthèse des cobalamines et/ou des cobamides, et enfin à récupérer les cobalamines et/ou les cobamides produits. Un tel procédé est applicable en particulier à tous les microorganismes producteurs cités page 4, et plus spécifiquement aux microorganismes du genre P. denitrificans, Rhizobium melitoti, ou Agrobactérium tumefaciens. Dans un mode de réalisation préféré, le microorganisme est P. denitrificans, et en particulier, la souche SC510. Concernant les microorganismes potentiellement producteurs, les séquences d'ADN utilisées seront celles correspondant aux étapes de la biosynthèse que le microorganisme ne peut réaliser.

A l'aide de la présente invention et par les diverses stratégies exposées ci-dessus, une amélioration de la production de cobalamines et/ou de cobamides ou de leurs précurseurs pour tout microorganisme producteur, ou potentiellement producteur, de cobalamines et/ou de cobamides, pourra être obtenue. Il suffira de cultiver ce microorganisme recombiné dans des conditions appropriées pour la production de cobalamines et pour l'expression des séquences d'ADN introduites. Cette culture pourra se faire en batch ou bien en continu, et la purification des cobalamines pourra se faire par les méthodes déjà utilisées au niveau industriel (Florent, 1986). Ces méthodes comprennent entre autre :
i) la solubilisation des cobalamines et leur conversion en leur forme cyano (par exemple en traitant le moût de fermentation à la chaleur, avec du cyanure de potassium en présence de nitrite de sodium), puis
ii) la purification des cyanocobalamines en différentes étapes qui peuvent être par exemple
   a) l'adsorption sur différents substrats tels que l'amberlite IRCSO, le Dowex 1X2 ou l'amberlite XAD 2 suivie d'une élution avec un mélange eau-alcool ou eau phénol, puis
   b) l'extraction dans un solvant organique et enfin
   c) la précipitation ou cristallisation à partir de la phase organique soit par addition de réactifs ou dilution dans un solvant approprié, soit par évaporation.

La présente invention montre de plus qu'il est possible, par les techniques d'ADN recombinant, d'améliorer la production de cobalamines d'une bactérie productrice de cobalamines en cumulant des améliorations. Ceci revient à obtenir une première amélioration comme cela est décrit ci-dessus puis à améliorer cette amélioration toujours à l'aide des techniques d'ADN recombinant, c'est-à-dire par exemple en amplifiant des gènes de biosynthèse des cobalamines.

Un autre objet de la présente invention concerne les polypeptides impliqués dans la biosynthèse des cobalamines et/ou des cobamides. En particulier, la présente invention a pour objet tous les polypeptides, ou des dérivés ou des fragments de ces polypeptides, qui sont codés par les séquences d'ADN décrites précédemment, et qui sont impliqués dans la voie de biosynthèse des cobalamines et/ou des cobamides. La séquence en acides aminés de ces polypeptides est décrite, ainsi que certaines de leurs caractéristiques physicochimiques. Une activité enzymatique ou des propriétés spécifiques ont également été associées à chacun d'entre-eux.

A cet égard, l'invention a pour objet les polypeptides intervenant dans la transformation du précorrine-3 en acide cobyrinique a,c-diamide, et plus particulièrement dans le transfert d'un groupement méthyl du SAM aux positions C1, C5, C11, C15, et C17.

L'invention a aussi pour objet les polypeptide:
. intervenant dans la transformation de l'acide cobyrique en cobinamide, ou
. possèdant une activité S-adénosyl-L-méthionine:précorrine-2 méthyl transférase (SP2MT), ou
. possèdant une activité acide cobyrinique et/ou hydrogénobyrinique a,c-diamide synthase, ou
. possèdant une activité précorrin-8x mutase, ou
. possèdant une activité nicotinate-nucléotide: dimethylbenzimidazole phosphoribosyltransferase, ou
. possèdant une activité cobalamine-(5'-phosphate) synthase, ou
. possèdant une activité cobyric acid synthase, ou
. possédant une activité cob(I)alamin adénosyltransférase, ou
. possédant une activité precorrin-6x réductase, ou
. intervenant dans la transformation de l'acide hydrogénobyrinique a,c-diamide en acide cobyrinique a,c-diamide.

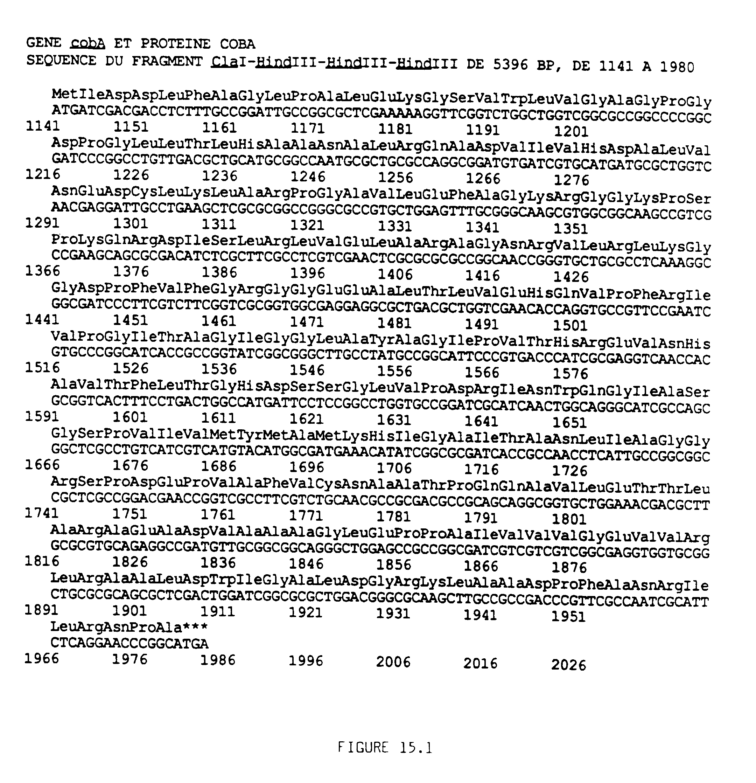

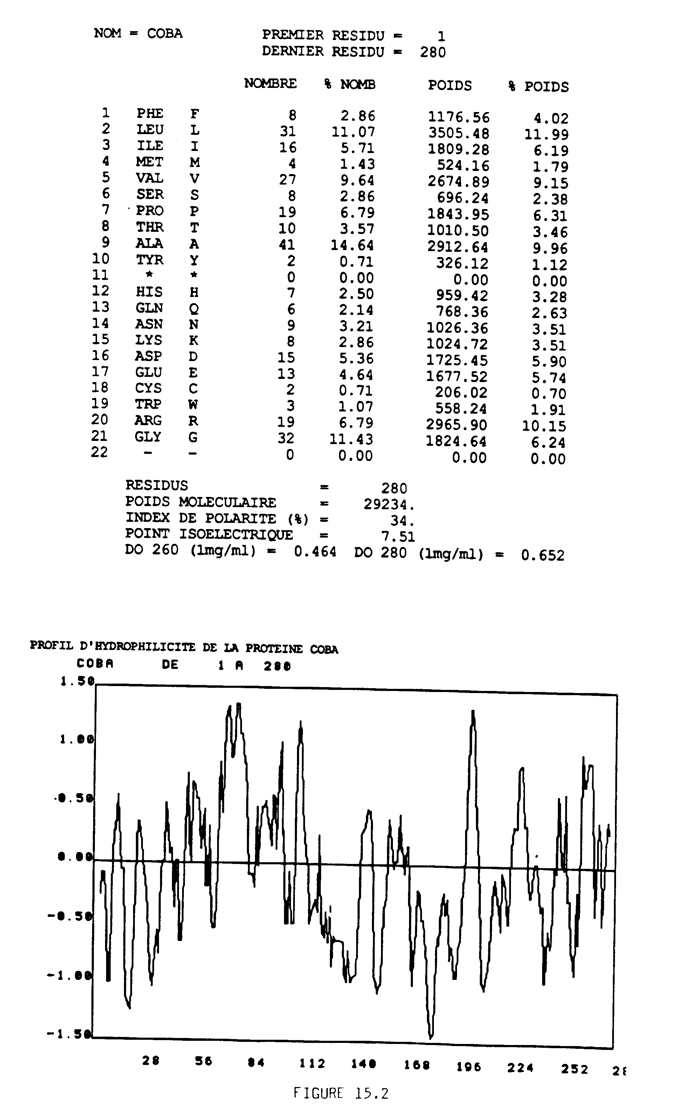

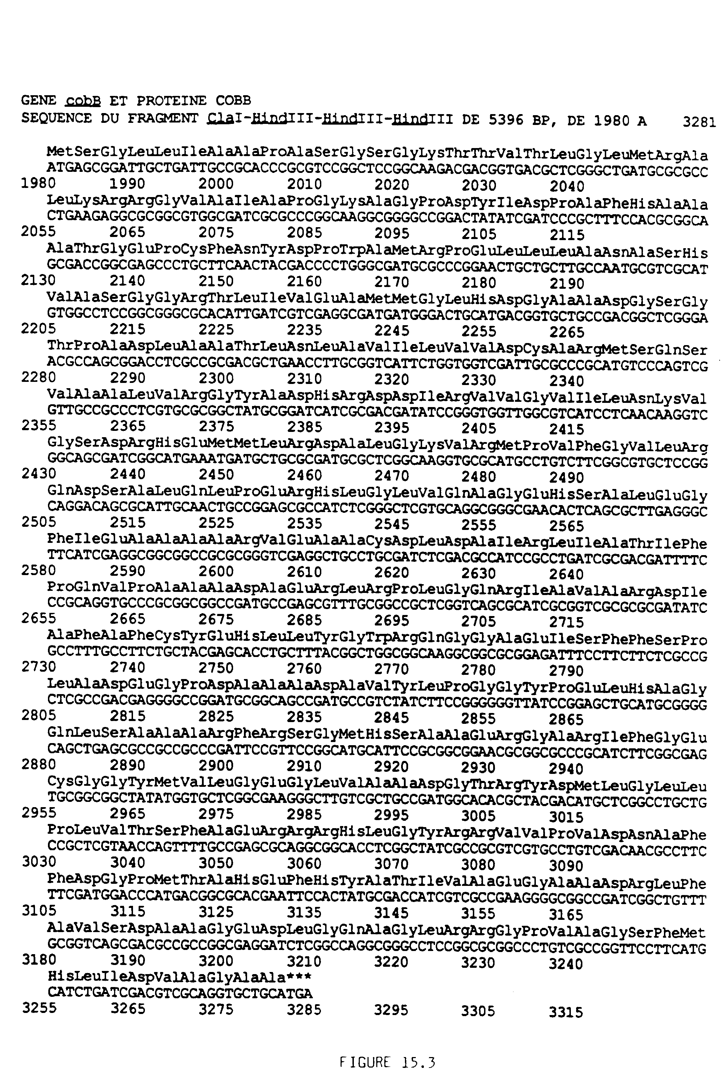

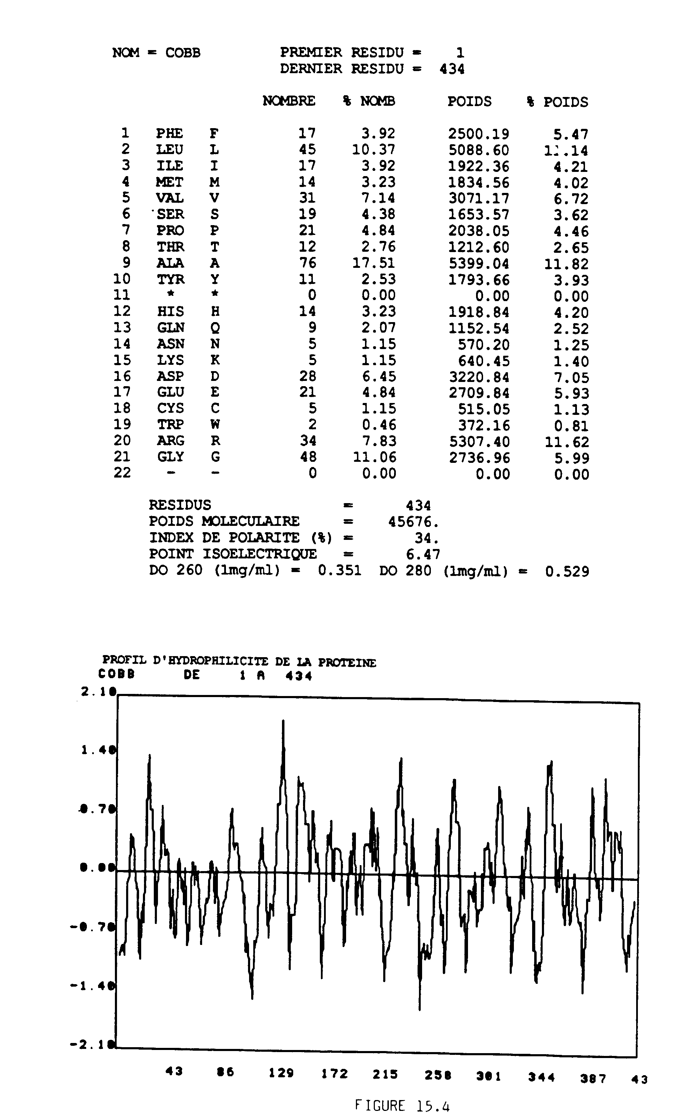

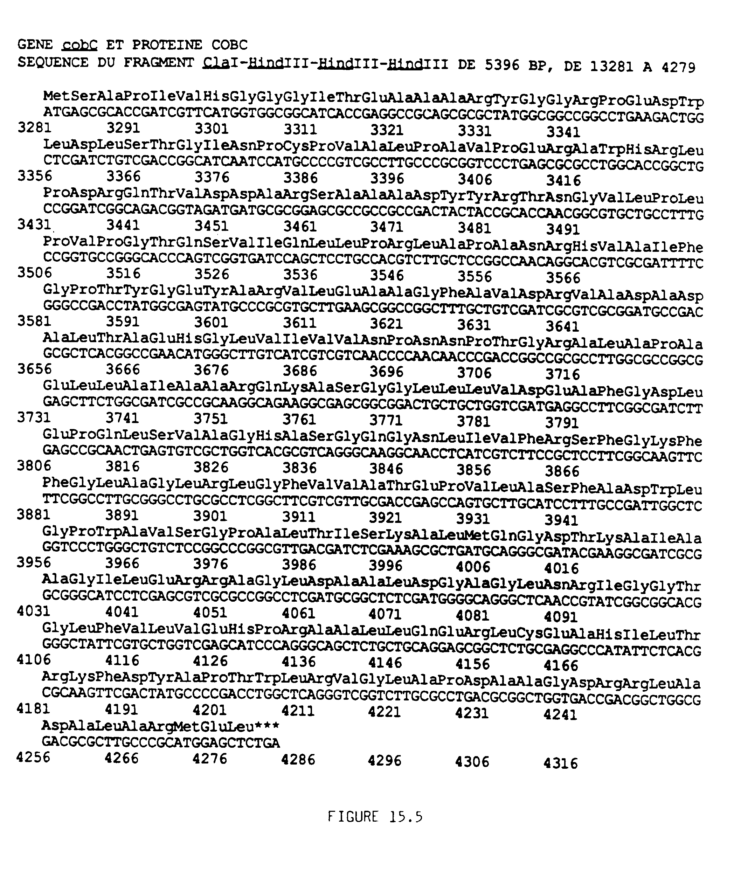

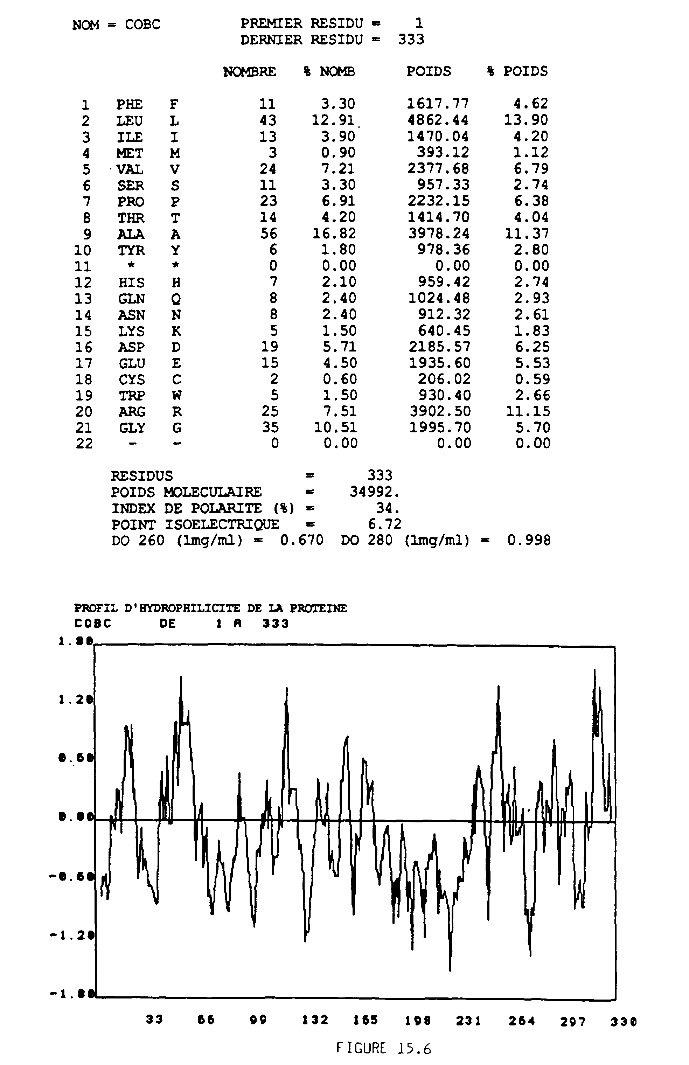

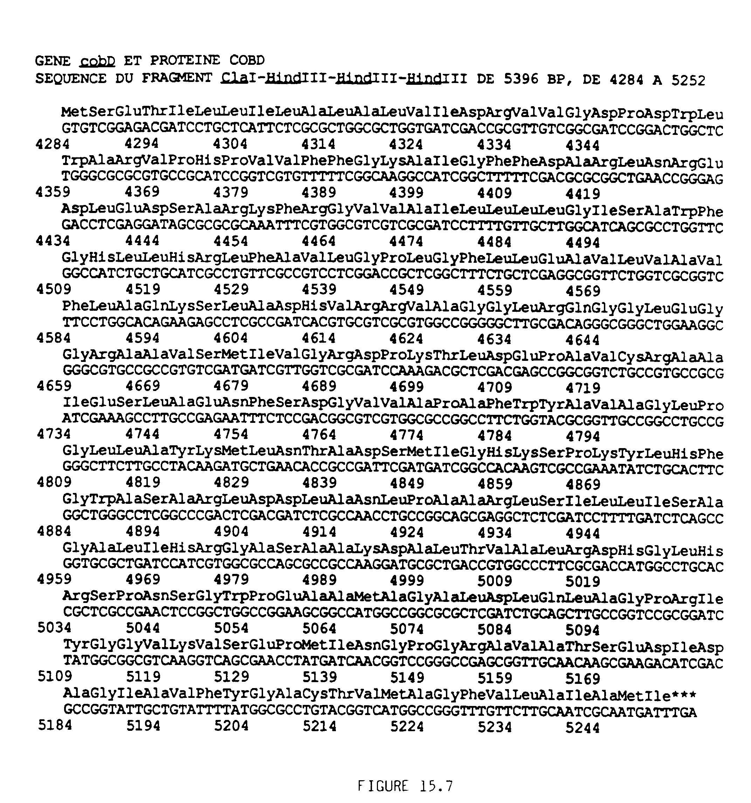

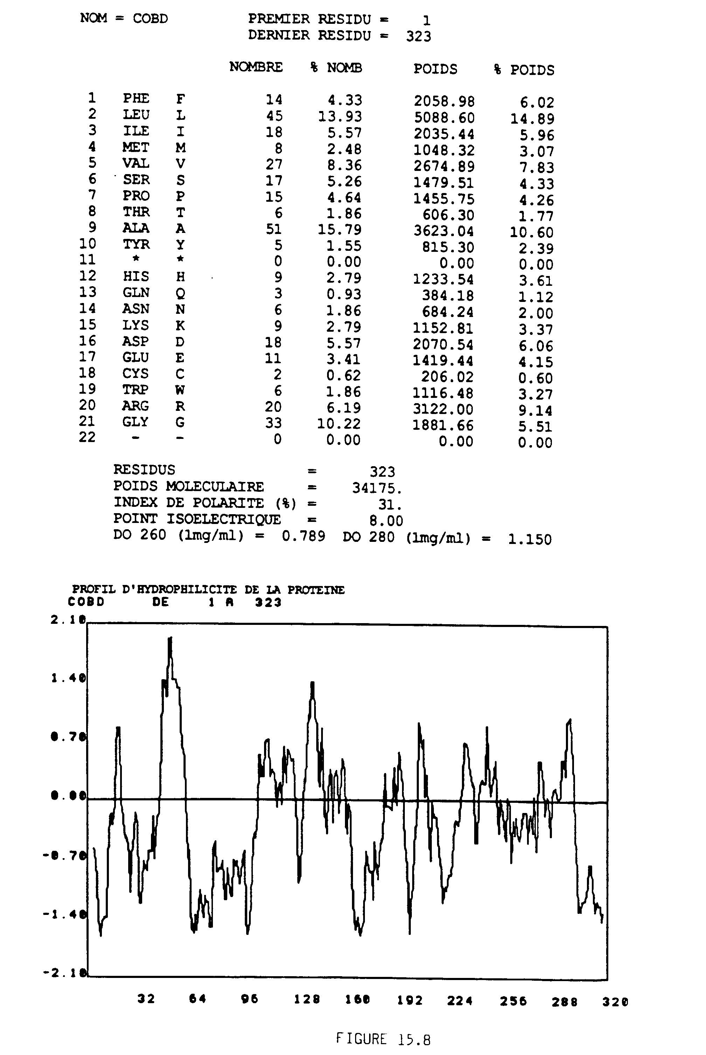

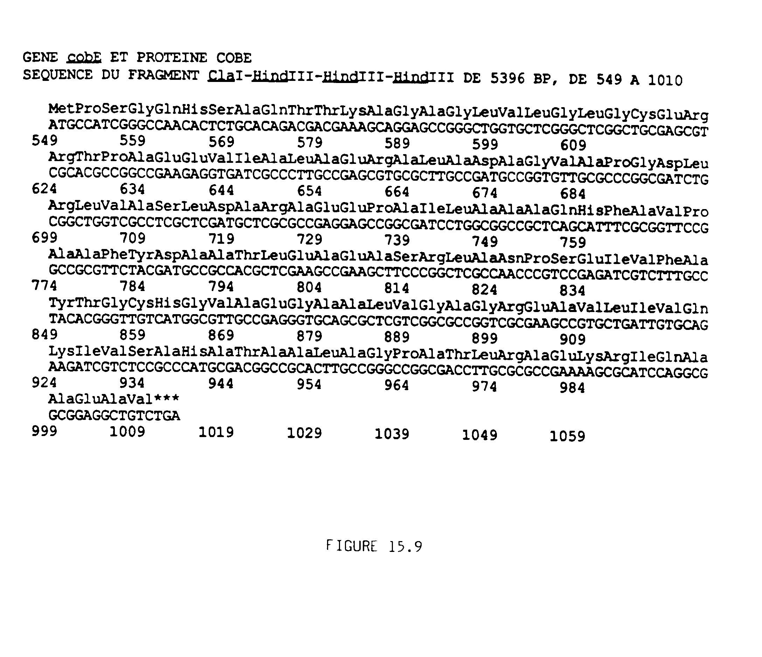

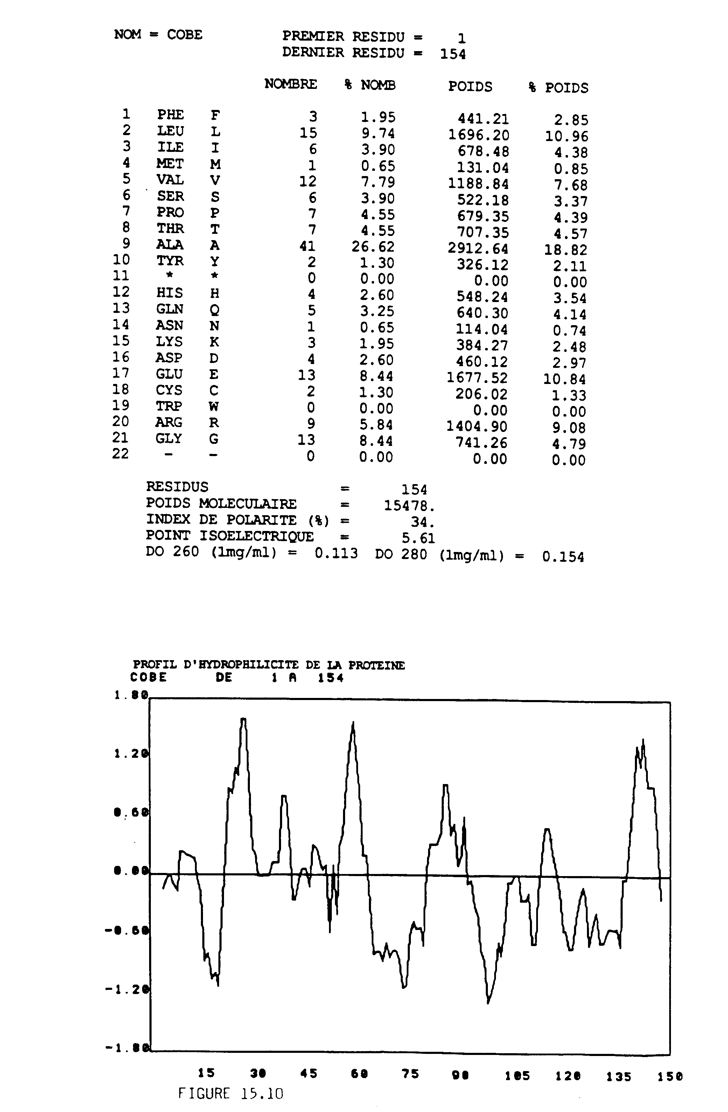

Avantageusement, l'invention a pour objet un polypeptide choisi parmi les protéines COBA, COBB, COBC,COBD, COBE, COBF, COBG, COBH, COBI, COBJ, COBK, COBL, COBM, COBN, COBO, COBP, COBQ, COBS, COBT, COBU, COBV, COBW, COBX et CORA présentées aux figures 15, 16, 40, 41 et 47.

De plus, l'utilisation des sondes d'hybridation décrites précédemment permet, à partir de gènes isolés dans d'autres microorganismes, de caractériser et d'isoler les polypeptides isofonctionnels d'autres microorganismes. De cette manière, la présente invention montre que la séquence d'une protéine COB de Pseudomonas denitrificans est significativement homologue aux séquences de protéines d'autres microorganismes présentant le même type d'activité. Entre ces protéines COB catalysant la même réaction chez des microorganismes différents, seules les distances évolutives ont introduit des variations (Wein-Hsiung et al., 1985). La présente invention à egalement pour objet ces polypeptides isofonctionnels.

L'attribution d'une activité enzymatique particulière est le résultat d'une analyse qui peut être effectuée selon diverses stratégies. En particulier, des études d'affinité in vitro, vis-à-vis du SAM (S-adénosyl-L-méthionine), permettent d'attribuer à une protéine capable de fixer le SAM, une activité méthyltransférase, et donc son implication dans une des étapes de transfert des groupements méthyl qui se produisent entre l'uro'gen III et l'acide cobyrinique. Un autre moyen d'apprécier l'activité de ces polypeptides consiste à doser les intermédiaires de la voie de biosynthèse des cobalamines qui sont accumulés chez des mutants incapables d'exprimer ces polypeptides (identifiés par des expériences de complémentation). Ces analyses permettent de déduire que le polypeptide en question a pour substrat l'intermédiaire accumulé, ce qui permet de situer et de définir son activité dans la voie de biosynthèse. La présente invention décrit également un procédé de dosage des activités enzymatiques de la voie de biosynthèse, applicable à toute souche productrice de cobalamines et/ou de cobamides. Ces dosages permettent de purifier à partir de toute souche productrice de ces composés, l'activité enzymatique dosée. A partir de cette activité purifiée, la séquence NH2-terminale de la protéine COB en question, ou bien celle des sous-unités de cette protéine, peut être effectuée, ce qui permet d'identifier le ou les gènes de structure qui codent pour l'activité en question. Pour Pseudomonas denitrificans, les gènes de structure qui codent pour des activités de la voie de biosynthèse sont identifiés en trouvant pour chaque séquence NH2-terminale, la protéine COB ayant la même séquence NH2-terminale.

La présente invention décrit aussi un procédé permettant l'identification et le dosage, chez des souches productrices de cobalamines ou des mutants non producteurs, d'intermédiaires de la voie de biosynthèse des cobalamines ou d'autres corrinoïdes. Ces intermédiaires peuvent être dosés aussi bien dans des moûts de culture que dans les cellules elles-mêmes. Les intermédiaires qui peuvent être dosés sont tous les corrinoïdes qui se trouvent dans la voie de biosynthèse après l'acide cobyrinique à savoir outre l'acide cobyrinique, l'acide cobyrinique monoamide, l'acide cobyrinique diamide, l'acide cobyrinique triamide, l'acide cobyrinique tétraamide, l'acide cobyrinique pentaamide, l'acide cobyrique, le cobinamide, le cobinamide phosphate, le GDP-cobinamide, le coenzyme B₁₂-phosphate et le coenzyme B₁₂. Les formes cyano et coenzyme de ces produits peuvent être dosées par cette technique.

D'autres objets et avantages de la présente invention apparaîtront à la lecture des exemples et des dessins suivants, qui doivent être considérés comme illustratifs et non limitatifs.

### DEFINITION DES TERMES EMPLOYES ET ABREVIATIONS

ACDAS: acide cobyrinique a,c-diamide synthase
ADN recombinant: ensemble de techniques qui permettent soit d'associer au sein du même microorganisme des séquences d'ADN qui ne le sont pas naturellement, soit de mutagéniser spécifiquement un fragment d'ADN.
ATP: adénosine 5'-triphosphate
BSA: sérum albumine bovine
CLHP: Chromatographie liquide à haute performance.
cluster: groupe de gènes.
Cob: correspond au phénotype de niveau réduit (au moins 10 fois moins que le contrôle) de production de cobalamines.
Codon stop: codon de terminaison de traduction
Corrinoïdes : dérivés de l'acide cobyrinique possédant le noyau corrine.
dGTP: 2'-désoxyguanosine 5'-triphosphate
DMBI: diméthylbenzimidazole
dNTP: 2'-désoxyribonucléosides 5'-triphosphates
DTT: dithiothréitol
gène cob: gène impliqué dans la biosynthèse du cobinamide à partir de l'uro'gen III.
gène cor: gène impliqué dans la biosynthèse des corrinoïdes à partir de l'uro'gen III.
kb: kilobases
NN:DMBI PRT :
ORF: phase ouverte
pb: paires de bases
Protéine COB: protéine intervenant soit comme catalyseur dans la voie de biosynthèse des cobalamines, soit comme protéine régulatrice dans le réseau de régulation des gènes cob, soit les deux.
Protéine COR: protéine intervenant soit comme catalyseur dans la voie de biosynthèse des corrinoïdes, soit comme protéine régulatrice dans le réseau de régulation des gènes cor, soit les deux.
SAM: S-adénosyl-L-méthionine.
SDS: sodium dodécyl sulfate
SP2MT: SAM-L-méthionine:précorrine-2 méthyltransférase
SUMT: SAM:uro'gen III méthyltransférase
Uro'gen III: uroporphyrinogène III.

### Légendes des figures:

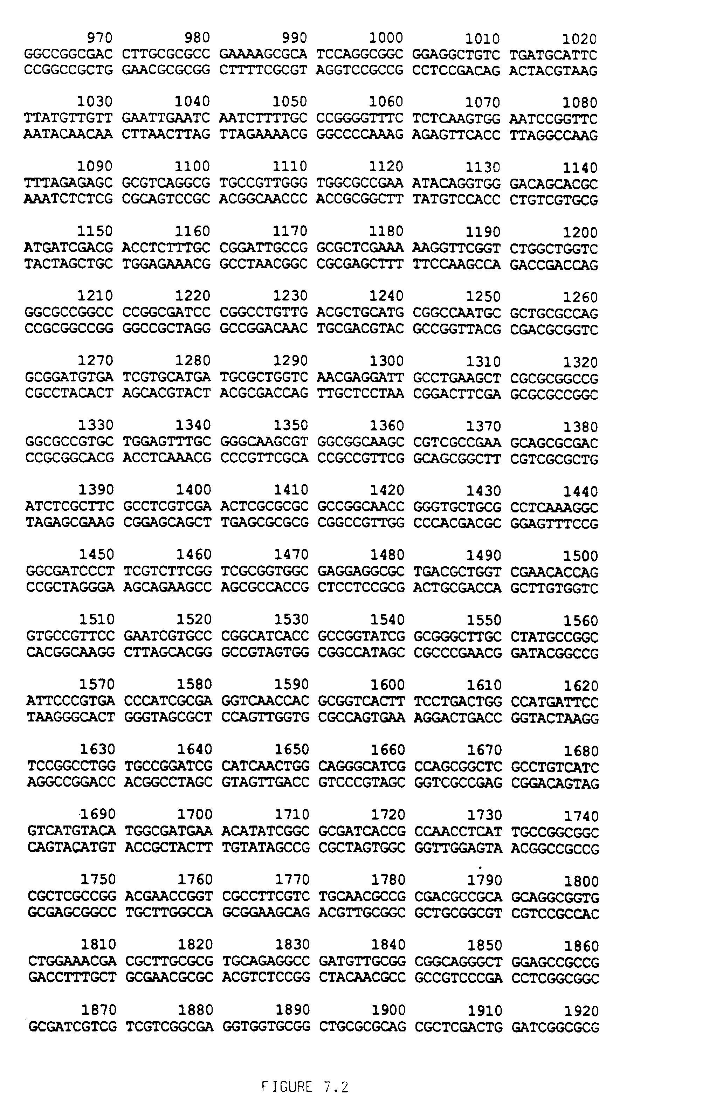

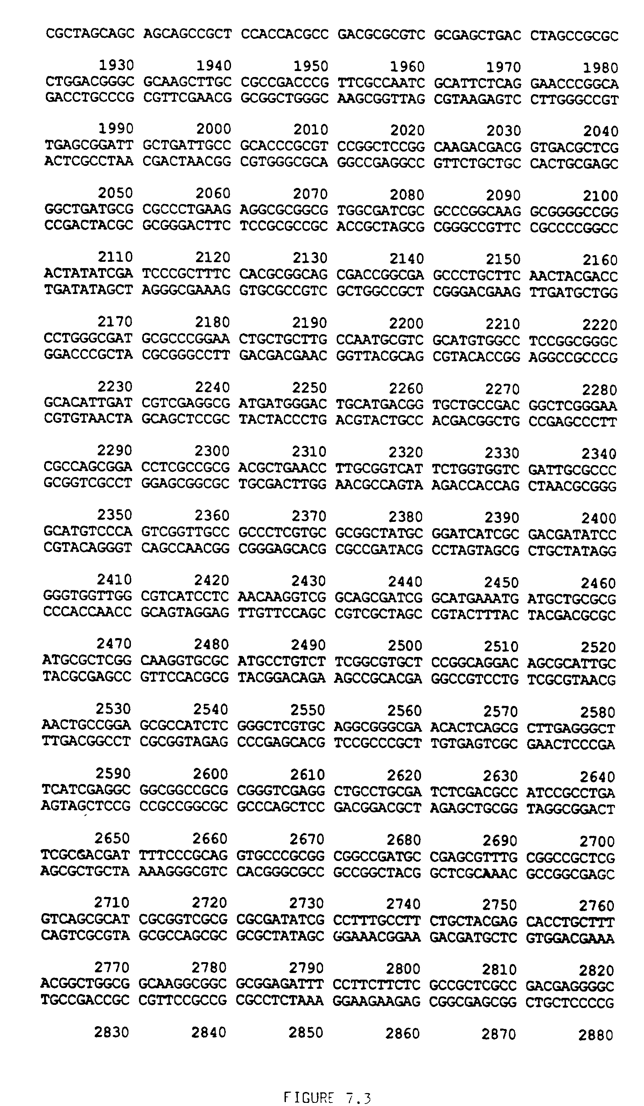

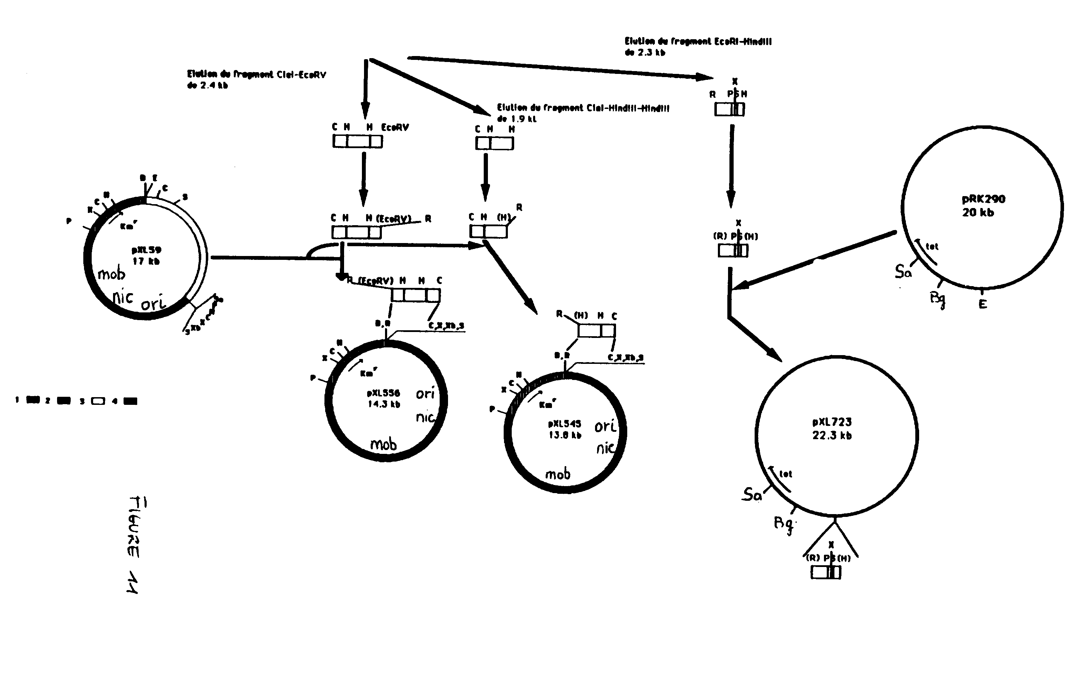

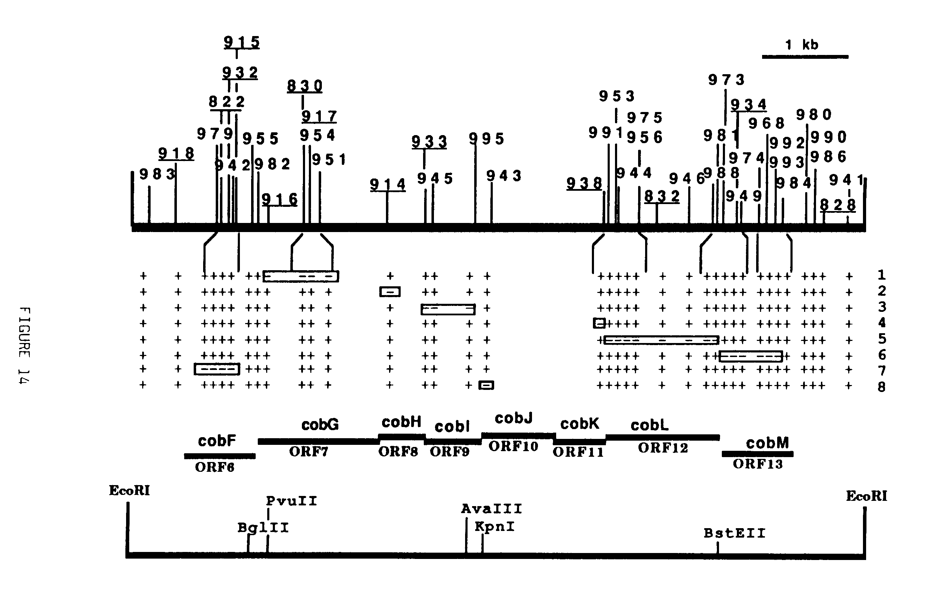

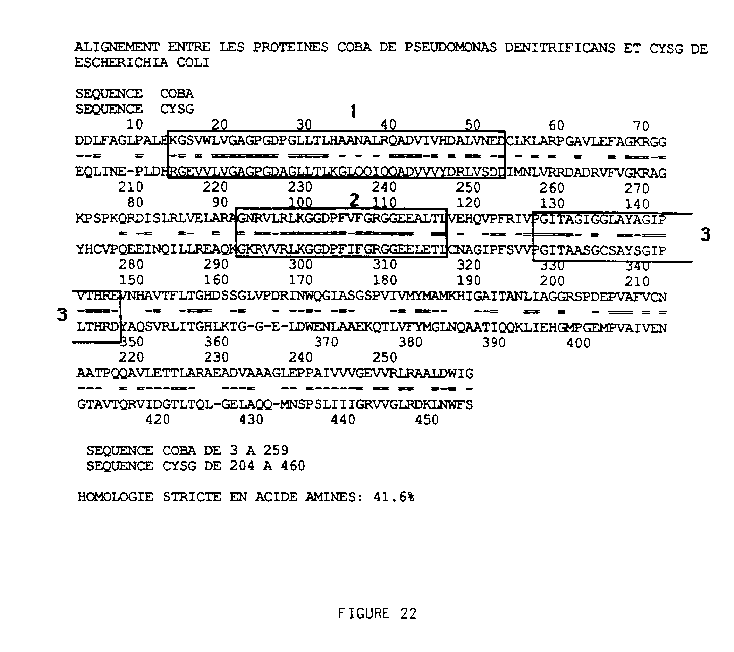

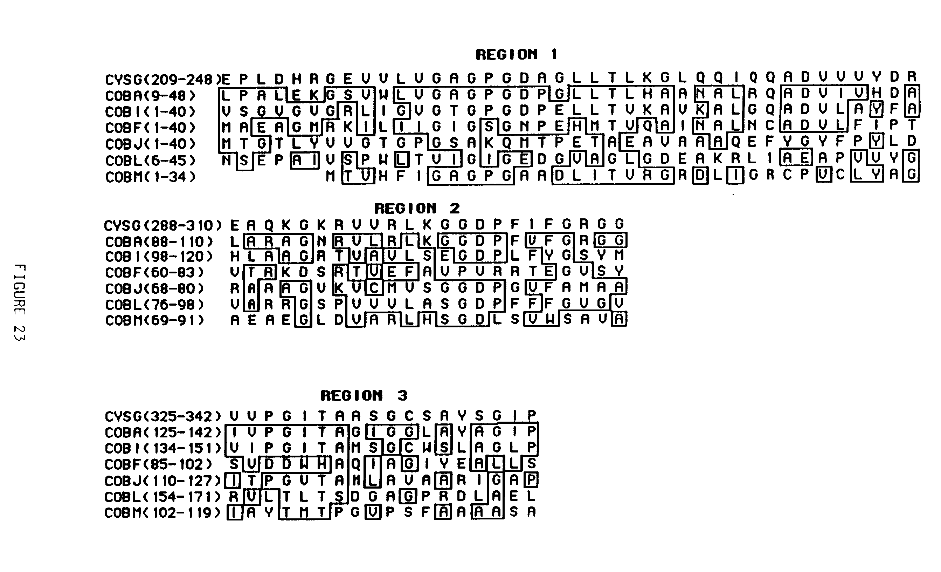

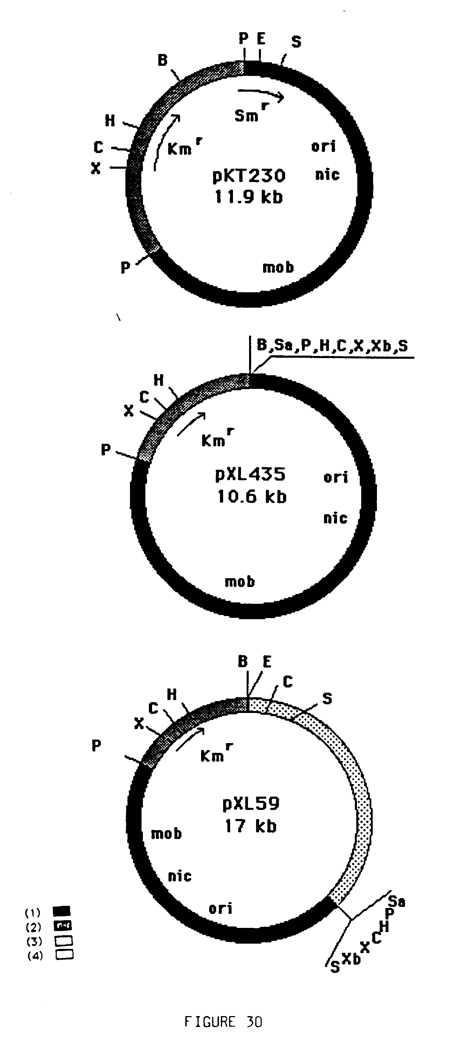

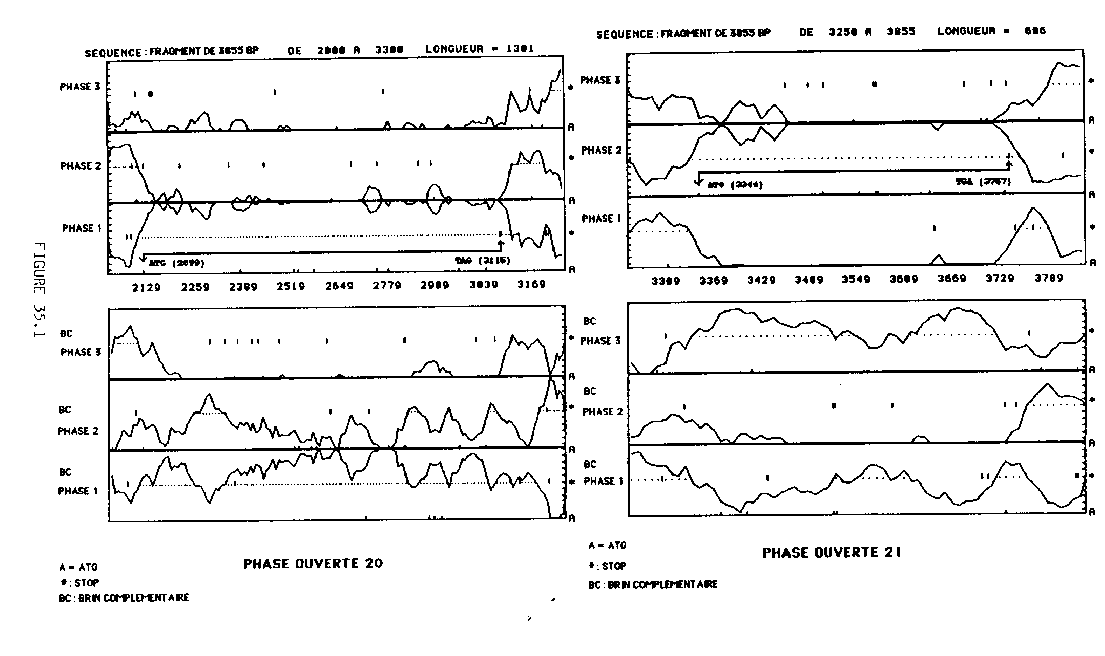

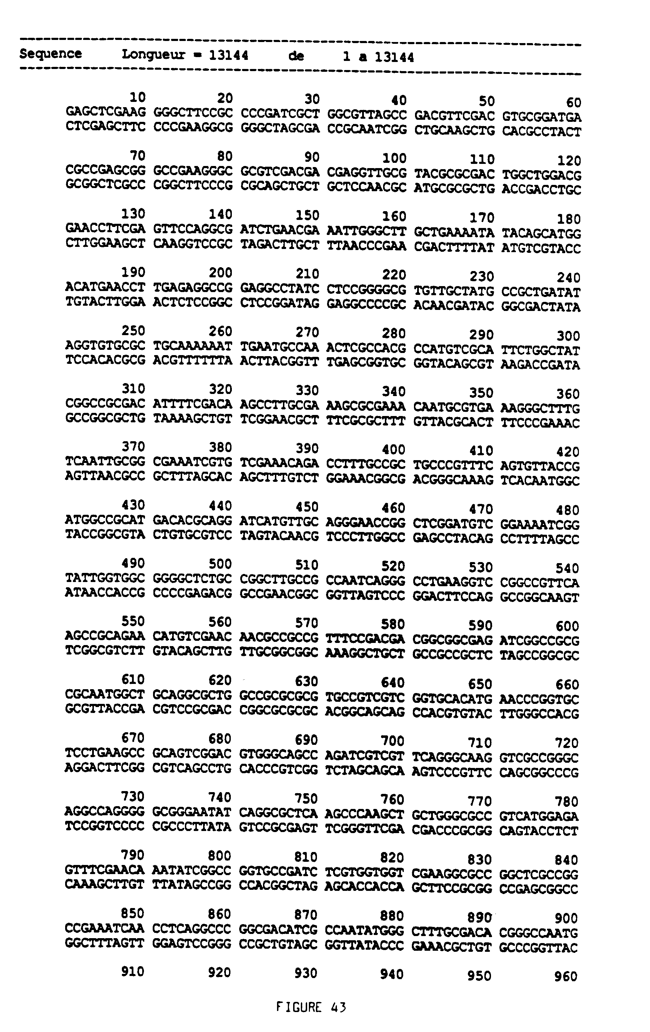

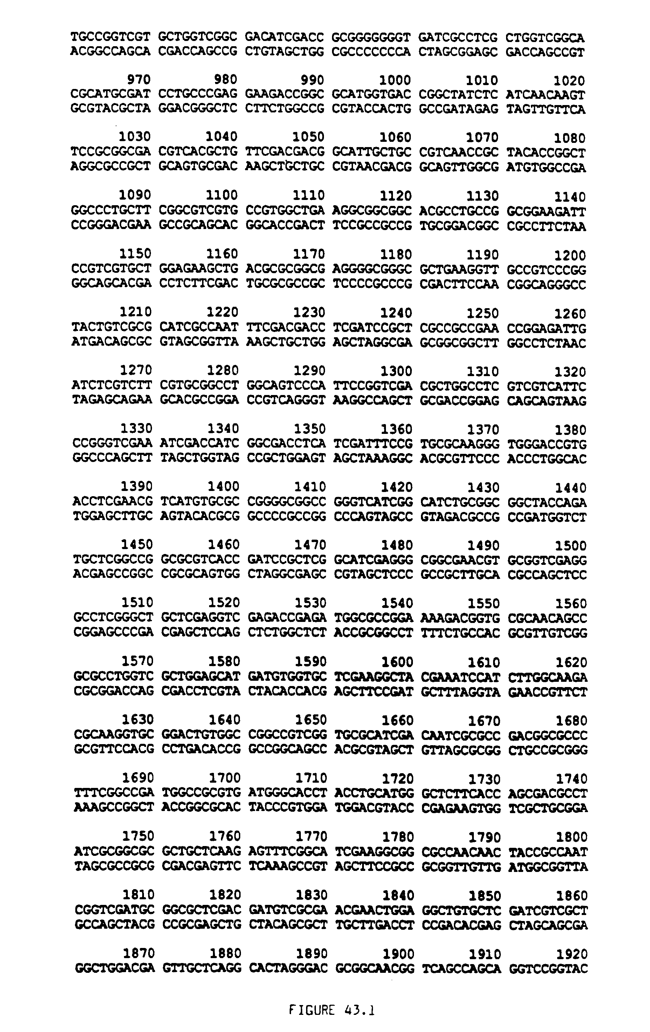

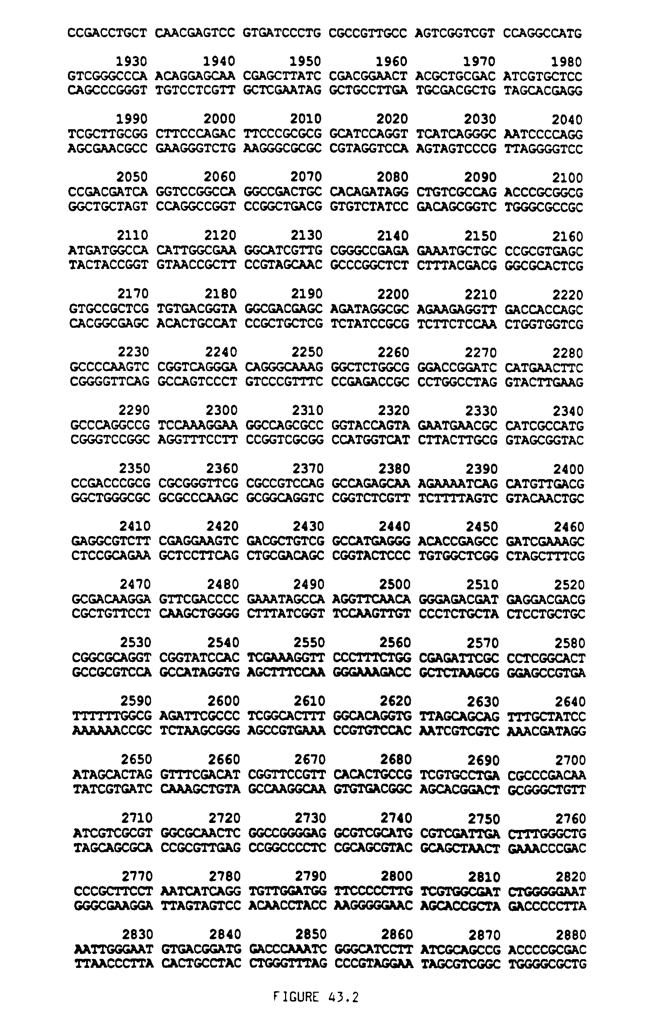

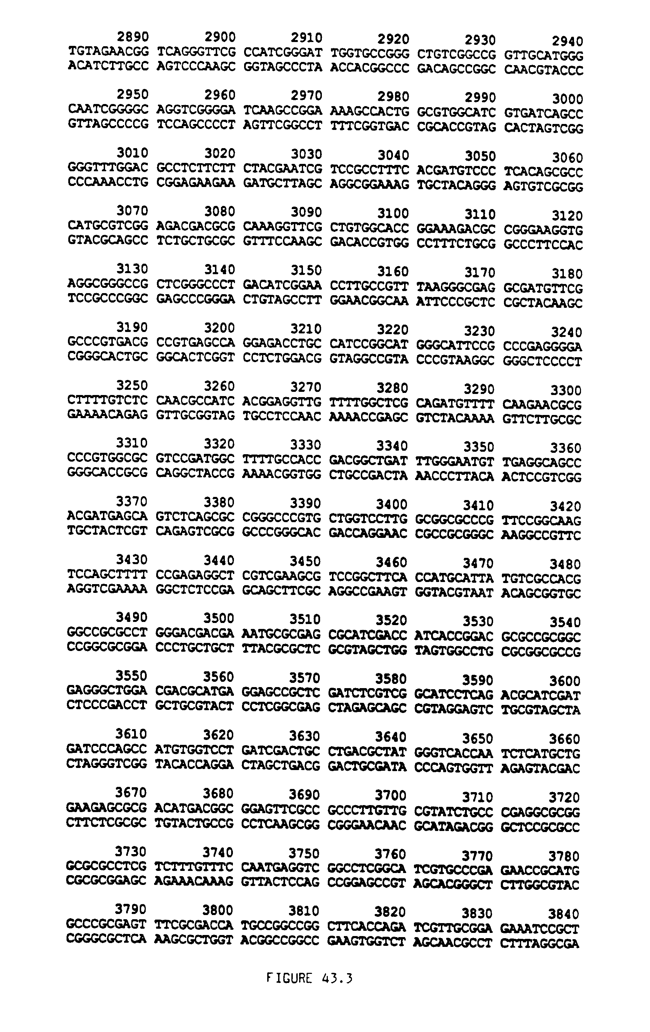

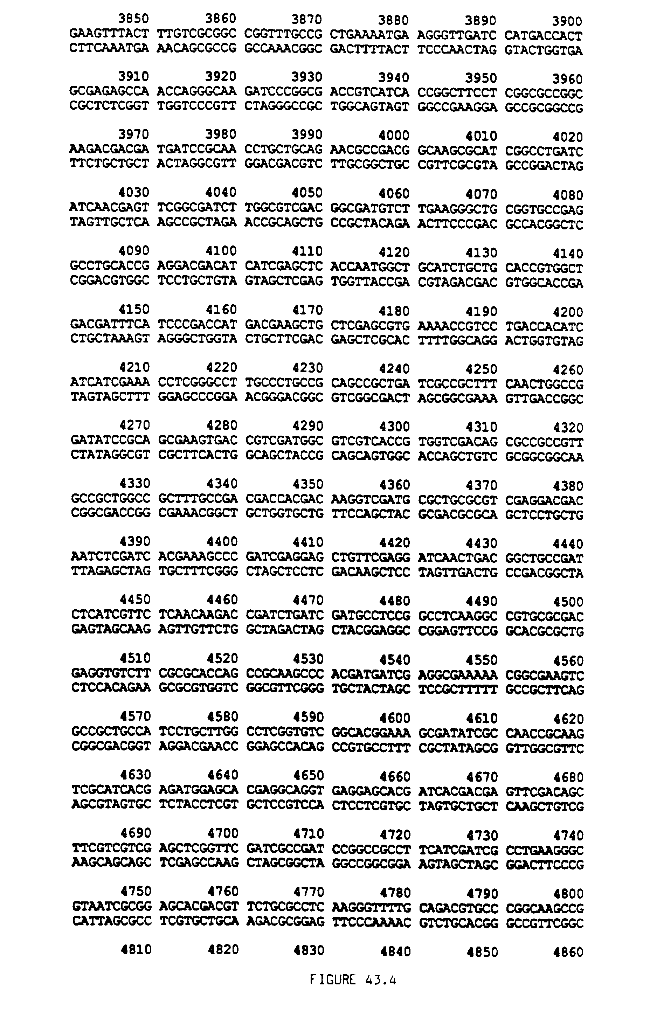

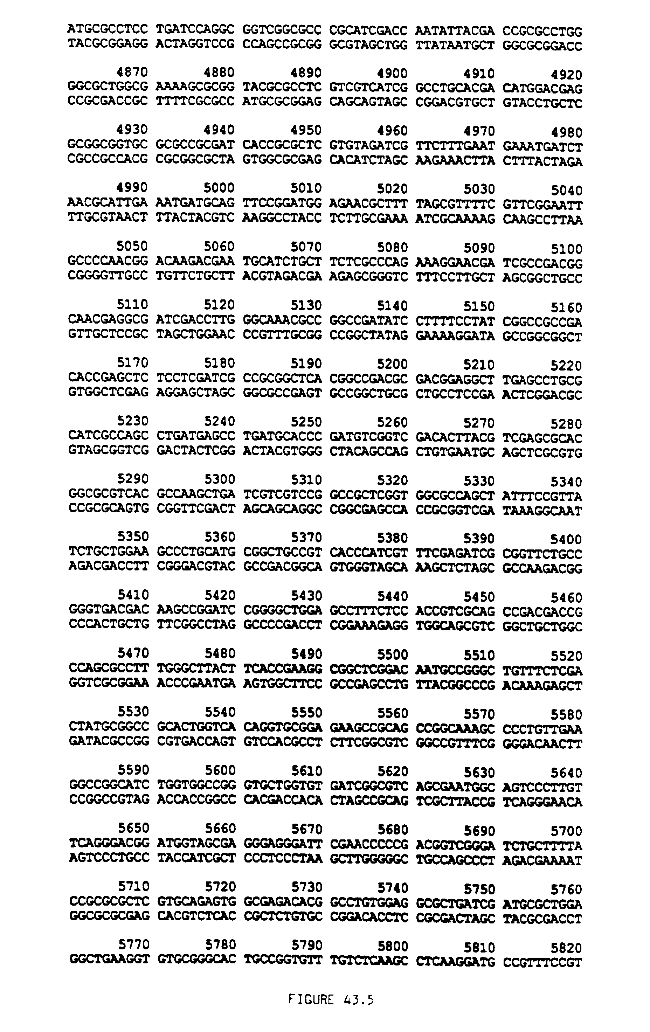

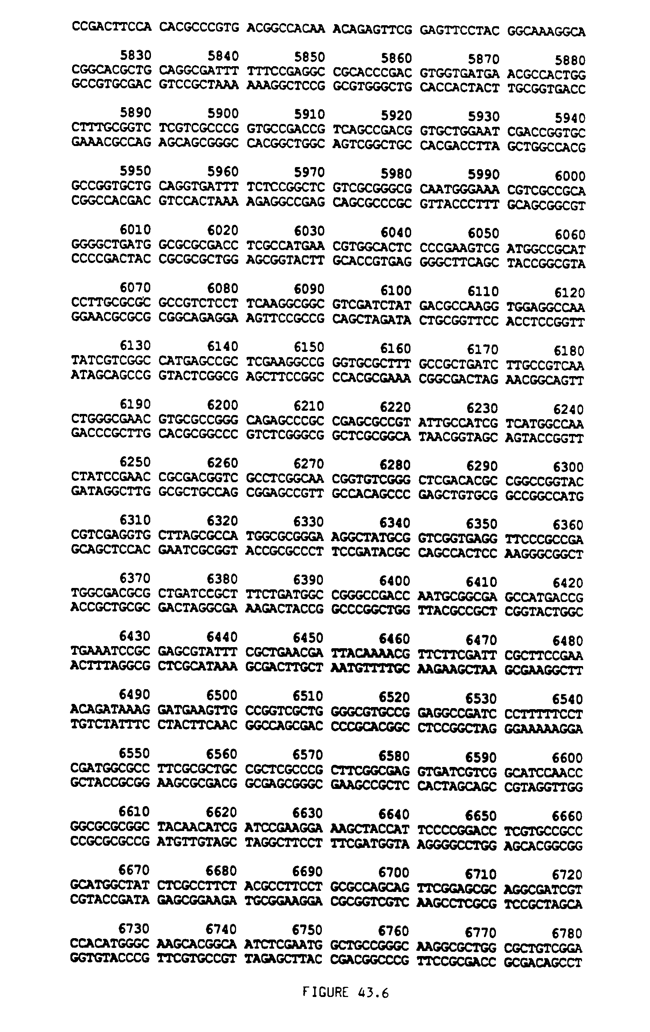

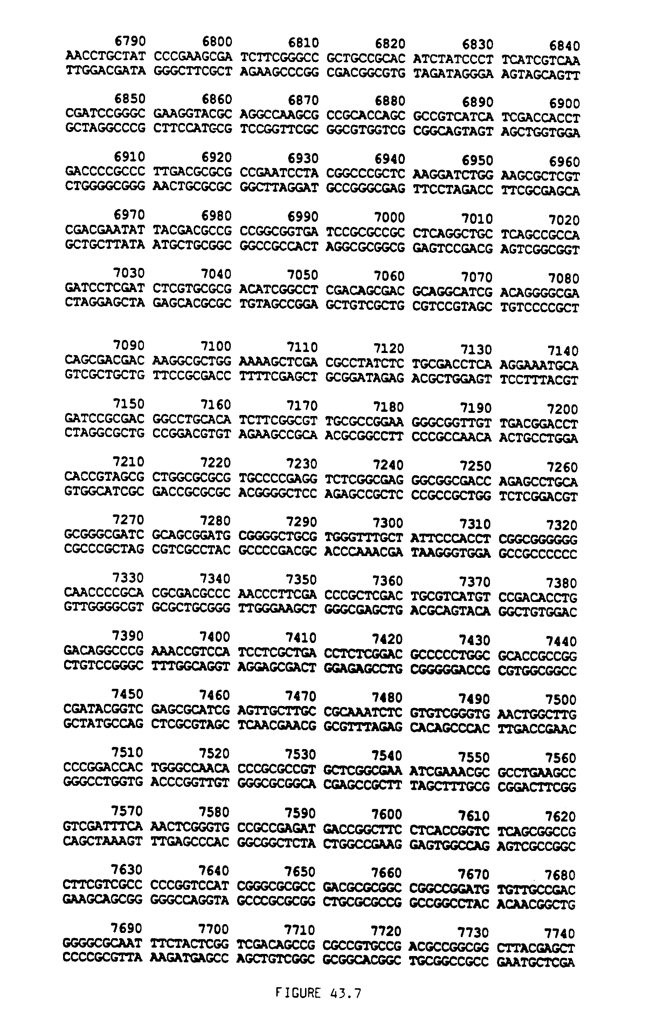

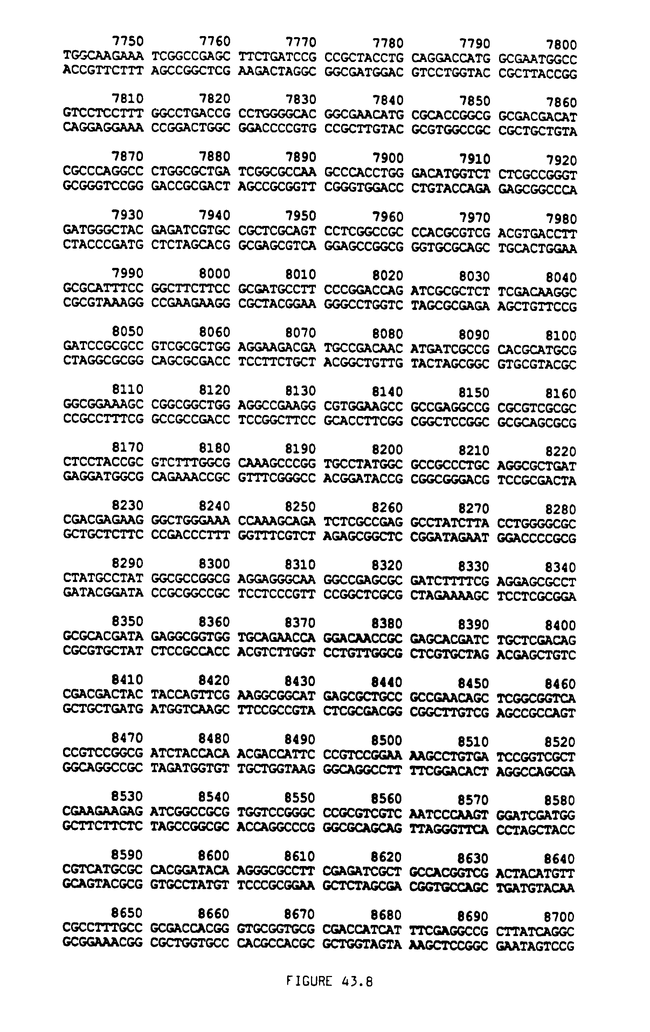

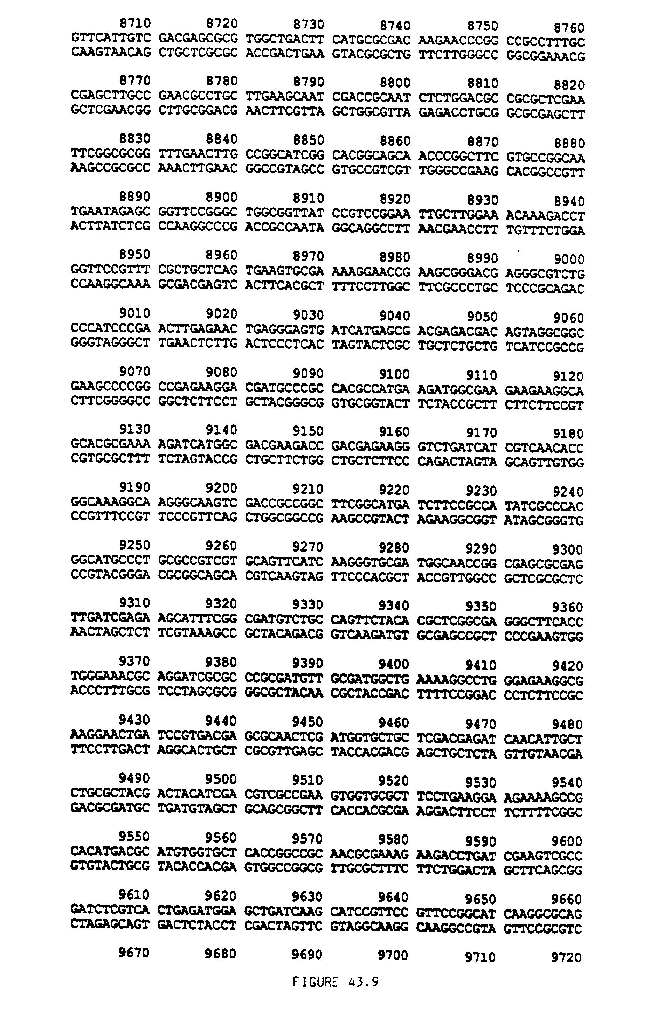

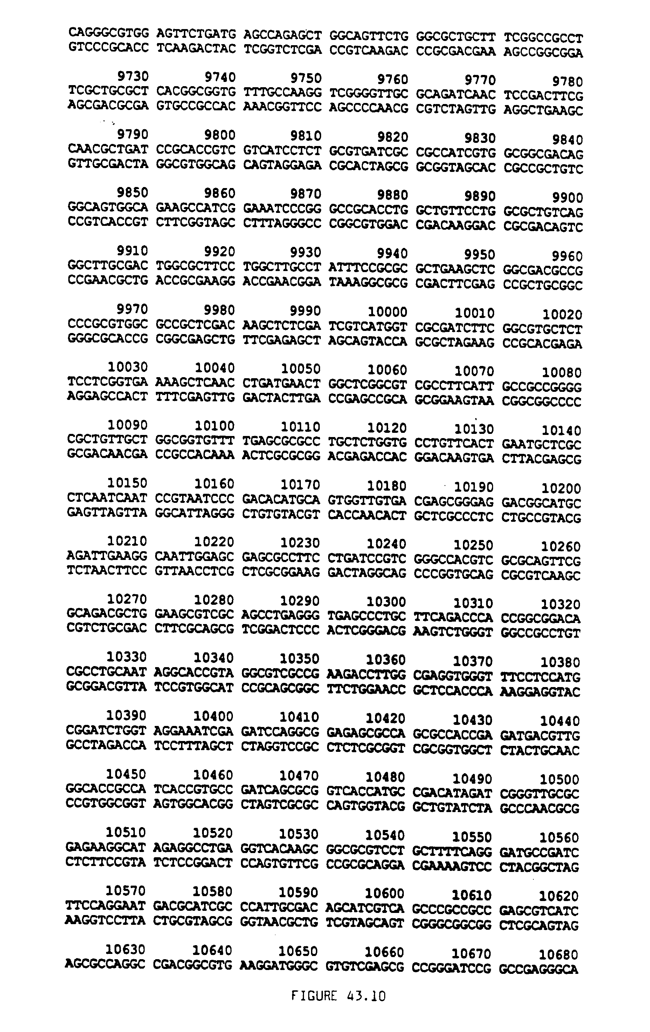

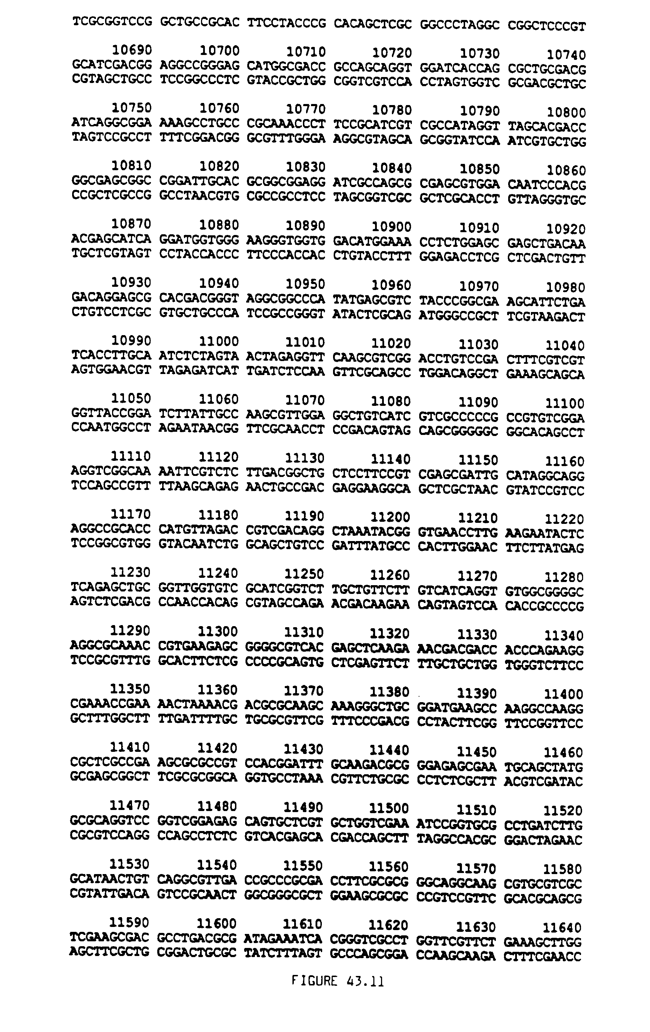

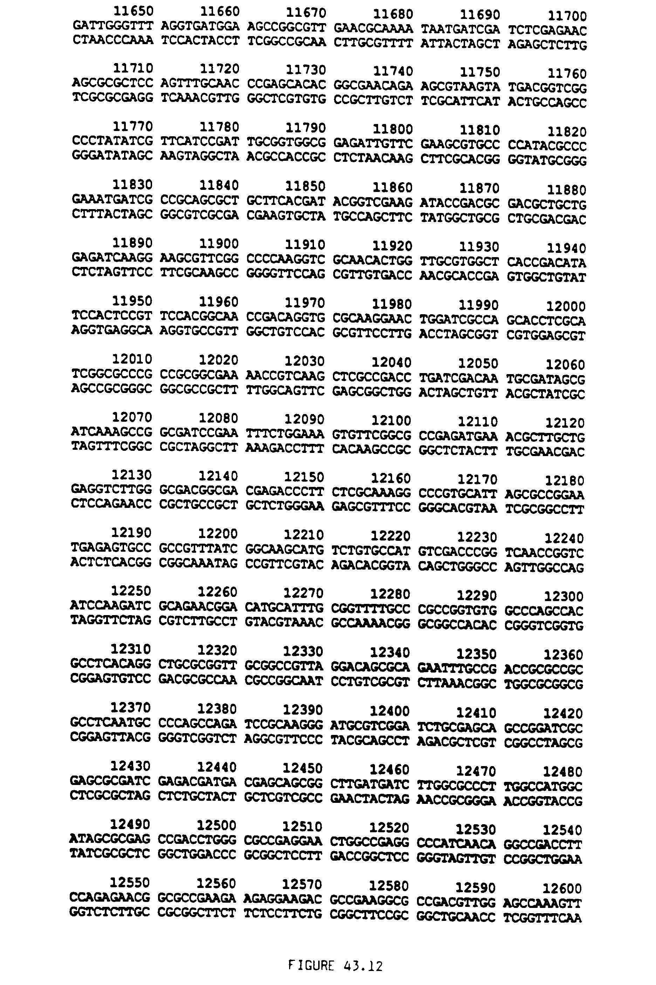

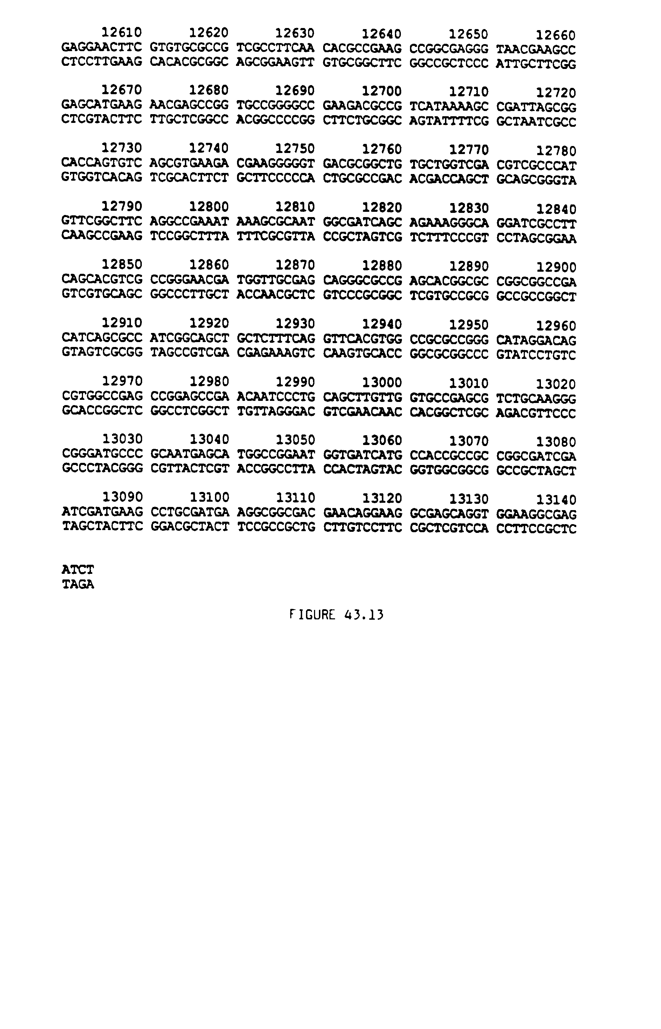

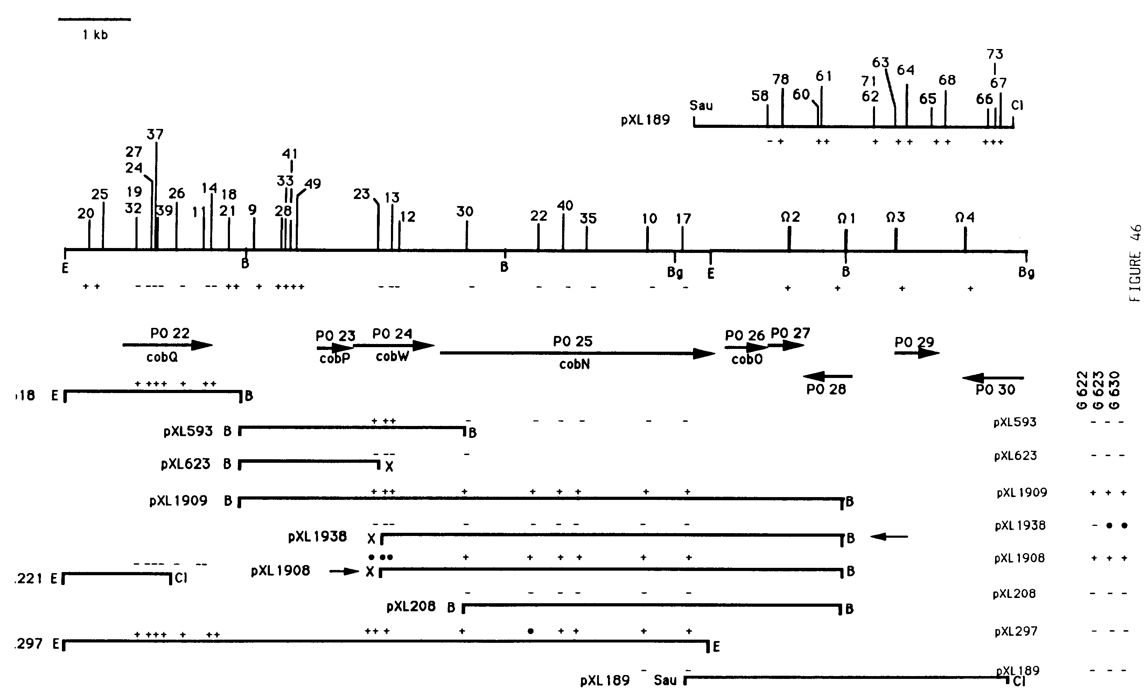

Figure 1:
   Structure du coenzyme B12; le groupement 5'-déoxyadénosyl est remplacé par un groupement CH3 pour la méthylcobalamine, par un groupement cyano pour la cyanocobalamine, par un groupement hydroxyl pour l'hydroxocobalamine.
Figure 2:
   Biosynthèse des cobalamines et différentes étapes de cette biosynthèse suivant la littérature. X: ligands axiaux du cobalt; le ligand en a peut être différent du ligand en b. R: ligand en a du cobalt qui définit le type de cobalamine (voir figure 1).
Figure 3:
   Structures de l'uro'gen III, du précorrine-1, du précorrine-2 et du précorrine-3.
Figure 4:
   Formules développées de l'uro'gen III et de l'acide cobyrinique. D'après la littérature, entre l'uro'gen III et l'acide cobyrinique il se produit 8 transferts de méthyles SAM-dépendants successivement en C2, C7, C20, C17, C12, C1, C15 et C5, une décarboxylation en C12, l'élimination du carbone en C20 et l'insertion de l'atome de cobalt. X: ligands axiaux du cobalt; le ligand en a peut être différent du ligand en b.
Figure 5:
   Dernières étapes de la biosynthèse des cobalamines. Afin de clarifier le schéma des détails du noyau corrine ont été omis. Les cinq étapes enzymatiques sont représentées: 1, cobinamide kinase; 2, cobinamide phosphate guanylyltransférase; 3, cobalamine 5'-phosphate synthase; 4, cobalamine 5'-phosphate phosphohydrolase; 5, nicotinate nucléotide:DMBI phosphoribosyltransférase.
Figure 6:
   Cartes de restriction des fragments ClaI-HindIII-HindIII-HindIII de 5.4 kb; EcoRI de 8.7 kb; SalI-SalI-SalI-SalI-SalI-BglI de 4748 pb et SstI-SstI-BamHI de 3855 pb . Ne figurent que les 20 enzymes de restriction qui coupent le moins fréquemment l'ADN. Les sites de coupure de chaque enzyme sont indiqués par un trait vertical.
Figure 7:
   Séquence nucléotidique des deux brins du fragment ClaI-HindIII-HindIII-HindIII de 5378 pb de Pseudomonas denitrificans. Le brin situé en haut est à lire 5' vers 3' dans le sens gauche droite qui correspond à l'orientation gauche droite du fragment de la carte de restriction présentée à la figure 6. Le site ClaI se trouve à la position 23 (début du site de coupure) car sur cette séquence se trouvent des sites de restriction PstI, SalI et XbaI qui sont apparus lors des clonages dans des multisites en vue du séquençage. La séquence du fragment ClaI-HindIII-HindIII-HindIII commence donc à la position 23.
Figure 8:
   Séquence nucléotidique des deux brins du fragment EcoRI de 8753 pb de Pseudomonas denitrificans. Le brin situé en haut est à lire 5' vers 3' dans le sens gauche droite qui correspond à l'orientation gauche-droite du fragment de la carte de restriction présentée à la figure 6.
Figure 9:
   Analyse des probabilités des phases codantes d'après usage des codons en utilisant le programme de Staden et MacLachlan (1982) sur les 6 phases de lecture de la séquence du fragment ClaI-HindIII-HindIII-HindIII de 5378 pb. Pour les phases qui appartiennent au même brin codant, la phase la plus probable correspond à celle où une ligne pointillée, non interrompue par des codons stop, est plaçée sous la ligne de probabilité de cette phase.
   1. Séquence allant du nucléotide 1 au nucléotide 1200. Grâce à cette analyse, la phase ouverte 1 est identifiée. Elle commence à l'ATG en position 549 et se termine au TGA en position 1011.
   2. Séquence allant du nucléotide 1000 au nucléotide 2200. Grâce à cette analyse, la phase ouverte 2 est identifiée. Elle commence à l'ATG en position 1141 et se termine au TGA en position 1981.
   3. Séquence allant du nucléotide 1800 au nucléotide 3400. Grâce à cette analyse, la phase ouverte 3 est identifiée. Elle commence à l'ATG en position 1980 et se termine au TGA en position 3282.
   4. Séquence allant du nucléotide 3000 au nucléotide 4500. Grâce à cette analyse, la phase ouverte ouverte 4 est identifiée. Elle commence à l'ATG en position 3281 et se termine au TGA en position 4280.
   5. Séquence allant du nucléotide 3800 au nucléotide 5378. Grâce à cette analyse, la phase ouverte 5 est identifiée. Elle commence au GTG en position 4284 et se termine au TGA en position 5253.
Figure 10:
   Analyse des probabilités des phases codantes d'après usage des codons en utilisant le programme de Staden et MacLachlan (1982) sur les 6 phases de lecture du fragment EcoRI de 8753 pb. Pour les phases qui appartiennent au même brin codant, la phase la plus probable correspond à celle où une ligne pointillée, non interrompue par des codons stop, est plaçée sous la ligne de probabilité de cette phase.
   1. Séquence allant du nucléotide 650 au nucléotide 1650. Grâce à cette analyse, la phase ouverte 6 est identifiée. Elle commence à l'ATG en position 736 et se termine au TAG en position 1519.
   2. Séquence allant du nucléotide 1400 au nucléotide 3100. Grâce à cette analyse, la phase ouverte 7 est identifiée. Elle commence à l'ATG en position 1620 et se termine au TAG en position 2997.
   3. Séquence allant du nucléotide 2700 au nucléotide 3700. Grâce à cette analyse, la phase ouverte 8 est identifiée. Elle commence à l'ATG en position 3002 et se termine au TGA en position 3632.
   4. Séquence allant du nucléotide 3500 au nucléotide 4100. Grâce à cette analyse, la phase ouverte 9 est identifiée. Elle commence au GTG en position 3631 et se termine au TGA en position 4366.
   5. Séquence allant du nucléotide 4150 au nucléotide 5150. Grâce à cette analyse, la phase ouverte 10 est identifiée. Elle commence à l'ATG en position 4365 et se termine au TGA en position 5127.
   6. Séquence allant du nucléotide 5000 au nucléotide 6000. Grâce à cette analyse, la phase ouverte 11 est identifiée. Elle commence à l'ATG en position 5893 et se termine au TAG en position 5110.
   7. Séquence allant du nucléotide 5700 au nucléotide 7200. Grâce à cette analyse, la phase 12 est identifiée. Elle commence à l'ATG en position 5862 et se termine au TAA en position 7101.
   8. Séquence allant du nucléotide 7000 au nucléotide 8000. Grâce à cette analyse, la phase ouverte 13 est identifiée. Elle commence à l'ATG en position 7172 et se termine au TTG en position 7931.
Figure 11:
   Construction des plasmides pXL556, pXL545 et pXL723. Un fragment ClaI-EcoRV de 2.4 kb contenant les gènes cobA et cobE est excisé du fragment de 5,4 kb puis purifié. Un "linker" EcoRI est ajouté au site EcoRV, puis le fragment est inséré dans le pXL59 entre les sites ClaI-EcoRI. Le plasmide ainsi construit est nommé pXL556. La construction est comparable pour le pXL545 : un fragment ClaI-HindIII-HindIII de 1.9 kb est excisé du fragment de 5,4 kb puis purifié. Ce fragment contient uniquement le gène CobE. Un "linker" EcoRI est ajouté au site HindIII, puis le fragment est inséré dans le pXL59 entre les sites ClaI-EcoRI.
   Le pXL723 est construit comme suit: un fragment EcoRI-HindIII de 2.3 kb est excisé du fragment de 5,4 kb, purifié, puis les extrémités sont remplies par le grand fragment de l'ADN polymérase I de E. coli. Ce fragment est cloné dans le pRK290 (Ditta et al., 1981) digéré par EcoRI puis traité avec le large fragment de l'ADN polymérase I de E. coli afin de remplir les extrémités. Les sites de restrictions qui sont figurés entre parenthèse correspondent à des sites qui ont disparu après traitement avec le large fragment de l'ADN polymérase I de E. coli.
   1 , fragment PstI-SstI de RSF1010 (De Graff et al., 1978); 2, fragment PstI-BamHI de pACYC177 (Bagdasarian et al., 1981); 3, fragment BamHI-SstI contenant l'opéron lactose de E. coli sans son promoteur, l'opérateur, le site d'initiation de la traduction et les 8 premiers codons non essentiels de lacZ (Casadaban et al.,1983); 4, fragment Sau3AI de Pseudomonas putida KT2440 (Bagdasarian et al., 1981); ori, origine de réplication; nic, site de relaxation; mob, locus essentiel pour la mobilisation; Kmr gén-e de résistance à la kanamycine (Bagdasarian et al., 1981); B, BamHI; C, ClaI; E, EcoRI; H, HindIII; P, PstI; S, SstI; Sa, SalI; X, XhoI; Xb, XbaI.
Figure 12:
   Etudes des insertions des transposons Tn5Spr et Tn5 sur le fragment de 5378 bp. Les insertions du transposon Tn5 sur le plasmide pXL723 sont figurées comme à la figure 14; celles du transposon Tn5Spr, dans le chromosome de la souche SBL27 Rifr, sont encadrées; les insertions dans le chromosome de SC510 Rifr de cassettes portant le gène de résistance à la kanamycine (1630 et 1631) sont représentées avec une flèche, selon l'orientation de la transcription du gène de résistance à la kanamycine, sous le numéro de l'insertion. Les phases ouvertes déduites de la séquence sont portées sur cette figure (de cobA à cobE); des signes + ou - sont représentés sous chaque insertion de transposon ou de cassette de résistance pour indiquer que l'insertion est inactivationnelle (-) ou non (+) soit pour la complémentation de différents mutants (cas des insertions de transposons Tn5) ou que l'insertion abolit la production de cobalamines de la souche dans laquelle elle a lieu. Il y a une absence de complémentation lorsque le mutant recombiné synthétise moins de trois fois moins de cobalamines que le niveau de synthèse de la souche dont le mutant dérive. Les inserts des plasmides pXL545Ω, pXL1500, pXL1397 et pXL302 sont représentés avec les sites de restriction se trouvant à leurs extrémités. Ces inserts sont clonés dans les plasmides à large spectre d'hôte pXL435 et pXL59 (Cameron et al., 1989).
   Le plasmide pXL545Ω correspond au plasmide pXL545 décrit à la figure 11 avec en plus le fragment BamHI de 2 kb du pHP45Ω (Prentki et Krisch) contenant un gène de résistance à la spectinomycine cloné au site BamHI du pXL545.
   Le plasmide pXL1500 correspond au fragment BglII-SstI de 4.2 kb présenté sur cette figure cloné aux sites BamHI et SstI du pKT230 (Bagdasarian et al., 1981) présenté à la figure 30; le pXL1397 correspond au fragment HindIII-SstI de 2.4 kb désigné sur la figure inséré entre les sites HindIII et SstI du multisite du pXL435 (Cameron et al., 1989) décrit sur la figure 30; le plasmide pXL302 correspond au fragment EcoRI-HindIII de 2.3 kb tel qu'il est décrit sur la figure inséré entre les site EcoRI et HindIII du pXL59 (Cameron et al., 1989), décrit sur la figure 30, le site HindIII utilisé étant le site se trouvant dans le multisite de clonage du pXL59; le pXL723 est décrit à la figure 11 tout comme le pXL545.
   Des signes + ou - sont représentés au dessus de chacun de ces inserts pour indiquer s'il y a complémentation par le plasmide en question des insertions chromosomiques représentées au-desssus. C, ClaI; E, EcoRI; H, HindIII; RV, EcoRV; Sau, Sau3AI; S, SstI.
Figure 13:
   Construction des plasmides pXL253 et pXL367. Le fragment EcoRI de 8.7 kb est excisé puis purifié à partir du plasmide pXL151. Il est cloné au site EcoRI du pKT230 pour donner le pXL253. Ce même fragment est inséré au site EcoRI du pRK290 (Ditta et al., 1981) pour donner le pXL367. 1, fragment PstI-SstI de RSF1010 (De Graff et al., 1978); 2, fragment PstI-BamHI de pACYC177 (Bagdasarian et al., 1981); ori, origine de réplication; nic, site de relaxation; mob , locus essentiel -pour la mobilisation (Bagdasarian et al., 1981); B, BamHI; C, ClaI; E, EcoRI; H, HindIII; P, PstI; S, SstI; Sa, SalI; X, XhoI; Xb, XbaI; tetr gène de résistance à la tétracycline; Kmr , gène de résistance à la kanamycine.
Figure 14:
   Etudes des insertions des transposons Tn3lacZ et Tn5 sur le fragment EcoRI de 8.7 kb cloné dans pRK290 (Ditta et al, 1980). Les insertions des transposons Tn3lacZ sont soulignées au contraire de celles des transposons Tn5. Les phases ouvertes déduites de la séquence (cobF à cobM) sont portées sur cette figure et les huit groupes d'insertions inactivationnelles (numérotés de 1 à 8 sont présentés; des signes + ou - sont représentés sous chaque insertion de transposon pour indiquer que l'insertion est inactivationnelle (-) ou non (+) pour la complémentation de différents mutants). Il y a une absence de complémentation lorsque le mutant recombiné synthétise moins de trois fois moins de vitamine B12 que le niveau de synthèse de la souche dont le mutant dérive. Ces groupes d'insertions inactivationnelles correspondent aux mutants suivants: 1, G615; 2, G614 et G616; 3, G613 et G164; 4, G620; 5, G638; 6, G610 et G609; 7, G612; 8, G611. Ces mutants sont des mutants Cob d'Agrobacterium tumefaciens déjà décrits (Cameron et al, 1989). Une carte de restriction du fragment de 8.7 kb est portée au bas de la figure.
Figure 15:
   Les séquences codantes de chacun des gènes du fragment de 5,4 kb, respectivement cobA à cobE, sont indiquées. La séquence des protéines COBA à COBE codées par ces séquences figurent sous leur séquence codante respective, cobA à cobE.la composition en acides aminés de chaque protéine, en nombre et en pourcentage, respectivement de COBA à COBE, sont présentées ainsi que le poids moléculaire, l'index de polarité, le point isoélectrique, la densité optique à 260 nm et à 280 nm d'une solution à 1 mg/ml de protéine purifiée. Le profil d'hydrophilicité de chaque protéine, respectivement de COBA à COBE, est représenté; il a été calculé d'après le programme de Hopp et Woods (1981). Les valeurs positives correspondent à des régions de la protéine qui sont hydrophiles. En abscisse la position des acides aminés est indiquée tandis qu'en ordonnée la valeur de l'index d'hydrophilicité est figurée; lorsque cette valeur est positive, cela indique que la région de la protéine est hydrophile.
Figure 16:
   Les séquences codantes de chacun des gènes du fragment de 8,7 kb, respectivement cobF à cobM, sont indiquées. Les séquences des protéines COBF à COBM codées par ces séquences, figurent sous leur séquence. La légende est identique à celle de la figure 15. NB. Nous avons fait commencer la protéine COBF à l'ATG situé à la position 736; il est possible que l'ATG situé à la position 751 soit le véritable codon d'initiation de cette protéine.
Figure 17:
   Réaction catalysée par l'acide cobyrinique a,c-diamide synthase. L'ACDAS catalyse l'amidation des fonctions acides carboxyliques des chaines acétate périphériques a et c de l'acide cobyrinique (hydrogénobyrinique) pour donner l'acide cobyrinique diamide (hydrogénobyrinique diamide) ; le donneur du groupement amine utilisé dans le test enzymatique est la L-glutamine; il donne par désamination l'acide L-glutamique. X correspond aux ligands axiaux du cobalt qui peuvent être différents l'un de l'autre.
Figure 18:
   Réaction catalysée par la SP2MT. La SP2MT catalyse le transfert d'un méthyle du SAM au dihydrosirohydrochlorine ou précorrine-2 pour donner le précorrine-3. Le groupement méthyle est transféré à la position C20 du noyau porphyrine.
Figure 19:
   Structure de l'acide hydrogénobyrinique et de l'acide hydrogénobyrinique a,c-diamide.
Figure 20:
   Affinités des protéines COBA et COBF pour le SAM. Les courbes donnent en unités arbitraires la radioactivité à la sortie de la colonne de TSK-125 pour chaque protéine appliquée sur cette colonne. Les temps de rétention sont indiqués en minutes et le pic de radioactivité corrrespondant au SAM libre est observé au temps de 10 min 30 sec.
Figure 21:
   Comparaison des séquences de COBA et de COBI. Seules les régions 1, 2 et 3, de forte homologie, sont présentées. Les signes = sont plaçés entre les résidus identiques et - entre les résidus homologues (H K R, L I V M, A G S T, Y F W, D E Q N B Z, P, C).
Figure 22:
   Comparaison des séquences primaires des protéines COBA de Pseudomonas denitrificans et CYSG de E. coli. L'alignement a été fait suivant le programme de Kanehisa, 1984. Les signes = sont placés entre les résidus identiques et - entre les résidus homologues (H K R, L I V M, A G S T, Y F W, D E Q N B Z, P, C). Les régions 1, 2, 3 correspondent aux zones de forte homologie entre les protéines.
Figure 23:
   Comparaison des séquences de CYSG de E. coli avec des protéines COB de Pseudomonas denitrificans (COBA, COBF, COBI, COBJ, COBL et COBM). Les comparaisons portent sur les régions 1, 2 et 3, de fortes homologies, qui existent entre CYSG, COBA et COBI. Les positions sur les séquences protéiques des régions présentant des homologies sont présentées sur la figure. Nous avons considéré les groupes de résidus homologues suivants: H K R, L I V M, A G S T, Y F W, D E Q N B Z, P, C. Si, à une même position, il se trouve au moins 3 résidus homologues, nous avons encadré ces acides aminés.
Figure 24:
   Construction des plasmides pXL1148 et pXL1149. Le pXL1148 est construit comme suit: le fragment BamHI-BamHI-SstI-SstI de 1.9 kb du fragment de 8,7 kb contenant les gènes cobH et cobI est purifié, et des "linkers" XbaI et EcoRI sont plaçés respectivement aux extrémités BamHI et SstI. Ce fragment est ensuite inséré entre les sites XbaI et EcoRI du plasmide à large spectre d'hôte pXL59 (Cameron et al., 1989) pour donner le plasmide pXL1148. Le pXL1149 est construit comme le pXL1148 si ce n'est que le fragment initialement purifié est le fragment BamHI-BamHI-SstI de 1.5 kb au lieu du fragment contenant en plus le petit fragment SstI de 400 pb utilisé pour le pXL1148. Le fragment subit ensuite les mêmes traitements enzymatiques et le même clonage dans le pXL59. 1, fragment PstI-SstI de RSF1010 (De Graff et al., 1978); 2, fragment PstI-BamHI de pACYC177 (Bagdasarian et al., 1981); 3, fragment BamHI-SstI contenant l'opéron lactose de E. coli sans promoteur, opérateur, site d'initiation de la traduction et les 8 premiers codons non essentiels de lacZ (Casadaban et al.,1983); 4, fragment Sau3AI de Pseudomonas putida KT2440 (Bagdasarian et al., 1981);ori, origine de réplication; nic, site de relaxation; Kmr gène de résistance à la kanamycine; mob , locus essentiel pour la mobilisation (Bagdasarian et al., 1981); B, BamHI; C, ClaI; E, EcoRI; H, HindIII; P, PstI; S, SstI; Sa, SalI; X, XhoI; Xb, XbaI.
Figure 25:
   Protéines totales des souches SC510 Rifr, SC510 Rifr pKT230, SC510 Rifr pXL1148, SC510 Rifr pXL1149 analysées en PAGE-SDS à 10% comme cela est décrit. Les bactéries ont été cultivées pendant 4 jours en milieu PS4, puis des lysats des protéines totales ont été effectués. Piste 1, SC510 Rifr; piste 2, SC510 Rifr pXL1149; piste 3, SC510 Rifr pXL1148; piste 4, SC510 Rifr pKT230. Les masses moléculaires des marqueurs de masse moléculaire sont indiquées. Les positions où les protéines COBI et COBH migrent sont indiquées.
Figure 26:
   Construction des plasmides pXL1496 et pXL1546. Le plasmide pX1496 permet de surexprimer la protéine COBF chez E. coli et le plasmide pXL1546 permet de surexprimer COBF chez Pseudomonas denitrificans. Le fragment EcoRI-XhoI de 2.2 kb est excisé et purifié à partir du fragment de 8.7 kb. Il est cloné au site EcoRI du phage M13mp19 pour donner le plasmide pXL1405. Ensuite un site NdeI est introduit par mutagénèse dirigée, comme cela est décrit précédemment, à la position 733 de ce fragment; de cette manière une site NdeI se trouve juste sur le codon d'initiation présumé du gène cobF. Le nouveau plasmide ainsi obtenu est nommé pXL1406. Un fragment NdeI-SphI-SphI de 1.5 kb, contenant le gène cobF à partir de son codon d'initiation présumé, est purifié après digestion partielle par les enzyme appropriées et ligaturé avec les fragments appropriés du plasmide pXL694 (fragment EcoRI-NdeI de 120 pb contenant des signaux d'expression de E. coli -voir texte- et fragment EcoRI-SphI de 3.1 kb contenant le gène de résistance à l'ampicilline, les fonctions de réplication du plasmide ainsi que les terminateurs de l'opéron rrnB de E. coli comme cela est décrit dans le texte). Le plasmide ainsi construit est nommé pXL1496. Le pXL1546 est construit comme suit: le fragment EcoRI-BamHI-BamHI de 2 kb du pXL1496 est purifié par digestion partielle avec les enzymes appropriées; ce fragment contient les signaux d'expression de E. coli, suivis du gène cobF , puis la partie 5' du gène cobG, elle-même suivie des terminateurs de l'opéron rrnB de E. coli comme cela est décrit dans le texte; ce fragment est cloné dans le plasmide multihôte pKT230 (Bagdasarian et al., 198-1) décrit sur la figure 30. B, BamHI; C, ClaI; E, EcoRI; H, HindIII; P, PstI; S, SstI; Sa, SalI; X, XhoI; Xb, XbaI; Kmr, gène de résistance à la kanamycine; Amp, gène de résistance à l'ampicilline.
Figure 27:
   Protéines totales des souches SC510 Rifr, SC510 Rifr pKT230, SC510 Rifr pXL1546 analysées en PAGE-SDS à 10% comme cela est décrit. Les bactéries ont été cultivées pendant 4 jours en milieu PS4, puis des lysats des protéines totales ont été effectués. Piste 1, SC510 Rifr; piste 2, SC510 Rifr pKT230; piste 3, SC510 Rifr pXL1546. Les masses moléculaires des marqueurs de masse moléculaire sont indiquées. La position où la protéine COBF migre est indiquée.
Figure 28:
   Protéines totales des souches E. coli B et E. coli B pXL1496 analysées en PAGE-SDS à 10 % comme cela est décrit. Piste 1, E. coli pXL1496 cultivée en absence de tryptophane; piste 2, E. coli pXL1496 cultivée dans les mêmes conditions en présence de tryptophane; piste 3, E. coli cultivée en absence de tryptophane; piste 4, E. coli cultivée dans les mêmes conditions en présence de tryptophane. Les masses moléculaires des marqueurs sont indiquées. La position de migration de la protéine COBF est indiquée.
Figure 29:
   Construction des plasmides pXL525 etpXL368. Le pXL368 est construit comme suit: le fragment EcoRV-ClaI de 2.4 kb (contenant les gènes cobA et cobE) est purifié à partir du plasmide pXL556 (B. Cameron et al., 1989) ce qui permet d'obtenir ce fragment avec un site BamHI et un site XbaI aux extrémités; ce fragment est cloné dans le pXL203 aux sites BamHI et XbaI. Pour la construction du pXL525 un "linker" XbaI est ajouté au site EcoRI situé à l'extrémité droite du fragment EcoRI de 8.7 kb; ce fragment EcoRI-XbaI de 8.7 kb est ensuite cocloné avec le fragment EcoRI-XbaI de 2.4 kb provenant du pXL556 et contenant cobA et cobE. Les sites de restrictions qui sont figurés entre parenthèse correspondent à des sites qui ont disparu après traitement avec le large fragment de l'ADN polymérase I de E. coli. 1 , fragment PstI-SstI de RSF1010 (De Graff et al., 1978); 2, fragment PstI-BamHI de pACYC177 (Bagdasarian et al., 1981); ori, origine de réplication; nic, site de relaxation; mob , locus essentiel pour la mobilisation; Kmr, gène de résistance à la kanamycine (Bagdasarian et al., 1981); B, BamHI; C, ClaI; E, EcoRI; H, HindIII; P, PstI; S, SstI; Sa, SalI; X, XhoI; Xb, XbaI; tet, gène de résistance à la tétracycline; Ampr et Amp, gène de résistance à l'ampicilline.
Figure 30:
   Plasmides, du groupe d'incompatibilité Q, à large spectre d'hôte chez les bactéries gram-négatives. Ces plasmides sont décrits dans une précédente publication (Cameron et al., 1989) et sont utilisés dans la présente invention. 1, fragment PstI-SstI de RSF1010 (De Graff et al., 1978); 2, fragment PstI-BamHI de pACYC177 (Bagdasarian et al., 1981); 3, fragment BamHI-SstI contenant l'opéron lactose de E. coli sans promoteur, opérateur, site d'initiation de la traduction et les 8 premiers codons non essentiels de lacZ (Casadaban et al.,1983); 4, fragment Sau3AI de Pseudomonas putida KT2440 (Bagdasarian et al., 1981);ori, origine de réplication; nic, site de relaxation; Kmr gène de résistance à la kanamycine; Smr, gène de résistance à la streptomycine ; mob , locus essentiel pour la mobilisation (Bagdasarian et al., 1981); B, BamHI; C, ClaI; E, EcoRI; H, HindIII; P, PstI; S, SstI; Sa, SalI; X, XhoI; Xb, XbaI.
Figure 31:
   Temps de rétention de différents étalons corrinoïdes (1 mg/étalon) sur le système de séparation de décrit à l'exemple 9. La colonne utilisée est une colonne de Nucléosil C-18 (Macherey-Nagel). Au niveau de chaque pic d'absorbance un numéro est représenté correspondant au corrinoïde décrit ci-dessous. Le temps de rétention est porté en abscisse et l'absorbance à 371 nm se trouve en ordonnée. 1, acide cobyrinique; 2, acide cobyrinique a-amide; 3, acide cobyrinique g-amide; 4, acide cobyrinique -a,g-diamide; 5, acide cobyrinique c-amide; 6, acide cobyrinique c,g-diamide; 7, acide cobyrinique a,c-diamide; 8, acide cobyrinique triamide; 9, acide cobyrinique tetraamide; 10, acide cobyrinique pentaamide; 11, acide cobyrique; 12, GDP-cobinamide; 13, cobinamide phosphate; 14, cobinamide; 15, cyanocobalamine 5'-phosphate; 16, cyanocobalamine.
Figure 32:
   Séquence nucléotidique des deux brins du fragment SalI-SalI-SalI-SalI-SalI-BglI de 4748 pb de Pseudomonas denitrificans. Le brin situé en haut est à lire de 5' vers 3' dans le sens gauche droite qui correspond à l'orientation gauche droite du fragment de la carte de restriction présentée sur la figure 6.
Figure 33:
   Séquence nucléotidique des deux brins du fragment SstI-SstI-BamHI de 3855 pb de Pseudomonas denitrificans. Le brin situé en haut est à lire de 5' vers 3' dans le sens gauche droite qui correspond à l'orientation gauche droite du fragment de la carte de restriction présentée sur la figure 6.
Figure 34:
   Analyse des probabilités des phases codantes d'après l'usage des codons en utilisant le programme de Staden et MacLachlan (1982) sur les six phases de lecture du fragment SalI-SalI-SalI-SalI-SalI-BglI de 4748 pb. Pour les phases qui appartiennent au même brin codant, la phase la plus probable correspond à celle où une ligne pointillée, non interrompue par des codons stop, est placée sous la ligne de probabilité de cette phase. 4a. Analyse de la séquence correspondant aux nucléotides 200 à 800. Cette analyse permet d'identifier la phase ouverte 14. Elle commence à l'ATG en position 660 et se termine au TGA en position 379. 4b. Analyse de la séquence correspondant aux nucléotides 800 à 1500. Cette analyse permet d'identifier la phase ouverte 15. Elle commence au GTG en position 925 et se termine au TAA en position 1440. 4c. Analyse de la séquence correspondant aux nucléotides 1450 à 2600. Cette analyse permet d'identifier la phase ouverte 16. Elle commence à l'ATG en position 1512 et se termine au TGA en position 2510. 4d. Analyse de la séquence correspondant aux nucléotides 2500 à 4650. Cette analyse permet d'identifier la phase ouverte 17. Elle commence au GTG en position 2616 et se termine au TGA en position 4511.
Figure 35:
   Analyse des probabilités des phases codantes d'après l'usage des codons en utilisant le programme de Staden et MacLachlan (1982) sur les six phases de lecture du fragment SstI-SstI-BamHI de 3855 pb. Pour les phases qui appartiennent au même brin codant, la phase la plus probable correspond à celle où une ligne pointillée, non interrompue par des codons stop, est placée sous la ligne de probabilité de cette phase. 5a. Analyse de la séquence correspondant aux nucléotides 1 à 905. Cette analyse permet d'identifier la phase ouverte 18. Elle commence à l'ATG en position 809 et se termine au TGA en position 108. 5b. Analyse de la séquence correspondant aux nucléotides 955 à 2105. Cette analyse permet d'identifier la phase ouverte 19. Elle commence à l'ATG en position 1971 et se termine au TGA en position 1063. 5c. Analyse de la séquence correspondant aux nucléotides 2000 à 3300. Cette analyse permet d'identifier la phase ouverte 20. Elle commence à l'ATG en position 2099 et se termine au TAG en position 3115. 5d. Analyse de la séquence correspondant aux nucléotides 3250 à 3855. Cette analyse permet d'identifier la phase ouverte 21. Elle commence à l'ATG en position 3344 et se termine au TGA en position 3757.
Figure 36:
   Construction des plasmides pXL233, pXL843 et pXL1558 à partir du pXL154. Les plasmides sont construits de la manière suivante. Le fragment EcoRI de 3,5 kb, contenant le gène cobS tronqué et la séquence en amont, est excisé du pXL154, puis purifié et cloné dans le site EcoRI du pKT230. Le plasmide ainsi construit est nommé pXL233. Le fragment EcoRI-XhoI-XhoI de 3,5 kb, contenant le gène cobT et la séquence en aval, est excisé et purifié à partir du pXL154 par digestions partielles. Le fragment EcoRI-EcoRI-EcoRI de 4,3 kb contenant le gène cobS et la séquence en amont est excisé et purifié à partir du pXL154, puis ligaturé au fragment de 3,5 kb précédent. Le fragment EcoRI-XhoI de 8 kb environ ainsi obtenu est cloné dans les sites EcoRI et SalI du pXL59 pour générer le plasmide pXL843. Le plasmide pXL1558 est construit de la façon suivante: le fragment HindIII-HindIII de 12 kb est excisé du pXL154 et purifié puis les extrémités sont remplies par le grand fragment de l'ADN polymérase I de E. coli. Cet insert est cloné dans le pRK290 (Ditta et al. 1981) digéré par EcoRI puis traité avec le large fragment de l'ADN polymérase I de E. coli pour rendre les extrémités franches. Les sites de restriction qui sont figurés entre parenthèse correspondent à des sites qui ont disparus au cours du clonage. 1, fragment PstI-SstI de RSF1010 (Degraff et al., 1978); 2, fragment PstI-BamHI de pACYC177 (Bagdasarian et al., 1981); B, BamHI; C, ClaI; E, EcoRI; H, HindIII; P, PstI; S, SstI; Sa, SalI; X, XhoI; Xb, XbaI; Tet gène de résistance à la tétracycline; Kmr gène de résistance à la kanamycine; Smr, gène de résistance à la streptomycine.
Figure 37:
   Etude des insertions du transposon Tn5Sp sur l'insert HindIII-HindIII de 12 kb du pXL154. Les insertions du transposon sont cartographiées sur l'insert HindIII-HindIII de 12 kb, cloné sur le pXL1558. Les insertions chromosomiques dans la souche SC510 Rifr sont encadrées, celle qui ne l'est pas, est introduite dans la souche SBL27 Rifr. Un signe plus ou moins est représenté sous chaque insertion pour indiquer le phénotype Cob de la souche ayant cette insertion. L'absence de complémentation (ou la complémentation) de la souche G2035 par des plasmides pXL1558::Tn5Sp est indiquée par des signes moins (ou plus) au dessous de chaque insertion. Les inserts des plasmides, décrits dans la figure 36, sont représentés. Les signes plus (ou moins), sur ces plasmides et alignés avec les insertions au transposon, schématisent la complémentation (ou (l'absence) de la souche mutée au transposon par le plasmide. Les phases ouvertes déduites de la séquence sont aussi portées sur cette figure (ORF14 à 17 ainsi que les gènes cob correspondants (cobS et cobT). E : EcoRI; H : HindIII; X : XhoI.
Figure 38:
   Construction des plasmides pXL1286, pXL1303, pXL1324, pXL1490B et pXL1557 à partir du pXL519. La position du fragment séquencé est présentée dans la partie supérieure de la figure au dessus de la carte de restiction du cluster; il s'agit d'un SstI-SstI-BamHI de 3.9 kb. Les plasmides sont construits de la manière suiante. Le fragment BglII-EcoRI de 2 kb, contenant le gène cobU et la la séquence en aval, est excisé du pXL519 puis purifié et cloné aux sites BamHI et EcoRI du pKT230 pour générer le plasmide pXL1286. Le fragment SstI-EcoRI de 2,7 kb, contenant le gène cobV tronqué, le gène cobU et la séquence en aval, est excisé du pXL519 puis purifié et cloné aux sites SstI et EcoRI du pKT230 pour générer le plasmide pXL1324. Le fragment SstI-SstI de 1,6 kb, contenant le gène cobV tronqué et la séquence en amont, est excisé du pXL519 puis purifié et cloné au site SstI du pKT230 pour générer le plasmide pXL1303. Le fragment SstI-SstI-BamHI de 3.85 kb est purifié après digestion totale du pXL519 par BamHI et digestion partielle par SstI. Ce fragment est ensuite cloné aux sites BamHI et SstI du pKT230 pour générer le pXL1490B. Le plasmide pXL1557 est construit de la façon suivante: le fragment HindIII-BamHI de 9 kb est excisé du pXL519 et purifié puis les extrémités sont remplies par le grand fragment de l'ADN polymérase I de E. coli. Cet insert est cloné dans le pRK290 (Ditta et al. 1981) digéré par EcoRI puis traité avec le large fragment de l'ADN polymérase I de E. coli pour rendre les extrémités franches. Les sites de restriction qui sont figurés entre parenthèse correspondent à des sites qui ont disparus au cours du clonage. 1, fragment PstI-SstI de RSF1010 (Degraff et al., 1978); 2, fragment PstI-BamHI de pACYC177 (Bagdasarian et al., 1981); B, BamHI;Bg, BglII; C, ClaI; E, EcoRI; H, HindIII; P, PstI; S, SstI; Sa, SalI; X, XhoI; Xb, XbaI; Tetr gène de résistance à la tétracycline;Kmr gène de résistance à la kanamycine; Smr, gène de résistance à la streptomycine.
Figure 39:
   Etude des insertions du transposon Tn5Sp sur l'insert HindIII-BamHI de 9 kb du pXL519. Les insertions du transposon sont cartographiées sur l'insert HindIII-BamHI de 9 kb, cloné sur le pXL1557. Les insertions chromosomiques dans la souche SC510 Rifr sont encadrées, celles qui ne le sont pas, sont introduites dans la souche SBL27 Rifr. Un signe plus ou moins est représenté sous chaque insertion pour indiquer le phénotype Cob de la souche ayant cette insertion. L'absence de complémentation (ou la complémentation) de la souche G2040 par des plasmides pXL1557::Tn5Sp est indiquée par des signes moins (ou plus) au dessous de chaque insertion. Les inserts des plasmides, décrits dans la figure 6, sont représentés. Les signes plus (ou moins), sur ces plasmides et alignés avec les insertions au transposon, schématisent la complémentation (ou l'absence) de la souche mutée au transposon par le plasmide. Les phases ouvertes déduites de la séquence sont aussi portées sur cette figure (ORF18 à 21) ainsi que les gènes cob correspondant (cobU et cobV).
Figure 40:
   Séquences codantes de chacun des gènes du fragment de 4,8 kb, respectivement cobX, cobS et cobT, sont indiquées. La séquence des protéines COBX, COBS et COBT codées par ces séquences figure sous les séquences codantes respectives cobX, cobS et cobT. La légende est identique à celle de la figure 15.
Figure 41:
   Séquences codantes de chacun des gènes du fragment de 3,9 kb, respectivement cobU et cobV, sont indiquées. La séquence des protéines COBU et COBV codées par ces séquences figure sous les séquences codantes respectives cobU et cobV. La légende est identique à celle de la figure 15.
Figure 42:
   A.Protéines totales des souches E. coli BL21 pLysS pET3b, E. coli BL21 pLysS pXL1937 analysées sur PAGE-SDS 10%. Piste 1, BL21 pLyspET3b; piste 2, E. coli BL21 pLysS pXL1937.
   B. Protéines totales des souches : E.coli BL21, E.coli BL21 pXL1874 et E.coli BL21 pXL1875 analysées sur PAGE-SDS 10%. Piste 1, E.coli BL21; piste 2, E.coli BL21 pXL1874; piste E.coli BL21 pXL1875.
   Les masses moléculaires des marqueurs sont indiquées. La bande correspondant à la protéine surexprimée est indiquée par une flêche.
Figure 43:
   Séquence nucléotidique des deux brins du fragment SstI-SstI-SstI-SstI-BglII-BglII de 13144 pb de Pseudomonas denitrificans. Le brin situé en haut est à lire de 5' vers 3' dans le sens gauche droite qui correspond à l'orientation gauche droite du fragment de la carte de restriction présentée sur la figure 46.
Figure 44:
   Carte de restriction du fragment SstI-SstI-SstI-SstI-BglII-SstI-BglII de 13144 pb de Pseudomonas denitrificans. La ou les positions de sites de restriction courant sont indiquées par ordre croissant du nombre de coupure sur le fragment séquencé; les positions correspondent à la séquence présentée sur la figure 43.
Figure 45:
   Analyse des probabilités des phases codantes d'après l'usage des codons en utilisant le programme de Staden et MacLachlan (1982) sur les six phases de lecture du fragment SstI-SstI-SstI-SstI-BglII-SstI-BglII de 13144 pb de Pseudomonas denitrificans. Pour les phases qui appartiennent au même brin codant, la phase la plus probable correspond à celle où une ligne pointillée, non interrompue par des codons stop, est placée sous la ligne de probabilité de cette phase.
   1. Séquence correspondant aux nucléotides 1 à 2266. Cette analyse permet d'identifier la phase ouverte 22. Elle commence à l'ATG en position 429 et se termine au TAG en position 1884.
   2. Séquence correspondant aux nucléotides 2266 à 4000. Cette analyse permet d'identifier la phase ouverte 23. Elle commence à l'ATG en position 3364 et se termine au TGA en position 3886.
   3. Séquence correspondant aux nucléotides 3800 à 5000. Cette analyse permet d'identifier la phase ouverte 24. Elle commence à l'ATG en position 3892 et se termine au TAG en position 4954.
   4. Séquence correspondant aux nucléotides 5000 à 9000. Cette analyse permet d'identifier la phase ouverte 25. Elle commence à l'ATG en position 5060 et se termine au TAG en position 8885.
   5. Séquence correspondant aux nucléotides 9000 à 9700. Cette analyse permet d'identifier la phase ouverte 26. Elle commence à l'ATG en position 9034 et se termine au TGA en position 9676.
   6. Séquence correspondant aux nucléotides 9600 à 13144. Cette analyse permet d'identifier les phase ouvertes 27, 28, 29 et 30. Elles commencent respectivement aux ATG en position 9678, 10895, 11656 et 13059 et se terminent aux codons stop en position 10101, 10304, 12181 et 12366. Les phases ouvertes 28 et 30 se trouvent sur le brin complémentaire de celui codant correspondant à toutes les autres phases ouvertes.
Figure 46:
   Fragment EcoRI-BglII-EcoRI-BglII de 13.4 kb, positions des insertions des transposons Tn5Sp sur le fragment EcoRI de 9,1 kb, positions des insertions des transposons Tn5 sur l'insert du plasmide pXL189 ainsi que les inserts des divers plasmides utilisés lors des expériences de complémentation des souches SC510 Rif^{r} ::Tn5Sp. Les complémentations des mutants SC510 Rif^{r} ::Tn5Sp par les plasmides sont indiquées (+)-entre 5 % et 100 % du niveau de la souche parentale, SC510 Rif^{r}-, (∸)-complémentation partielle, entre 0.5 à 5 % du niveau de SC510 Rif^{r}-, ou (-)-absence de complémentation, c'est à dire, moins de mille fois moins que SC510 Rif^{r}- positionnés juste au dessus des traits schématisant l'insert des plasmides et alignés avec les sites d'insertion des mutant correspondants. Au dessous de la cartographie des insertions des transposons Tn5 sur l'insert du plasmide pXL189 est représentée la complémentation (+), ou l'absence de complémentation (-) de ces plasmides mutants pour les mutants d'Agrobacterium tumefaciens G632 et G633. Sur la partie droite de la figure se trouve un tableau représentant la complémentation des mutants G622, G623 et G630 (Cameron et al., 1989) par différents plasmides; (+)-complémentation totale, 100 % du niveau de la souche parentale, C58C9 Rif^{r}-, (∸)-complémentation partielle, entre 10 et 50 % du niveau de C58C9 Rif^{r}-, ou (-)-absence de complémentation-.
   Les différents plasmides dont l'insert est représenté sont construits comme suit (les fragments sont excisés soit du pXL156 soit du pXL157):
   pXL618 correspond au fragment EcoRI-BamHI de 2.5 kb cloné aux mêmes sites de pKT230 (Bagdasarian et al., 1981);
   pXL593 correspond au fragment BamHI de 3.1 kb cloné au site BamHI de pKT230 (Bagdasarian et al., 1981);
   pXL623 correspond au fragment BamHI-XhoI de 1.9 kb cloné aux sites BamHI-SalI de pXL59 (Cameron et al., 1989);
   pXL1909 correspond au fragment BamHI-BamHI-BamHI de 8.4 kb cloné au site BamHI de pKT230 (Bagdasarian et al., 1981);
   le pXL221 correspond au fragment EcoRI-ClaI de 1.6 kb cloné aux mêmes site de pXL59 (le site ClaI dans lequel est cloné ce fragment est le site ClaI du multisite de pXL59 (Cameron et al., 1989);
   les pXL1908 et 1938 correspondent au même insert, fragment XhoI-BamHI-BamHI de 6.5 kb, auquel des linkers XbaI ont été ajoutés; cet insert est cloné dans les deux orientations au site XbaI de pXL435 (Cameron et al., 1989); une flèche positionnée sur la figure indique la position du gène de résistance à la kanamycine vis à vis des extrémités de l'insert des deux plasmides;
   pXL208 correspond au fragment BamHI de 5.2 kb cloné au site BamHI de pKT230 (Bagdasarian et al., 1981);
   pXL297 correspond au fragment EcoRI de 9.1 kb cloné au site EcoRI de pKT230 (Bagdasarian et al., 1981).
   Les phases ouvertes (PO) définies par le séquençage du fragment (PO 22 à 30) sont représentées ainsi que les gènes cob correspondants; une flèche indique la polarité de la transcription.
   E, EcoRI; B, BamHI; Bg, BglII; Cl, ClaI; Sau, Sau3AI; X, XhoI;
Figure 47:
   Séquences codantes de chacun des gènes du fragment de 13.4 kb, respectivement cobQ, cobP et cobW, cobN et cobO sont indiquées. La séquence des protéines COBQ, COBP, COBW, COBN et COBO codées par ces séquences figurent sous leur séquence codante respective cobO, cobP, cobW, cobN et cobO. La légende est identique à celle de la figure 15.
figure 48 :
   A-Séquence NH2-terminale de la SUMT de M. ivanovii et séquence des oligonucléotides 923 946, 947; -, signifie qu'à cette position, le résidu n'a pas pu être déterminé; pour l'oligonucléotide antisens les acides aminés indiqués au-dessus de la séquence correspondent aux anticodons présentés. B-présentation de l'amplification enzymatique d'un fragment interne au gène de structure de la SUMT de M. ivanovii avec les oligonuléotides 946 et 947.
Figure 49 :
   Constrution de la forme réplicative recombinante pG10. Le fragment de 615 pb obtenu par amplification est digéré par HindIII et EcoRI puis purifié comme cela est décrit. Ce fragment est ensuite mis à ligaturer avec la forme réplicative du phage M13mp19 digérée par les mêmes enzymes. Le clone recombinant est trouvé comme cela est décrit dans le texte.
Figure 50 :
   Autoradiographie d'un blot d'ADN génomique de M. ivanovii digéré par diverses enzymes, séparé par électrophorèse en gel d'agarose puis transféré sur membrane de Nylon comme cela est décrit précédemment. La membrane est hybridée avec la sonde pG10 comme cela est décrit précédemment. 1, HindIII-BglII; 2, KpnI-BglII; 3, EcoRI-BglII; 4, BglII-PstI. Les tailles des différents fragments qui hybrident avec la sonde sont représentés en kb.
Figure 51 :
   Séquence nucléotidique des deux brins du fragment de 955 pb de M. ivanovii. Le brin situé en haut est à lire de 5' vers 3' dans le sens gauche droite.
Figure 52:
   Séquence codante du géne corA de M. ivanovii obtenue à partir de la séquence de 955 pb. La séquence primaire de la protéine CORA est aussi représentée. Les acides aminés sont représentés au-dessus de leur codon et le codon stop est figuré par une étoile. Les principales propriétés physiques de la protéine CORA de M. ivanovii, à savoir, la composition en acides aminés, en nombre et en pourcentage, le poids moléculaire, l'index de polarité, le point isoélectrique, la densité optique à 280 nm d'une solution à 1 mg/l de protéine purifiée. Le profil d'hydrophobicité de la protéine CORA de M. ivanovii; ce profil a été réalisé d'après le programme de Hopp et Woods (1981). Les valeurs positives correspondent à des régions de la protéine qui sont hydrophiles. Sont indiquées, la position des acides aminés en abscisse, et en ordonnée la valeur de l'index d'hydrophilicité; si cette valeur est positive, cela indique que dans cette région la protéine est hydrophile.
Figure 53 :
   Comparaison des séquences primaires des protéines COBA de P. denitrificans et CORA de M. ivanovii. Les protéines ont été alignées grâce au progamme de Kanehisa (1984). =, acides aminés identiques; -, acides aminés homologues d'après les critères définis précédemment (voir figure 22 et 23).
Figure 54 :
   Construction des plasmides pXL1832 et pXL1841. Les légendes décrites plaçées sur la figure permettent de suivre les les constructions.

### Techniques générales de clonage, de biologie moléculaire et de biochimie.

Les méthodes classiques de biologie moléculaire telles que la centrifugation d'ADN plasmidique en gradient de chlorure de césium-bromure d'éthidium, les digestions par des enzymes de restriction, l'électrophorèse sur gel, l'électroélution des fragments d'ADN à partir de gels d'agarose, la transformation dans E. coli, etc, sont décrites dans la littérature (Maniatis et al.,1982, Ausubel et al., 1987).

Les enzymes de restriction ont été fournies par New-England Biolabs (Biolabs), Bethesda Reseach Laboratories (BRL) ou Amersham Ltd (Amersham). Les oligonucléotides "linkers" ont été fournis par Biolabs. Pour les ligatures, les fragments d'ADN sont séparés selon leur taille sur des gels d'agarose 0.7 % ou acrylamide 8 %, purifiés par électroélution, extraits au phénol, précipités à l'éthanol puis incubés dans un tampon Tris-HCl pH 7.4 50 mM, MgCl2 10 mM, DTT 10 mM, ATP 2 mM, en présence d'ADN ligase du phage T4 (Biolabs). Si nécessaire, les fragments d'ADN ayant des extrémités 5' proéminentes sont déphosphorylés par un traitement à la phosphatase alcaline d'intestin de veau (CIP, Pharmacia) à 37°C pendant 30 mn dans le tampon suivant: glycine 100 mM, MgCl2 1 mM, ZnCl2 1 mM, pH 10.5. La même technique est utilisée pour la déphosphorylation des extrémités 3' proéminentes ou franches, mais le traitement est de 15 mn à 37°C puis de 15 mn à 56 °C. L'enzyme est inactivée par chauffage du mélange réactionnel à 68°C pendant 15 mn -en présence de SDS 1% et de NaCl 100 mM suivi d'une extraction au phénol-chloroforme et d'une précipitation à l'éthanol. Le remplissage des extrémités 5' proéminentes est effectué par le fragment de Klenow de l'ADN polymérase I d'E. coli (Biolabs). La réaction est effectuée à température ambiante pendant 30 mn dans un tampon Tris-HCl pH 7.2 50 mM, dNTPs 0.4 mM, MgSO4 10 mM, DTT 0.1 mM, BSA 50 mg/ml. Le remplissage des extrémités 3' proéminentes est effectué en présence de l'ADN polymérase du phage T4 (Biolabs) selon les recommandations du fabriquant. La digestion des extrémités proéminentes est effectuée par traitement limi-té à la nucléase S1 (BRL) selon les recommandations du fabriquant. Des oligonucléotides "linkers" sont ajoutés sur des extrémités de fragments d'ADN comme cela est déjà décrit (Maniatis et al, 1982). La mutagénèse in vitro par oligodéoxynucléotides est effectuée selon la méthode développée par Taylor et al., 1985, en utilisant le kit distribué par Amersham. Les ADN ligaturés sont utilisés pour transformer la souche rendue compétente: E. coli MC1060 [ Δ(lacIOPZYA)X74, galU, galK, strAr, hsdR] pour les plasmides ou E. coli TG1[Δ(lac proA,B), supE, thi, hsdD5/ F' traD36, proA+, B+, lacIq, lacZΔM15] pour les formes réplicatives de phages dérivés du bactériophage M13. Les ADN plasmidiques sont purifiés suivant la technique de Birnboim et Doly, 1979. Les minipréparations d'ADN plasmidique sont faites suivant le protocole de Klein et al., 1980. Les préparations d'ADN chromosomiques de bactéries gram-négatives sont réalisées comme cela a été déjà décrit (Cameron et al., 1989). Les sondes radioactives sont préparées par translation de coupure suivant la méthode déjà détaillée (Rigby et al., 1977). Les hybridations entre des séquences d'ADN ainsi que l'immobilisation des acides nucléiques sur membranes de nitrocellulose sont effectuées comme cela a été décrit (Cameron et al., 1989). Lors de clonages pour lesquels il y a une faible probabilité de trouver le clone recombinant recherché, ceux-ci sont trouvé après hybridation sur filtre comme cela est déjà décrit (Maniatis et al., 1982). La séquence nucléotidique de fragments d'ADN est réalisée par la méthode de terminaison de chaînes (Sanger et al., 1977). Dans le mélange réactionnel le dGTP est remplacé par le 7-déaza-dGTP, ceci afin d'éviter les compressions de bandes lors de l'électrophorèse sur gel d'acrylamide, provoquées par le pourcentage en GC élevé de l'ADN. Les milieux de culture utilisés pour la partie bactériologique ont déjà été présentés (Maniatis et al., 1982). Les cultures en milieu PS4 sont réalisées comme cela est déjà décrit (Cameron et al., 1989); les souches de Pseudomonas denitrificans SC510 Rifr et G2 Rifr sont cultivées en milieu PS4 comme suit: des Erlenmeyers de 250 ml contenant 25 ml de milieu PS4, avec si besoin l'antibiotique sélectif pour le plasmide porté par chaque souche, sont ensemencés avec une dilution au 1/100 de préculture saturée en milieu L (Miller 1972), avec, si besoin est, l'antibiotique sélectif pour le 5 plasmide porté par chaque souche; ces cultures sont incubées 6 jours à 30°C, puis les moûts sont analysés pour leur titre en cobalamines ou bien l'activité enzymatique de certaines enzymes de la voie. Les souches d'Agrobacterium tumefaciens, Pseudomonas putida et de Rhizobium meliloti sont cultivées à 30°C; sauf spécifications, elles sont cultivées en milieu L. Les conjugaisons bactériennes sont réalisées comme cela est déjà décrit (Cameron et al., 1989). Les extraits de protéines totales sont réalisés comme cela est déjà décrit (Ausubel et al., 1987). Les électrophorèses (SDS-PAGE) d'analyse des protéines en gel d'acrylamide en conditions dénaturantes sont effectuées comme cela est déjà décrit (Ausubel et al., 1987). L'appareil PhastSystem (Pharmacia) utilisant le système de tampons discontinus de Laemli (Laemli, 1970) est aussi utilisé; différents gels sont utilisés en fonction des poids moléculaires des protéines à analyser ainsi que leur pureté: PhastGel gradient 8-25 PhastGel Homogeneous 12.5 La coloration est effectuée soit au bleu de Coomassie à l'aide du PhastGel Blue R (Pharmacia), soit au nitrate d'argent en utilisant le PhastGel silver Kit (Pharmacia) en se conformant aux instructions du fabriquant. Les séquences NH2-terminales des protéines sont déterminées par la technique de dégradation d'Edman en utilisant un séquenceur automatisé (Applied Biosystems modèle 407A) couplé à un appareil de CLHP pour l'identification des dérivés phénylthiohydantoïnes.

### EXEMPLE 1 - Isolement de fragments d'ADN de P.denitrificans contenant des gènes Cob

Cet exemple décrit l'isolement de fragments d'ADN de Pseudomonas denitrificans portant des gènes Cob. Ces fragments ont été mis en évidence par des expériences de complémentation de mutants Cob d'A.tumefaciens et P.putida (Cameron et al., 1989).

Ces mutants Cob ont été obtenus par mutagénèse avec la N-méthyl-N'-nitro-N-nitrosoguanidine selon la technique de Miller (Miller et al., 1972), ou par insertions du transposon Tn5. De cette manière, des souches incapables de synthétiser les cobalamines ont été mises en évidence, et en particulier, le mutant cob G572 de P.putida, et les mutants Cob G159, G161, G164, G169, G171, G258, G609, G610, G611, G612, G613, G614, G615, G616, G620, G622, G623, G630, G632, G633, G634, G638, G642, G643, G2034, G2035, G2037, G2038, G2039, G2040, G2041, G2042 et G2043 de A.tumefaciens.

Parallèlement, une banque d'ADN génomique de P.denitrificans est réalisée dans un vecteur mobilisable à large spectre d'hôte, pXL59, par digestion, en présence d'enzymes de restriction, de 5 µg d'ADN (Cameron et al., 1989).

Par complémentation, plusieurs plasmides ont pu être isolés, permettant de complémenter les mutants cob de P.putida et d'A.tumefacien. Parmi ceux-ci on notera plus particulièrement les plasmides pXL151 , pXL154, pXL156, pXL157 et pXL519 .

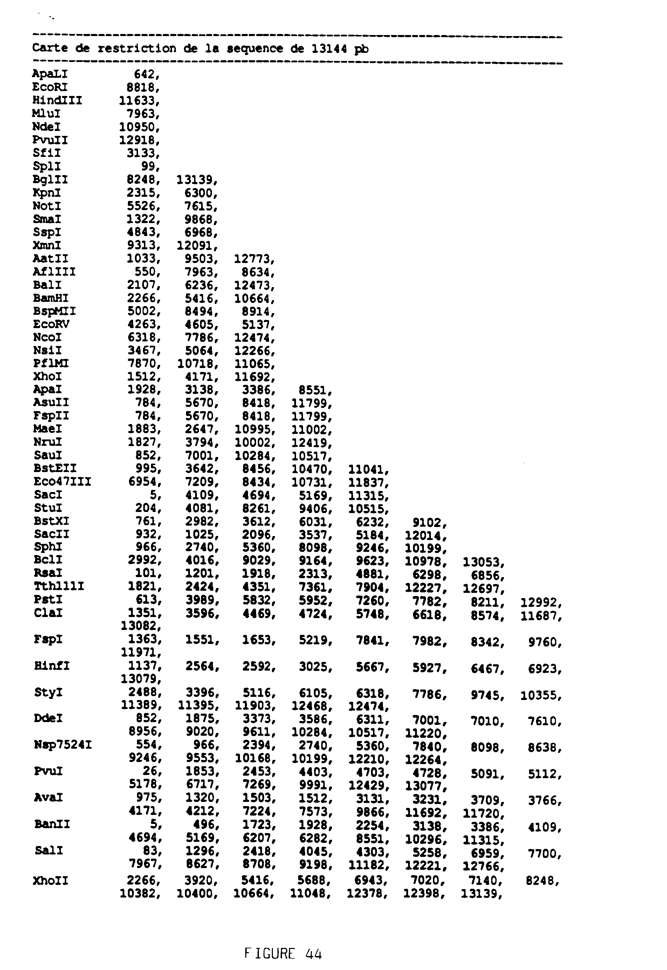

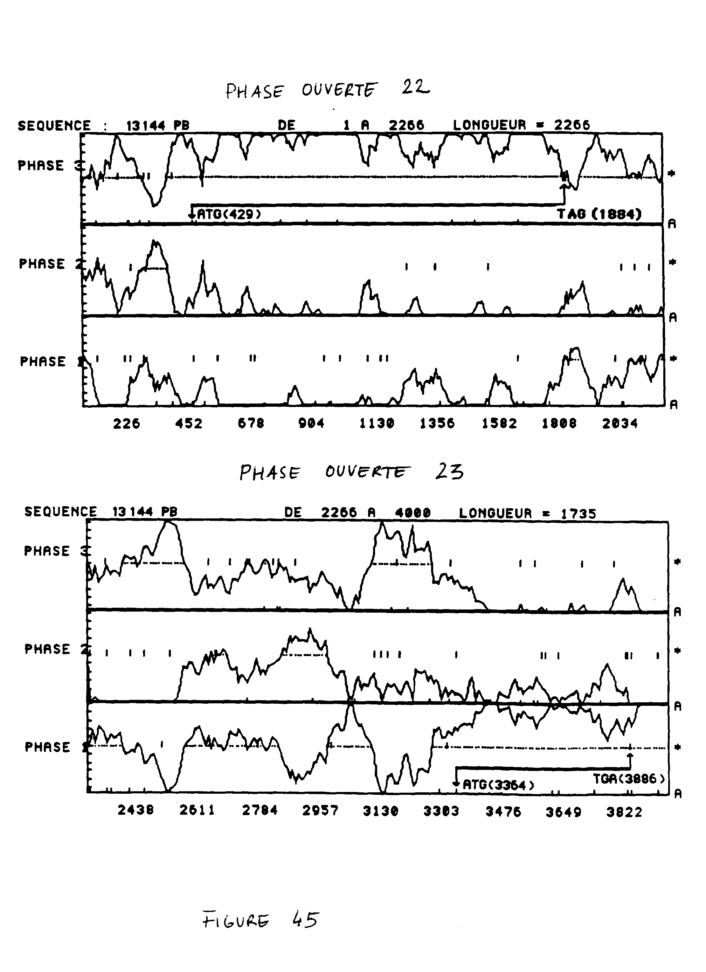

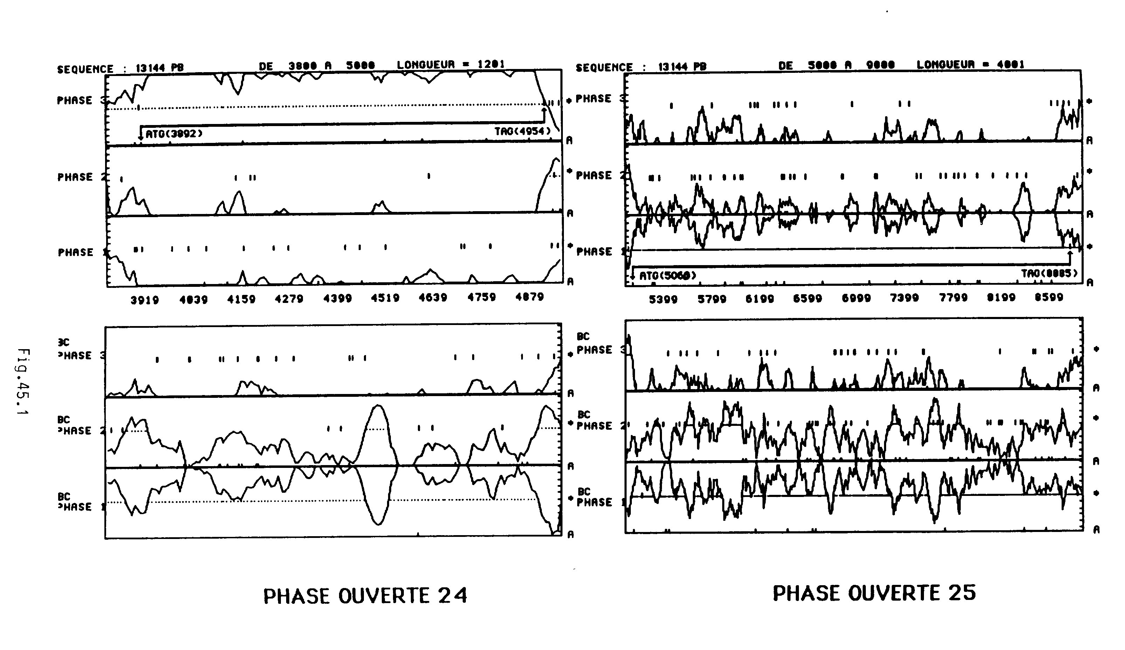

Ces plasmides ont été isolés et des fragments d'ADN ont pu être excisés, purifiés et analysés par restriction. Ces fragments sont présentés sur les figures 6 et 44: Un fragment ClaI-HindIII-HindIII--HindIII de 5,4 kb, un fragment EcoRI-EcoRI de 8,7 kb, un fragment SalI-SalI-SalI-SalI-SalI-BglI de 4,8 kb, un fragment SstI-SstI-BamHI de 3,9 kb et un fragment EcoRI-BglII-EcoRI-BglII de 13,4 kb.

### EXEMPLE 2 - Séquençage des fragments d'ADN isolés

Cet exemple illustre le séquençage de fragments d'ADN portant des gènes cob de Pseudomonas denitrificans SC510.

### 2.1. Séquençage d'un fragment ClaI-HindIII-HindIII-HindIII de 5,4 kb

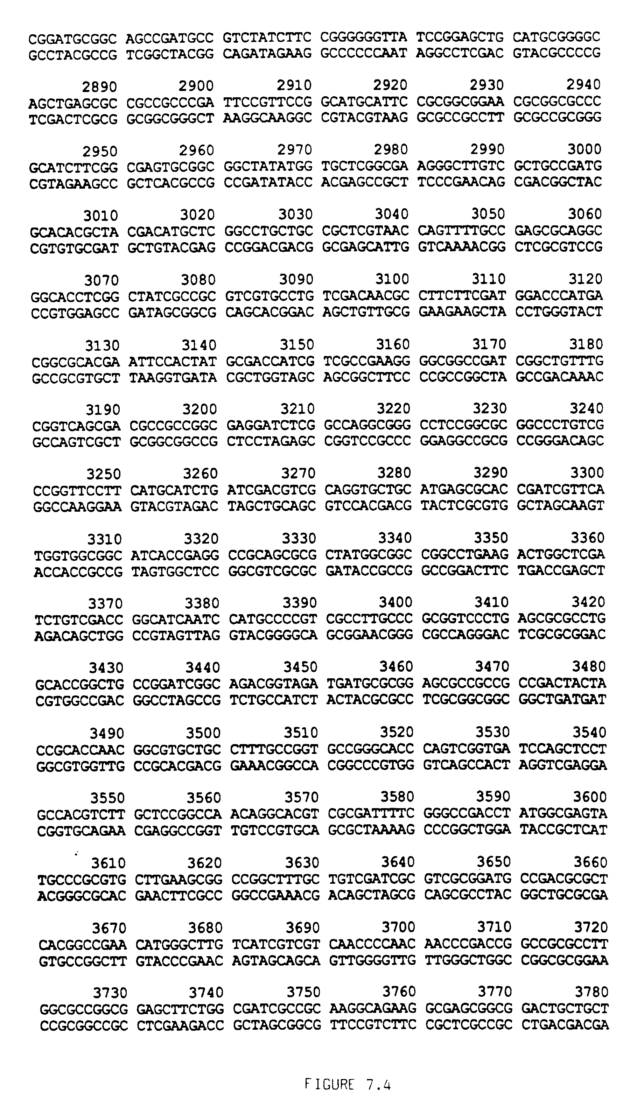

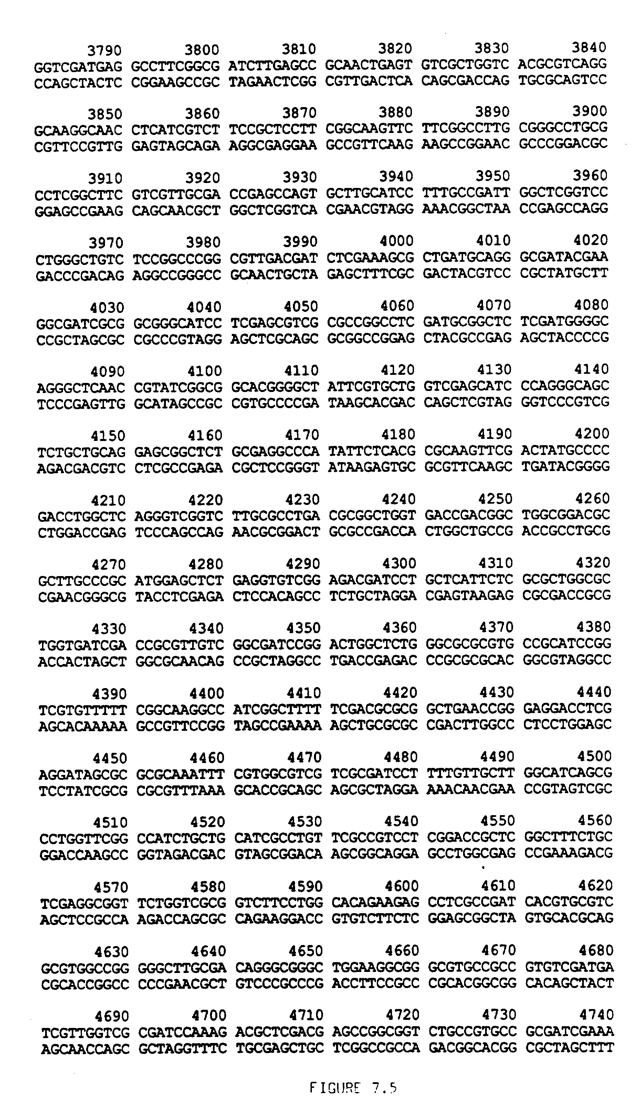

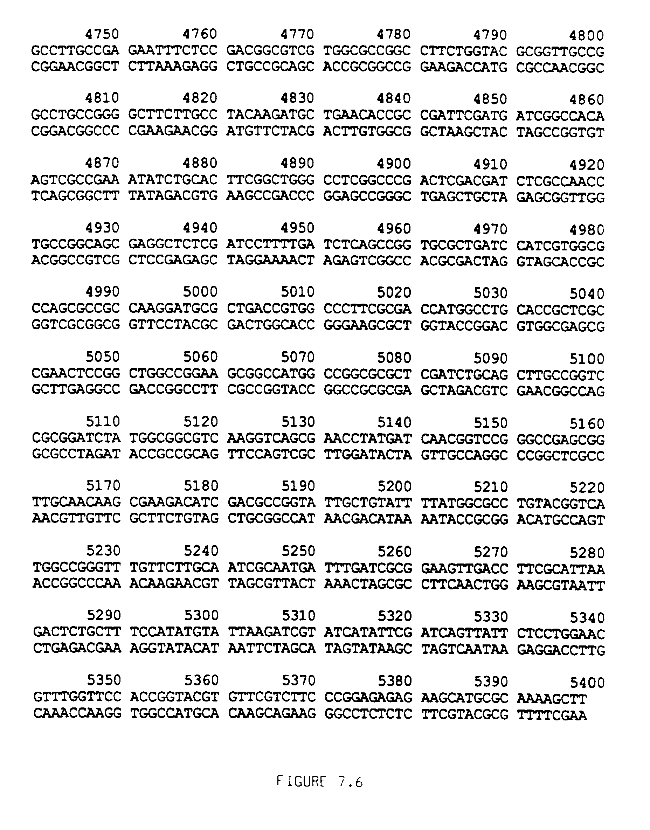

Ce fragment est contenu dans le plasmide pXL157 décrit dans l'exemple 1. Après excision, des sous fragments du fragment de 5,4 kb ont été clonés dans les phages M13mp18, ou M13mp19 (Norrander et al., 1983), ou M13tg130, ou M13tg131 (Kieny et al., 1983) dans les deux orientations. Des délétions ont été ensuite réalisées in vitro par la méthode de Henikoff (1987). Ces délétions ont ensuite été séquencées avec le "primer universel" comme amorce de synthèse des réactions de terminaison de chaîne. Le recouvrement entre ces différentes délétions a permis d'établir la séquence totale, sur les deux brins, du fragment de 5,4 kb (figure 7). Ce fragment comprend 5378 pb. Sur la séquence décrite à la figure 7 figurent, avant le site ClaI, trois sites de restriction (PstI, SalI et XbaI) qui sont apparus lors du clonage du fragment en question en vue du séquençage dans des multisites de clonage. Lorsque par la suite nous nous référerons, dans la présente invention, à la séquence de ce fragment ClaI-HindIII-HindIII-HindIII, ce sera à la séquence présentée à la figure 7 où les 22 premières bases ne correspondent pas à de l'ADN de Pseudomonas denitrificans (ainsi toutes les positions de site de restriction ou de début de phase ouverte se réfèrent à la séquence présentée à la figure 7).

### 2.2 Séquence nucléotidique d'un fragment EcoRI-EcoRI de 8,7 kb

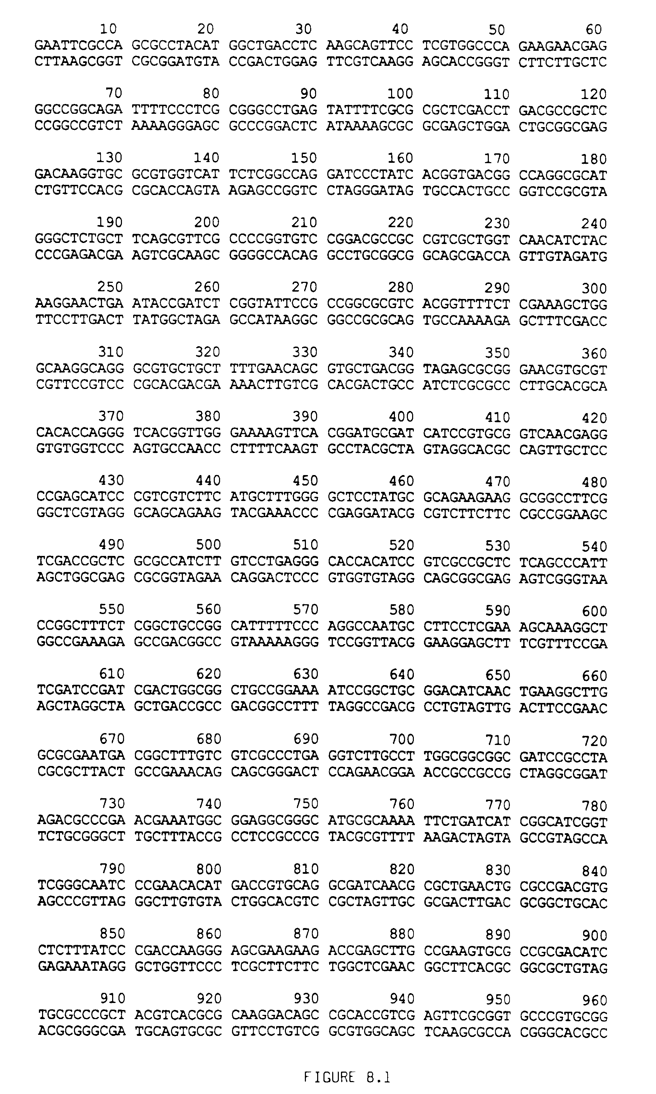

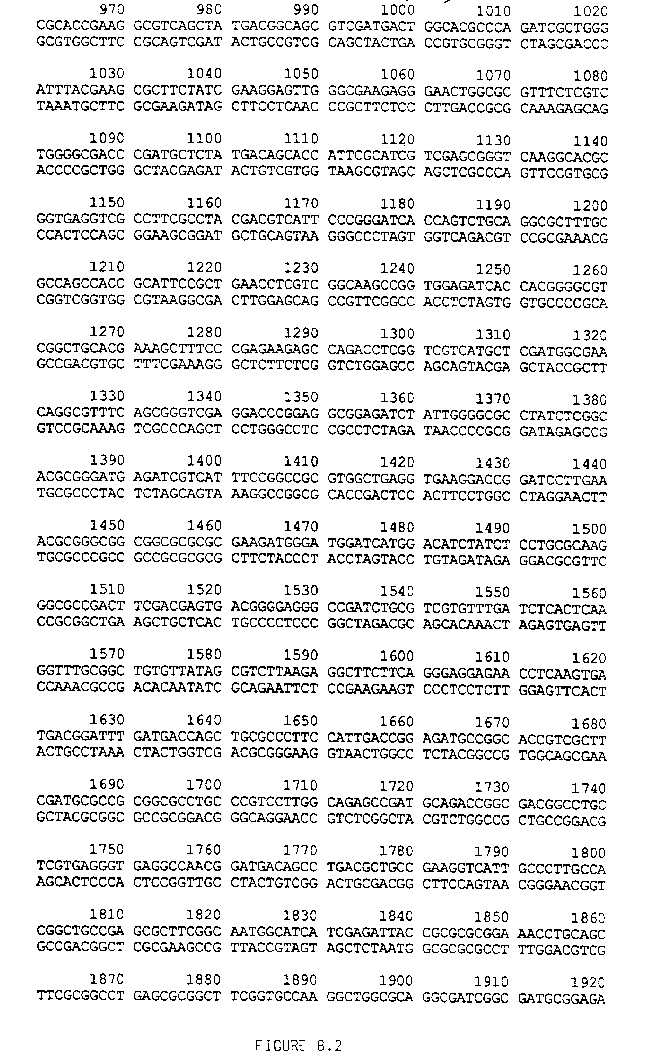

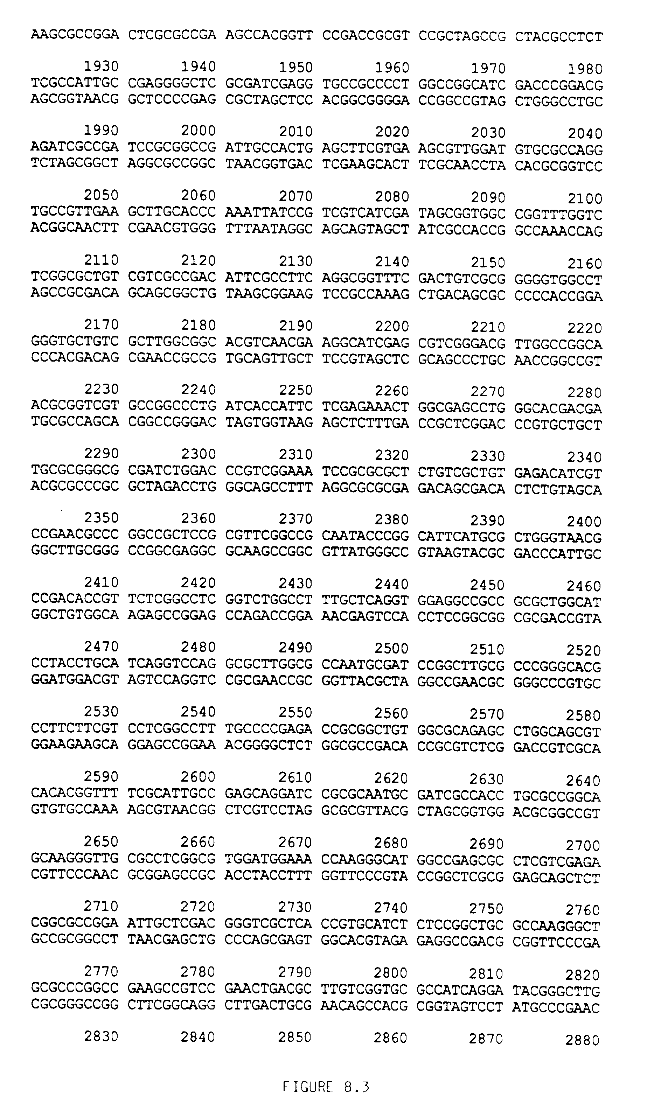

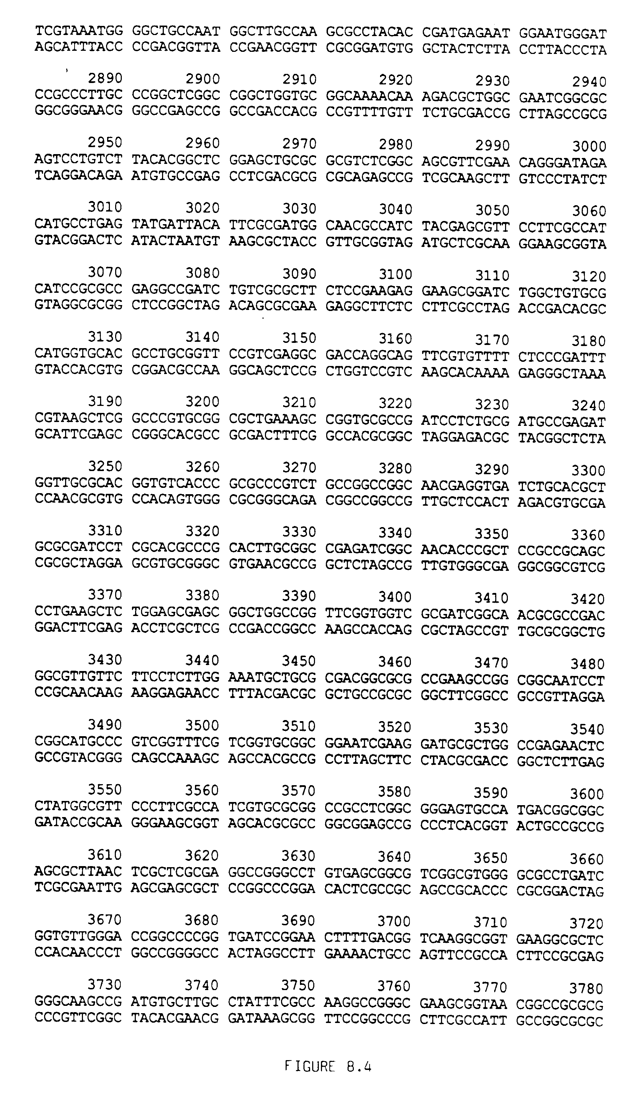

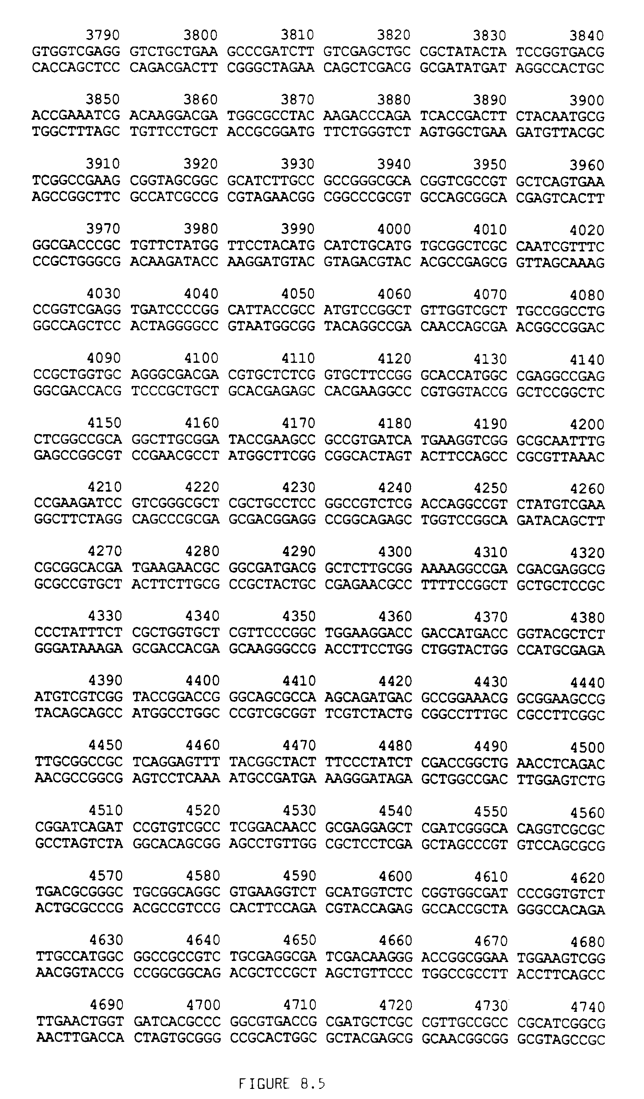

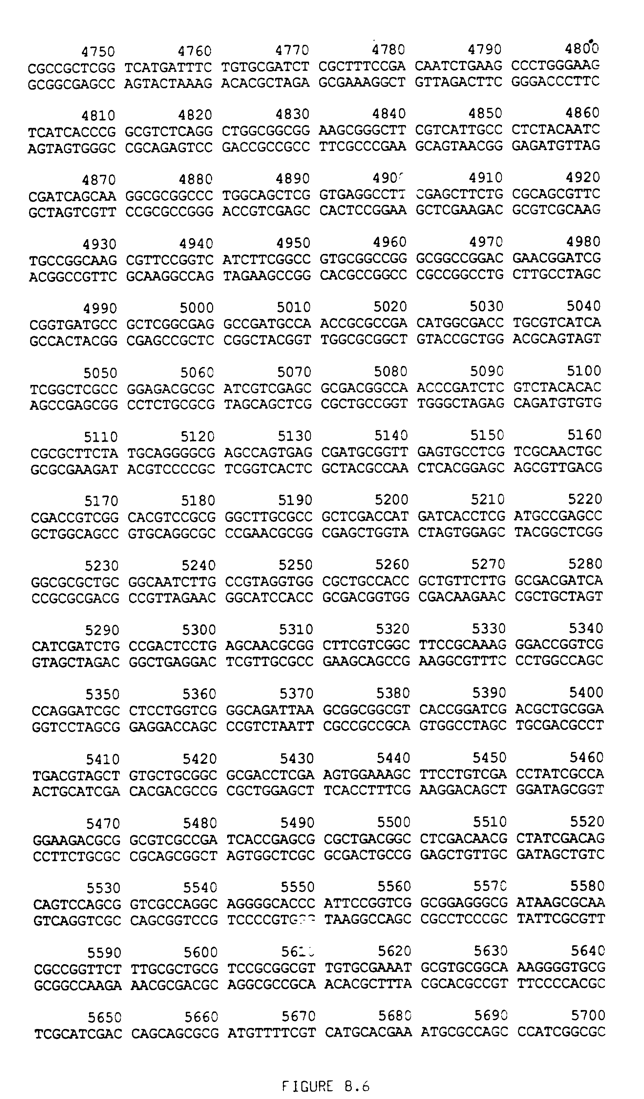

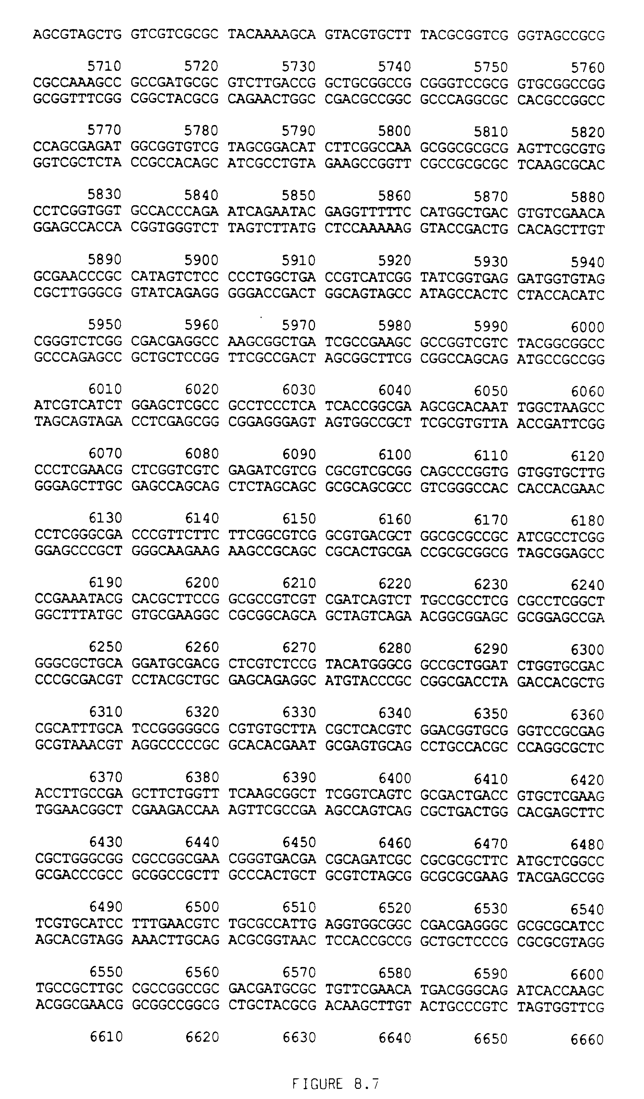

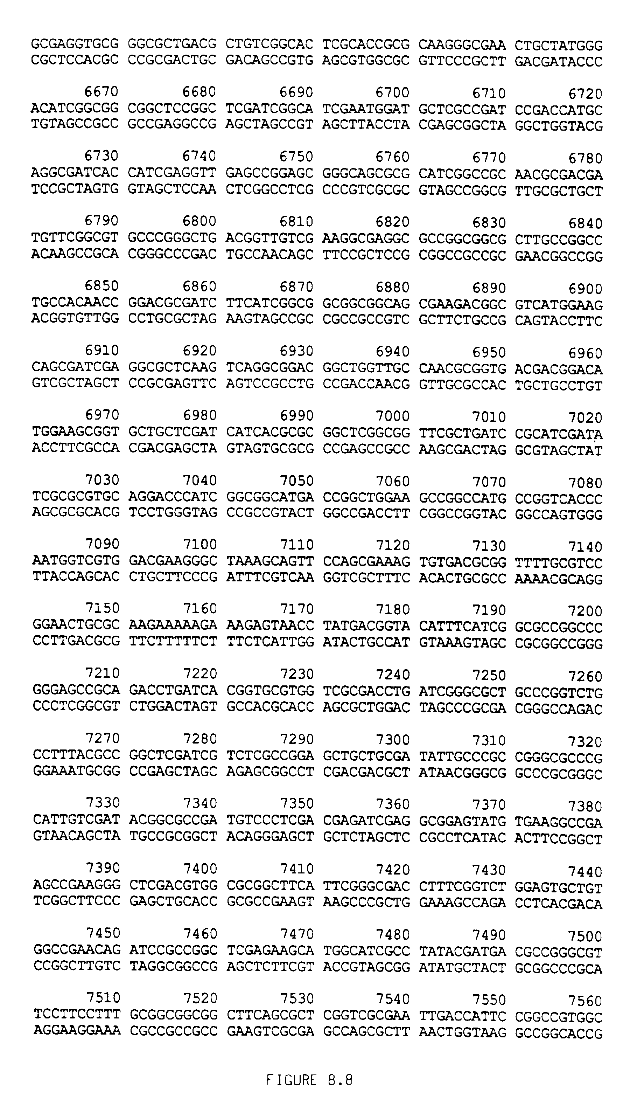

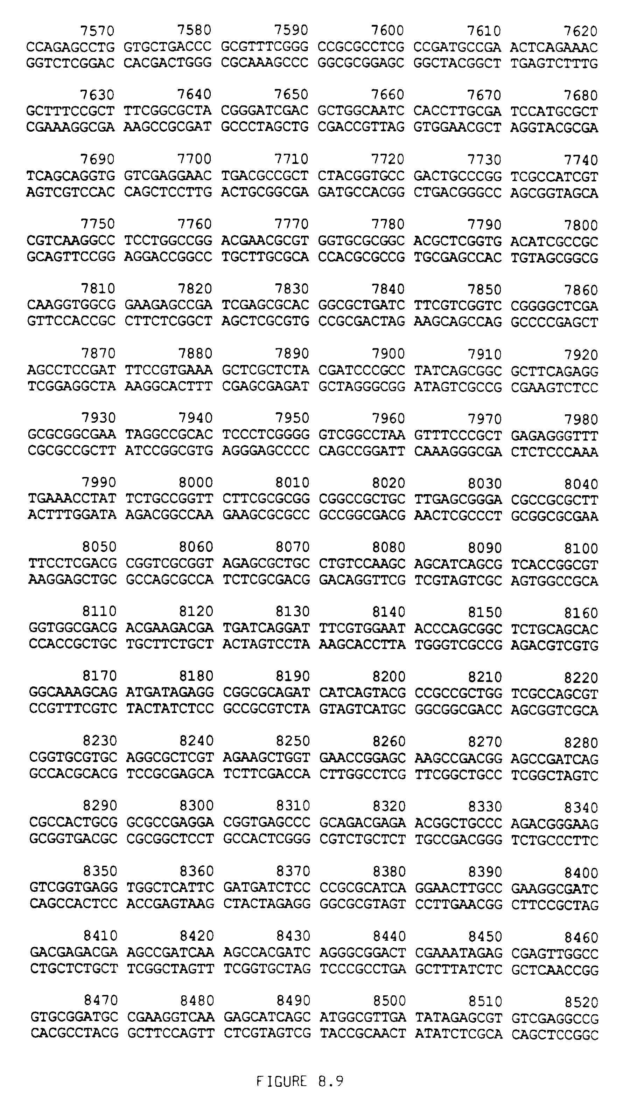

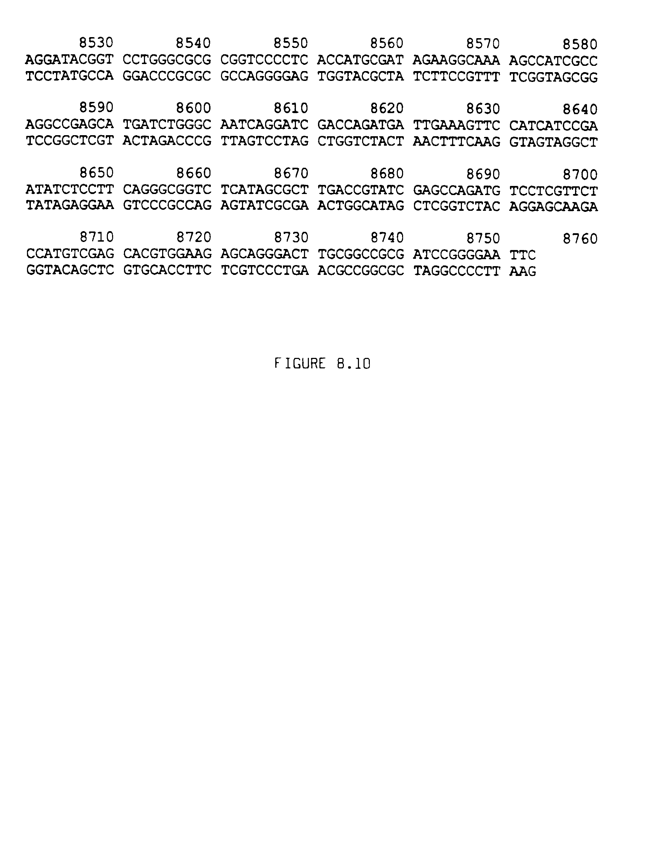

Ce fragment est porté par le pXL151 décrit dans l'exemple 1. Le site EcoRI ainsi que les 70 pb adjacentes situées à la droite de ce fragment proviennent du pXL59 qui est le vecteur qui a servi à construire le pXL151 par clonage d'un fragment Sau3AI de Pseudomonas denitrificans SC510. Après excision, des sous fragments du fragment de 8,7 kb ont été clonés dans les phages M13mp18, ou M13mp19 (Norrander et al., 1983), ou M13tg130, ou M13tg131 dans les deux orientations (Kieny et al., 1983). Des délétions ont été ensuite réalisées in vitro par la méthode de Henikoff (1987). Ces délétions ont ensuite été séquencées avec le "primer universel" comme amorce de synthèse des réactions de terminaison de chaîne. Le recouvrement entre ces différentes délétions a permis d'établir la séquence totale, sur les deux brins, du fragment de 8,7 kb (figure 8). Ce fragment comprend 8753 pb.

### 2.3. Séquençage d'un fragment SalI-SalI-SalI-SalI-SalI-BglI de 4,8 kb.

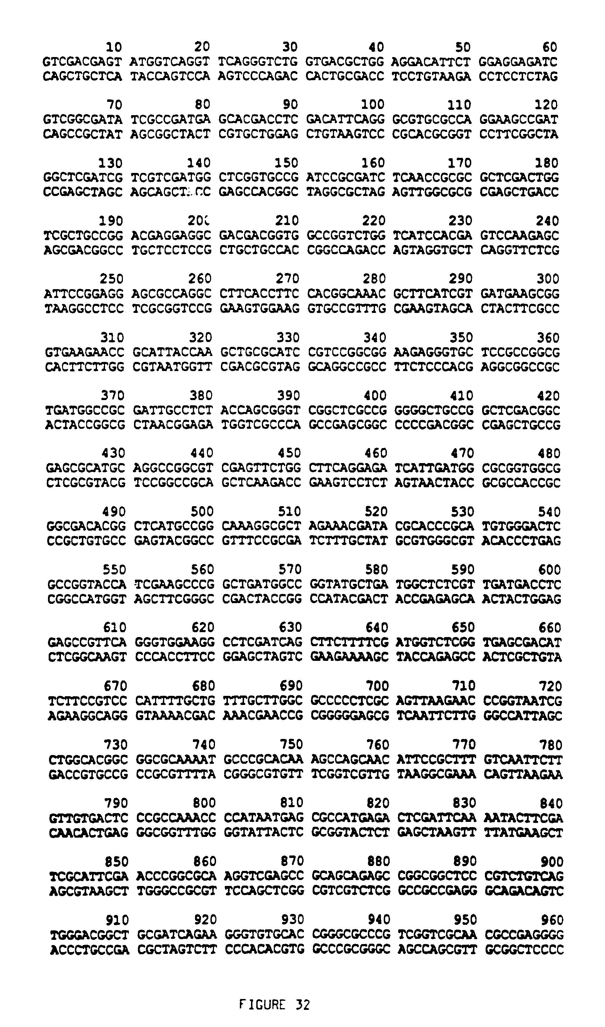

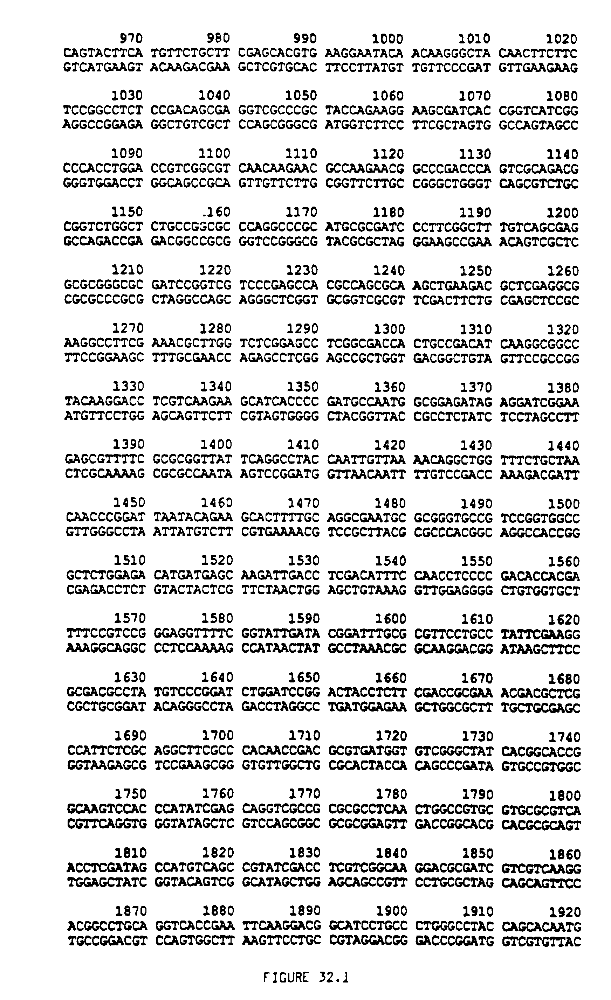

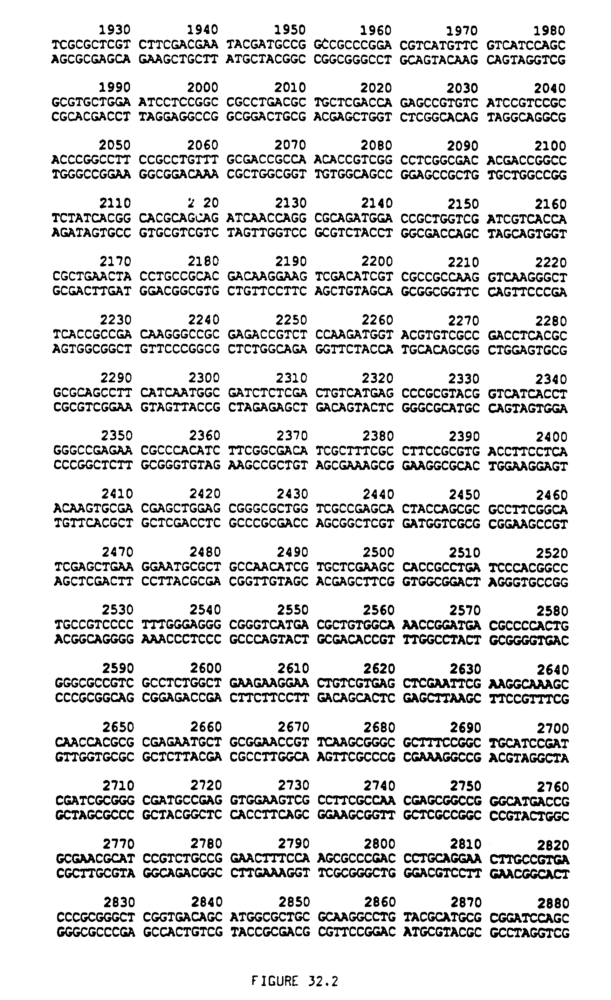

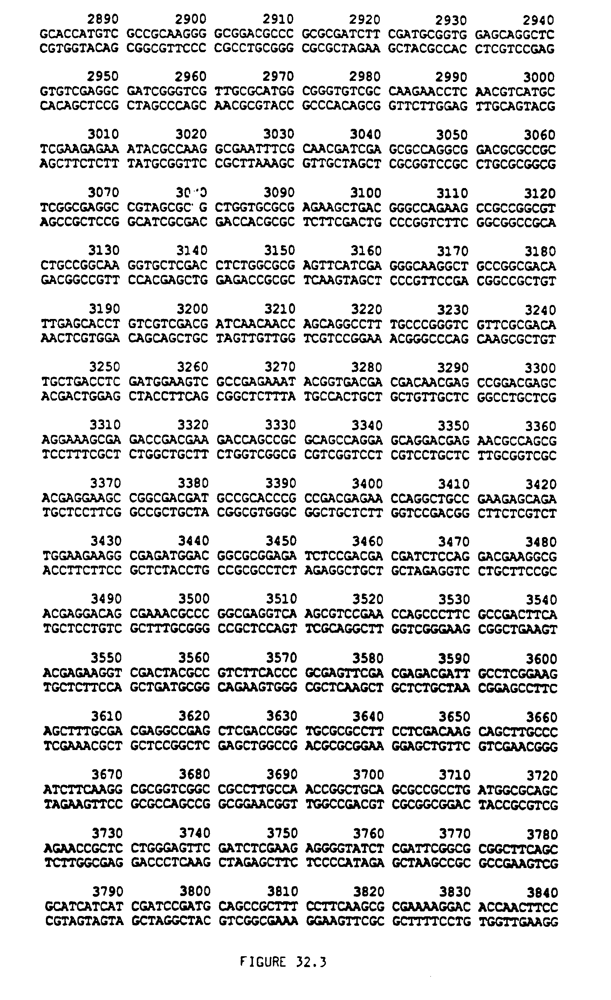

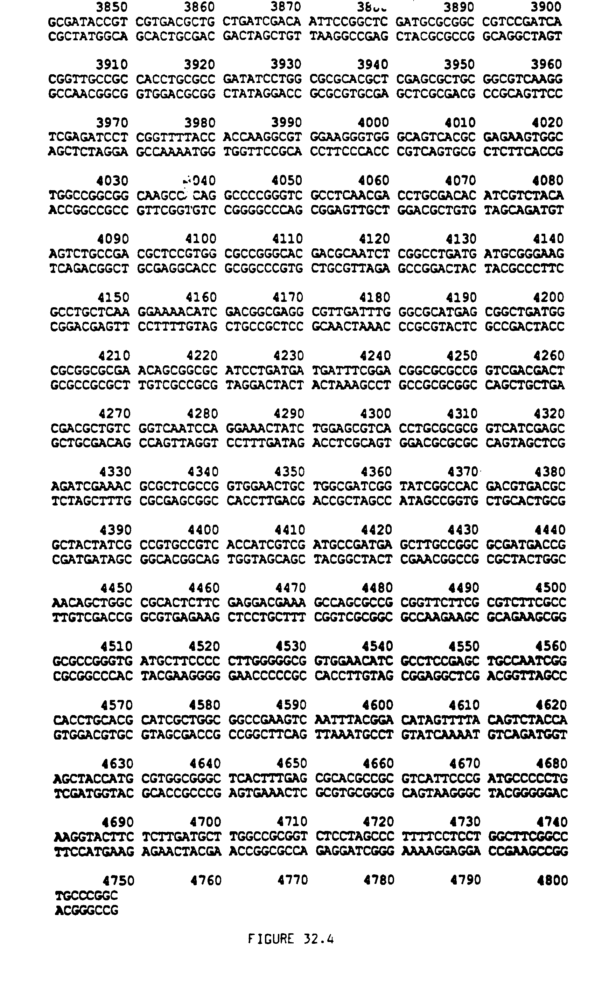

Ce fragment est contenu dans le plasmide pXL154 décrit dans l'exemple 1. Le protocole est identique à celui utilisé dans l'exemple 2.2. La séquence totale sur les deux brins du fragment de 4,8 kb est présentée sur la figure 32. Ce fragment contient 4749 pb.

### 2.4. Séquence nucléotidique d'un fragment SstI-SstI-BamHI de 3,9 kb.

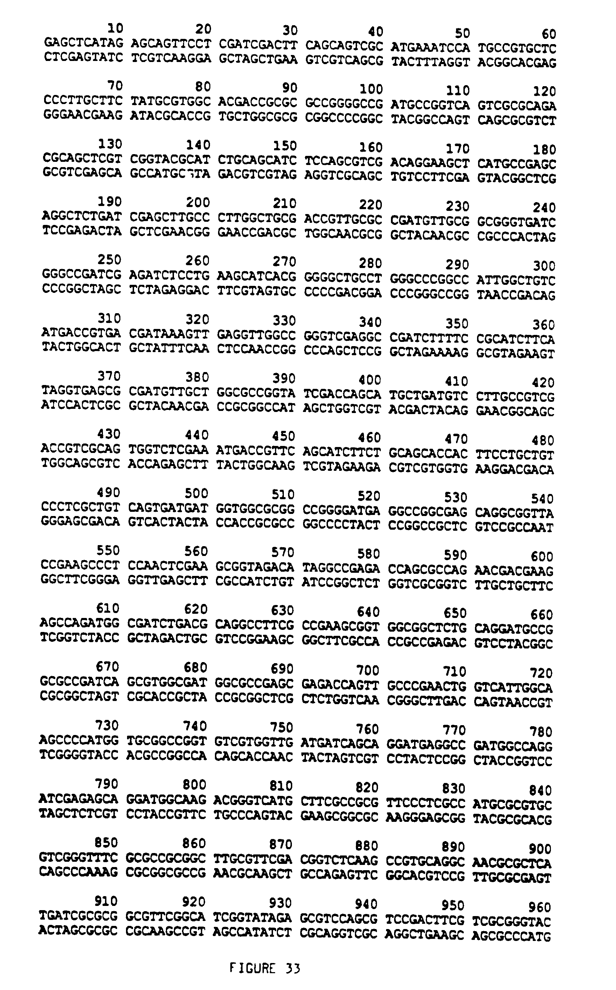

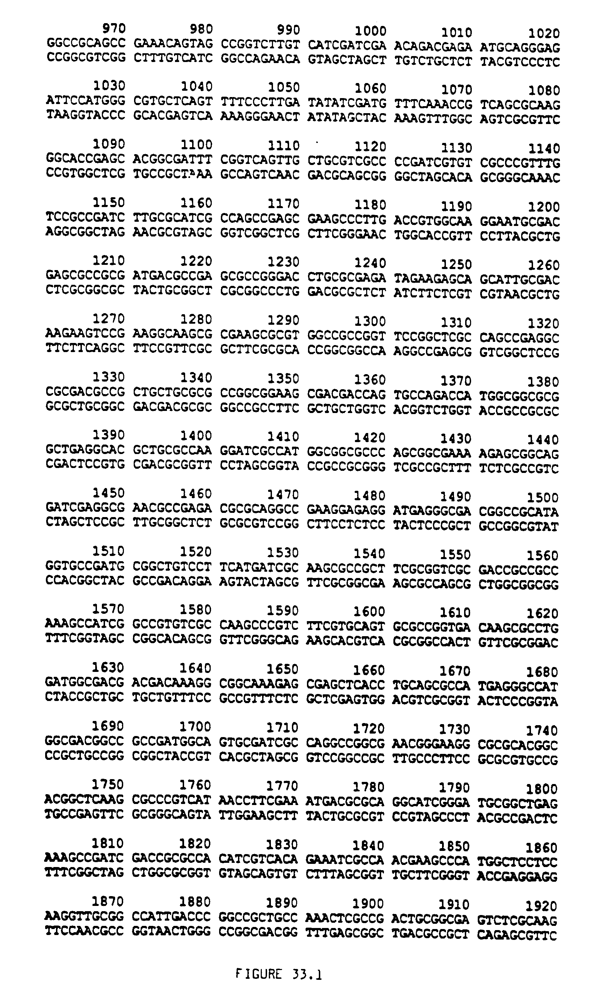

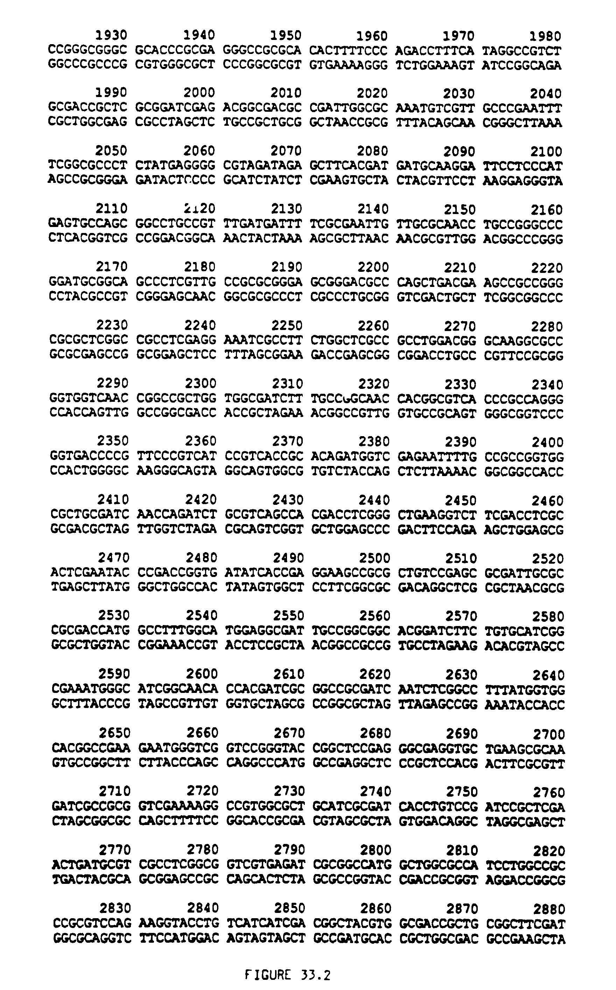

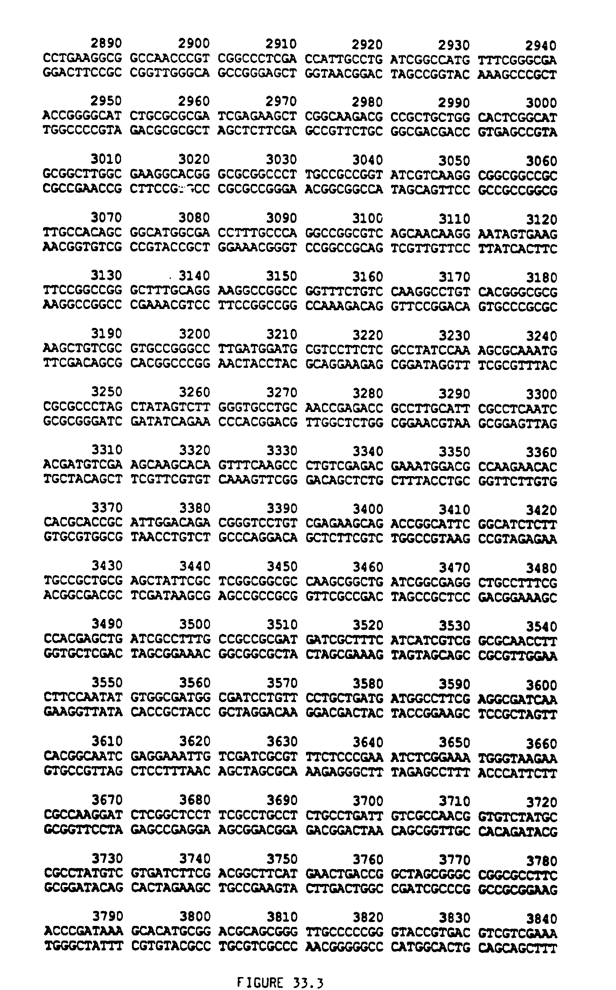

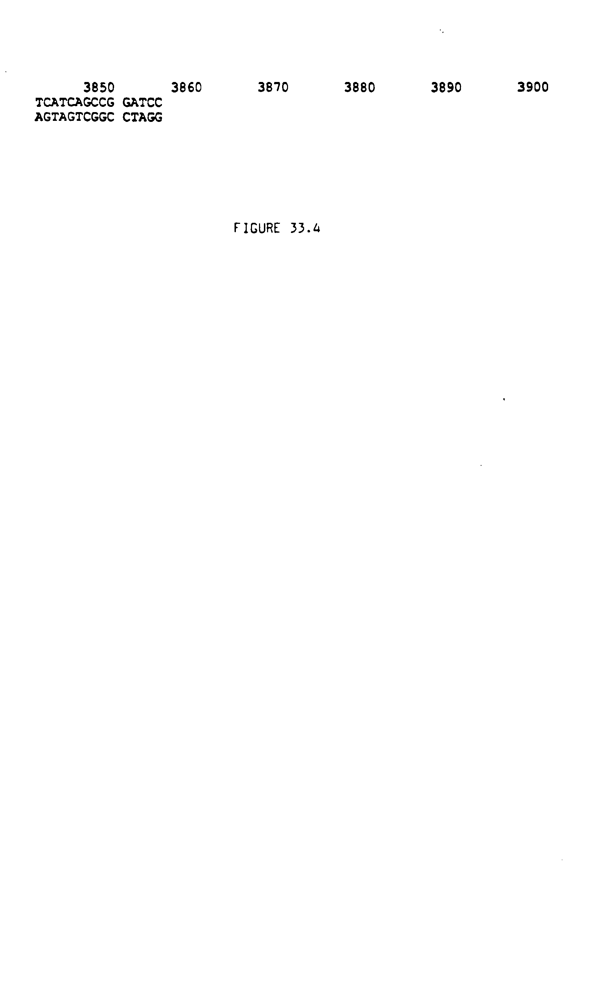

Ce fragment est inclus dans le plasmide pXL519 décrit dans l'exemple 1. Le protocole est identique à celui utilisé dans l'exemple 2.2. La séquence totale sur les deux brins du fragment de 3,9 kb est présentée sur la figure 33. Ce fragment contient 3855 pb.

### 2.5. Séquence nucléotidique d'un fragment EcoRI-BglII-EcoRI-BglII de 13.4 kb.

Ce fragment est contenu dans les plasmides pXL156 et pXL157 décrits dans l'exemple 1. Le protocole utilisé est identique à celui de l'exemple 2.2. La séquence sur les deux brins du fragment de 13.15 kb est présentée sur la figure 43. Elle correspond à la séquence totale du fragment de 13.4 kb mis à part 250 pb, correspondant à un fragment EcoRI-SstI, se trouvant à l'extrémité gauche de celui-ci.

A partir de ces séquences nucléotidiques, des cartes de restriction ont été obtenues pour les enzymes qui coupent le moins fréquemment (figures 6 et 44). Le pourcentage en bases GC de l'ADN de Pseudomonas denitrificans SC150 est relativement élevé (65,5 %) et se traduit par des compressions sur les gels de séquence. Pour éviter ces problèmes deux solutions sont apportées :
i) l'utilisation du 7-déaza-dGTP au lieu du dGTP dans les réactions de séquence pour diminuer les structures secondaires qui se forment pendant l'électrophorèse dans le gel de séquence et
ii) le séquençage des deux brins.

### EXEMPLE 3 - Analyse de ces séquences nucléotidiques : détermination des phases ouvertes

Les séquences nucléotidiques des fragments ClaI-HindIII-HindIII-HindIII de 5,4 kb (figure 7), EcoRI-EcoRI de 8,7 kb (figure 8), SalI-SalI-SalI-SalI-SalI-BglI de 4,8 kb (figure 32), SstI-SstI-BamHI de 3,9 kb (figure 33) et EcoRI-BglII-EcoRI-BglII de 13,4 kb (figure 43) permettent de définir des phases ouvertes. Comme il s'agit d'ADN à haut pourcentage en GC, les phases ouvertes sont nombreuses vu la faible fréquence de codons d'arrêt de traduction. Une étude de la probabilité des phases codantes d'après usage des codons en utilisant la méthode de Staden et MacLachlan (1982) est réalisée. Elle caractérise les phases ouvertes qui ont la meilleure probabilité d'être codantes par rapport aux autres phases du même brin d'ADN, cette probabilité est fonction de l'usage des codons de gènes déjà séquencés provenant des bactéries du genre Pseudomonas. De cette manière :
3.1. Cinq phases ouvertes sont caractérisées pour le fragment ClaI-HindIII-HindIII-HindIII de 5,4 kb. Elles sont nommées phases 1 à 5 et leurs positions sur la séquence du fragment de 5,4 kb sont les suivantes (sur la séquence 5'→3' du site ClaI vers les sites HindIII):

**Tableau :**

| Phases ouvertes probables du fragment ClaI-HindIII-HindIII-HindIII de 5,4 kb. Les positions sur la séquence correspondent aux positions sur la séquence décrite à la figure 7 ; le brin codant est le brin 5'→3' correspondant au brin supérieur sur cette figure. | | | |
|---|---|---|---|
| Numéro de la phase | Codon de démarrage de la traduction | Codon stop | Poids moléculaire en kD de la protéine codée |
| 1 | 549 | 1011 | 15.5 |
| 2 | 1141 | 1981 | 29.2 |
| 3 | 1980 | 3282 | 45.7 |
| 4 | 3281 | 4280 | 35.0 |
| 5 | 4284 | 5253 | 34.1 |

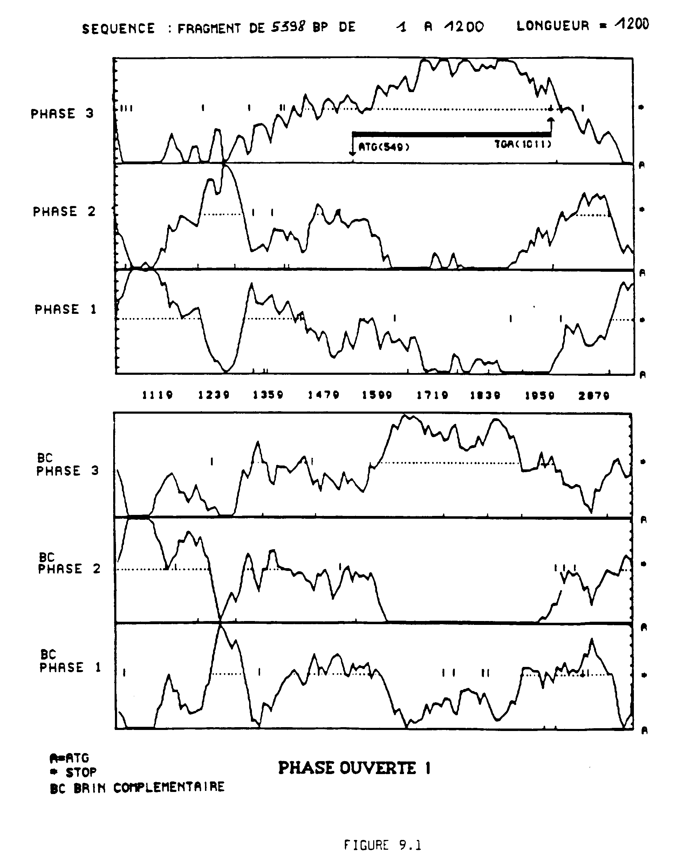

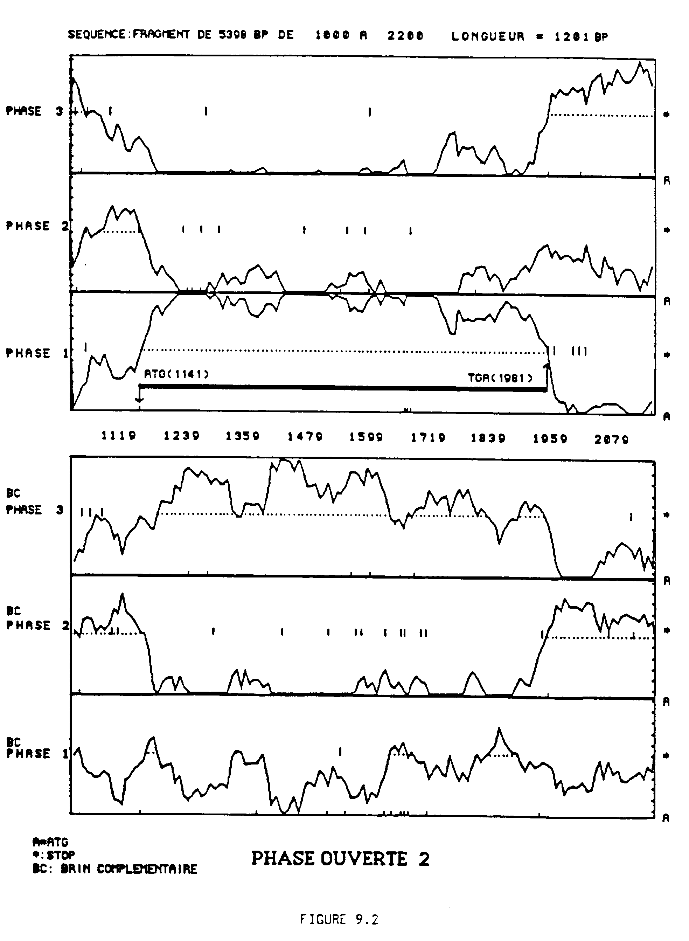

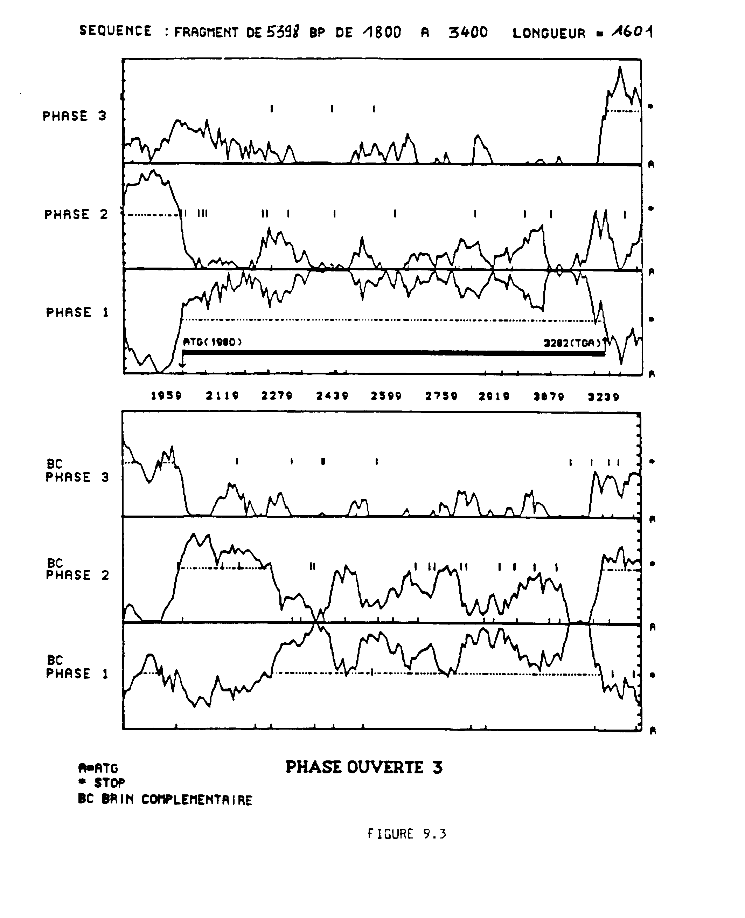

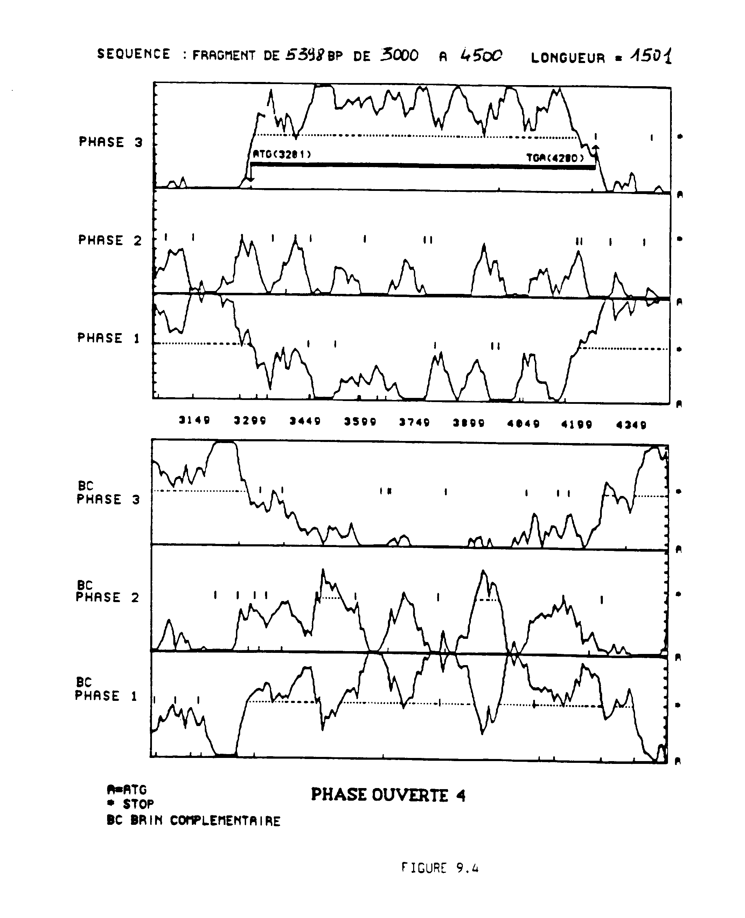

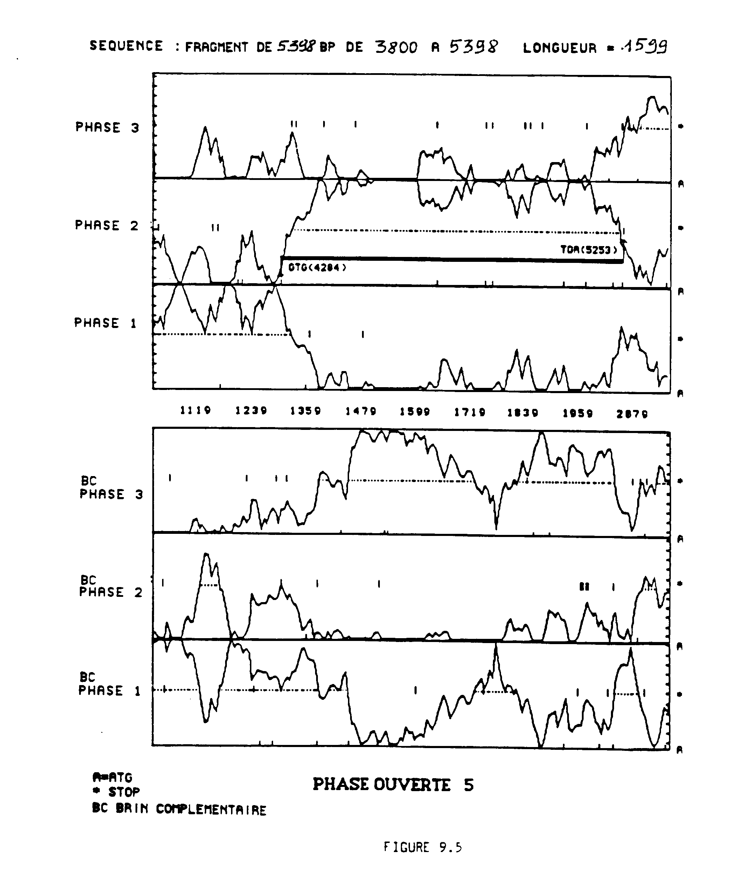

Les représentations des probabilités que ces phases ouvertes soient des phases codantes, avec parallèlement celles observées sur les autres phases (5 au total) sont portées sur la figure 9. Ces cinq phases sont codées par le même brin. Quatre d'entre elles (phases ouvertes 1 à 4) présentent les caractéristiques de phases codantes en couplage traductionnel (Normak et al., 1983), à savoir que le codon d'initiation de traduction de la phase x+1 chevauche le codon de terminaison traduction de la phase x ou bien que ceux-ci sont très proches.
**3.2.** Huit phases sont caractérisées pour le fragment EcoRI-EcoRI de 8,7 kb. Elles sont nommées phases 6 à 13 et leurs positions sur la séquence du fragment de 8,7 kb sont portées sur le tableau ci-dessous.

**Tableau :**

| Phases ouvertes probables du fragment EcoRI 8,7 kb. Lespositions sur la séquence correspondent aux positions sur la séquence décrite sur la figure 8 ; sur cette figure, le brin codant est le brin supérieur sauf pour la phase 11. | | | |
|---|---|---|---|
| Numéro de la phase de traduction | Codon de démarrage | Codon stop | Poids moléculaire en kD de la protéine codée |
| 6 | 736 | 1519 | 28.9 |
| 7 | 1620 | 2997 | 46.7 |
| 8 | 3002 | 3632 | 22.0 |
| 9 | 3631 | 4366 | 25.8 |
| 10 | 4365 | 5127 | 27.1 |
| 11 | 5893 | 5110 | 28.0 |
| 12 | 5862 | 7101 | 42.9 |
| 13 | 7172 | 7931 | 26.8 |

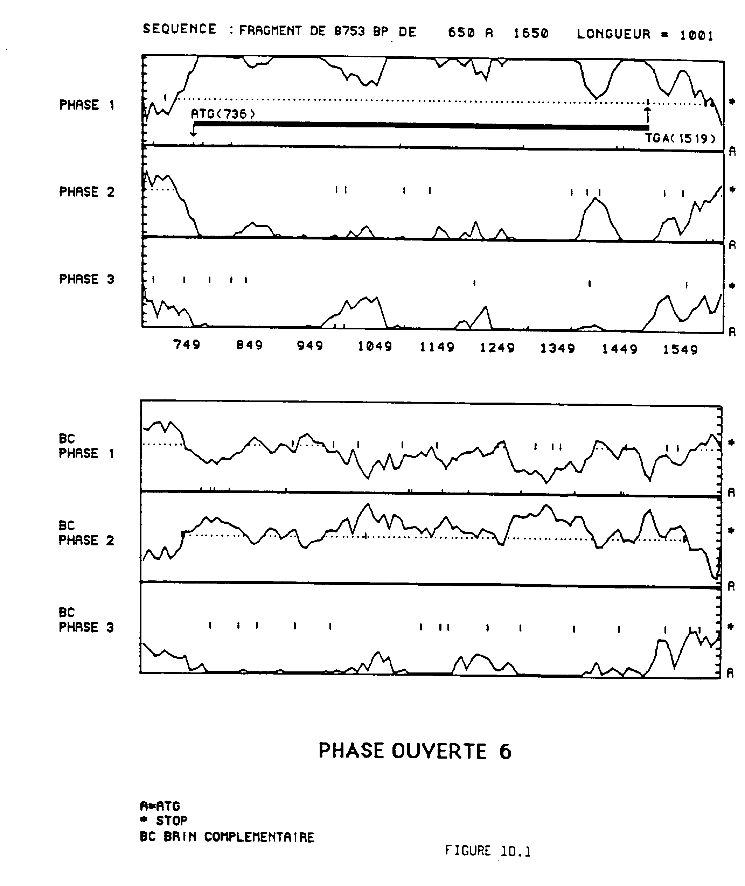

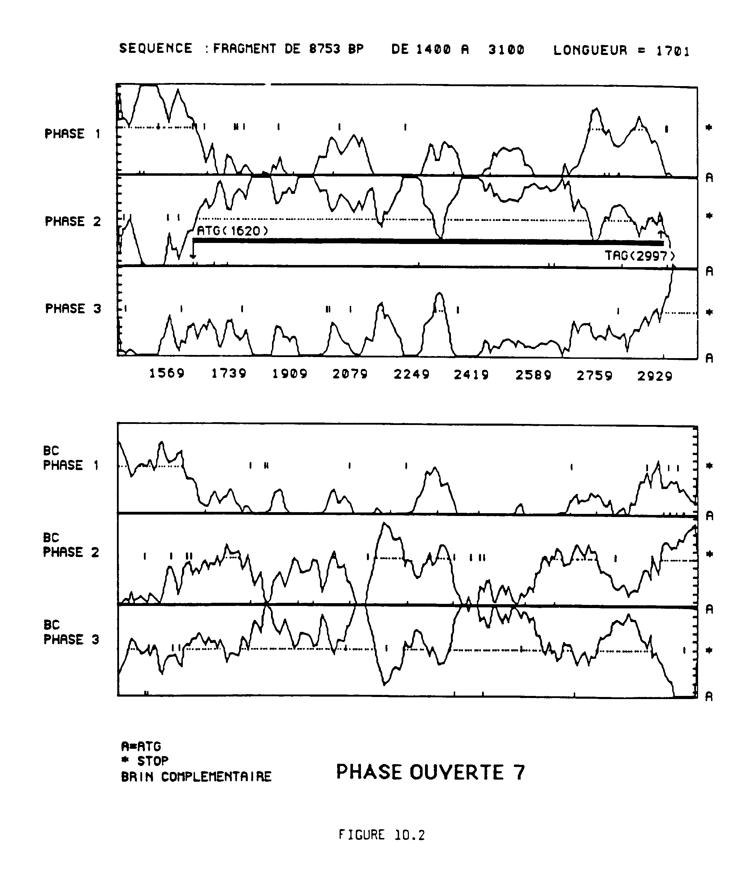

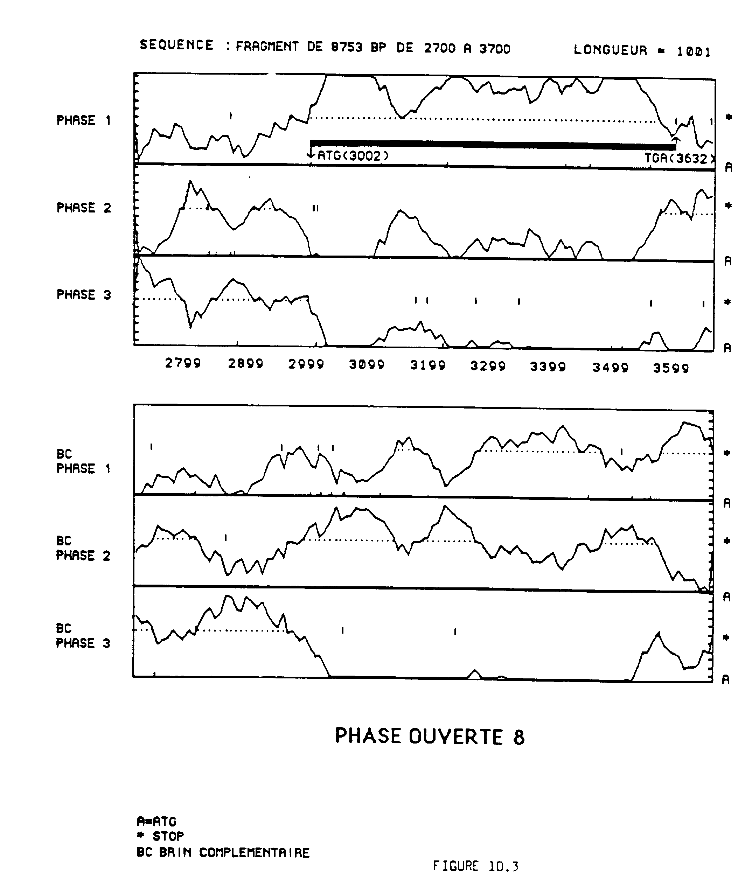

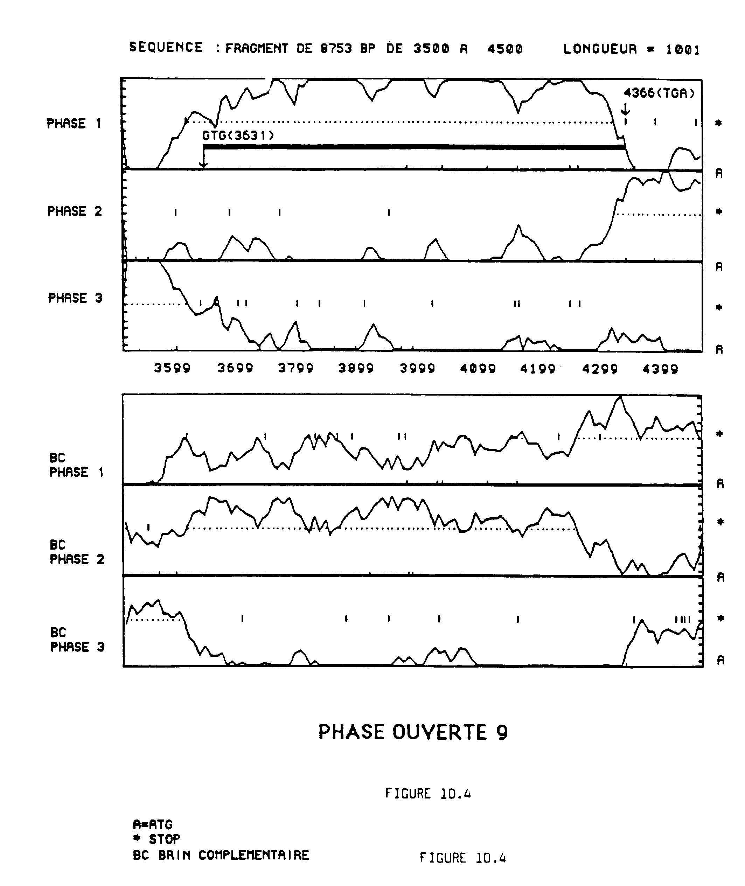

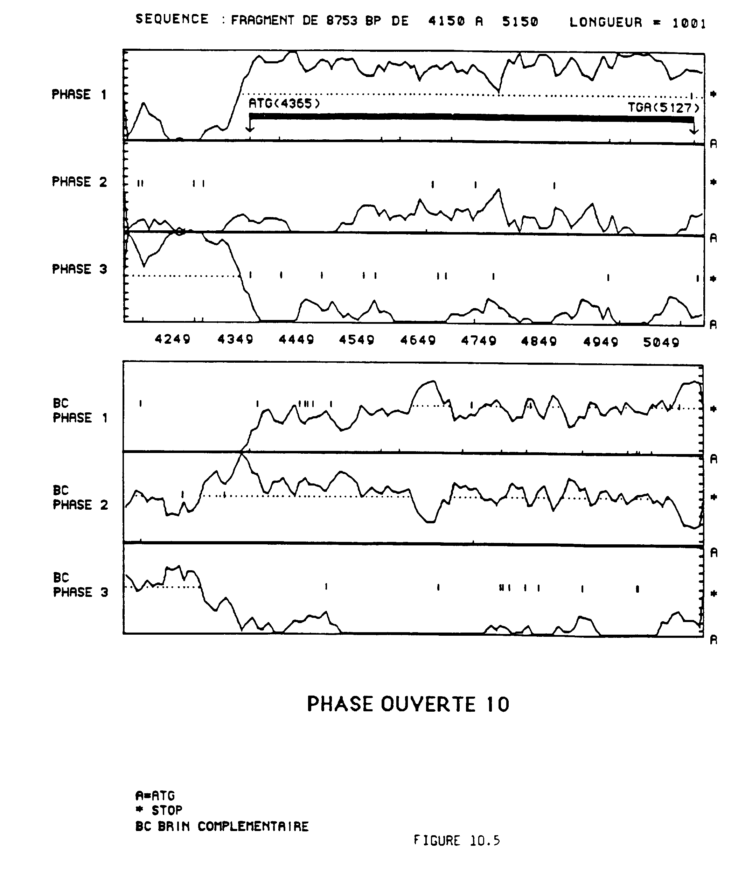

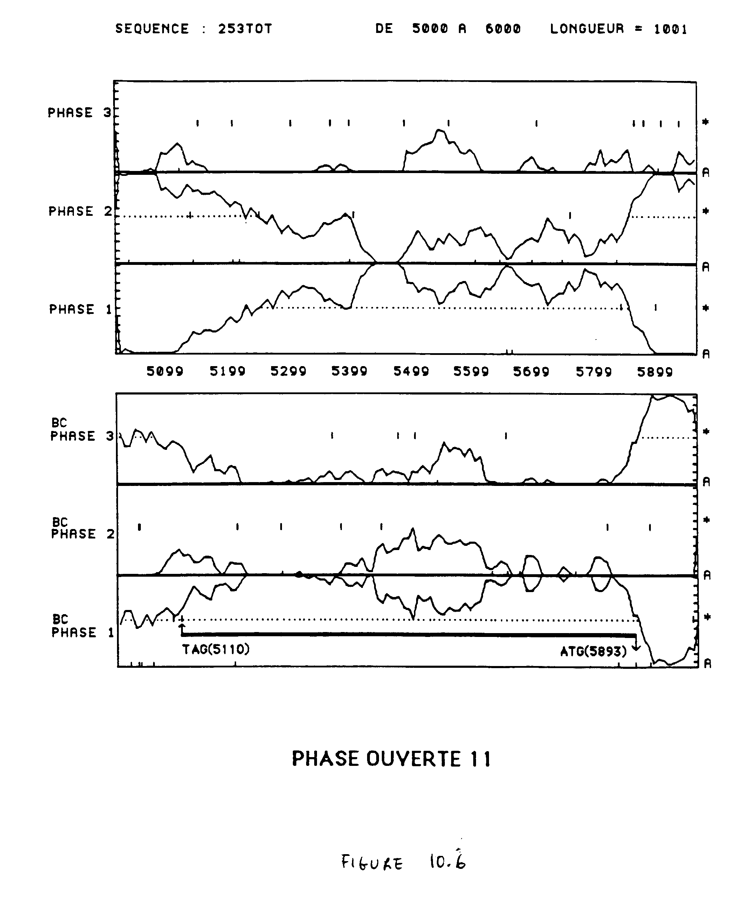

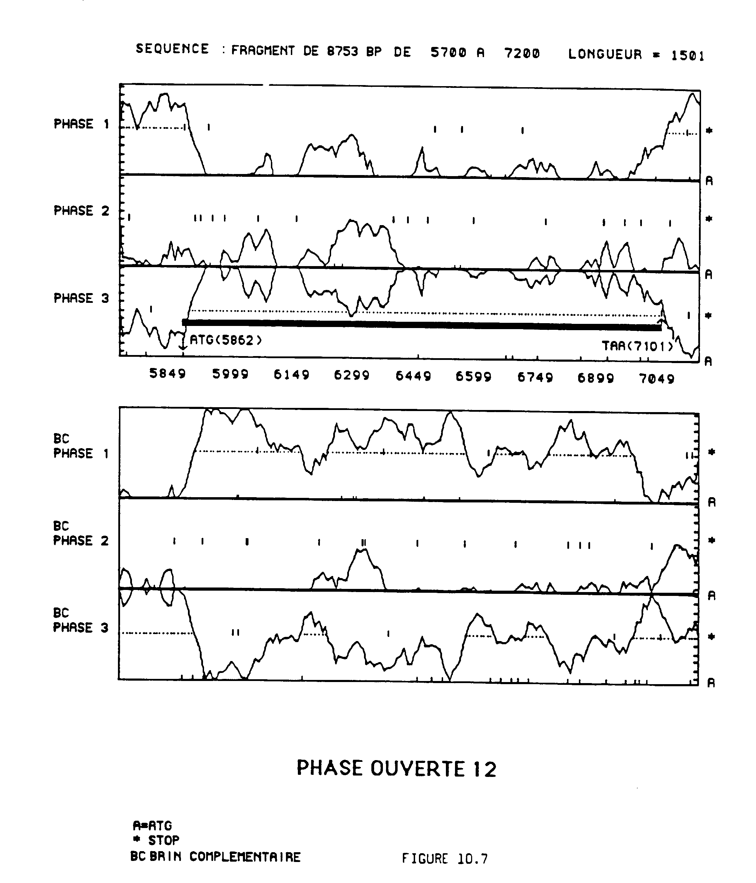

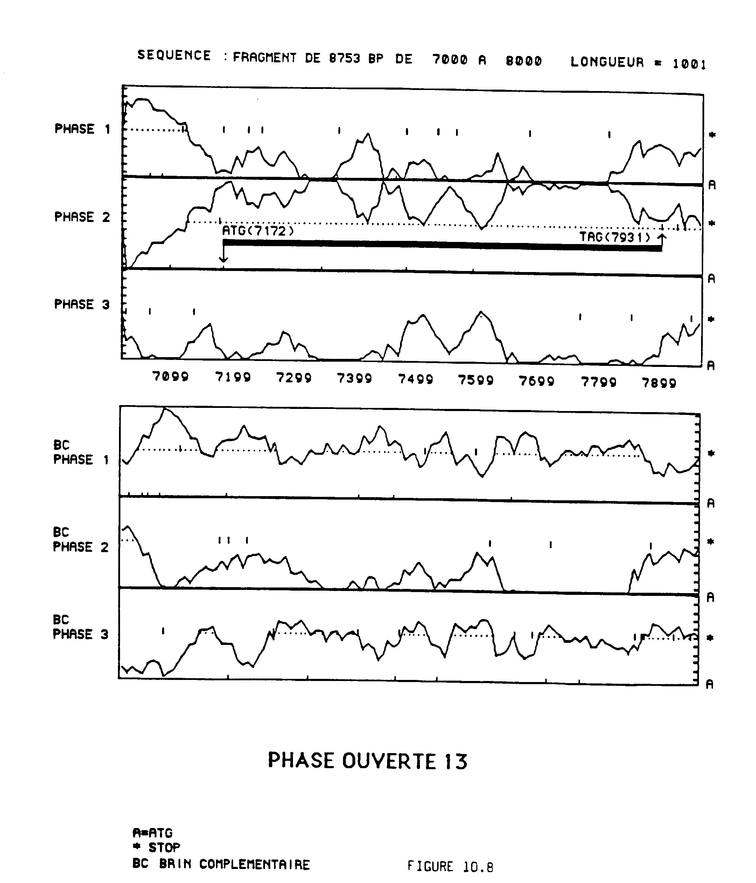

Les représentations des probabilités de ces phases ouvertes, avec parallèlement celles observées sur les autres phases (6 phases au total) sont portées sur la figure 10. A l'exception de la phase 11, ces phases sont codées par le même brin. Quatre d'entre elles (de 7 à 10) présentent les caractéristiques de phases codantes en couplage traductionnel (Normark et al., 1983), à savoir que le codon d'initiation de traduction de la phase x+1 chevauche le codon de terminaison de traduction de la phase x ou bien que ceux-ci sont très proches.
**3.3.** Quatre phases ouvertes sont caractérisées pour le fragment SalI-SalI-SalI-SalI-SalI-BglI de 4,8 kb. Elles sont nommées phases 14 à 17 et leurs positions sur la séquence du fragment de 4,8 kb sont les suivantes (sur la séquence 5'- 3' des sites SalI vers le site BglI):

**Tableau:**

| Phases ouvertes probables du fragment SalI-SalI-SalI-SalI-SalI-BglI de 4,8 kb. Les positions sur la séquence correspondent aux positions décrites sur la figure 32 où le brin supérieur est donné dans son orientation 5'- 3'. Les phases 15, 16 et 17 sont codées par le brin supérieur au contraire de la phase 14 | | | |
|---|---|---|---|
| Numéro de la phase | Codon de phase démarrage de traduction | Codon stop | Poids moléculaire en D de la protéine codée |
| 14 | 660 | 379 | 10286 |
| 15 | 925 | 1440 | 18941 |
| 16 | 1512 | 2510 | 36983 |
| 17 | 2616 | 4511 | 70335 |

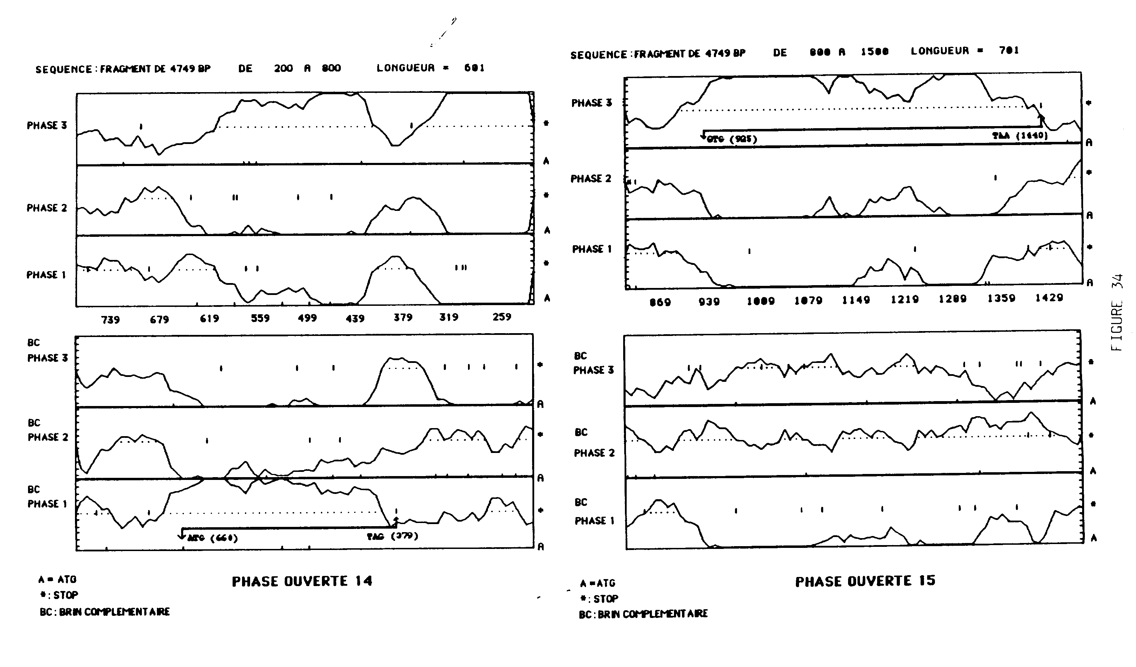

Les représentations des probabilités que ces phases ouvertes soient codantes, avec parallèlement celles observées sur les autres phases (au total 4) sont portées sur la figure 34. Les phases 15, 16 et 17 sont codées par le même brin, la phase 14 par le brin complémentaire.
**3.4.** Quatre phases sont caractérisées pour le fragment SstI-SstI-BamHI de 3,9 kb. Elles sont nommées 18 à 21 et leurs positions sur la séquence du fragment de 3,9 kb sont portées sur le tableau ci-dessous.

**Tableau:**

| Phases ouvertes probables du fragment SstI-SstI-BamHI de 3,9 kb. Les positions sur la séquence correspondent aux positions décrites sur la figure 33 où la polarité du brin supérieur est 5'- 3'. Les phases 18 et 19 sont codées par le brin inférieur au contraire des phases 20 et 21. | | | |
|---|---|---|---|
| Numéro de la phase | Codon de démarrage de traduction | Codon stop | Poids moléculaire en D de la protéine codée |
| 18 | 809 | 108 | 25148 |
| 19 | 1971 | 1063 | 30662 |
| 20 | 2099 | 3115 | 34682 |
| 21 | 3344 | 3757 | 14802 |

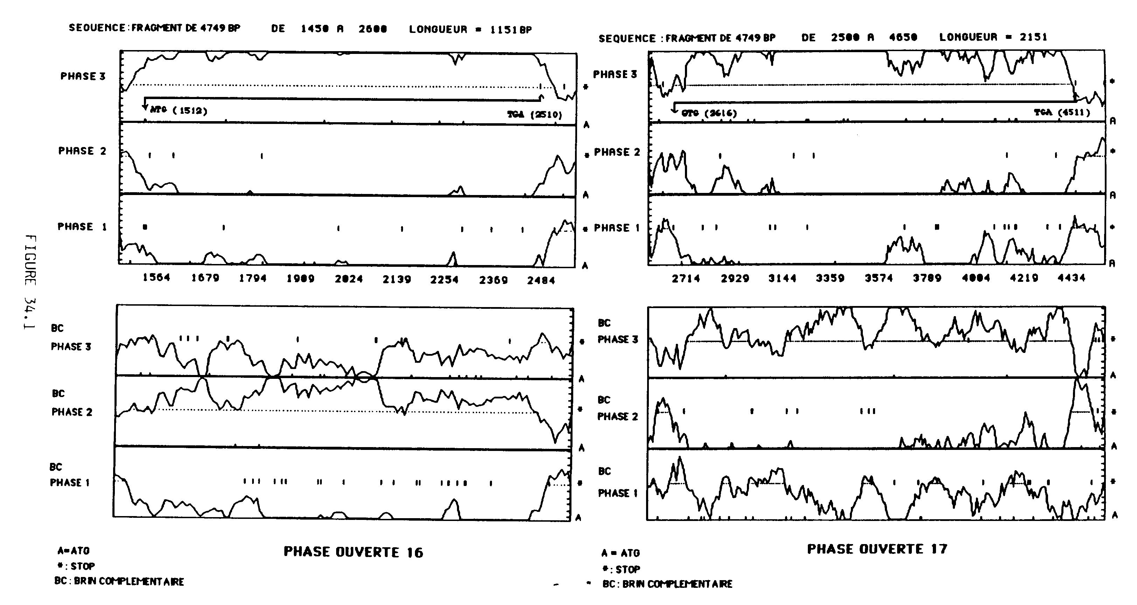

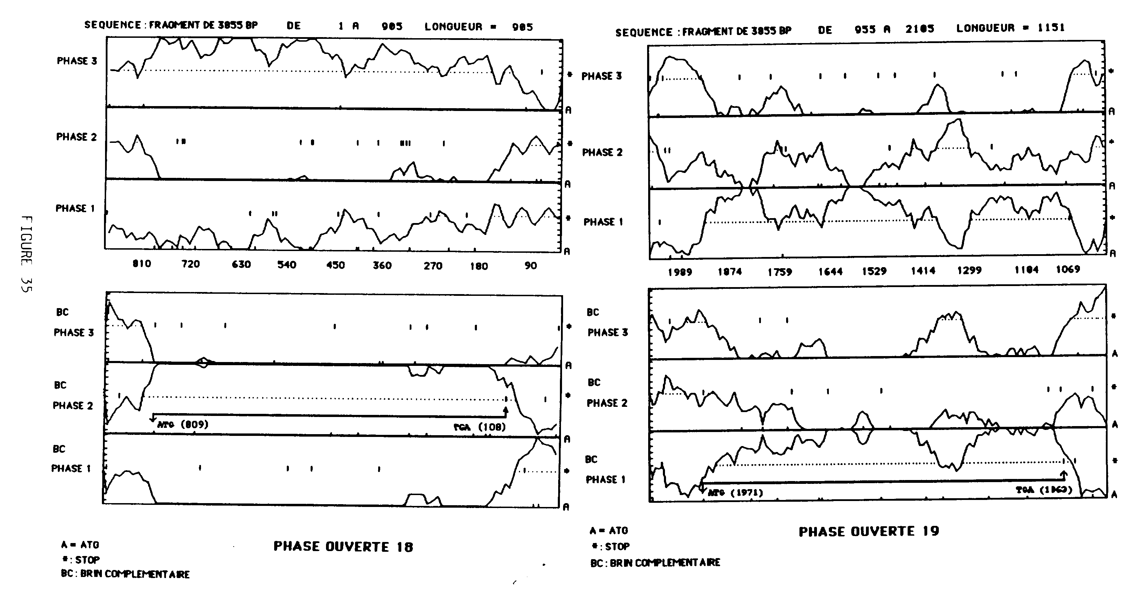

Les représentations des probabilités que ces phases ouvertes soient codantes, avec parallèlement celles observées sur les autres phases (au total 4) sont portées sur la figure 35. Les phases 19 et 20 sont transcrites de façon divergente.
**3.5.** Neuf phases ouvertes sont caractérisées pour le fragment EcoRI-BglII-EcoRI-BglII de 13.1 kb. Elles sont nommées phases 22 à 30 et leurs positions sur la séquence du fragment de 13,1 kb sont les suivantes (sur la séquence 5'- 3' du site EcoRI vers le site BglII):

**Tableau:**

| Phases ouvertes probables du fragment EcoRI-BglII-EcoRI-BglII de 13.1 kb. Les positions sur la séquence correspondent aux positions décrites sur la figure 43 où le brin supérieur est donné dans son orientation 5'- 3'. Les phases 22, 23, 24, 25, 26, 27 et 29 sont codées par le brin supérieur au contraire des phases 28 et 30. | | | |
|---|---|---|---|
| Numéro de la phase de traduction | Codon de démarrage | Codon stop | Poids moléculaire en D de la protéine codée |
| 22 | 429 | 1884 | 51 982 |
| 23 | 3364 | 3886 | 19442 |
| 24 | 3892 | 4954 | 38121 |
| 25 | 5060 | 8885 | 138 055 |
| 26 | 9034 | 9676 | 24 027 |
| 27 | 9678 | 10101 | 14 990 |
| 28 | 10835 | 10306 | 21 057 |
| 29 | 11656 | 12181 | 19 183 |
| 30 | 13059 | 12368 | 24 321 |

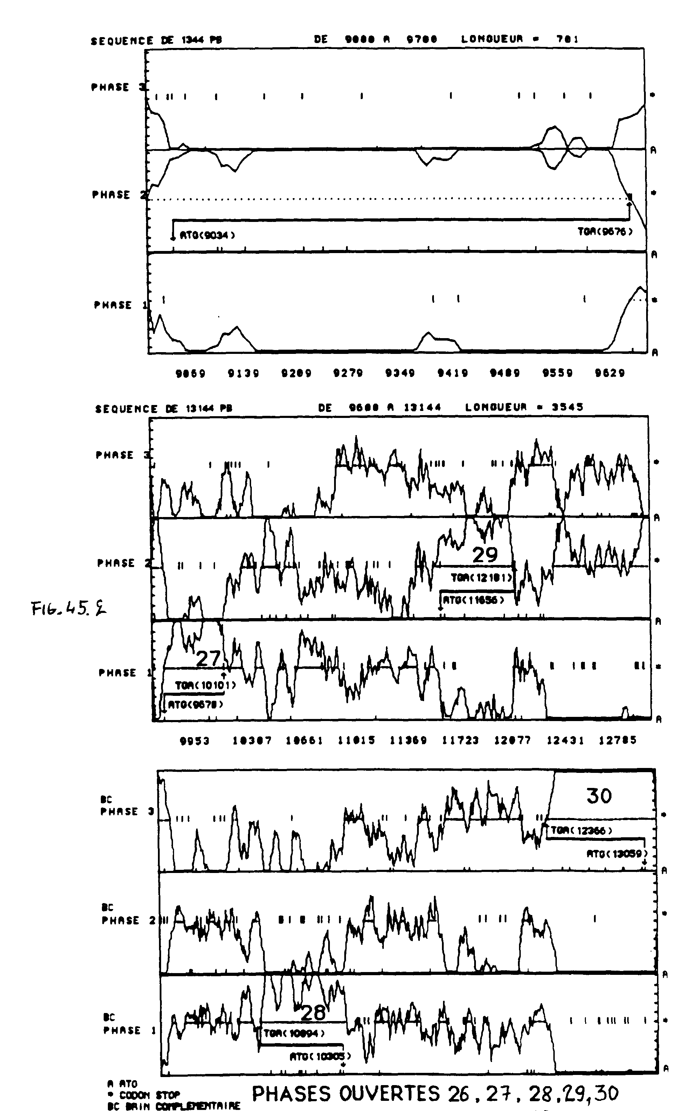

Les représentations des probabilités que les phases ouvertes 22, 23, 24, 25, et 26 soient codantes, avec parallèlement celles observées sur les autres phases (au total 5 phases) sont portées sur la figure 45. Ces 5 phases sont codées par le même brin.

### EXEMPLE 4 - Etudes génétiques sur les fragments d'ADN portant des gènes cob

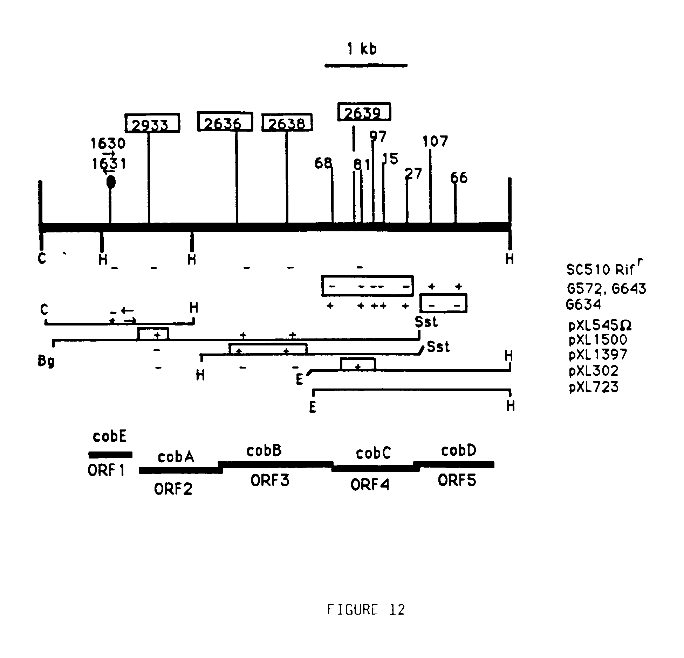

Cet exemple montre la relation qui existe entre les différentes phases ouvertes identifiées précédemment et les gènes impliqués dans la biosynthèse des cobalamines et/ou des cobamides portés par ces mêmes fragments. Ces gênes sont identifiés par une étude génétique comme décrit ci-dessous.
**4.1 - Etude génétique du fragment de 5,4 kb**
   Le plasmide pXL723 est le plasmide pRK290 (Ditta et al., 1980) contenant le fragment EcoRI-HindIII de 2264 pb, correspondant à la partie droite du fragment étudié, cloné au site EcoRI du pRK290 (figure 11). La construction des autres plasmides utilisés dans cette étude (pXL302, pXL1397, pXL545, pXL545Ω, pXL556 et pXL1500) est décrite dans la légende des figures 11 et 12.
   Des insertions ont été obtenues sur le plasmide pXL723 en utilisant la technique de Bruijn et Lupski, 1984. Les insertions du transposon Tn5 sur le plasmide pXL723 ont été sélectionnées, puis cartographiées dans le fragment de 5,4 kb (figure 12). Le pXL723 complémente le mutant Cob G572 de Pseudomonas putida et le mutant Cob G634 d'Agrobacterium tumefaciens. Ces insertions se classent en deux groupes d'insertions inactivationnelles: soit celles qui ne permettent plus de complémenter le mutant Cob G572, soit celles qui abolissent la complémentation du mutant Cob G634 (figure 12). Les insertions qui inactivent la complémentation du mutant G572 sont cartographiées dans la phase ouverte 4 (il s'agit des insertions 15, 27, 68, 81 et 97) ; la phase ouverte 4 correspond donc à un gène cob. Celui-ci est nommé cobC. Les insertions qui inactivent la complémentation du mutant G634 sont cartographiées dans la phase 5 (ce sont les insertions 66 et 107, figure 12); la phase ouverte 5 correspond donc à un gène cob. Celui-ci est nommé cobD. Par ailleurs des insertions avec un transposon Tn5Sp^{r} ont été réalisées. Le transposon Tn5Sp^{r} a été construit au laboratoire en clonant au site BamHI du transposon Tn5 (Jorgensen et al., 1979) une cassette BamHI contenant le gène de résistance à la spectinomycine provenant du plasmide pHP45Ω (Prentki et Krisch, 1984). Ces insertions ont été faites dans le chromosome de la souche de Pseunomonas denitrificans SBL27 Rif^{r}. La souche SBL27 est une souche de Pseudomonas denitrificans dont SC510 dérive par plusieurs mutagénèses. SBL27 produit 10 fois moins de cobalamines que SC510 sur milieu PS4. Sur 10000 clones de la souche SBL27 Rif^{r}, portant chacun une insertion du transposon, plus de 30 d'entre eux avaient perdu la capacité de synthétiser des cobalamines. Certains de ces clones possédaient une insertion dans le fragment étudié dans cet exemple. Ces insertions ont été cartographiées par analyses de restriction selon la méthode de Southern (Southern, 1975). Les sites d'insertions du transposon chez ces différents mutants sont portés sur la figure 12. Une de ces insertions numérotée 2639 se trouve dans le gène cobC; cette insertion est complémentée par le plasmide pXL302 qui porte un fragment contenant le gène cobC (figure 12). Deux insertions, nommées 2636 et 2638, sont dans la phase ouverte 3. Ces mutants sont bloqués dans la biosynthèse des cobalamines, et ils sont complémentés par le plasmide pXL1397 qui ne contient que la phase ouverte 3, mais non complémentés par le plasmide pXL302 qui contient les gènes cobC et cobD (figure 12). Ces deux insertions sont donc dans un autre gène. A la phase ouverte 3 nous associons le gène cobB. Une insertion 2933 est placée dans la phase ouverte 2 ; elle est complémentée par le plasmide pXL1500 qui contient la phase ouverte 2 ; cette insertion est non complémentée par le plasmide pXL1397 qui contient le gène cobB et qui complémente les deux insertions dans cobB. Il s'agit donc ici d'une insertion dans un autre gène; à la phase ouverte 2 nous associons un gène nommé cobA.
   Une cassette de résistance à la kanamycine provenant du plasmide pUC4K (Barany et al., 1985) a été introduite au site NotI du fragment ClaI (position 0 sur la séquence)-RsaI (position 1686 sur la séquence) cloné dans un plasmide pUC8 (Viera et Messing, 1982) ; il s'agit du site NotI situé à la position 771 dans la phase 1 (voir la séquence sur la figure 7); deux insertions ont été retenues correspondant chacune à une orientation différente de la cassette de résistance. Ces fragments portant chacun une insertion de la cassette de résistance ont été clonés dans le plasmide pRK404 (Ditta et al.) pour donner les plasmides pXL1630 et 1631. Ces plasmides ont été introduits par transfert conjugatif dans la souche de Pseudomonas denitrificans SC510 Rif^{r}, puis par une série de cultures-dilutions en absence de l'antibiotique sélectif pour le plasmide (la tétracycline), des doubles recombinants ayant échangé le fragment plasmidique avec le fragment chromosomique et ayant perdu le plasmide ont été trouvés. Deux souches ont ainsi été caractérisées :
   i) l'une est nommée SC510:1631 Rif^{r}, dans cette souche, la cassette de résistance à la kanamycine se trouve insérée, dans le chromosome au site NotI (se trouvant dans la phase 1); les polarités des transcriptions du gène de résistance à la kanamycine et celle de la phase ouverte 1 sont opposées,
   ii) l'autre insertion est nommée SC510:1630 Rif^{r}; la cassette de résistance est insérée au même site, mais la transcription du gène de résistance à la même polarité que celle de phase ouverte 1 entière.

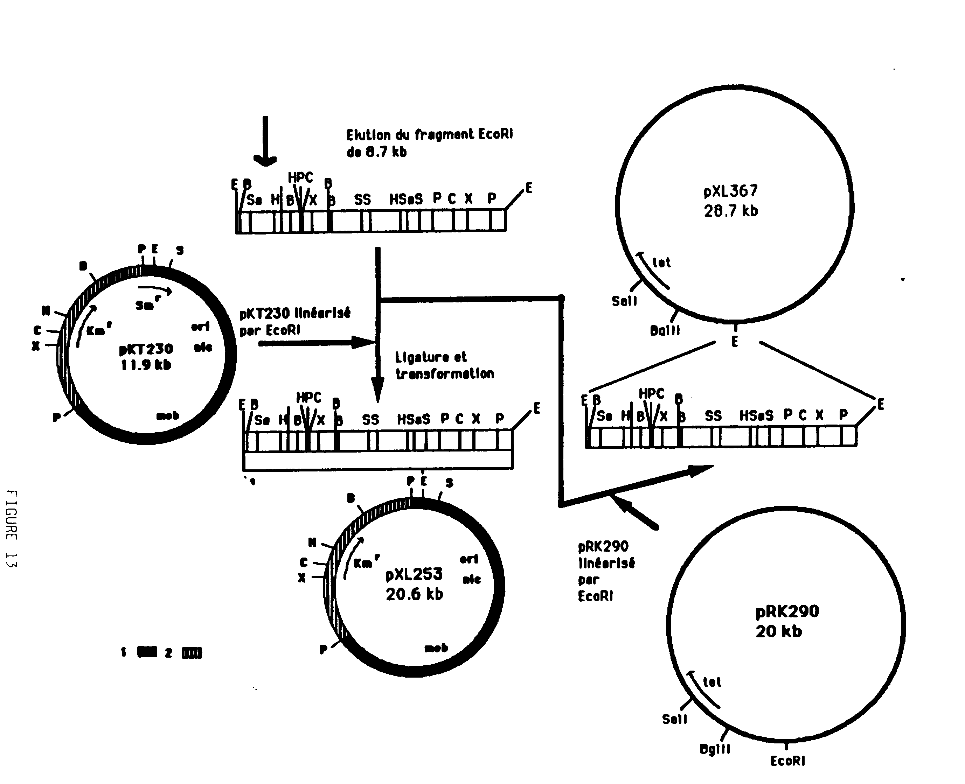

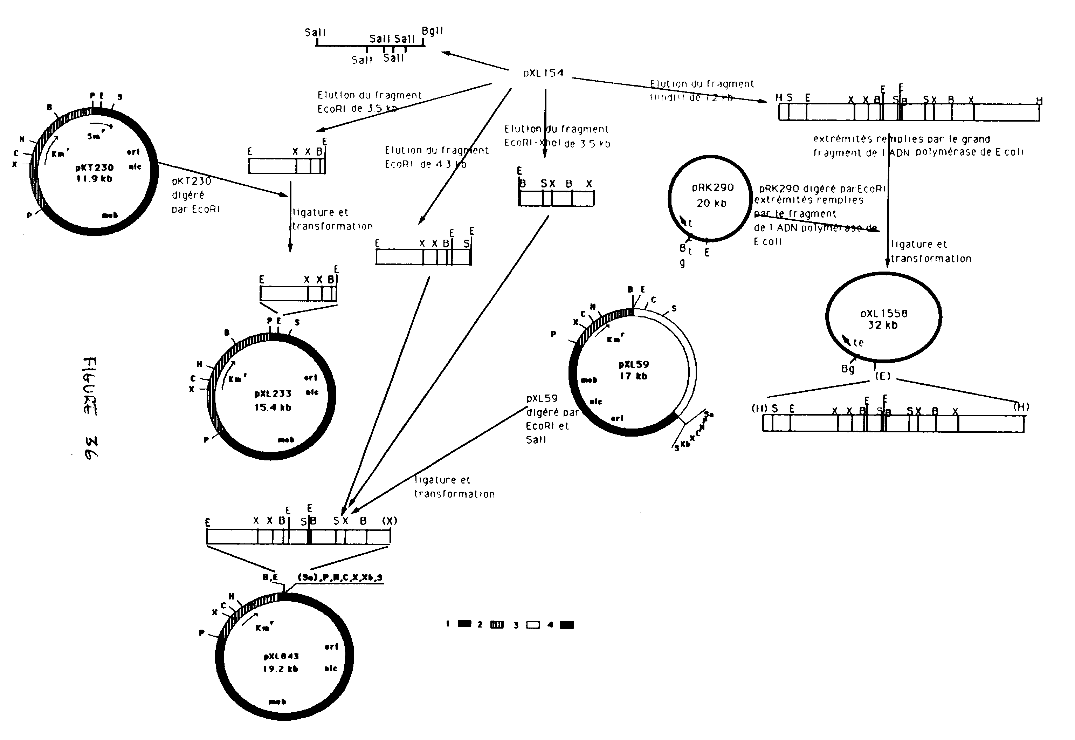

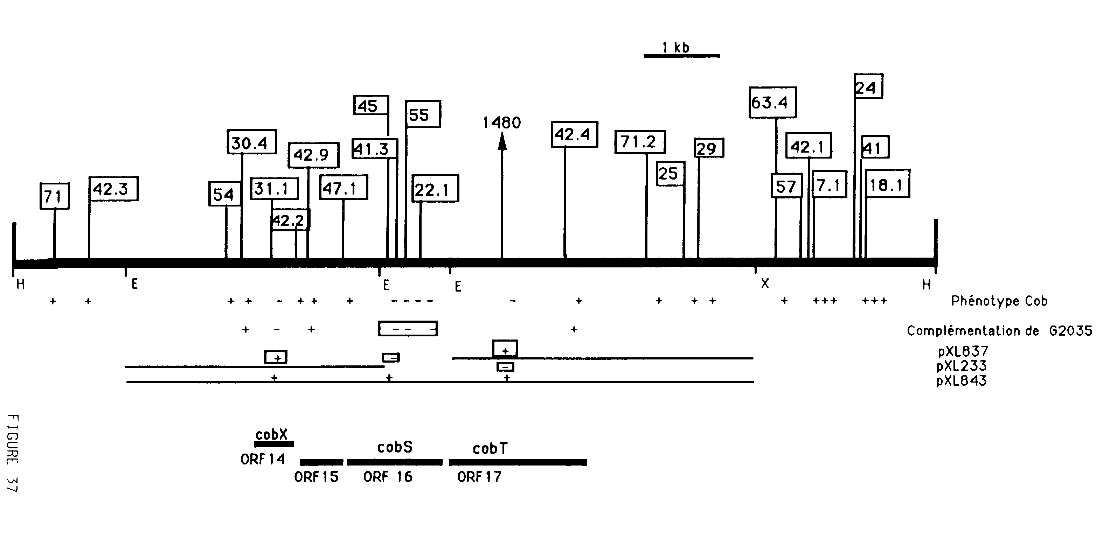

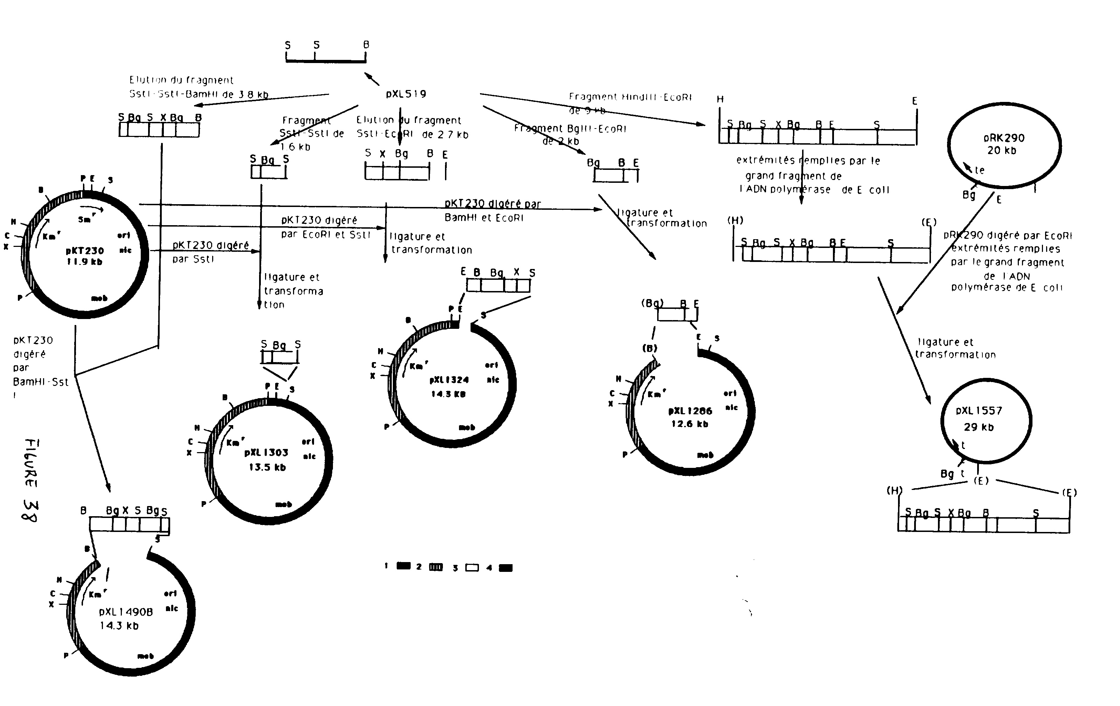

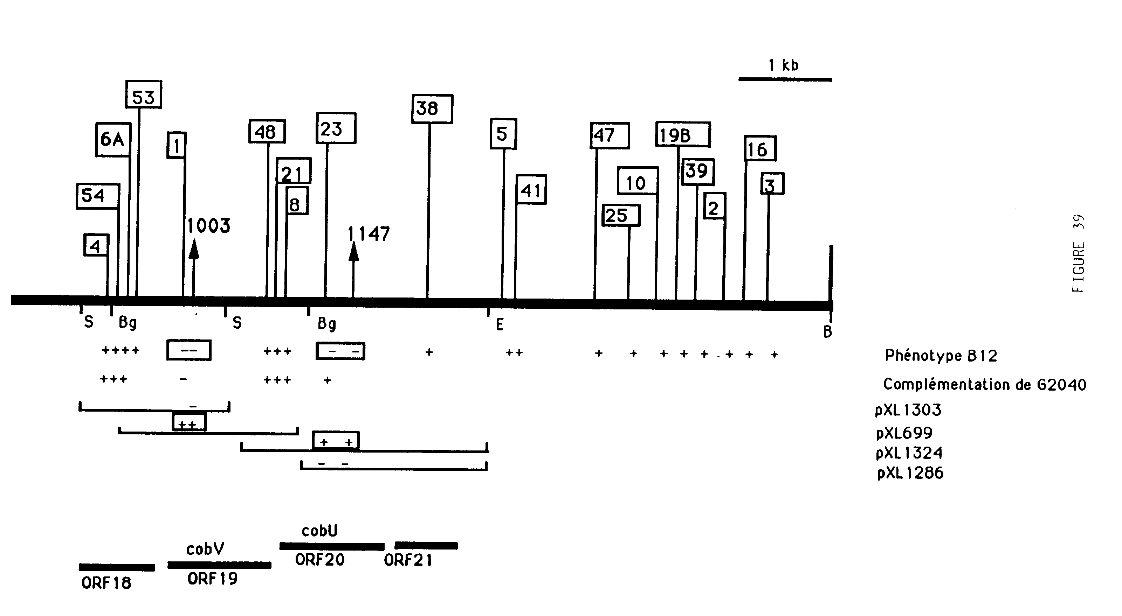

   Ces deux souches ont toutes les deux un taux de synthèse de cobalamines au moins 100 fois inférieur à celui de SC510. Le plasmide pXL545Ω correspond au plasmide pXL545 dans lequel la cassette de résistance à la spectinomycine, du plasmide pHP45Ω a été insérée au niveau du site BamHI. Ce plasmide (figure 12) qui contient le fragment ClaI-HindIII de 814 pb (où seule la phase ouverte 1 est entière) complémente seulement le mutant SC510:1630 Rif^{r}. Ceci suffit à définir un nouveau gène puisque ce mutant est complémenté par un plasmide qui contient uniquement la phase ouverte 1 entière. La phase ouverte 1 correspond à un gène de la voie de biosynthèse des cobalamines et/ou des cobamides. Ce gène est nommé cobE. L'absence de complémentation du mutant SC510:1631 Rif^{r} par le plasmide pXL545Ω est peut être due au fait que les gènes cobA, cobB, cobC, cobD et cobE, ou une partie d'entre eux, appartiennent au même opéron et que l'insertion dans cobE qui conserve une transcription dans le sens de transcription de l'opéron peut être complémentée seulement par l'expression en trans du gène cobE. Au contraire le mutant SC510:1631 Rif^{r} lui ne peut être complémenté que par un plasmide qui permet l'expression en trans des gènes cobA à cobE.
   Le fragment ClaI-HindIII-HindIII-HindIII de 5,4 kb contient donc cinq gènes cob nommés cobA, cobB, cobC, cobD et cobE.
**4.2 - Etudes génétiques du fragment de 8,7 kb**
   Le plasmide pXL367 est le pRK290 (Ditta et al., 1980) contenant le fragment EcoRI de 8,7 kb cloné au site EcoRI (figure 13).
   Des insertions du transposon Tn5 sur le plasmide pXL367 ont été sélectionnées en utilisant la technique déjà décrite (de Bruijn et Lupski, 1984). Les insertions dans le fragment de 8,7 kb ont été cartographiées. De la même manière des insertions du transposon Tn3lacZ ont été obtenues selon la méthode déjà décrite (Stachel et al., 1985) et cartographiées. 29 insertions du transposon Tn5 et 13 insertions du transposon Tn3lacZ ont ainsi été cartographiées. La position précise de ces insertions sur le fragment de 8,7 kb est portée à la figure 14. Des plasmides, portant chacun une seule insertion dans le fragment de 8,7 kb, ont été introduits par transferts conjugatifs dans les mutants Cob d'Agrobacterium tumefaciens G164, G609, G610, G611, G612, G613, G614, G615, G616, G620, G638. Ces mutants sont tous complémentés par le pXL367. Les insertions qui ne permettent plus la complémentation des différents mutants ont été recherchées. Elles correspondent à une insertion dans le gène responsable de la complémentation du mutant correspondant. Les résultats des complémentations des différents mutants pour leur caractère de production de cobalamines (phénotype Cob) sont portés sur la figure 14. Si le mutant recombiné produit moins de trois fois moins de cobalamines que ne produit le même mutant avec le plasmide pXL367, il est considéré comme étant non complémenté. Sur les mutants étudiés, G164, G609, G610, G611, G612, G613, G614, G615, G616, G620, G638, on observe huit classes différentes d'insertions inactivationnelles de transposons conduisant à un phénotype muté. Ces classes caractérisent des insertions par l'absence de complémentation d'un ou plusieurs mutants par les plasmides pXL367 portant ces mêmes insertions. Chaque classe correspond donc à un gène muté. On observe que les insertions appartenant à une même classe sont positionnées les unes à côté des autres. Huit classes d'insertions sont ainsi observées qui permettent de définir huit gènes. Chaque classe d'insertions définit un fragment minimum qui doit être contenu dans le gène correspondant. La figure 14 démontre un parfait recoupement entre les régions délimitées par chaque classe, au niveau de la carte de restriction, et les phases ouvertes décrites ci-dessus (exemple 3). On constate en effet que, pour chaque classe d'insertions, les transposons sont toujours insérés dans une partie du fragment de 8,7 kb qui est contenue dans une seule phase ouverte. A chaque classe d'insertions, est donc associée une phase ouverte et une seule. Les phases ouvertes indiquées ci-dessus codent donc chacune pour une protéine impliquée dans la voie de biosynthèse des cobalamines et/ou des cobamides. Les phases ouvertes correspondent chacune à des gènes impliqués dans la biosynthèse des cobalamines et/ou des cobamides. Ces phases ouvertes sont appelées cobF, cobG, cobH, cobI, cobJ, cobK, cobL et cobM pour les phases 6 à 13 respectivement. La position de ces gènes par rapport à la carte de restriction est représentée sur la figure 14.
**4.3 - Etude génétique du fragment de 4,8 kb.**
   Le plasmide pXL1558 est le plasmide pRK290 (Ditta et al., 1980) contenant le fragment HindIII-HindIII de 12 kb du pXL154 (Cameron et al., 1989) cloné au site EcoRI du pRK290 (figure 36). La construction des autres plasmides utilisés dans cette étude (pXL233 et pXL843) est décrite dans la légende de la figure 36.
   Des insertions Tn5Sp ont été obtenues sur le plasmide pXL1558. Tout d'abord une souche contenant un transposon Tn5Sp a été construite; ceci a été fait en transformant la souche C2110 (Stachel et al., 1985) à l'aide du plasmide pRK2013Tn5Sp (Blanche et al., 1989); le plasmide pRK2013Tn5Sp ayant une origine de réplication ColE1 ne se réplique pas dans la souche C2110 qui est polA-. Les colonies obtenues après transformation qui sont résistantes à la spectinomycine ont donc le transposon Tn5Sp dans leur chromosome; une colonie est ensuite réisolée, puis l'insertion du transposon est ensuite transduite à l'aide du phage P1 chez la souche MC1060 comme cela est décrit précédemment (Miller, 1972). La souche MC1060 Tn5Sp est transformée par le plasmide pXL1558; le plasmide pXL1558 est ensuite mobilisé par conjugaison à l'aide du pRK2013 chez C600 Rifr. Des conjugants résistants à la tétracycline (pour le plasmide pXL1558) et à la spectinomycine (pour le transposon) sont ensuite sélectionnés; de tels conjugants doivent contenir le plasmide pXL1558 dans lequel le transposon Tn5Sp s'est inséré. Des insertions portées sur le plasmide pXL1558, et plus précisément dans le fragment de 12 kb sont ensuite cartographiées par digestion de restriction; 23 insertions sont ainsi obtenues et cartographiées sur le fragment de 12 kb; la position de ces différentes insertions sur le fragment est présentée sur la figure 37. Ces 23 insertions ont été introduites sur le chromosome de la souche SC510 Rifr après transfert conjugatif du p-XL1558::Tn5Sp, puis introduction du plasmide pR751. Le plasmide pR751 est un plasmide, résistant à la triméthoprime, du même groupe d'incompatibilité que le pXL1558 (incP, Thomas et Smith, 1987). Par culture non sélective pour le pXL1558 (absence de tétracycline) mais sélective pour le pR751 et le transposon (présence de triméthoprime et de spectinomycine) l'échange de la mutation portée par le pXL1558::Tn5Sp avec le chromosome ainsi que la ségrégation du pXL1558 sont obtenus par la technique d'échange de marqueur par double recombinaison homologue comme cela est déjà décrit (Schell et al., 1988). Les souches ainsi sélectionnées portent le transposon dans leur chromosome. La double recombinaison homologue est vérifiée par la méthode de Southern (Southern, 1975). De cette manière 23 souches SC510 Rifr::Tn5Sp dans le fragment de 12 kb ont été identifiées.
   D'autre part une autre insertion Tn5Sp obtenue par mutagénèse au hasard du transposon Tn5Sp dans la souche SBL27 Rifr (Blanche et al., 1989) a été cartographiée sur le fragment de 12 kb par analyse de restriction selon la méthode de Southern (Southern, 1975), voir figure 37; cette souche est nommée SBL27 Rifr::Tn5Sp 1480.
   Le taux de synthèse de cobalamines est déterminé pour ces 24 souches cultivées en milieu PS4 selon le protocole déjà décrit (Cameron et al., 1989) et le phénotype Cob- est attribué aux souches produisant au moins 1000 fois moins (resp. 100) de vitamine B12 que la souche parente SC510 Rifr (resp.SBL27 Rifr), figure 37. Il est ainsi observé que 6 de ces insertions chromosomiques conduisent à un phénotype CobD chez P. denitrificans; il s'agit des insertions 31.1, 41.3, 45, 55, 22.1 et 1480.
   Trois plasmides pXL233, pXL837 (Cameron et al.) et pXL843 sont introduits par transferts conjugatifs dans trois souches présentant le phénotype Cob- soient SC510 Rifr::Tn5Sp 31.1,SC510 Rifr::Tn5Sp 45, SBL27 Rifr::Tn5Sp 1480. Ces trois mutants ont chacun un profil de complémentation différent pour la synthèse des cobalamines. En effet SBL27 Rifr::Tn5Sp 1480 est complémenté par le pXL837 et le pXL843 mais pas par le pXL233; le mutant SC510 Rifr::Tn5Sp 45 n'est complémenté que par le pXL843; le mutant SC510 Rifr::Tn5Sp 31.1 est complémenté par Le plasmide pXL843 ainsi que par le plasmide pXL233 (voir figure 37). Les données présentées permettent donc, d'après les résultats sur les complémentations des trois mutants, de conclure que les trois mutants sont différents et que pour chacun d'entre eux, le transposon Tn5Sp s'est inséré dans un gène cob différent.
   D'autre part les plasmides pXL1558::Tn5Sp 41.3, pXL1558::Tn5Sp 45, et pXL1558::Tn5Sp 22.1 sont introduits par transferts conjugatifs dans la souche G2035 (Cameron et al., 1989) et ne la complémentent pas. Le plasmide pXL1558 complémente ce mutant au contraire du plasmide pXL1558::Tn5Sp 31.1.
   Les données de phénotype et complémentation nous permettent de définir 3 classes d'insertions; chacune de ces classes est représentée par les insertions suivantes : 31.1, classe 1; 45, 41.3, 55 et 22.1, classe 2; 1480 classe 3.
   Pour chaque classe d'insertions, les transposons sont toujours insérés dans une partie du fragment de 4,8 kb qui est contenue dans une seule phase ouverte (ORF14, ORF16 et ORF17, comme définies à l'exemple 3). A chaque classe d'insertions est associée une seule phase ouverte. Les phases ouvertes indiquées ci-dessus codent donc pour une protéine impliquée dans la voie de biosynthèse des cobalamines et/ou des cobinamides. Ces phases ouvertes sont appelées cobX, cobS et cobT pour les phases 14, 16 et 17. La position de ces gènes par rapport à la carte de restriction est représentée sur la figure 37. La phase ouverte 15 n'est pas un gène impliqué dans la biosynthèse du coenzyme B12.
**4.4 - Etudes génétiques du fragment de 3,9 kb**
   Le plasmide pXL1557 est le plasmide pRK290 (Ditta et al., 1980) contenant le fragment HindIII-BamHI de 9 kb du pXL519 cloné au site EcoRI du pRK290 (figure 38). La construction des autres plasmides utilisés dans cette étude (pXL1286, pXL1303, pXL1324) est décrite dans la légende de la figure 38. Par ailleurs, le fragment BglII-XhoI de 2 kb (positions sur la séquence présentée figure 33 251 et 2234) du plasmide pXL519 est cloné aux sites BamHI-SalI du plasmide pXL435 (Cameron et al) pour générer le plasmide pXL699.
   Des insertions Tn5Sp ont été obtenues sur le plasmide pXL1557 selon la technique décrite dans l'exemple 4.3. Les insertions du transposon Tn5Sp sur le plasmide pXL1557, alors nommées pXL1557::Tn5Sp, ont été sélectionnées. Celles qui sont cartographiées dans le fragment de 9 kb (figure 39) ont été introduites sur le chromosome de la souche SC510 Rifr après transfert conjugatif du pXL1557::Tn5Sp et échange de marqueur par double recombinaison homologue comme cela est décrit en 4.3.
   La double recombinaison homologue est vérifiée par la méthode de Southern (Southern, 1975). De cette manière 20 souches SC510 Rifr::Tn5Sp ont été identifiées.
   D'autre part deux autres insertions Tn5Sp obtenues par mutagénèse au hasard du transposon Tn5Sp dans la souche SBL27 Rifr (Blanche et al., 1989) ont été cartographiées sur le fragment de 9 kb par analyse de restriction selon la méthode de Southern (Southern, 1975), voir sur la figure 39 les insertions 1003 et 1147.
   Le taux de synthèse de cobalamines est déterminé pour ces 22 souches cultivées en milieu PS4 selon le protocole déjà décrit (Cameron et al., 1989) et le phénotype Cob- est attribué aux souches produisant 1000 fois moins (resp. 100) de vitamine B12 que la souche parente SC510 Rifr (resp.SBL27 Rifr), figure 39. Seules les 4 insertions 1, 1003, 23 et 1147 se traduisent par un phénotype Cob- chez P. denitrificans.
   Quatre plasmides pXL699, pXL1286, pXL1303 et pXL1324 sont introduits par transferts conjugatifs dans les quatre souches présentant le phénotype Cob- soient SC510 Rifr::Tn5Sp 1, SBL27 Rifr::Tn5Sp 1003, SC510 Rifr::Tn5Sp 23 et SBL27 Rifr::Tn5Sp 1147. Le plasmide pXL699 complémente les deux premiers mutants (SC510 Rifr::Tn5Sp 1, SBL27 Rifr::Tn5Sp 1003) mais le plasmide pXL1303 ne les complémente pas; le plasmide pXL1324 complémente les deux autres mutants (SC510 Rifr::Tn5Sp 23 et SBL27 Rifr::Tn5Sp 1147) mais le plasmide pXL1286 ne -les complémente pas.
   D'autre part le plasmide pXL1557::Tn5Sp 1, est introduit par transfert conjuguatif dans la souche G2040 et ne la complémente pas alors que les plasmides pXL1557, pXL1557::Tn5Sp 6A, pXL1557::Tn5Sp 54, pXL1557::Tn5Sp 48, pXL1557::Tn5Sp 21, pXL1557::Tn5Sp 8, pXL1557::Tn5Sp 23 aussi introduits par transferts conjugatifs, la complémentent (voir figure 39).
   Les données de phénotype et complémentation permettent de définir 2 classes d'insertions. Pour chaque classe d'insertions, les transposons sont toujours insérés dans une partie du fragment de 3,9 kb qui est contenue dans une seule phase ouverte (ORF 19 et 0RF20 comme définie à l'exemple 3).
   A chaque classe d'insertions est associée une seule phase ouverte. Les phases ouvertes indiquées ci-dessus codent pour une protéine impliquée dans la voie de biosynthèse des cobalamines et/ou des cobinamides. Ces phases ouvertes sont appelées cobV et cobU pour les phases 19 et 20. Les phases 18 et 21 ne sont pas des gènes impliqués dans la voie de biosynthèse du coenzyme B12. La position de ces gènes par rapport à la carte de restriction est représentée sur la figure 39. Les insertions 48, 21 et 8 sont cartographiées entre les gènes cobU et cobV
**4.5 - Etudes génétiques du fragment de 13,4 kb.**
   4.5.1. Etudes sur le fragment EcoRI-BglII de 4327 pb.
      Le plasmide pXL189 (Cameron et al., 1989), qui contient au moins un gène cob porte un insert de 3.1 kb qui excepté 300 pb correspond à un fragment EcoRI-ClaI de 4,26 kb (voir figure 45). Le pXL189 a été soumis à une mutagénèse au transposon Tn5, comme cela est décrit précédemment (De Bruijn et Lupski (1984). 13 insertions ont ainsi été cartographiées dans l'insert du pXL189 comme cela est présenté sur la figure 46. Ces 13 plasmides mutants ainsi que pXL189 ont été conjugués chez deux mutants d'A. tumefaciens, G632 et G633, qui sont des mutants complémentés par pXL189 (Cameron et al., 1989). Seule l'insertion 58 s'est avérée être une insertion inactivationnelle. Ce résultat montre que les deux mutants G632 et G633 correspondent à une mutation dans un même gène et que d'autre part le seul gène de P. denitrificans qui puisse être responsable de leur complémentation correspond à la phase ouverte 26 (voir figure 46) puisque l'insertion 58 est cartographiée dans cette phase ouverte; de plus il s'agit de la seule insertion parmi les 13, qui est cartographiée dans cette phase ouverte. Un gène cob, nommé cobO est donc associé à la phase ouverte 26.
      Pour savoir si les quatre autres phases ouvertes (phases ouverte 27 à 30) identifiées sur ce fragment correspondent à des gènes cob, une cassette de résistance à la spectinomycine, du plasmide pHP45Ω (Prentki et Krisch, 1984) a été spécifiquement insérée dans chacun de ces gènes puis introduite dans le chromosome de P. denitrificans SC510 Rif^{r} par recombinaison homologue de manière à obtenir des mutants d'insertions dans chacune de ces phases ouvertes. Pour ce faire, le fragment EcoRI-ClaI (positions respectives 8818 et 13082 sur la séquence présentée sur la figure 43) a été utilisé. Ce fragment, qui porte les phases ouvertes 27 à 30, a été purifié à partir du pXL157 (Cameron et al., 1989); un linker EcoRI a été ajouté sur l'extrémité ClaI après que celle-ci ait été remplie par le fragment Klenow de l'ADN polymérase d'E. coli. Ce fragment a ensuite été cloné dans le plasmide pUC13 (Viera et al., 1982) au site EcoRI. Le plasmide ainsi construit a été appelé pXL332. Des insertions de la cassette de résistance à la spectinomycine du plasmide pHP45n (Prentki et Krisch, 1984) ont été réalisées sur le pXL332. Ces insertions ont été faites séparément aux sites SmaI (position 9868, phase ouverte 27), BamHI (position 10664, phase ouverte 28), ClaI (position 11687, phase ouverte 29) et NcoI (position 12474, phase ouverte 30) par digestions totales ou partielles du pXL332 avec les enzymes correspondantes puis, si cela est nécessaire, remplissage de ces extrémités avec le fragment de Klenow de l'ADN polymérase d'E. coli, puis ligature avec le fragment de SmaI 2 kb du pHP45Ω (Prentki et Krisch, 1984) contenant un gène de résistance à la spectinomycine; ces insertions sont nommées respectivement Ω2, Ω1, Ω3 et Ω4 comme cela est présenté sur la figure 46. Les fragments EcoRI portant ces différentes insertions ont ensuite été clonés sur le pRK404 (Ditta et al., 1985) à l'un des deux sites EcoRI. Les 4 plasmides portant ces différentes insertions ont ensuite été introduits par conjugaison chez SC510 Rif^{r}, comme cela est décrit précédemment. Le plasmide pR751 (Thomas et Smith, 1987) a ensuite été introduit dans les transconjuguants. L'échange des mutations portées par les 4 différents dérivés de pRK404 et le chromosome de SC510 Rif^{r} a pu être sélectionné comme cela est décrit (voir exemple 4.3). 4 souches ont ainsi été obtenues. Ces souches portent chacune une insertions de la cassette de résistance dans une des quatre phases ouvertes 27 à 30. Ces insertions ont été vérifiées par analyse de l'ADN génomique par Southern blot (Southern, 1975). La production de cobalamines de ces différentes souches a été étudiée. Elles ont toutes montré un phénotype Cob+ par culture en milieu PS4. Ce résultat indique que ces phases ouvertes n'interviennent pas dans la biosynthèse du coenzyme B12. Toutefois il est possible qu'une ou plusieurs de ces phases codent pour des protéines qui interviennent par exemple dans la transformation du coenzyme B12 en méthylcobalamine par exemple, c'est à dire la synthèse d'une autre cobalamine voir d'un autre corrinoïde.
   4.5.2 Etude du fragment EcoRI-EcoRI de 9.1 kb.
      Différents plasmides sont utilisés dans cette étude; le plasmide pXL1560 est le plasmide pRK290 (Ditta et al., 1980) contenant le fragment EcoRI-EcoRI de 9,1 kb kb du pXL156 (exemple 1) cloné au site EcoRI du pRK290 (voir figure 46). La construction des autres plasmides utilisés dans cette étude (pXL618, pXL593, pXL623, pXL1909, pXL1938, pXL1908, pXL221, pXL208, pXL297) est décrite dans la légende de la figure 45.
      Des insertions Tn5Sp ont été obtenues sur le plasmide pXL1560. La souche MC1060 Tn5Sp transformée par le plasmide pXL1560 a servi à obtenir des insertions du transposon Tn5Sp dans le fragment pXL1560; 27 insertions ont ainsi été obtenues et cartographiées sur le fragment de 9,1 kb; la position de ces différentes insertions sur le fragment est présentée sur la figure 4. Ces 27 insertions ont été introduites sur le chromosome de la souche SC510 Rif^{r} après transfert conjugatif des pXL1560::Tn5Sp, puis introduction du plasmide pR751. Le plasmide pR751 est un plasmide, résistant à la triméthoprime, du même groupe d'incompatibilité que le pXL1560 (incP, Thomas et Smith, 1987). Par culture non sélective pour le pXL1560 (absence de tétracycline) mais sélective pour le pR751 et le transposon (présence de triméthoprime et de spectinomycine) l'échange de la mutation portée par le pXL1560::Tn5Sp avec le chromosome ainsi que la ségrégation du pXL1560 sont obtenus; cette technique d'échange de marqueur par double recombinaison homologue est équivalente à celle déjà décrite par Schell et al., 1988. Les souches ainsi sélectionnées portent le transposon dans leur chromosome.
      La double recombinaison homologue est vérifiée par la méthode de Southern (Southern, 1975). De cette manière 27 souches SC510 Rif^{r}::Tn5Sp possédant chacune une insertion différente du transposon Tn5Sp dans le fragment de 9.1 kb ont été identifiées.
      Le taux de synthèse de cobalamines est déterminé pour ces 27 souches cultivées en milieu PS4, et le phénotype Cob⁻ est attribué aux souches produisant au moins 1000 fois moins de vitamine B12 que la souche parente SC510 Rif^{r}, figure 46. Il est ainsi observé que 18 de ces insertions chromosomiques, sur les 27, conduisent à un phénotype Cob- chez P. denitrificans comme cela est montré sur la figure 46. Les insertions 19, 32, 24, 27, 37, 39, 26, 11 et 14 sont cartographiées dans la phase ouverte 22 (voir figure 46). Toutes ces insertions sont complémentées par le plasmide pXL618 qui ne contient que la phase ouverte 22. Nous en déduisons que la phase ouverte 22 correspond à un gène cob que nous appelons cobO. Aucune insertion n'a été obtenue dans la phase ouverte 23; cependant le plasmide pXL623 qui ne contient que cette phase ouverte (voir figure 46), complémente deux mutants cobd'Agrobacterium tumefaciens, G642 et G2043 (Cameron et al., 1989). La phase ouverte 23 correspond donc à un gène cob nommé cobP. Les insertions 23, 13, 12, 30, 22, 40, 35, 10 et 17 qui sont cartographiées dans les phases ouvertes 24 et 25 entraînent un phénotype Cob- chez SC510 Rif^{r}. Il semble donc qu'il s'agisse de deux phases ouvertes dont le produit est impliqué dans la biosynthèse des cobalamines. Toutefois on ne peut exclure que ces insertions aient des effets polaires sur les gènes positionnés en 3' tels que cobO. Il convient donc d'étudier la complémentation de ces mutants afin de voir si leur phénotype Cob- ne résulte pas d'un effet polaire.
      Les mutants Cob d'Agrobacterium tumefaciens G622, G623 et G630 complémentés par le pXL156 ont été étudiés. Ces mutants ne sont pas complémentés par le plasmide pXL189 (Cameron et al., 1989) qui contient cobO comme seul gène cob. Au contraire ils sont complémentés par le plasmide pXL1908 qui contient cobO et la phase ouverte 25 en plus des phases ouvertes 27 à 30 (voir figure 45). Ces dernières ne peuvent être responsables de la complémentation de ces mutants puisque les protéines pour lesquelles elles codent n'interviennent pas dans la voie du coenzyme B₁₂. Les complémentations observées ne peuvent donc être que le seul fait de la phase ouverte 25. De plus les mutants SC510 Rif^{r} Tn5Sp cartographiés dans cette même phase ouverte (il s'agit des mutants 22, 40, 35, 10 et 17) sont complémentés par le plasmide pXL1908, voir figure 46, (portant cobO et la phase 25) alors qu'au moins deux d'entre eux ne sont pas complémentés par le pXL189 qui ne contient que cobO comme gène cob. Ces résultats montrent clairement que la phase ouverte 25 est un gène cob; ce gène cob est nommé cobN. Les mutants SC510 Rif^{r} Tn5Sp 23, 13 et 12, qui ont le phénotype Cob-, sont cartographiés dans la phase ouverte 24. Ces mutants ne sont pas complémentés par le plasmide pXL623 qui ne contient que le gène cobP. Au contraire ces mutants sont complémentés par le plasmide pXL593 qui contient cobP et la phase ouverte 24 ce qui indique que la phase ouverte 24 est responsable de leur complémentation. La phase ouverte 24 est donc un gène cob qui est nommé cobW.

### EXEMPLE 5 - Gènes et protéines.

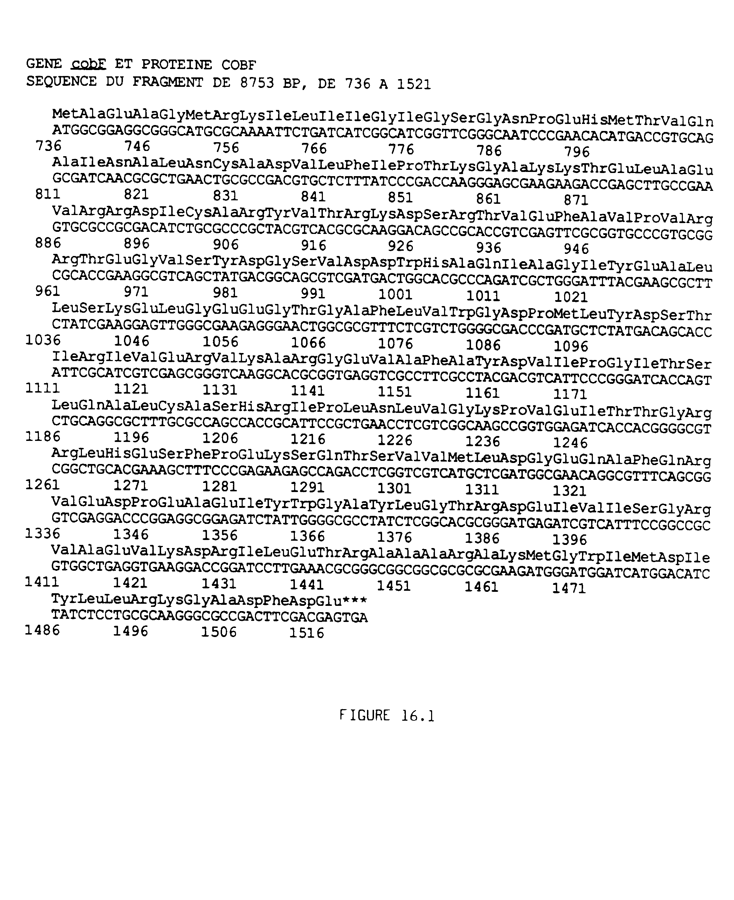

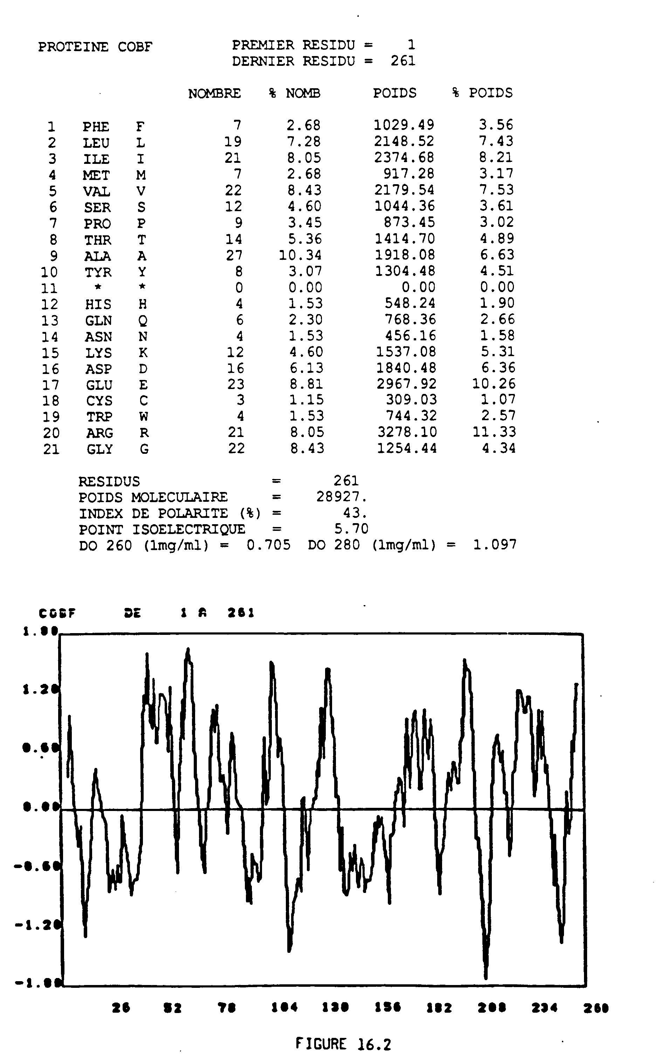

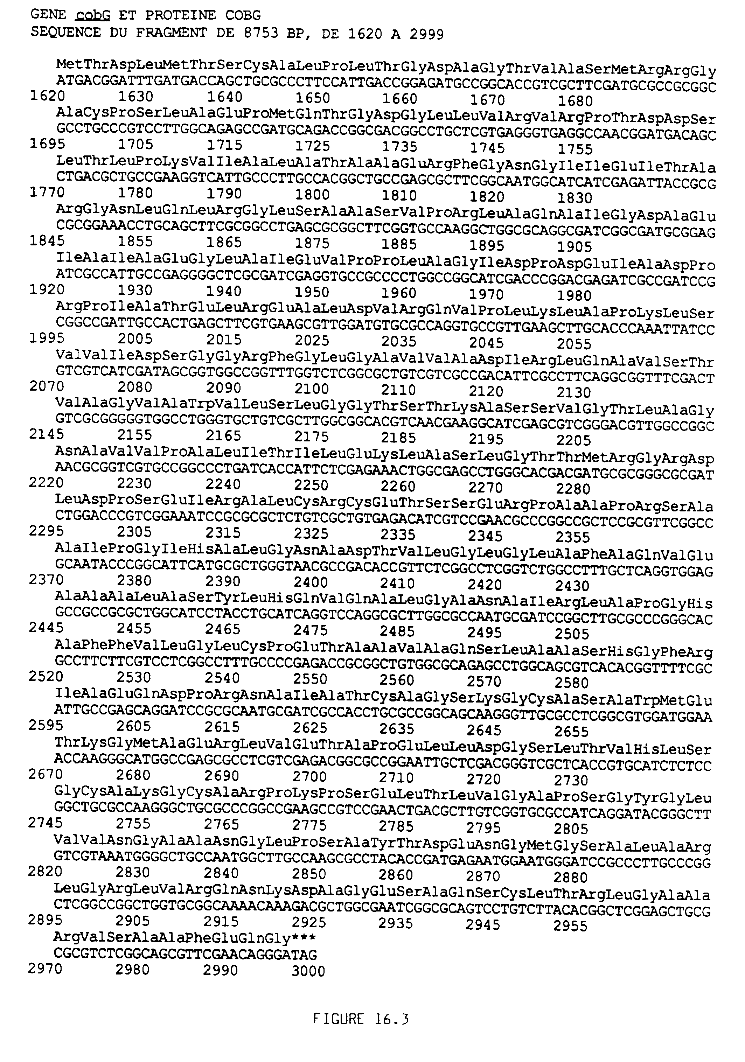

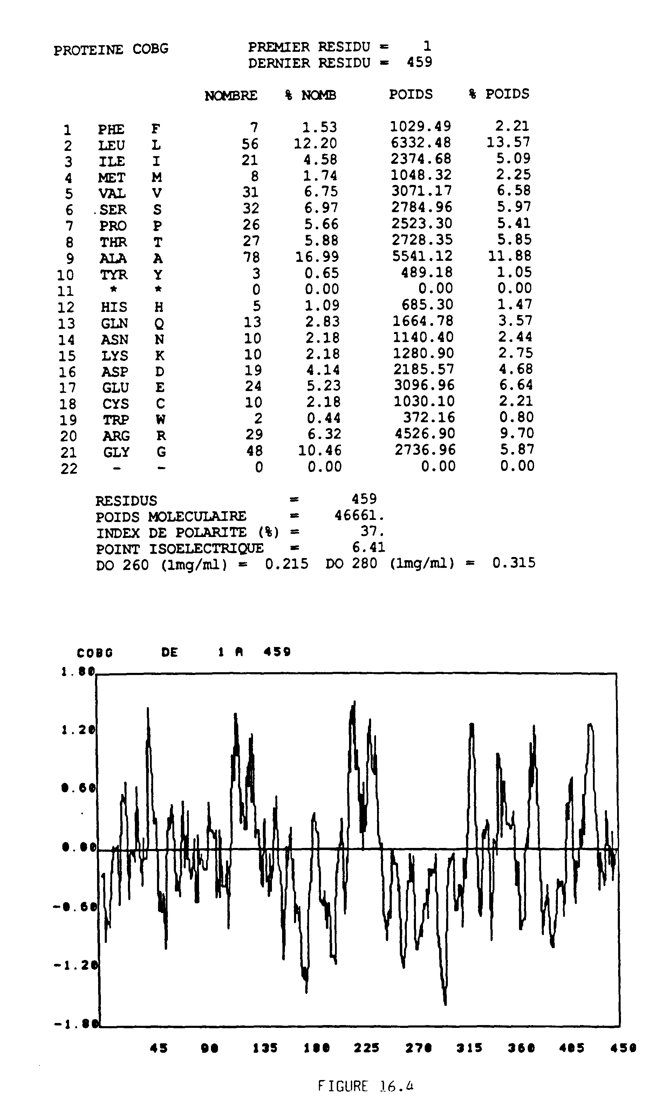

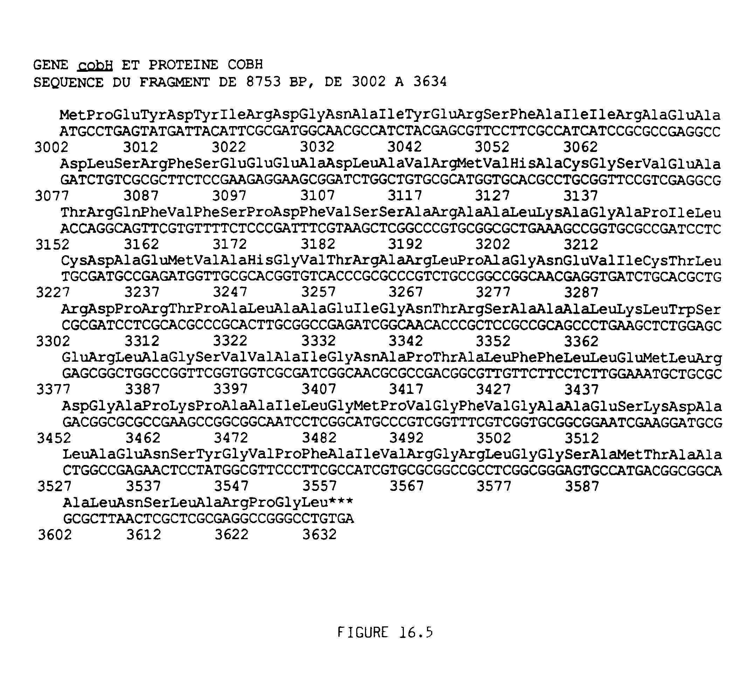

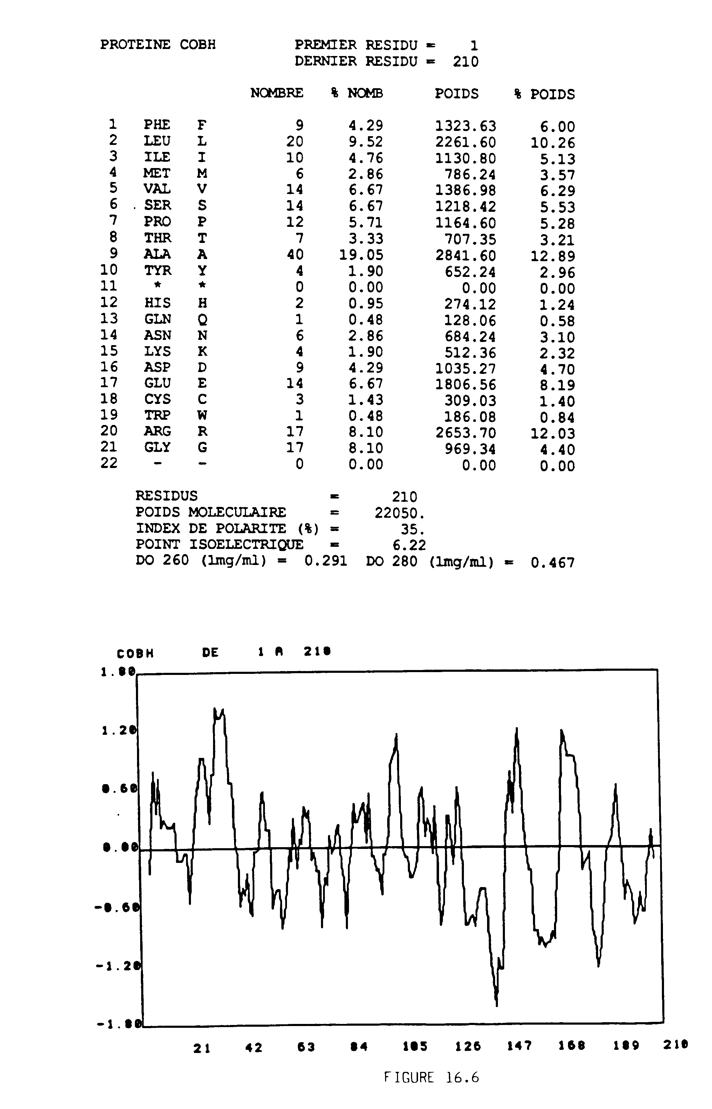

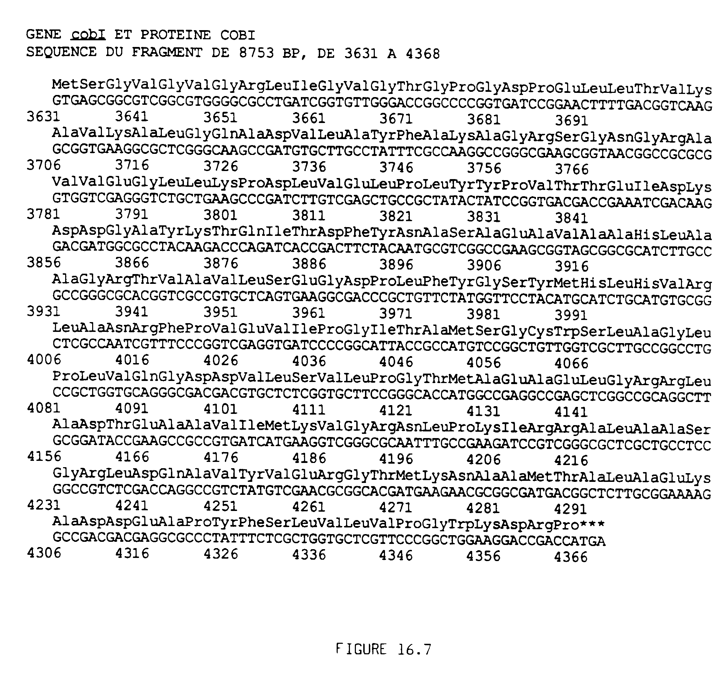

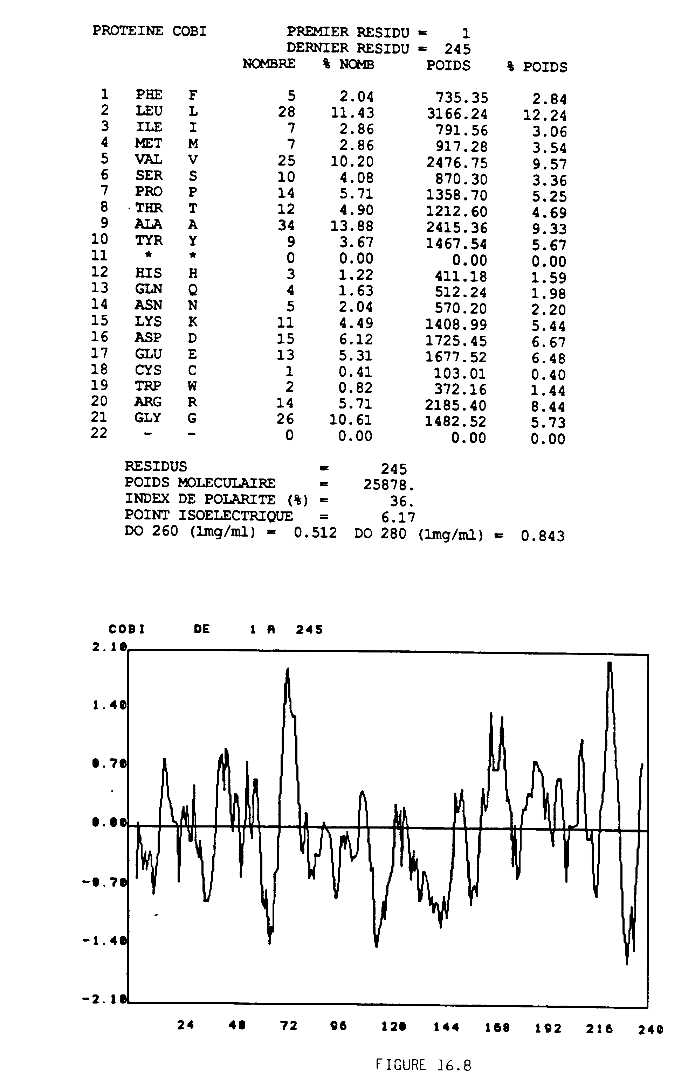

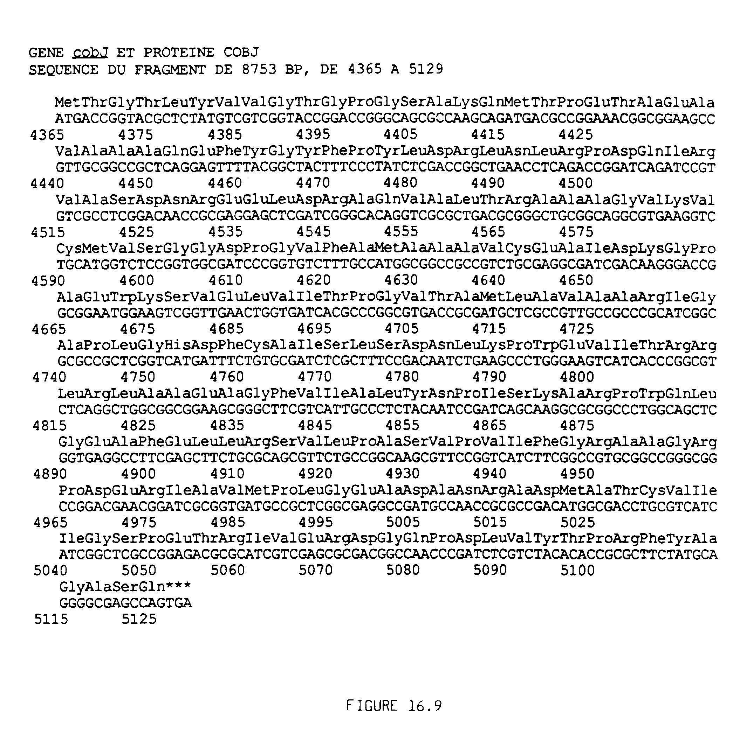

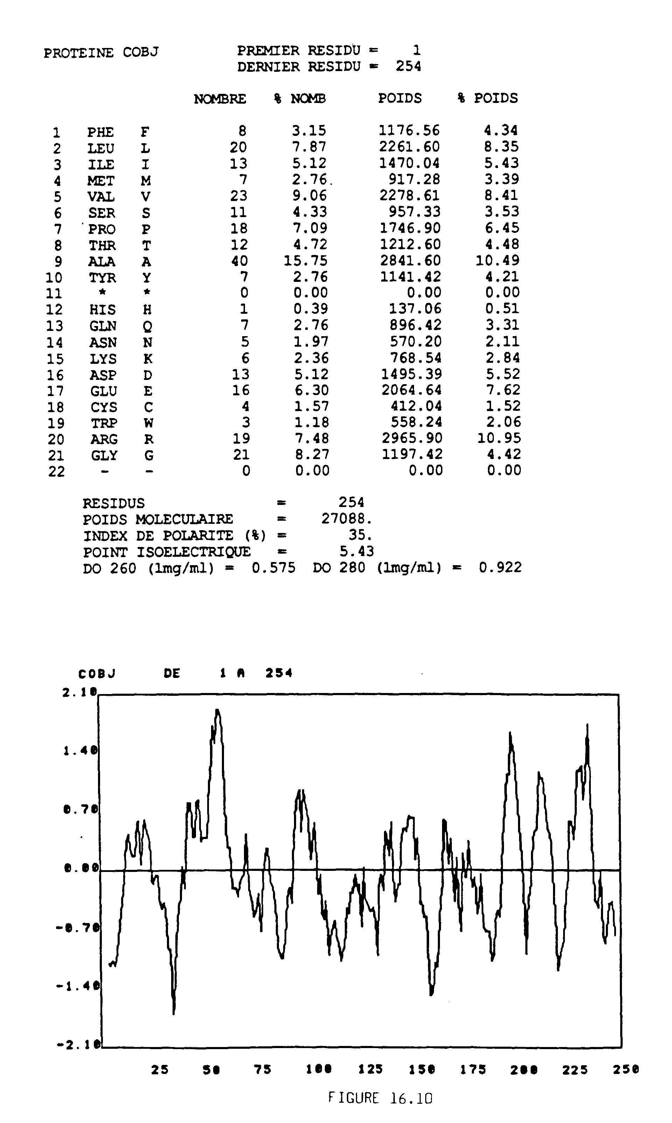

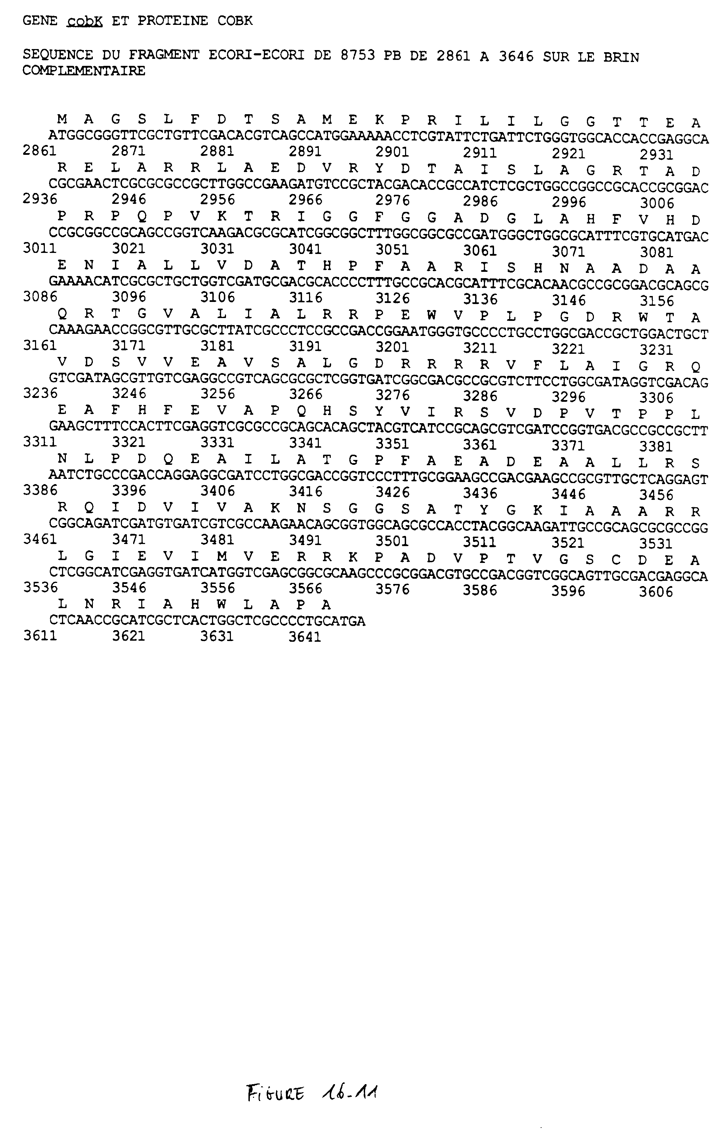

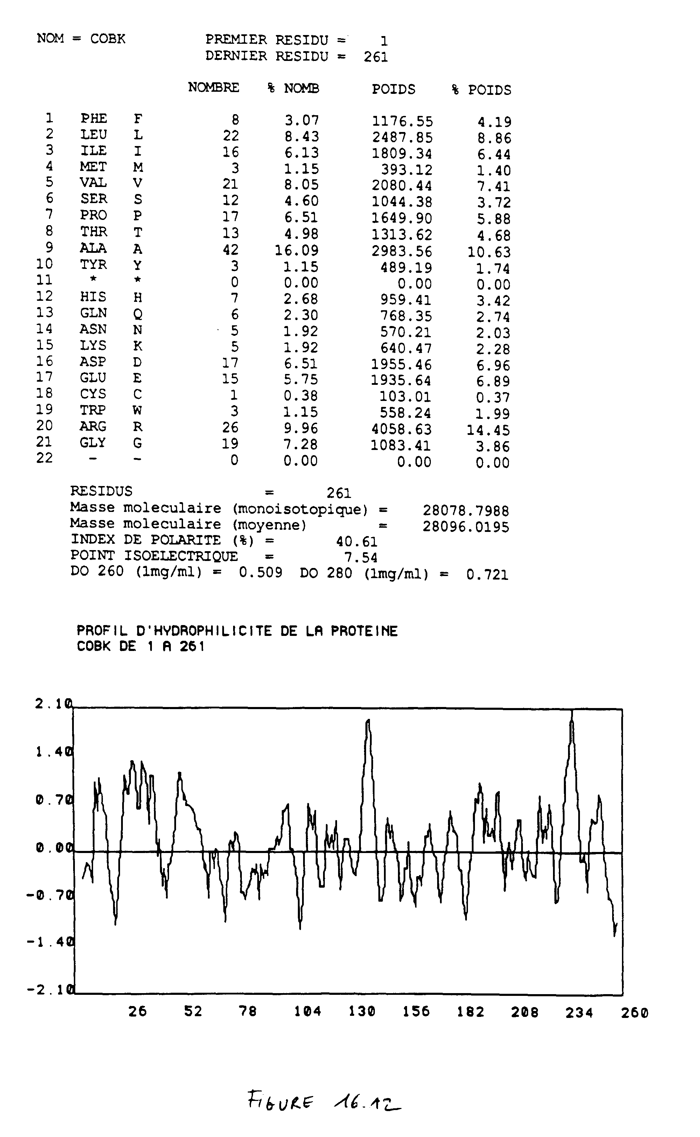

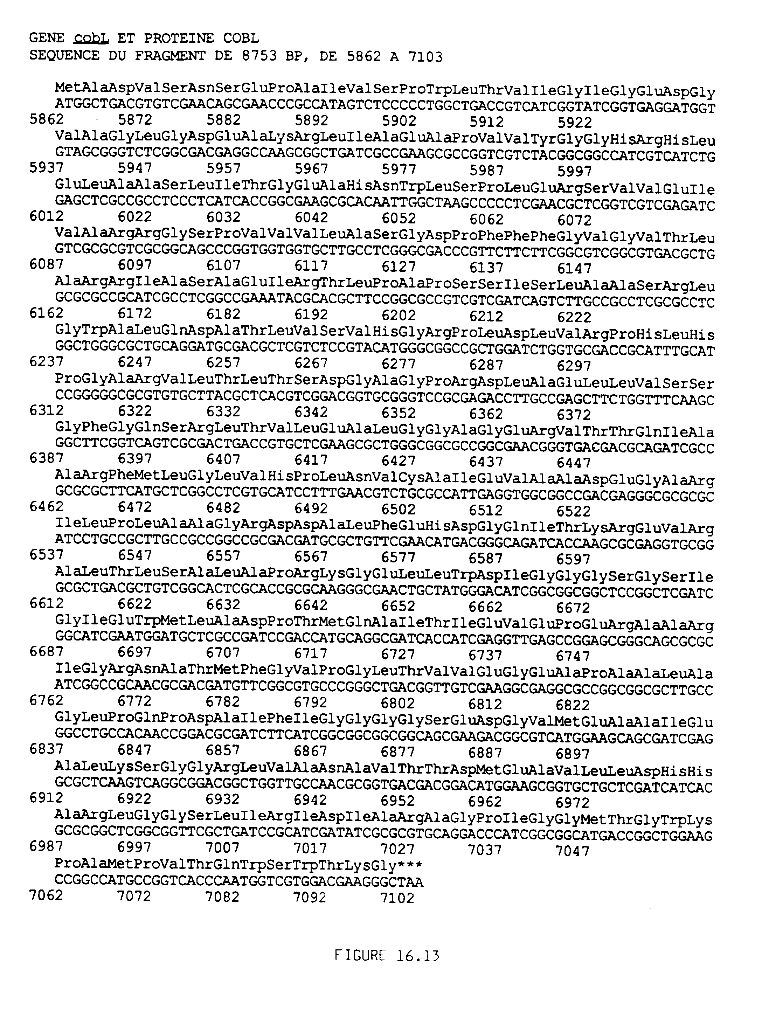

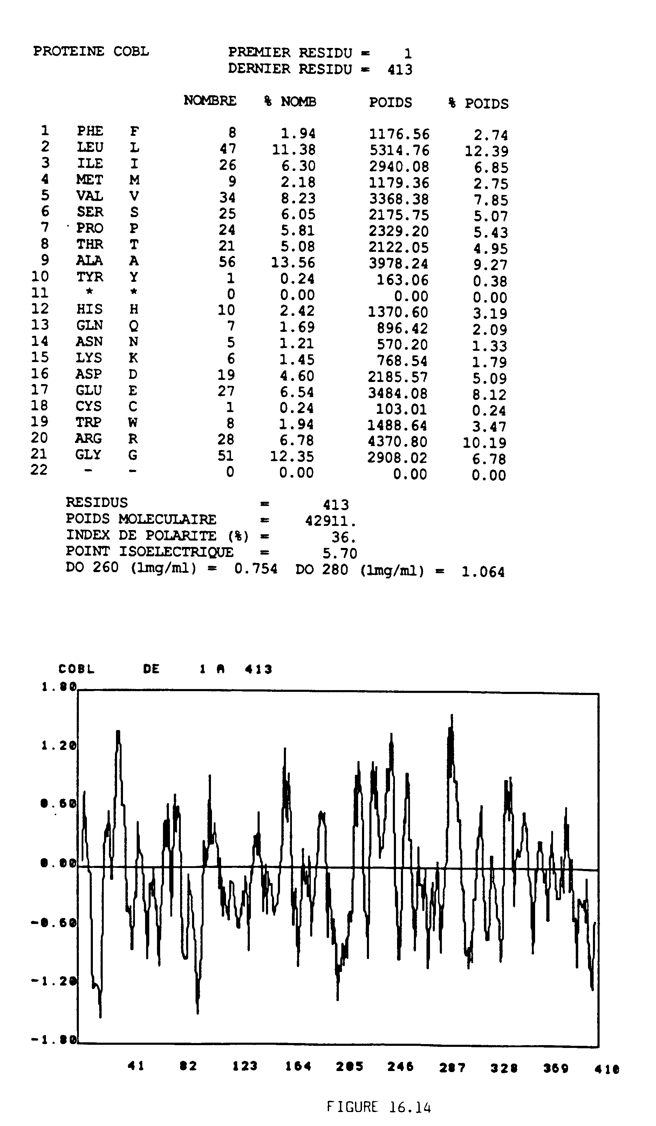

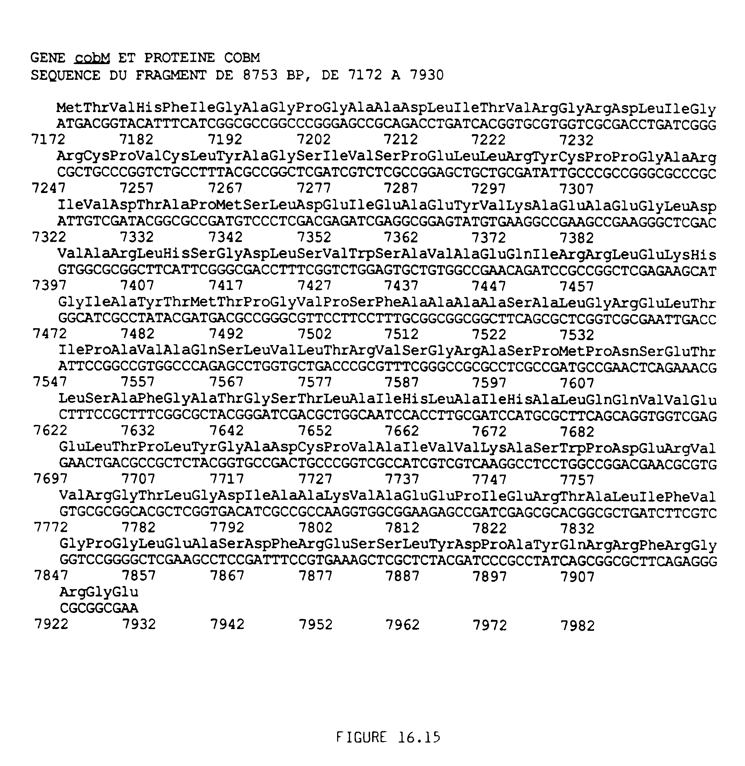

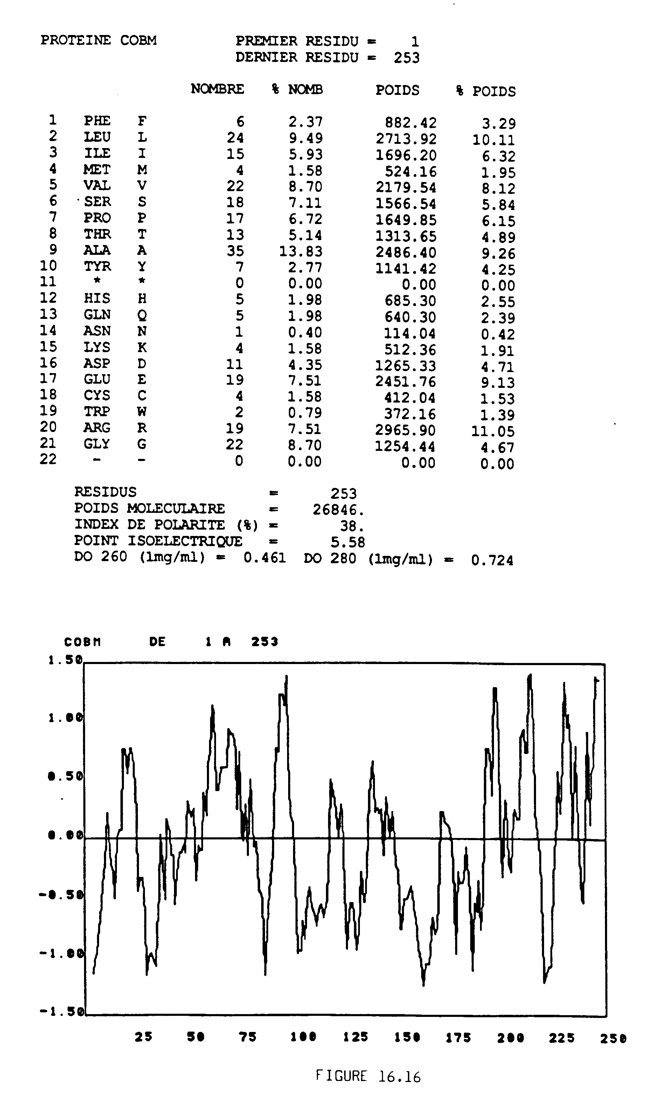

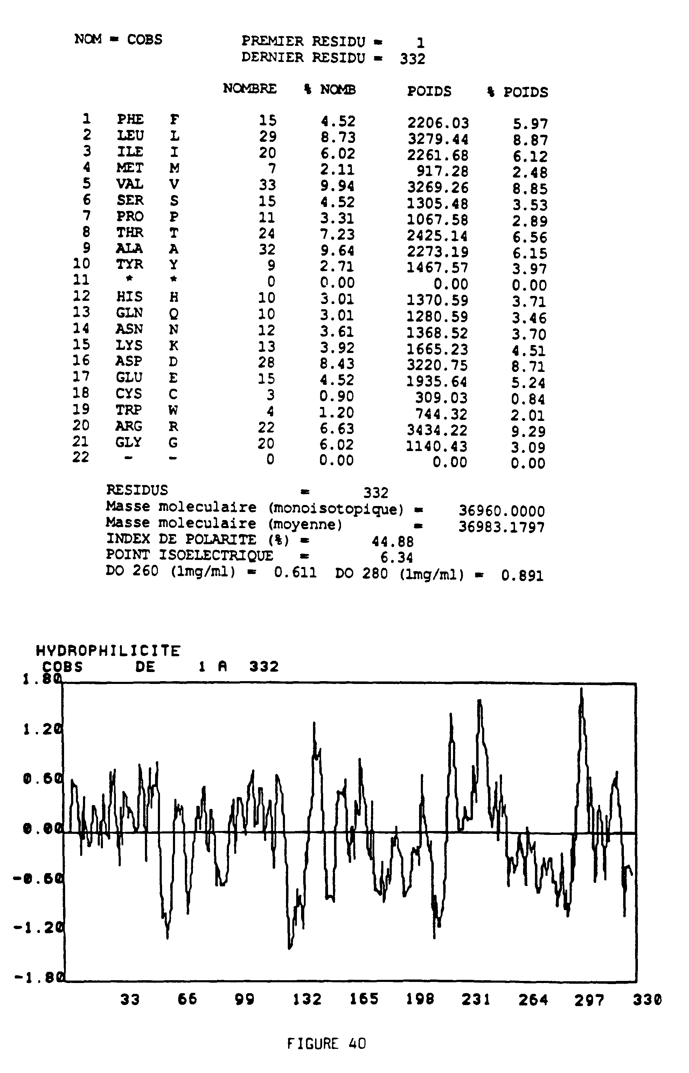

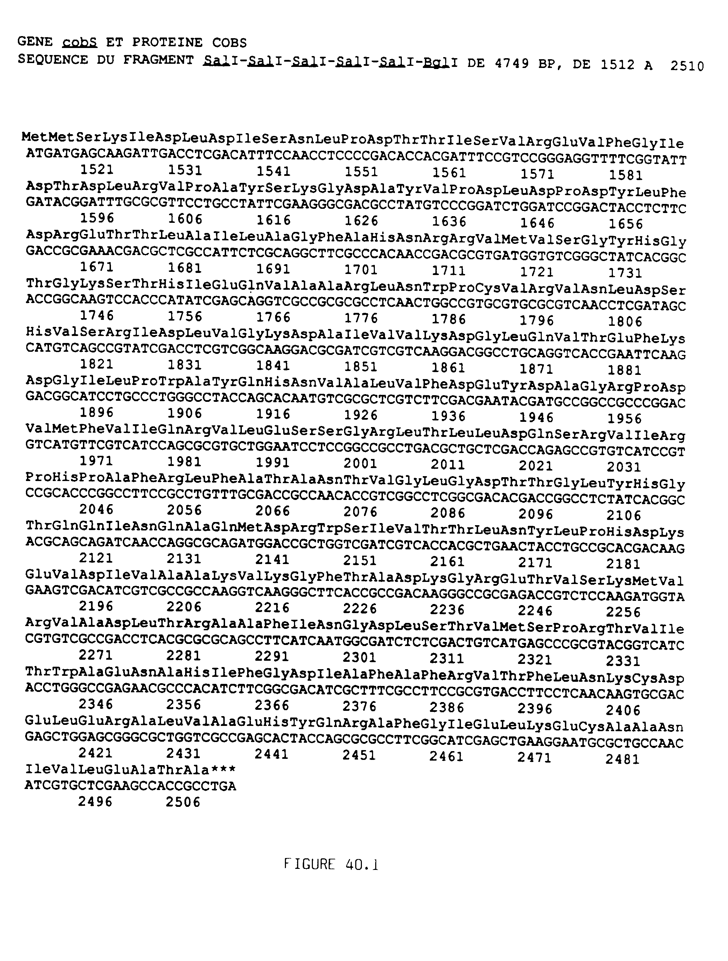

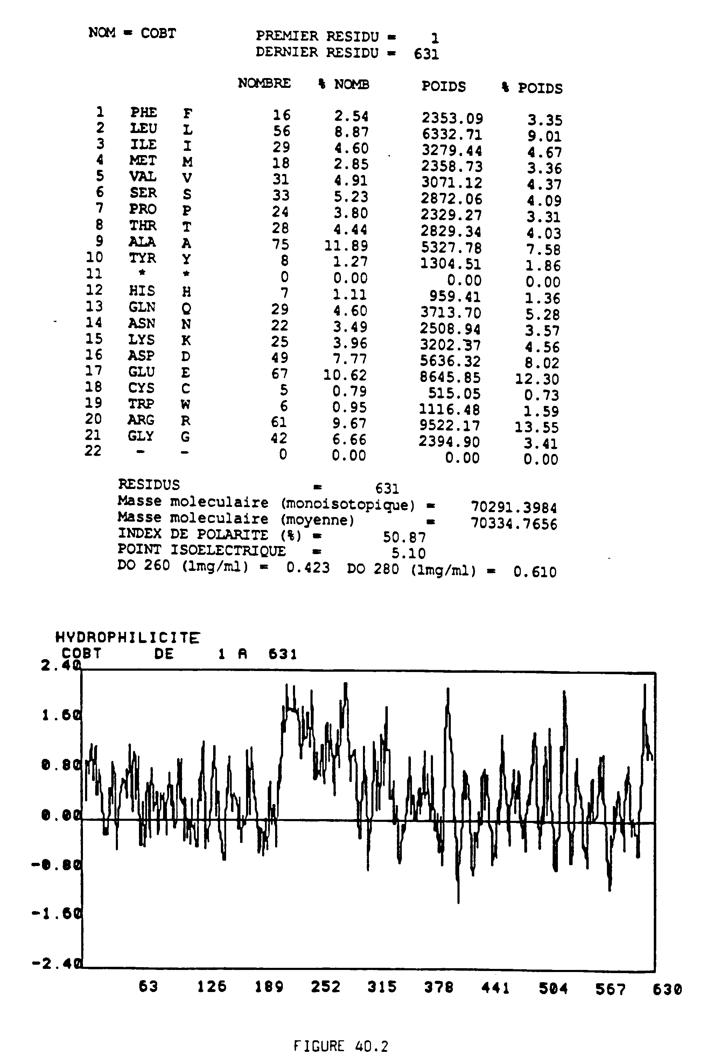

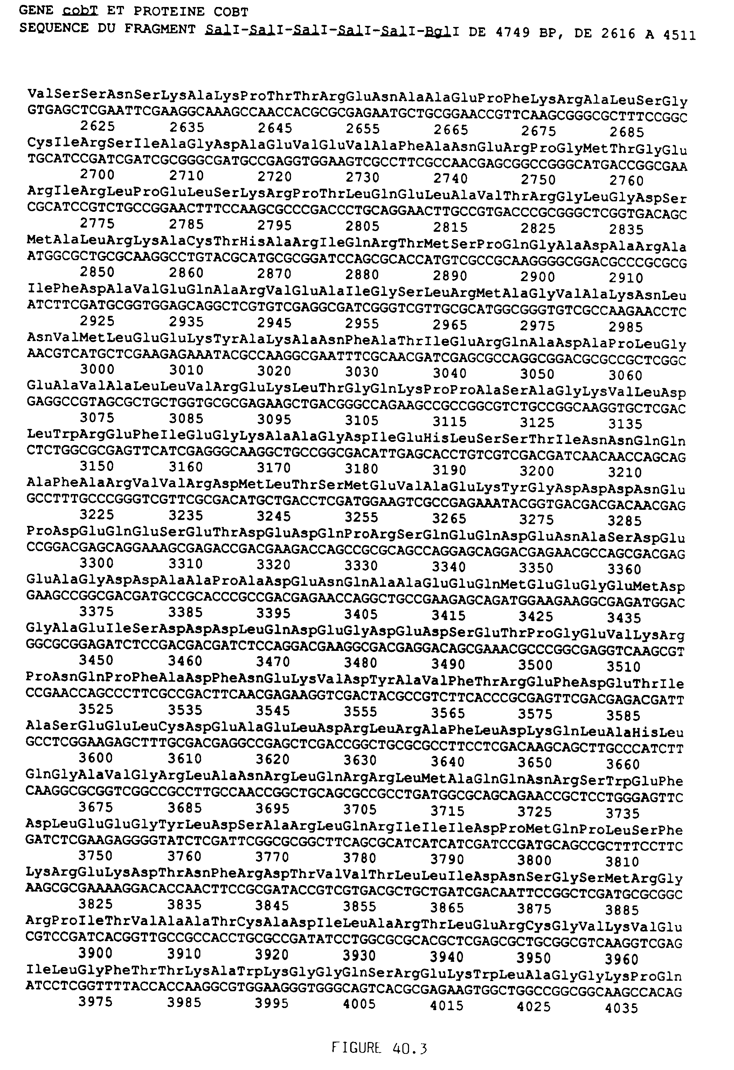

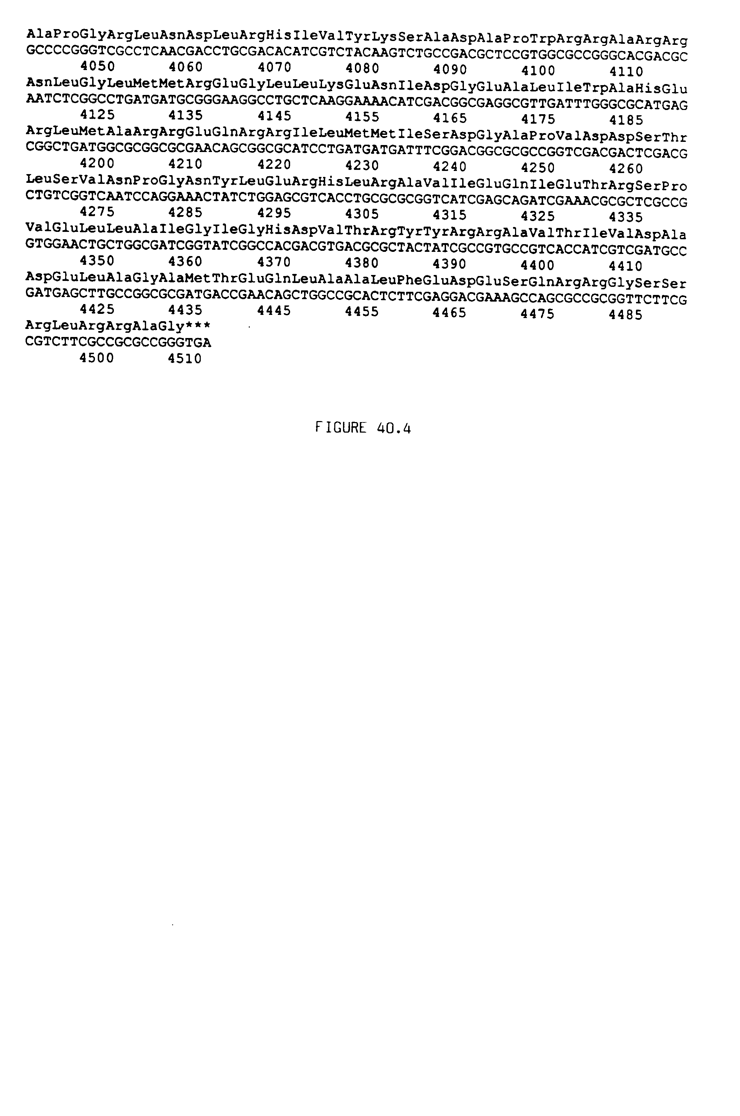

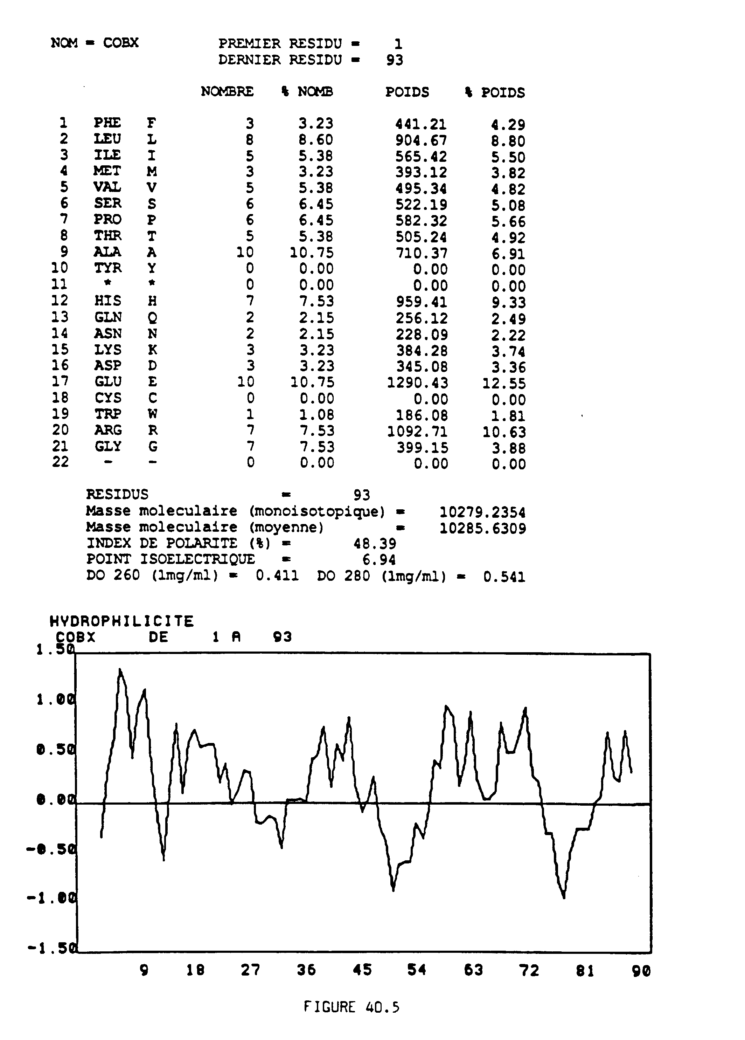

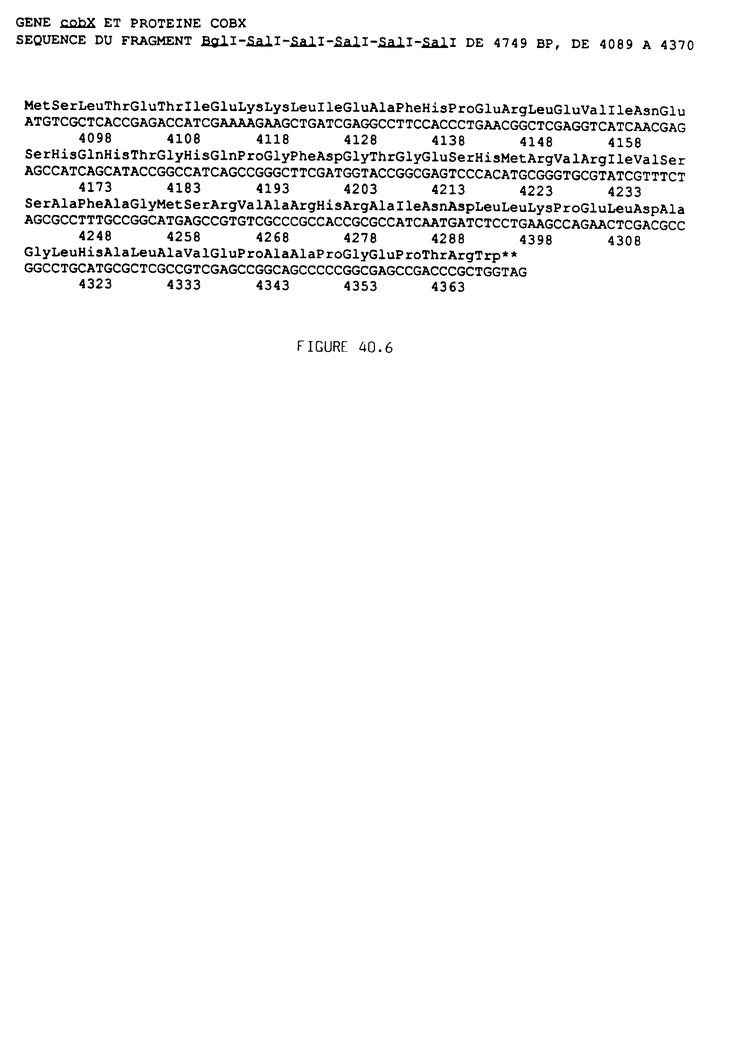

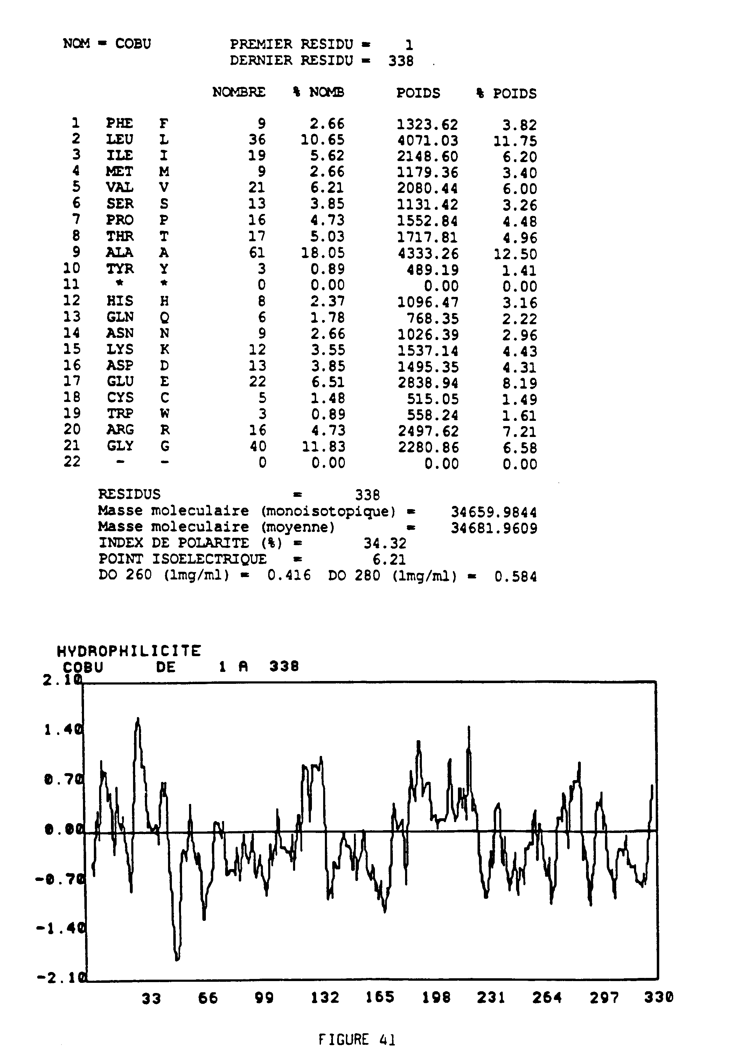

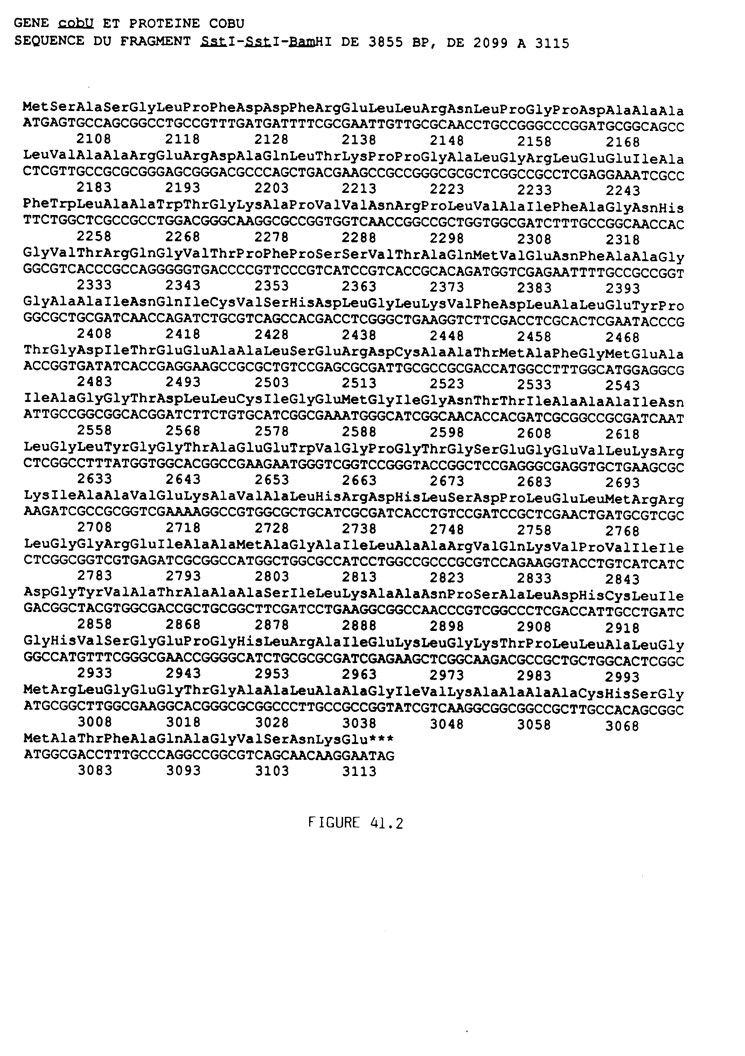

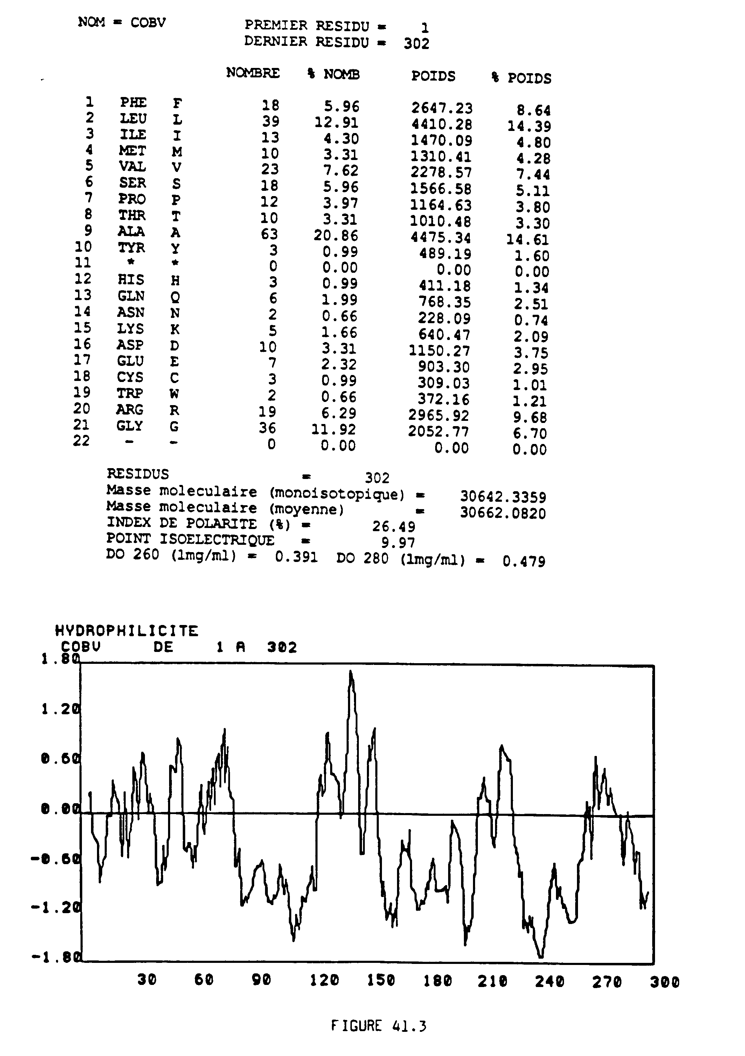

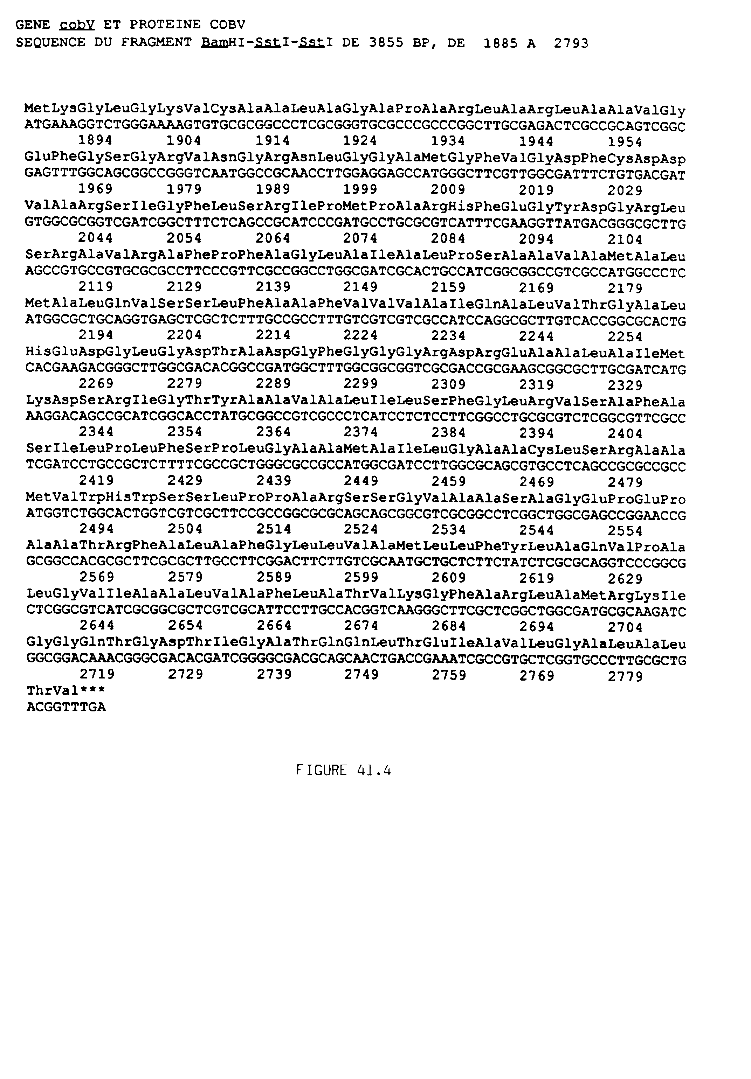

**5.1 - Fragment de 5,4 kb**
   Cinq gènes (cobA, cobB, cobC, cobD et cobE) sont donc définis sur le fragment ClaI-HindIII-HindIII-HindIII de 5,4 kb. Ils codent respectivement pour les protéines COB suivantes: COBA, COBB, COBC, COBD et COBE. Les parties codantes des gènes (cobA à cobE) sont décrites à la figure 15, ainsi que les séquences des protéines COBA à COBE. Des propriétés de chacune de ces protéines sont aussi présentées (composition en acides aminés, point isoélectrique, index de polarité et profil d'hydrophilicité).
**5.2 - Fragment de 8,7 kb**
   Huit gènes sont donc définis sur le fragment de 8,7 kb. Ces gènes cobF à cobM, codent respectivement pour les protéines COB suivantes : COBF, COBG, COBH, COBI, COBJ, COBK, COBL, COBM. Les parties codantes des gènes (cobF à cobM) sont décrites sur la figure 16, ainsi que les séquences des protéines COBF à COBM. Des propriétés de chacune de ces protéines sont aussi présentées (composition en acides aminés, poids moléculaire, point isoélectrique, index de polarité et profil d'hydrophilicité).
**5.3 - Fragment de 4,8 kb**
   Trois gènes (cobX, cobS, cobT) sont définis sur le fragment SalI-SalI-SalI-SalI-SalI-BglI de 4,8 kb. Ils codent respectivement pour les protéines suivantes: COBX, COBS et COBT. Les parties codantes de ces gènes sont décrites sur la figure 40, ainsi que les séquences des protéines COBX, COBS et COBT. Arbitrairement, l'ATG a la position 1512 de cobS a été choisi comme codon d'initiation plutôt que celui situé à la position 1485 (voir figure 32). Des propriétés de chacune de ces protéines sont aussi représentées (composition en acides aminés, point isoélectrique, index de polarité et profil d'hydrophobicité). COBT présente une poche hydrophile correspondant aux acides aminés 214 à 305.
**5.4 - Fragment de 3,9 kb**
   Deux gènes (cobU et cobV) sont définis sur le fragment SstI-SstI-BamHI de 3,9 kb. Ils codent respectivement pour les protéines suivantes: COBU et COBV. Les parties codantes de ces gènes sont décrites sur la figure 41, ainsi que les séquences des protéines COBU à COBV. Des propriétés de chacune de ces protéines sont aussi représentées (composition en acides aminés, point isoélectrique, index de polarité et profil d'hydrophobicité).
**5.5 - Fragment de 13,4 kb**
   Cinq gènes cob sont définis sur le fragment de 13.4 kb (cobO, cobP, cobW, cobN et cobO et cobV). Ils codent respectivement pour les protéines suivantes: COBQ, COBP, COBW, COBN et COBO. Les parties codantes de ces gènes (cobO, cobP, cobW, cobN et cobO) sont décrites sur la figure 46, ainsi que les séquences des protéines COBQ, COBP, COBW, COBN et COBO. Des propriétés de chacune de ces protéines sont aussi représentées (composition en acides aminés, point isoélectrique, index de polarité et profil d'hydrophobicité).
   D'après les profils d'hydrophilicité, qui ont été réalisés suivant les programmes de Hopp et Woods (1981), toutes les protéines COB, à l'exception de COBV, sont vraisemblablement des protéines solubles, par opposition à des protéines membranaires, puisque l'on constate l'absence de grands domaines hydrophobes. COBV est soit une protéine membranaire, puisque l'on constate 4 longs domaines hydrophobes (voir figure 41) soit une protéine cytoplasmique ayant d'importants domaines hydrophobes.
   Pour toutes les séquences en acides aminés des protéines COB, il est indiqué comme premier acide aminé en position NH₂-terminale une méthionine. Il est entendu que celle-ci peut être excisée in vivo (Ben Bassat et Bauer, 1984). On sait que des règles concernant l'excision in vivo de la méthionine NH₂-terminale par la méthionine aminopeptidase ont été proposées (Hirel et al., 1989).
   Par ailleurs, ces séquences protéiques ont été comparées aux protéines de Genpro, qui est une extraction protéique de Genbank (version 59) augmentée des parties codantes putatives supérieures à 200 acides aminés, suivant le programme de Kanehisa (1984). Aucune homologie significative n'a pu être mise en évidence avec les paramètres utilisés sur la version 59 de Genbank, sauf pour COBT. En effet, la protéine COBT présente un "core d'acide aminés acides" entre les positions (en acides aminés) 224 et 293 (voir figure 40); dans cette portion de la protéine, plus d'un acide aminé sur 2 est un résidu acide glutamique ou aspartique; ce noyau d'acides aminés acides rend la protéine homologue sur cette région, suivant le programme de Kanehisa (1984), à d'autres protéines ayant elles aussi un tel noyau acide. Les protéines les plus homologues sont : la protéine GARP de Plasmodium falciparum (Triglia et al., 1988), la troponine T cardiaque du rat (Jin et Lin, 1989), la prothymosine humaine et de rat (Eschenfeld et Berger, 1986), une protéine du rat androgène-dépendante se liant à la spermine (Chang et al., 1987), les protéines "mid-size neurofilament subunit" humaine, de rat et de poulet (Myers et al., 1987, Levy et al., 1987, Zopf et al., 1987). La fonctionalité de ces noyaux riches en résidus acides n'est pas connue; cependant ce noyau acide devrait soit permettre la fixation de cation métalliques tels que le Co++, ce qui rendrait à la protéine COBT un rôle de métallothionéine à cobalt, ou bien permettre des interactions avec d'autres protéines.

### EXEMPLE 6 - Etudes enzymatiques

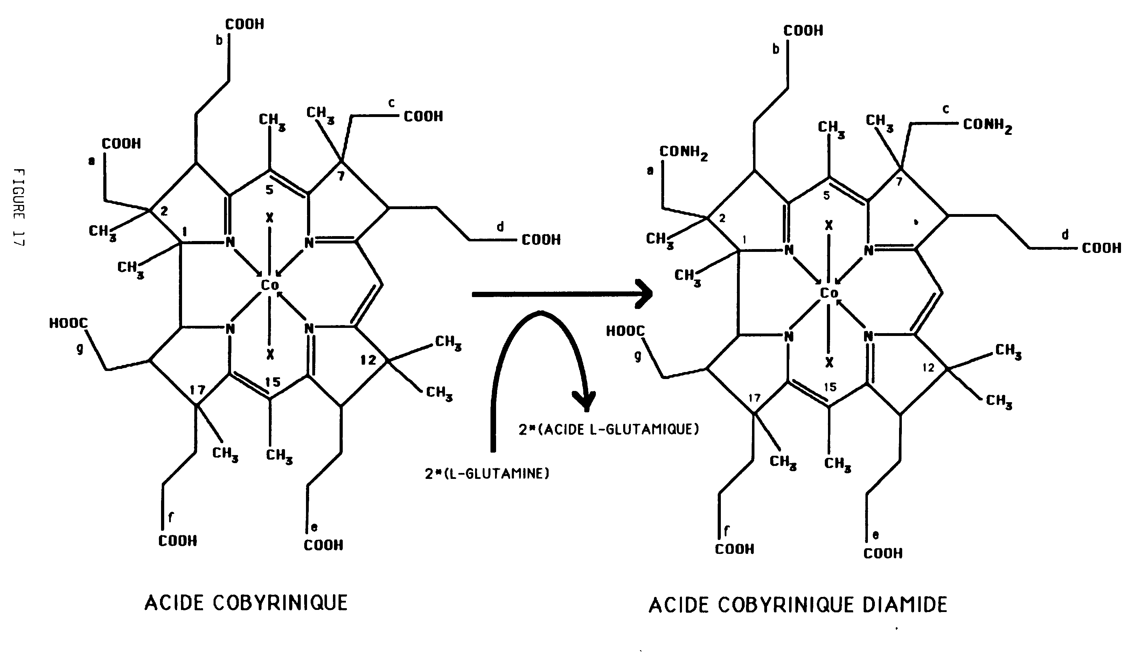

**6.1 - Identification de protéines COB et de leurs gènes à partir d'activités enzymatiques**
Cet exemple décrit comment, à partir d'une protéine purifiée, après avoir établi sa séquence NH₂-terminale, il est possible de trouver le gène de structure correspondant parmi des gènes cob séquencés.
**6.1.1. Identification de la protéine COBA codée par le gène cobA**
La purification de la SUMT de Pseudomonas denitrificans a été décrite (F. Blanche et al., 1989). La séquence NH₂-terminale de la protéine ainsi purifiée a pu être réalisée selon la technique décrite ci-dessus. Les dix premiers acides aminés ont été identifiés : 
1 2 3 4 5 6 7 8 9 10
Met Ile Asp Asp Leu Phe Ala Gly Leu Pro
La séquence NH₂-terminale de la protéine COBA (figure 15) correspond exactement à cette séquence. Le poids moléculaire de la SUMT purifiée estimé par électrophorèse PAGE-SDS à 12,5 % est de 30 000. La protéine COBA a un poids moléculaire déduit de sa séquence de 29 234 (figure 15). Les correspondances entre les séquences NH₂-terminales et les poids moléculaires indiquent clairement que la protéine COBA correspond à la SUMT. Le gène cobA est le gène de structure de la SUMT.
**6.1.2. Identification de la protéine COBB codée par le gène CobB**
a) Dosage de l'activité acide cobyrinique a,c-diamide synthase
Cet exemple illustre le dosage d'une activité de la voie de biosynthèse des corrinoïdes qui n'a encore jamais été décrite. Il s'agit de l'acide cobyrinique a,c-diamide synthase (ACDAS) qui catalyse l'amidation de deux fonctions acides carboxyliques du noyau corrine ou descobaltocorrine aux positions a et c (figure 17). Le donneur de groupement NH₂ est la L-glutamine et la réaction consomme 1 molécule d'ATP par amidation de chaque fonction acide carboxylique. Le dosage qui est décrit ci-dessous s'applique à la réaction de diamidation de l'acide cobyrinique; avec quelques modifications (détection en CLHP à 330 nm en particulier) il s'applique à la réaction de diamidation de l'acide hydrogénobyrinique.
Le mélange d'incubation (250 µl de tris-HCl 0,1 M pH 7,6) contenant ATP (1 mM), MgCl₂ (2,5 mM), glutamine (1 mM), acide cobyrinique (25 µM) ou acide hydrogénobyrinique (5 µM), cobyrinique a,c-diamide synthase (environ 1 unité d'activité) est incubé durant 1 heure à 30°C. A la fin de l'incubation, 125 µl d'une solution aqueuse de KCN (2,6 g/l) et 125 µl d'HCl 0,2 M sont ajoutés au mélange qui est ensuite chauffé à 80°C pendant 10 minutes puis centrifugé 5 minutes à 5 000 g. 50 µl du surnageant de centrifugation sont analysés en CLHP. Ils sont injectés sur une colonne Nucleosil 5-C₁₈ de 25 cm et élués avec un gradient de 0 à 100 % de tampon B dans A en 30 minutes ; tampon A : phosphate de potassium 0,1 M pH 6,5, 10 mM KCN ; tampon B : phosphate de potassium 0,1 M pH 8, 10 mM KCN/acétonitrile (1/1). Les corrinoïdes sont détectés grâce à leur absorption UV à 371 nm. L'unité d'activité enzymatique est définie comme la quantité d'enzyme nécessaire pour synthétiser 1 nmole de groupements amides par heure dans les conditions décrites.
b) Purification de l'activité acide cobyrinique a,c-diamide synthase de Pseudomonas denitrificans
Cette expérience illustre comment une protéine de Pseudomonas denitrificans intervenant dans la voie de biosynthèse des cobalamines peut être purifiée.
A partir du dosage décrit à l'exemple 6.1.2 a), la purification de l'acide cobyrinique a,c-diamide synthase de Pseudomonas denitrificans est réalisée comme décrit ci-dessous.
Dans une expérience typique de purification, 7 g de cellules humides de la souche SC 510 Rif^{r} dans laquelle on a introduit le plasmide pXL1500 (voir exemple 4.1. pour la description du pXL1500 ainsi que la figure 12) sont suspendues dans 30 ml de Tris-HCl 0,1 M pH 7,7 et soniquées durant 15 minutes à 4°C. L'extrait brut est ensuite récupéré par centrifugation 1 heure à 50 000 g puis 10 ml de cet extrait sont injectés sur une colonne de Mono Q HR 10/10 équilibrée avec le même tampon. Les protéines sont éluées avec un gradient linéaire de KCl (0 à 0,5 M). Les fractions contenant l'activité enzymatique sont regroupées et concentrées à 2,5 ml. Après dilution avec 1 ml de Tris-HCl 25 mM pH 7,7, les protéines sont fractionnées sur une Mono Q HR 5/5 en utilisant le gradient de KCl (0 à 0,5 M) précédent. Les fractions actives sont regroupées, 1 ml de Tris-HCl 0,1 M pH 7,7 contenant 1,7 M de sulfate d'ammonium est ajouté à l'échantillon qui est ensuite chromatographié sur une colonne de Phényl-Superose (Pharmacia) avec un gradient décroissant de sulfate d'ammonium (1,0 M à 0 M). Les fractions contenant l'activité recherchée sont rassemblées et chromatographiées sur une colonne Bio-Gel HPHT (Bio-Rad) avec un gradient de phosphate de potassium (0 à 0,35 M).
Après cette étape, l'enzyme est pure à plus de 95 %. Elle ne présente aucune protéine contaminante en PAGE-SDS. La pureté de la protéine est confirmée par l'unicité de la séquence NH2-terminale. Son poids moléculaire dans cette technique est de 45 000. Les différentes étapes de purification de l'ACDAS, avec leur facteur de purification et leur rendement sont portées sur le tableau ci-dessous.

**Tableau :**

| Purification de l'ACDAS | | | | | |
|---|---|---|---|---|---|
| Etape de purification | vol (ml) | Protéines (mg) | Activité spé. (u/mg de protéines) | Rendement | Facteur de purification¹ |
| Extrait brut | 10 | 200 | 8.5 | - | - |
| MonoQ 10/10 | 12 | 15.1 | 108 | 96 | 12.7 |
| MonoQ 5/5 | 3 | 3.75 | 272 | 60 | 32 |
| Phényl-Superose | 1 | 0.865 | 850 | 43 | 100 |
| Bio-Gel HPHT | 2 | 0.451 | 1320 | 35 | 155 |

| | | | | | |
|---|---|---|---|---|---|
| ¹ce facteur est calculé d'après l'augmentation de l'activité spécifique des fractions au cours de la purification. | | | | | |

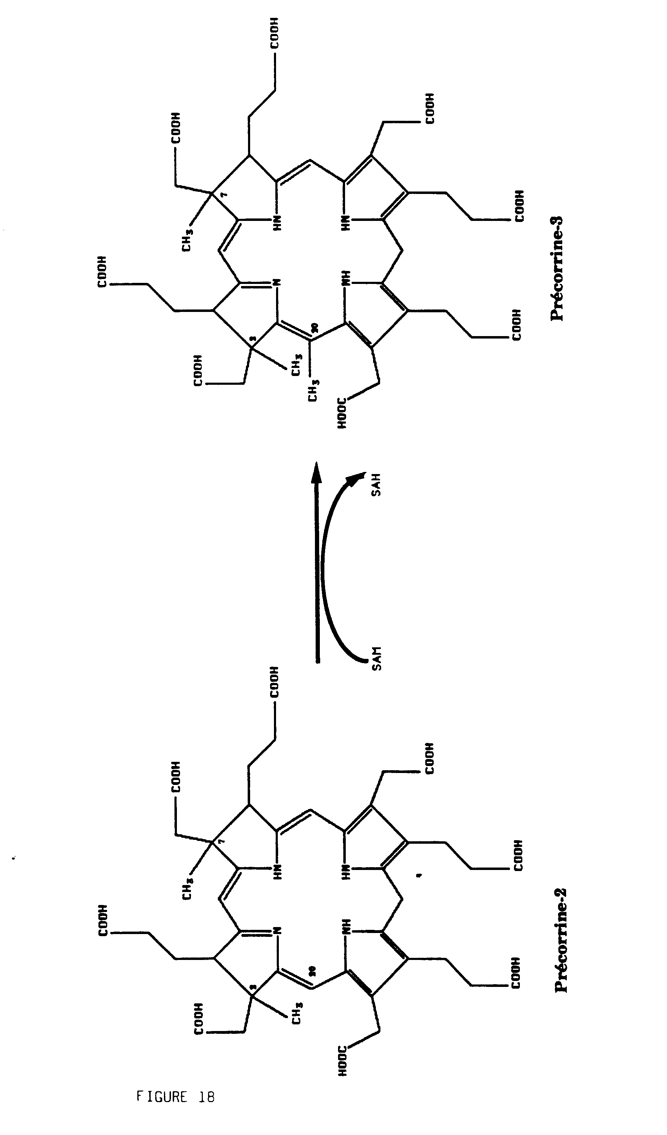

c) Séquence NH₂-terminale de l'acide cobyrinique a,c-diamide synthase de Pseudomonas denitrificans et identification du gène de structure de Pseudomonas denitrificans codant pour cette activité
Cet exemple illustre comment la séquence NH₂-terminale d'une protéine qui intervient dans la voie de biosynthèse des cobalamines permet d'identifier le gène de structure qui code pour cette protéine.
La séquence NH₂-terminale de l'acide cobyrinique a,c-diamide synthase de Pseudomonas denitrificans purifiée comme décrit à l'exemple 6.1.2 b) a été réalisée comme décrit précédemment. 15 résidus ont été identifiés : 
1 2 3 4 5 6 7 8 9 10 11 12 13 14 15
Ser Gly Leu Leu Ile Ala Ala Pro Ala Ser Gly Ser Gly Lys Thr
La séquence NH₂-terminale de la protéine COBB (figure 15) correspond exactement à cette séquence, si ce n'est que dans la séquence présentée sur la figure 15, une méthionine précède la séquence peptidique déterminée par séquençage direct. Il en résulte que la méthionine aminoterminale est certainement excisée in vivo par la méthionine aminopeptidase (Ben Bassat et Bauer, 1987). Le poids moléculaire de l'ACDAS purifiée estimé par électrophorèse PAGE-SDS à 12,5 % est de 45 000. La protéine COBB a un poids moléculaire déduit de sa séquence de 45 676 (figure 15). Les correspondances entre les séquences NH2-terminales et les poids moléculaires indiquent clairement que la protéine COBB correspond à l'ACDAS. Le gène cobB est le gène de structure de l'ACDAS.
**6.1.3. Identification de la protéine COBI codée par le gêne CobI**
a) Dosage d'une activité S-adénosyl-L-méthionine:précorrine-2 méthyltransférase
Cet exemple illustre le dosage d'une activité enzymatique de la voie de biosynthèse des corronoïdes qui n'a encore jamais été décrite. Il s'agit de la S-adénosyl-L-méthionine:-2 méthyltransférase (SP₂MT) qui catalyse le transfert d'un groupement méthyl de la S-adénosyl-L-méthionine (SAM) au précorrine-2 pour donner le précorrine-3 (figure 18). Les facteurs II et III, produits d'oxydation respectivement du précorrine-2 et du précorrine-3 ont déjà été purifiés à partir d'extraits cellulaires de Propionibacterium shermanii (Battersby et MacDonald, 1982, Scott et al., 1984); le précorrine-2 et le précorrine-3 sont reconnus comme des intermédiaires présumés de biosynthèse du coenzyme B₁₂ mais ils n'ont jamais été purifiés. Pour cette raison, l'activité correspondante n'a jamais été ni dosée, ni purifiée auparavant. Le substrat de la réaction enzymatique, précorrine-2, est une molécule très labile qu'il n'est pas possible de conserver car elle s'oxyde spontanément en présence de traces même infimes d'oxygène (Battersby et MacDonald, 1982). Le principe de ce test enzymatique repose donc sur la possibilité de générer extemporanément, à l'aide d'un extrait enzymatique de la souche SC510 Rif^{r} dans laquelle on a introduit le plasmide pXL1500, le précorrine-2 à partir du SAM et d'acide δ-aminolévulinique. L'incubation doit être effectuée en conditions de stricte anaérobie.
Les fractions contenant la SP₂MT sont incubées dans 1 ml de Tris-HCl 0,1 M pH 7,7 en présence de 5 mM DTT, 1 mM EDTA, 100 µM [methyl⁻³H]-SAM (1 µCi), 0,8 mM acide δ-aminolévulinique et 6 mg d'extrait enzymatique brut de la souche de Pseudomonas denitrificans SC510 Rif^{r} pXL1500 pendant 3 heures à 30°C. La souche SC510 Rif^{r} pXL1500 contient une forte activité SUMT (F. Blanche et al., 1989). Les composés tétrapyrroliques produits durant l'incubation sont fixés sur une colonne d'échangeur d'anions DEAE-Sephadex et estérifiés dans le méthanol à 5 % d'acide sulfurique en l'absence d'oxygène. Les dérivés diméthylés et triméthylés de l'uro'gen III sont ensuite séparés par chromatographie sur couche mince de silice en utilisant le dichlorométhane/méthanol (98,3/1,7) comme système éluant (F. Blanche et al., 1989). L'activité de la SP₂MT est exprimée par le rapport de la quantité de dérivés triméthylés obtenus sur l'ensemble des dérivés (di- et tri-) méthylés produits ramenée, à la quantité de protéine. L'extrait de SC510 Rif^{r} pXL1500 introduit dans le test ne présente pas d'activité SP₂MT détectable dans les conditions de dosage (le ratio précorrine-3 produits sur précorrine-2 produits durant le test est inférieur à 0,05).
b) Purification de la S-adénosyl-L-méthionine:précorrine-2 méthyltransférase de Pseudomonas denitrificans
Cette expérience illustre comment une protéine de Pseudomonas denitrificans intervenant dans la voie de biosynthèse de cobalamines peut être purifiée lorsqu'un dosage de l'activité en question existe.
La protéine est purifiée à partir de cellules de SC510 Rif^{r} contenant le plasmide pXL253. Il s'agit du plasmide pKT230 sur lequel a été inséré le fragment EcoRI de 8,7 kb (figure 13). Dans une expérience typique de purification, 50 g de cellules humides de la souche SC150 Rif^{r} dans laquelle a été introduit le plasmide pXL253 sont suspendues dans 250 ml de phosphate de potassium 0,1M pH 7,7, 5 mM DTT et soniquées durant 15 minutes à 4°C. Après centrifugation à 50 000 g durant 1 heure, le surnageant est passé à travers une colonne de DEAE-Sephadex (10 ml de gel) pour éliminer les composés tétrapyrroliques. Le pH de l'extrait brut ainsi obtenu est ajusté à pH 7,7 avec KOH 0,1 M. Les protéines précipitant entre 33 % et 45 % de saturation en sulfate d'ammonium sont collectées et dissoutes dans 40 ml de Tris-HCl 0,1M pH 7,7, 5 mM DTT. Cette solution est passée à travers une colonne de Sephadex G-25 éluée avec du Tris-HCl 10 mM pH 7,7, 5 mM DTT et les protéines collectées sont injectées sur une colonne de DEAE-Trisacryl-M. Les protéines sont éluées avec un gradient linéaire de 0 à 0,25 M KCl et les fractions contenant l'activité SP2MT sont regroupées et passées une seconde fois à travers une colonne de Sephadex G-25 comme ci-dessus. La fraction protéique est injectée sur une colonne d'Ultrogel HA (IBF) équilibrée dans le Tris-HCl 10 mM pH 7,7, 5 mM DTT. Les protéines sont éluées avec un gradient linéaire de 0 à 50 mM de phosphate de potassium pH 7,8 contenant 5 mM DTT. Les fractions contenant l'activité recherchée sont regroupées et injectées sur une MonoQ HR 5/5 (Pharmacia) équilibrée avec du Tris-HCl 50 mM pH 7,7, 5 mM DTT. La SP₂MT est éluée avec un gradient linéaire (0 à 0,25 M) de KCl. A la sortie de l'étape MonoQ, l'électrophorèse en PAGE-SDS (12,5 %) avec coloration aux sels d'argent révèle que l'enzyme est pure à plus de 99 %. Ceci est confirmé par l'unicité de la séquence NH₂-terminale de la protéine. Le poids moléculaire calculé à partir de l'électrophorèse en conditions dénaturantes (PAGE-SDS à 12,5 %) est de 26 500. Les étapes de purification de la SP₂MT avec leurs rendements, sont décrites dans le tableau ci-dessous.

**Tableau :**

| Purification de la SP2MT | | | |
|---|---|---|---|
| Etape de purification | Vol (ml) | Protéines (mg) | Facteur de purification¹ |
| Extrait brut | 300 | 6000 | - |
| Précipitation (33-45 %) | 40 | 1530 | 3.9 |
| DEAE-Trisacryl-M | 57 | 355 | 16.9 |
| Ultrogel HA | 30 | 71 | 85 |
| MonoQ HR 5/5 | 12 | 33.5 | 179 |

| | | | |
|---|---|---|---|
| ¹ce facteur est calculé d'après le rendement en protéines. | | | |

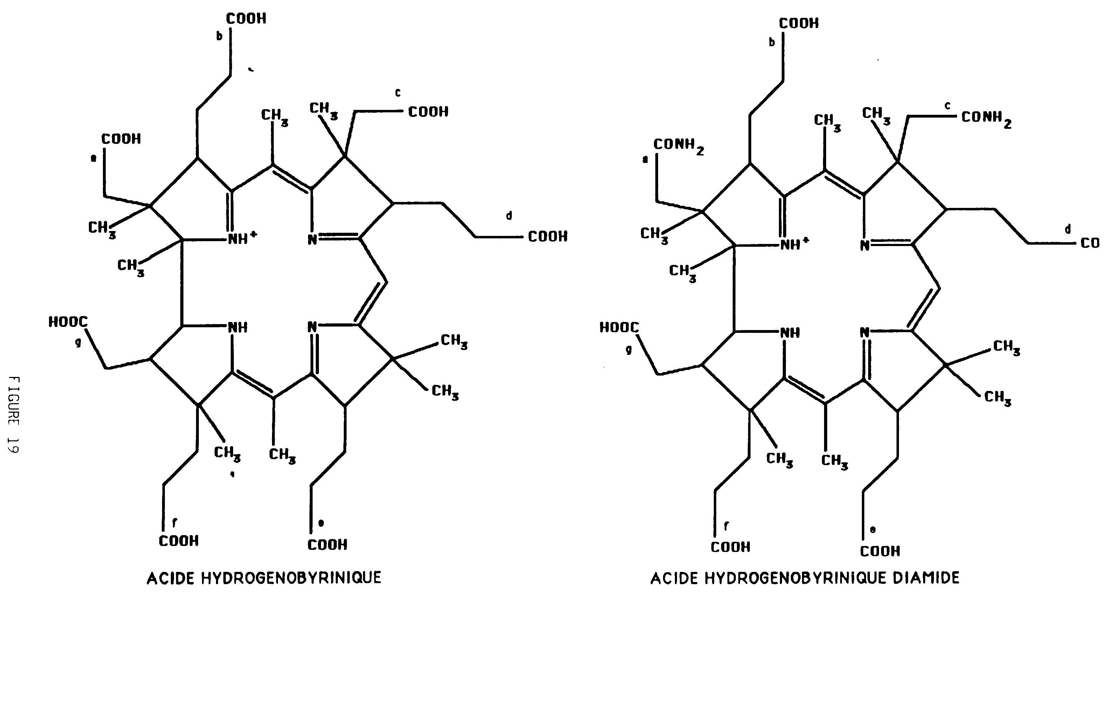

c) Séquence NH₂-terminale de la SP₂MT et identification du gène de structure codant pour cette activité
Cet exemple illustre comment la séquence NH₂-terminale d'une protéine qui intervient dans la voie de biosynthèse permet d'identifier le gène de structure qui code pour cette protéine. Dans l'exemple présent, il s'agit du gène de structure de la SP₂MT.
La séquence NH₂-terminale de la protéine purifiée a été réalisée comme décrit précédemment. Les 15 premiers acides aminés ont été identifiés : 
1 2 3 4 5 6 7 8 9 10 11 12 13 14 15
Ser Gly Val Gly Val Gly Arg Leu Ile Gly Val Gly Thr Gly Pro
La séquence NH₂-terminale de la protéine COBI (figure 16) correspond exactement à cette séquence, si ce n'est que dans la séquence présentée sur la figure 16 une méthionine précède la séquence peptidique déduite de la séquence nucléotidique. Il en résulte que la méthionine aminoterminale est certainement excisée in vivo par la méthionine aminopeptidase (Ben Bassat et Bauer, 1987). Le poids moléculaire de la SP2MT purifiée estimé par électrophorèse PAGE-SDS à 12,5 % est de 26 500. La protéine COBI a un poids moléculaire déduit de sa séquence en acides aminés de 25 878 (figure 16). Les correspondances entre les séquences NH₂-terminales et les poids moléculaires indiquent clairement que la protéine COBI correspond à la SP₂MT. Le gène cobI est le gène de structure de la SP₂MT.
**6.1.4. Identification de la protéine COBH codée par le gène cobH**
a) Dosage de l'activité precorrin-8x mutase.
Cet exemple illustre le dosage d'une activité enzymatique de la voie de biosynthèse des cobalamines qui n'a jusqu'à ce jour jamais été décrite. Il s'agit de la precorrin-8x mutase. Cette enzyme catalyse le transfert du groupement methyl de la position C-11 à la position C-12 lors de la transformation du precorrin-8x en acide hydrogénobyrinique (voir la nomenclature des carbones FIG. 19; PL. 68). Plus généralement, c'est l'enzyme catalysant le transfert du groupement méthyl de C-11 vers C-12, conduisant ainsi au noyau corrine. L'enzyme est ici appelée mutase, bien qu'il ne soit pas formellement démontré que le transfert du groupement méthyl soit intramoléculaire, même si cela est très vraissemblable.
L'activité enzymatique est mise en évidence par la transformation du precorrin-8x (5 pM) en acide hydrogénobyrinique au cours d'incubations en présence de fractions enzymatiques dans du Tris-HCl 0.1 M pH 7.7, 1 mM EDTA, à 30°C durant 1 h. En fin d'incubation, la réaction est stoppée par chauffage à 80°C durant 10 min et après centrifugation à 3000 x g durant 10 min, l'acide hydrogénobyrinique formé présent le surnageant est analysé par CLHP (Cf exemple 6.1.2.a).
b) Purification de la precorrin-8x mutase.
La purification de la precorrin-8x mutase de Pseudomonas denitrificans est réalisée comme décrit çi-dessous.
Durant cette purification, toutes les solutions tampons sont ajustées à pH 7.7.
Dans une expérience typique de purification, 50 g de cellules de la souche SC510 Rifr portant le plasmide pXL253 (plasmide pKT230 sur lequel a été cloné, au site EcoRI, le fragment de 8.7 kb, figure 13), obtenues après culture en milieu PS4, sont resuspendues dans 200 ml de tampon phosphate de potassium 0.1 M, et soniquées durant 12 min. Après centrifugation à 50 000 x g durant 1 heure, le surnageant est passé à travers une colonne de DEAE-Sephadex (10 ml de gel) pour éliminer les composés tetrapyrroliques. Le pH de la solution est immédiatement ajusté à 7.7 avec une solution 1 M KOH. La fraction protéique précipitant entre 40 et 60% de saturation en sulfate d'ammonium est collectée par centrifugation et dissoute dans 50 ml de Tris-HCl 0.1 M. Cet échantillon est ensuite injecté sur une colonne d'Ultrogel AcA 54 (IBF, France)(volume de gel 1000 ml), et les protéines sont éluées à un débit de 60 ml/h avec du Tris-HCl 50 mM. Les fractions contenant l'activité sont regroupées et injectées sur une colonne de DEAE-Trisacryl M (IBF, France) équilibrée avec du Tris-HCl 50 mM, et les protéines sont éluées avec un gradient de 0 à 0.2 M KCl. Les fractions contenant la protéine à purifier sont regroupées, passées à travers une colonne de Sephadex G25 équilibrée en Tris-HCl 10 mM. La fraction protéique est injectée sur une colonne d'Ultrogel HA (IBF, France) équilibrée avec du Tris-HCl 10 mM, et les protéines sont éluées avec un gradient de 0 à 0.1 M de phosphate de potassium, puis la fraction active est chromatographiée sur une colonne de Phenyl-Sepharose CL 4B (Pharmacia) dans du phosphate de potassium 10 mM, éluée avec un gradient de 0.65 à 0 M de sulfate d'ammonium. Les fractions actives sont regroupées. La protéine ainsi obtenue est pure à plus de 95% (d'après les résultats d'électrophorèse PAGE-SDS à 12.5% et coloration aux sels d'argent). La pureté de la protéine est confirmée par l'unicité de la séquence N-terminale. Son poids moléculaire calculé à l'aide de cette technique est de 22 000. Les étapes de purification de la precorrin-8x mutase avec leurs rendements de purification sont décrites sur le tableau çi-dessous.

**tableau :**

| Purification de la precorrin-8x mutase. | | | |
|---|---|---|---|
| Etape de purification | Vol (ml) | Protéines (mg) | Facteur de purification¹ |
| Extrait brut | 250 | 6000 | - |
| Précipitation (40-60 %) | 50 | 2350 | 2.6 |
| Ultrogel ACA 54 | 70 | 655 | 9.2 |
| DEAE-Trisacryl M | 30 | 271 | 22 |
| Ultrogel HA | 22 | 93 | 65 |
| Phényl Sépharose | 12 | 31 | 194 |

| | | | |
|---|---|---|---|
| ¹ce facteur est calculé d'après le rendement en protéines | | | |

c) Séquence NH₂-terminale de la précorrin-8x mutase et identification de son gène de structure.
Cet exemple illustre comment la séquence NH₂-terminale d'une protéine qui intervient dans la voie de biosynthèse permet d'identifier le gène de structure qui code pour cette protéine.
La séquence NH₂-terminale de cette protéine a été réalisée comme décrit précédemment. 15 résidus ont été identifiés : 
1 2 3 4 5 6 7 8 9 10 11 12 13 14 15
Pro Glu Tyr Asp Tyr Ile Arg Asp Gly Asn Ala Ile Tyr Glu Arg
La séquence NH₂-terminale de la protéine COBH (figure 16) correspond exactement à cette séquence, si ce n'est que dans la séquence présentée sur la figure 16 une méthionine précède la séquence peptidique déterminée par le séquençage décrit ci-dessus. Il en résulte que la méthionine aminoterminale est certainement excisée in vivo par la méthionine aminopeptidase (Ben Bassat et Bauer, 1987). Le deuxième résidu étant une proline, cette excision est conforme aux -règles déjà énoncées (Hirel et al., 1989). Le poids moléculaire de la precorrin-8x mutase purifiée estimé par électrophorèse PAGE-SDS à 12,5 % est de 22 000. La protéine COBH a un poids moléculaire déduit de sa séquence de 22 050 (figure 16). Les correspondances entre les séquences NH₂-terminales et les poids moléculaires, de ces protéines, indiquent clairement que la protéine COBH correspond à la precorrin-8x mutase. cobH est le gène de structure de la precorrin-8x mutase.
d) Préparation, isolement et identification du precorrin-8x.
Dans une expérience typique de préparation du precorrin-8x, un extrait enzymatique brut de la souche SC510 Rifr pXL253 (1000 mg de protéines) est incubé en anaérobiose durant 20 h à 30°C dans 100 ml de tampon Tris-HCl 0.1 M pH 7.7 avec 1000 nmol de trimethylisobacteriochlorin préparé comme décrit précédemment (Battersby et al., 1982), EDTA (1 mM), ATP (100 µmol), MgCl2 (250 µmol), NADH (50 µmol), NADPH (50 µmol), SAM (50 µmol) et acide hydrogénobyrinique (20 µmol). En fin d'incubation, le precorrin-8x est le produit tétrapyrrolique formé majoritaire. Il est isolé et purifié par CLHP sur une colonne µBondapak C18 (Waters) en utilisant un gradient d'élution linéaire de 0 à 50% d'acétonitrile dans un tampon phosphate de potassium, pH 5,8. La masse du precorrin-8x (m/z = 880) et la masse de son dérivé methyl ester (m/z = 978) indiquent qu'il s'agit d'un composé ayant la même formule brute que l'acide hydrogénobyrinique. Les caractéristiques UV/Visible et de fluorescence sont très différentes de celles de l'acide hydrogénobyrinique et indiquent que la molécule possède deux chromophores séparés. La seule réaction enzymatique d'isomérisation entre precorrin-6x (Thibaut et al, 1990) et l'acide hydrogénobyrinique étant la migration du methyl de C-11 vers C-12, le precorrin-8x est le dernier intermédiaire avant l'acide hydrogénobyrinique et la réaction correspondante est la migration du méthyl de C-11 vers C-12, catalysée par la precorrin-8x mutase.
**6.1.5. Identification de la protéine COBU codée par le gène cobU**
a) Dosage de l'activité
nicotinate-nucleotide:dimethylbenzimidazole phosphoribosyltransferase (figure 5, réaction 5). Cet exemple illustre le dosage d'une activité enzymatique directement liée à la voie de biosynthèse des cobalamines. Il s'agit de la nicotinate-nucleotide:dimethylbenzimidazole phosphoribosyltransferase (NN:DMBI PRT) (EC 2.4.2.21). Les fractions contenant l'activité NN:DMBI PRT (environ 5 unités) sont incubées à 30°C durant 8 mn dans 500 µl de tampon glycine:NaOH 0.1 M pH 9.7 en présence de 1 mM NaMN (acide nicotinique mononucéotide) et 10 µM DMBI. La réaction est ensuite stoppée par chauffage à 80°C durant 10 mn, le mélange réactionnel est dilué par 4 volumes d'eau et 100 pl de cette solution sont injectés sur une colonne CLHP Nucleosil 5-C8 de 15 cm éluée avec un mélange 0.1 M phosphate de potassium pH 2.9:acétonitrile (93:7) à un débit de de 1 ml/min. Le α-ribazole 5'-phosphate est détecté et quantifié par fluorimétrie (excitation : 260 nm; émission >370 nm). L'unité d'activité enzymatique est définie comme la quantité d'enzyme nécessaire pour générer 1 nmol de α-ribazole 5'-phosphate par heure dans ces conditions.
b) Purification de l'activité NN:DMBI PRT de Pseudomonas denitrificans
Cette expérience illustre comment une protéine de P. denitrificans intervenant dans la voie de biosynthèse des cobalamines peut être purifiée. A partir du dosage décrit dans l'exemple 6.1.5.a), la purification de la NM:DMBI PRT de Pseudomonas denitrificans est réalisée comme décrit ci-desssous. Dans une expérience typique de purification on utilise 10 g de cellules humides de la souche SC510 Rifr dans laquelle le plasmide pXL1490B a été introduit comme cela est décrit précédemment. Le plasmide pXL1490B est décrit sur la figure 38; ce plasmide a été obtenu par clonage du fragment BamHI-SstI-SstI de 3.85 kb du pXL519 (voir figure 38). Ce plasmide porte donc les gènes cobU et cobV de P. denitrificans. Les cellules cultivées en milieu PS4, supplémenté en lividomycine, comme cela est décrit précédemment, sont récoltées aprés 96 heures de culture en milieu PS4. Elles sont resuspendues dans 25 ml de tampon 0.1M Tris-HCl pH 7.2 et soniquées durant 15 mn à 4°C. L'extrait brut est ensuite recupéré par centrifugation durant 1 h à 50 000 g puis passé à travers une colonne de DEAE-Trisacryl M (IBF, France) équilibrée avec le même tampon. 10 % de l'éluat (120 mg de protéines) est fractionné sur une colonne de Mon-o Q HR 10/10 en utilisant un gradient de KCl (de 0 à 0.6 M). Les fractions actives sont regroupées et concentrées à 2 ml par ultrafiltration puis après mélange avec un volume de tampon Tris-HCl 30 mM pH 7.2, l'échantillon est fractionné une seconde fois sur une Mono Q HR 5/5 comme précédemment. Les fractions actives sont regroupées puis l'échantillon est amené à une molarité de 1 M à l'aide de sulfate d'ammonium et chromatographié sur une colonne de Phényl-Superose HR 5/5 élué avec un gradient décroissant de sulfate d'ammonium (de 1 M à 0 M). Les fractions contenant l'activité recherchée sont rassemblées, concentrées par ultrafiltration, et chromatographiées sur une colonne de perméation de gel Bio-Sil 250, éluée avec du phosphate de sodium 20 mM-sulfate de sodium 50 mM pH 6.8.
Après cette étape, l'enzyme est pure à plus de 95 %. Elle ne présente aucune protéine contaminante en PAGE-SDS. Cette pureté est confirmée par l'unicité de la séquence NH2-terminale. Son poids moléculaire dans cette technique est de 35 000. Les différentes étapes de purification de la NN:DMBI PRT sont portées sur le tableau ci-dessous.

**Tableau:**

| Purification de la NN:DMBI PRT de P. denitrificans | | | | | |
|---|---|---|---|---|---|
| Etape de purification | vol (ml) | Protéines (mg) | Activité spé. (u/mg de protéines) | Rendement | Facteur de Facteur de purification¹ |
| Extrait brut | 6,0 | 120 | 2650 | - | - |
| MonoQ 10/10 | 6,0 | 12,07 | 13515 | 51,3 | 5,1 |
| MonoQ 5/5 | 3,0 | 6,19 | 20140 | 39,2 | 7,6 |
| Phényl-Superose | 1,5 | 2,60 | 35510 | 29,0 | 13,4 |
| Bio-Sil 250 | 1,2 | 1,92 | 39750 | 24,0 | 15,0 |

c) Séquence NH2-terminale de la NN:DMBI PRT de P. denitrificans et identification du gène de structure de Pseudomonas denitrificans codant pour cette activité. La séquence NH2-terminale de la NN:DMBI
PRT de Pseudomonas denitrificans purifiée comme décrit dans l'exemple 6.1.5b) a été réalisée selon la technique décrite ci-dessus. Les 15 premiers résidus ont été identifiés : 
1 2 3 4 5 6 7 8 9 10 11 12 13 14 15
Ser Ala Ser Gly Leu Pro Phe Asp Asp Phe Arg Glu Leu LeuArg
 La séquence NH2-terminale de la protéine COBU (figure 41) correspond à cette séquence, si ce n'est que dans la séquence présentée sur la figure 41, une méthionine précède le premier acide aminé de la séquence peptidique déterminée par séquençage direct. Il en résulte que la méthionine aminoterminale est certainement excisée in vivo par la méthionine aminopeptidase (Ben Bassat et Bauer, 1987). Le poids moléculaire de la N-transglycosidase purifiée estimé par électrophorèse PAGE-SDS à 12,5 % est de 35 000. La protéine COBU a un poids moléculaire déduit de sa séquence de 34 642 (figure 41). Les correspondances entre les séquences NH2-terminales et les poids moléculaires indiquent clairement que la protéine COBU correspond à la NN:DMBI PRT. Le gène cobU est le gène de structure de la NN:DMBI PRT. d)Spécificité de la NN:DBI PRT pour de DBI. Cet exemple illustre comment l'étude de la spécificité de la NN:DMBI PRT de P. denitrificans permet de faire biosynthétiser à P. denitrificans diverses cobamides en utilisant les propriétés catalytiques de la NN:DMBI PRT de P. denitrificans pour effectuer la synthèse de la base nucléotidique en question.
Le substrat de l'enzyme pour synthétiser des cobalamines est le 5,6-diméthylbenzimidazole. Le benzimidazole et le 5-méthylbenzimidazole, respectivement, sont des substrats de la réaction avec des vitesses de réaction de 157 % et de 92 %, respectivement, comparé au substrat naturel (5,6-diméthylbenzimidazole), la concentration en NaMN étant fixée à 2 mM. La spécificité de la NN:DMBI PRT de P. denitrificans est donc faible pour les substrats à noyau benzimidazole. On peut donc utiliser la souche de P. denitrificans SC510 Rifr (Cameron et al., 1989), la cultiver en milieu PS4 où le 5,6-diméthylbenzimidazole est remplaçé par du benzimidazole ou du 5-diméthylbenzimidazole, respectivement, afin de faire synthétiser à la bactérie de la Coα-(benzimidazolyl)-Coβ-cyanocobamide, Coα-(5-méthylbenzimidazolyl)-Coβ-cyanocobamide respectivement. Il est certain que d'autres cobamides pourraient être synthétisées de cette manière.
**6.1.6. Identification de la protéine COBV codée par le gène CobV.**
Cet exemple illustre comment le dosage d'une activité de la voie de biosynthèse du coenzyme B12 chez P. denitrificans, puis la purification partielle de cette activité peut permettre d'identifier le gène de structure de cette enzyme chez P. denitrificans.
a) dosage de l'activité GDP-cobinamide:α-ribazole (5'-phosphate) cobinamidephosphotransférase (ou cobalamine (5'-phosphate) synthase)
Cet exemple illustre le dosage d'une activité directement liée à la voie de biosynthèse des cobalamines. Il s'agit de la cobalamine (5'-phosphate) synthase. Les fractions contenant l'activité (environ 5 à 10 unités) sont incubées à l'obscurité à 30°C dans 500 µl de tampon Tris-HCl 0.3 M pH 9.0 en présence de 1 mM EDTA, 12.5 mM MgCl2, 50 µM α-ribazole 5'-phosphate et 20 µM GDP-cobinamide [sous forme 5'-deoxy-5'-adénosyl (Ado) ou coenzyme]. Après 15 mn d'incubation, 500 pl de cyanure de potassium 20 mM sont ajoutés et la solution est chauffée à 80°C durant 10 mn. Après centrifugation pour éliminer le matériel précipité, la vitamine B12 5'-phosphate présente dans le surnageant est dosée comme cela est décrit à l'exemple 9. Une unité de cobalamine (5'-phosphate) synthase est définie comme la quantité d'enzyme nécessaire pour générer 1 nmol de cobalamine 5'-phosphate par h dans les conditions décrites ci-dessus.
L'Ado-GDP-cobinamide est obtenu par incubation de l'Ado-cobinamide phosphate (Blanche et al., 1989) avec un extrait de SC510 Rifr pXL623 dans les conditions du dosage de la cobinamide phosphate guanylyltransferase (voir 6.1.11.b). Le α-ribazole et le α-ribazole-5'-phosphate sont isolés à partir de cultures de SC510 Rifr et purifiés par CLHP dans les conditions du dosage décrit dans l'exemple 6.1.5a).
b) Purification partielle de la cobalamine (5'-phosphate) synthase.
Cette expérience illustre comment une activité enzymatique de P. denitrificans intervenant dans la voie de biosynthèse des cobalamines de P. denitrificans peut être partiellement purifiée. A partir du dosage décrit ci-dessus, la purification de la cobalamine (5'-phosphate) synthase est réalisée. Pour ce faire, dans une expérience typique de purification, 10 g de cellules humides de la souche SC510 Rifr dans laquelle le plasmide pXL1490B a été introduit comme cela est décrit précédemment. Le plasmide pXL1490B est décrit sur la figure 38; ce plasmide correspond au fragment SstI-SstI-BamHI de 3.85 kb cloné dans le pKT230. Ce plasmide porte les gènes cobU et cobV de P. denitrificans. La présence de ce plasmide chez P. denitrificans SC510 Rifr conduit à une amplification de l'activité cobalamine (5'-phosphate) synthase d'un facteur 100 environ; il est donc probable que l'insert porté par le plasmide pXL1490B contient le gène de structure de cette enzyme; ce gène ne peut donc être que cobU ou cobV. Les cellules SC510 Rifr pXL1490B sont obtenues par culture en milieu PS4 supplémenté avec de la lividomycine comme cela est décrit ci-dessus. Les cellules sont centrifugées puis resuspendues dans 25 ml de tampon Tris-HCl 0.1 M (pH 8.3)-1 mM EDTA (tampon A) et soniqués pendant 15 mn à 4°C. L'extrait brut est ensuite récupéré par centrifugation 1 h à 50 000 g et passé à travers une colonne de Sephadex G-25 équilibrée avec du tampon A. La fraction protéique est récupérée et injectée par fraction de 300 µl (7.5 mg de protéines) sur une colonne de Superose 12 HR 10/30 éluée dans du tampon A. La fraction exclue est récupérée, mélangée à un volume égal de tampon A-1.0 M sulfate d'ammonium et chromatographiée sur une colonne de Phényl-Supérose HR 5/5. Les protéines sont éluées avec un gradient décroissant de sulfate d'ammonium (de 0.5 M à 0 M) dans du tampon A, suivi d'un plateau à 0 M de sulfate d'ammonium dans le but d'éluer l'activité cobalamine (5'-phosphate) synthase. La purification partielle de cette enzyme est décrite dans le tableau ci-dessous, sur la base de 75 mg de protéines introduits au départ dans le processus de purification.

**Tableau :**

| Purification partielle de la cobalamine (5'-phosphate) synthase de P. denitrificans. | | | | | |
|---|---|---|---|---|---|
| Etape de purification | vol (ml) | Protéines (mg) | Activité spé. (u/mg de protéines) | Rendement | Facteur de purification¹ |
| Extrait brut | 3.0 | 75 | 325 | - | - |
| Superose 12HR | 50.0 | 2.9 | 6 810 | 81 | 21 |
| Phenyl-Superose | 4.5 | 0.35 | 17 850 | 26 | 55 |

c) Spécificité de la cobalamine (5'-phosphate) synthase. Le Km pour le (Ado)GDP-cobinamide est de 0.9 µM. Toutefois, l'enzyme présente la même affinité et une vitesse de réaction pratiquement identiques pour la forme (CN, aq) du substrat. Le Km de l'enzyme pour le α-ribazole
5'-phosphate est de 2.7 µM environ. De plus, les préparations les plus pures de cobalamine (5'-phosphate) synthase catalysent la réaction du Ado-GDP-cobinamide avec le α-ribazole pour donner le coenzyme B12 et dans ces conditions aucune accumulation de cobalamine 5'-phosphate n'est observée. Le Km de l'enzyme pour le α-ribazole est de 7.8 µM. Des concentrations intracellulaires en α-ribazole 5'-phosphate et en α-ribazole de 30 et de 700 pM respectivement, ont été mesurées par CLHP au cours de la production de cobalamines de SC510 Rifr en milieu PS4 dans les conditions de cultures décrites dans l'exemple 6.1.5a). Ceci montre que le coenzyme B12 peut être généré directement à partir du Ado-GDP-cobinamide par la cobalamine (5'-phosphate) synthase sans l'intervention d'une cobalamine 5'-phosphatase.
L'absence d'accumulation ou la présence de traces de cobalamine 5'-phosphate dans les cultures de P. denitrificans SC510 Rifr confirme que le coenzyme B12 est produit par la réaction directe du Ado-GDP-cobinamide avec le α-ribazole in vivo.
Cette réaction directe a déjà été observée et décrite in vitro chez Propionibacterium shermanii (Ronzio et al., 1967; Renz, 1968). Comme le gène de structure de la cobalamine (5'-phosphate) synthase ne peut être que cobU ou cobV puisque l'amplification chez P. denitrificans d'un fragment portant ces deux gènes cob de P. denitrificans conduit à une augmentation par un facteur 100 de l'activité cobalamine (5'-phosphate) synthase et que le gène cobU est le gène de structure de la NN:DMBI PRT, cobV est donc le gène de structure de la cobalamine (5'-phosphate) synthase.
**6.1.7. Identification de la protéine COBK codée par le gène cobK.**
a) dosage de l'activité precorrin-6x réductase.
Cet exemple illustre le dosage d'une activité enzymatique nouvelle directement liée à la voie de biosynthèse des cobalamines. Il s'agit de la precorrin-6x réductase.
Les fractions contenant l'activité precorrin-6x réductase (environ 0.05 unités, U) sont incubées à 30°C durant 60 min dans 250 µl de tampon Tris-HCl 0.1 M pH 7.7 en présence de 1 mM EDTA, 500 pM NADPH, 25 µM [methyl-3H]SAM (80 µCi/µmol), 4 pM precorrin-6x (Thibaut et al., 1990), et 0.5 U de dihydroprecorrin-6x méthylase partiellement purifiée (voir préparation çi-dessous). La réaction est ensuite stoppée par chauffage à 80°C durant 5 min et, après centrifugation à 5000 x g durant 5 min, le surnageant est injecté sur une colonne de .DEAE-Sephadex (contenant 200 µl de gel). La colonne est ensuite lavée extensivement avec le tampon Tris-HCl, et les composés fixés sont élués avec 4 ml de 1 M HCl. La radioactivité dans cet éluat est comptée en scintillation liquide. L'unité d'activité enzymatique est définie comme la quantité d'enzyme nécessaire pour réduire 1 nmol de precorrin-6x par h dans ces conditions.
La dihydroprecorrin-6x methylase est partiellement purifiée à partir d'un extrait brut de SC510 Rifr pXL253, sur une colonne d'échange d'anions Mono Q HR 5/5 (Pharmacia). La colonne est éluée avec un gradient linéaire de 0 à 0.4 M de KCl dans du tampon 0.1 M Tris-HCl pH 7.7. L'activité enzymatique est éluée à 0.35 M KCl. Cette activité est détectée et quantifiée grâce au test d'activité de la precorrin-6x reductase défini çi-dessus (en présence de 0.5 U de precorrin-6x reductase dans le milieu d'incubation). Après l'étape de Mono Q, les fractions contenant de l'activité dihydroprecorrin-6x methylase sont totalement dépourvues d'activité precorrin-6x réductase. L'unité d'activité méthylase est définie comme la quantité d'enzyme nécessaire pour transférer 1 nmol de groupements méthyls sur le dihydroprecorrin-6x par h dans les conditions décrites çi-dessus.
b) purification de l'activité precorrin-6x réductase.
A partir du dosage décrit ci-dessus, la purification de la precorrin-6x réductase de Pseudomonas denitrificans est réalisée comme décrit çi-dessous.
Dans une expérience typique de purification, 100 g de cellules humides de la souche SC510 Rifr dans laquelle on a introduit le plasmide pXL253 (plasmide pKT230 sur lequel a été cloné, au site EcoRI, le fragment de 8.7 kb, figure 13) sont suspendues dans 200 ml de tampon Tris-HCl 0.1 M pH 7.7-1 mM EDTA (tampon A), et soniquées durant 15 min à 4°C. L'extrait brut est ensuite récupéré par centrifugation durant 1 h à 50 000 x g et passé en trois fois à travers une colonne de Sephadex G 25 équilibrée avec le tampon A. Les trois fractions exclues du gel sont poolées et ajustées à 1 1 avec le tampon A. Les protéines précipitant entre 25 et 40% de saturation en sulfate d'ammonium sont collectées par centrifugation et resuspendues dans 50 ml de tampon A et cette solution est désalée à travers une colonne de Sephadex G 25 equilibrée avec du tampon B (25 mM Tris-HCl-500 µM DTT-15% glycérol). La solution de protéines est ensuite injectée à 2.5 ml/min sur une colonne de Q Sepharose Fast Flow (Pharmacia) equilibrée avec du tampon B, et les protéines sont éluées avec un mélange tampon B-0.2 M KCL. Cette fraction est désalée sur une colonne de Sephadex G 25 équilibré avec du tampon C (50 mM Tris-HCl-500 µM DTT-15% glycérol). La solution protéique est ensuite fractionnée (100 mg de protéines à chaque chromatographie) sur une colonne Mono Q HR 10/10 (Pharmacia) à l'aide d'un gradient de 0 à 0.4 M KCL dans le tampon C, puis la fraction contenant l'activité est chromatographiée sur une colonne Phenyl-Superose HR 10/10 (Pharmacia) dans un gradient linéaire décroissant de sulfate d'ammonium (de 1 à 0 M). La fraction active est désalée et la precorrin-6x réductase est repurifiée sur une colonne Mono Q HR 5/5. Elle est éluée dans le tampon 50 mM Tris-HCl pH 8.1-500 µM DTT-15% glycérol, avec un gradient de 0 à 0.2 M de KCL. Pour parfaire la purification, la protéine est finalement chromatographiée sur une colonne Bio-Sil 250 (Bio-Rad) éluée avec 20 mM potassium phosphate-50 mM sodium sulfate, pH 6.8-500 µM DTT-15 % glycérol. Après cette étape, l'enzyme est pure à plus de 95%. Elle ne présente aucune protéine contaminante en PAGE-SDS, les protéines étant révélées au nitrate d'argent. Ce degré de pureté est confirmé par l'unicité de la séquence NH2-terminale. Son poids moléculaire dans cette technique est de 31 000. Les différentes étapes de purification de la precorrin-6x réductase, avec leur facteur de purification et leur rendement sont portées sur le tableau çi-dessous.

**Tableau:**

| Purification de la precorrin-6x reductase. | | | | | |
|---|---|---|---|---|---|
| Etape de purification | vol (ml) | Protéines (mg) | Activité spé. (u/mg de protéines) | Rendement | Facteur de purification¹ |
| Extrait brut | 270 | 9600 | 0.535 | - | - |
| S.A. 25 40% | 100 | 4160 | 1.14 | 92 | 2.1 |
| Q Sepharose | 150 | 1044 | 3.64 | 74 | 6.8 |
| Mono Q 10/10 | 55 | 67 | 24.5 | 32 | 46 |
| Phényl-superose | 10 | 2.2 | 325 | 14 . | 607 |
| Mono Q 5/5 | 2.5 | 0.082 | 5750 | 9.2 | 10750 |
| Bio-sil 250 | 1.0 | 0.055 | 7650 | 8.2 | 14300 |

c) Séquence NH2-terminale et séquences partielles internes de la precorrin-6x réductase de Pseudomonas denitrificans et identification du gène de structure de Pseudomonas denitrificans codant pour cette activité.
La séquence NH2-terminale de la precorrin-6x réductase de Pseudomonas denitrificans purifiée comme décrit ci dessus a été déterminée comme décrit précédemment. Six résidus ont été identifiés: Ala-Gly-Ser-Leu-Phe-Asp
De même, après digestion trypsique et séparation des fragment CLHP sur une colonne de phase inverse C-18, trois séquences internes ont été obtenues: Ile-Gly-Gly-Phe-Gly-Gly-Ala-Asp-Gly-Leu 
Arg-Pro-Glu-Trp-Val-Pro-Leu-Pro-Gly-Asp-Arg

Val-Phe-Leu-Ala-Ile-Gly
La séquence NH2-terminale de la protéine COBK (figure 16) correspond exactement à la séquence NH2-terminale de la precorrin-6x réductase, si ce n'est que dans la séquence présentée sur la figure 16, une méthionine précède la séquence peptidique déterminée par séquençage direct. Il en résulte que la méthionine aminoterminale est certainement excisée in vivo par la méthionine aminopeptidase (Ben Bassat et Bauer, 1987). De même, les trois séquences internes correspondent aux troisséquences 60 à 69, 112 à 122, et 143 à 148 de la protéine COBK. Le poids moléculaire de la precorrin-6x réductase purifiée est estimé par électrophorèse PAGE-SDS à 31 000. La protéine COBK a un poids moléculaire déduit de sa séquence de 28 000 (figure 16). Les correspondances entre les séquences NH2-terminales, internes, et les poids moléculaires indiquent clairement que la protéine COBK correspond à la precorrin-6x réductase. Le gène cobK est le gène de structure de la precorrin-6x réductase.
d) Réaction catalysée par la precorrin-6x réductase.
La réaction enzymatique de réduction du precorrin-6x est NADPH-dépendante de façon stricte chez P. denitrificans. Le NADPH ne peut pas être remplaçé par le NADH. Lorsque l'enzyme purifiée (ou une fraction active en cours de purification, ou même un extrait enzymatique brut) est incubée dans les conditions du dosage de l'activité , mais en l'absence de SAM et de dihydroprecorrin-6x méthylase, le produit de la réaction peut alors être purifié par CLHP dans le système décrit pour la purification du precorrin-6x (Cf exemple 6.1.4.d). Après désalage et estérification (méthanol sulfurique à 4%, 20°C, 24h, atmosphère argon), l'ester correspondant a une masse m/z = 1008. Le produit de la réaction catalysée par la precorrin-6x reductase est donc le dihydroprecorrin-6x, appelé aussi precorrin-6y.
**6.1.8. Identification de la protéine COBQ codée par le gène CobQ.**
a) Dosage de l'activité cobyric acid synthase.
Cet exemple illustre le dosage d'une activité enzymatique de la voie de biosynthèse des cobalamines qui n'a jusqu'à ce jour jamais été décrite. Il s'agit de la cobyric acid synthase. Cette enzyme catalyse l'amidation des fonctions acides carboxyliques périphériques en positions b, d, e, et g sur le noyau corrine (voir FIG. 19; PL. 68). Le donneur de groupements NH2 est la L-glutamine et chaque réaction d'amidation s'accompagne de la consommation d'une molécule d'ATP.
La fraction à doser est incubée à l'obscurité à 30°C pendant 60 min dans 250 µl de tampon 0.1 M Tris hydrochloride pH 7.5 contenant 1 mM DTT, 1 mM EDTA, 1 mM ATP, 2.5 mM MgCl2, 1 mM glutamine, 10 µM acide Ado-cobyrinique di- ou pentaamide. La réaction est stoppée en ajoutant 25 µl d'une solution aqueuse 0.1 M de cyanure de potassium. Après chauffage à 80°C durant 10 min et centrifugation à 3000 x g durant 10 min, les composés formés présents dans le surnageant sont analysés par CLHP. L'unité d'activité est définie comme la quantité d'enzyme necessaire pour générer 1 nmol de fonctions amides par h dans ces conditions.
Les acides 5'-deoxy-5'-adenosyl(Ado)-cobyriniques diamide et pentaamide sont isolés de cultures de la souche SC510 en milieu PS4, en utilisant la méthode dont le principe est décrit dans l'exemple 9.
b) Purification de la cobyric acid synthase.
A partir du dosage décrit à l'exemple 6.1.8 a), la purification de la cobyric acid synthase de Pseudomonas denitrificans est réalisée comme décrit çi-dessous.
Dans une expérience typique de purification, 6 g de cellules humides de SC510 Rifr dans laquelle on a introduit le plasmide pXL618 (voir exemple 4.5.2) sont soniqués dans 15 ml de tampon Tris-HCl 0.1 M pH 7.7, DTT 1 mM, EDTA 1mM. Après centrifugation (50 000 x g durant 1 h), l'extrait est amené à 20% de glycerol (vol/vol). A 8.5 ml de l'extrait brut (203.5 mg de protéines) sont ajoutés 24 ml de tampon Tris-HCl 10 mM, DTT 1 mM, glycerol 20%. La solution est injectée sur Mono Q HR 10/10 (Pharmacia) à 2 ml/min équilibrée avec du tampon Tris-HCl 50 mM pH 7.7, DTT 1 mM, glycérol 20%. Les protéines sont éluées avec un gradient linéaire de 0.5 M NaCl et les fractions actives regroupées et amenées à 1 mM EDTA. La solution est amenée à 0.85 M en sulfate d'ammonium et injectée sur une colonne Phenyl-Superose HR 5/5 (Pharmacia), équilibrée dans le tampon Tris-HCl pH 7.7, DTT 1 mM, sulfate d'ammonium 0.85 M et les protéines sont éluées avec un gradient linéaire décroissant de 0.85 M à 0 M de sulfate d'ammonium. Les fractions sont immédiatement amenées à 20% de glycérol. La fractionactive est concentré à 2.5 ml par ultrafiltration et chromatographiée sur une colonne PD 10 (Pharmacia) équilibrée et éluée avec du tampon Tris-HCl 50 mM pH 8.3, DTT 1 mM, glycérol 20% (vol/vol). La fraction protéique est recueillie et injectée sur une Mono Q HR 5/5 équilibrée avec le même tampon et les protéines sont éluées avec un gradient linéaire de 0.5 M NaCl. La chromatographie de perméation sur gel Bio-Sil 250 (Bio-Rad) en milieu tampon Tris-HCl 50 mM pH 7.5, DTT 1 mM, glycérol 20%, NaCl 0.1 M permet finalement d'obtenir une protéine pure à plus de 97%. Elle ne présente aucune protéine contaminante en PAGE-SDS. Cette pureté est confirmée par l'unicité de la séquence NH2-terminale. Son poids moléculaire dans cette technique est de 57 000. Les différentes étapes de purification de la cobyric acid synthase avec leur facteur de purification et leur rendement sont portées sur le tableau çi-dessous.

**Tableau:**

| Purification de la cobyric acid synthase. | | | | | |
|---|---|---|---|---|---|
| Etape de purification | vol (ml) | Protéines (mg) | Activité spé.U /mg A^{a} B^{b} | Rendement^{a} | Facteur de purification^{a} |
| Extrait brut | 8.5 | 203 | 114 / 118 | - | - |
| MonoQ 10/10 | 8.0 | 35.5 | 388 / 425 | 60 | 3.4 |
| Phényl-superose | 8.0 | 3.23 | 1988 / 2021 | 28 | 17 |
| MonoQ 5/5 | 1.0 | 1.20 | 4549 / 4085 | 24 | 40 |
| Bio-Sil 250 | 0.75 | 0.88 | 4992 / N.D. | 19 | 44 |

| | | | | | |
|---|---|---|---|---|---|
| a/ Avec l'acide Ado-cobyrinique a,c-diamide comme substrat | | | | | |
| b/ Avec l'acide Ado-cobyrinique pentaamide comme substrat ND = Non Déterminé | | | | | |

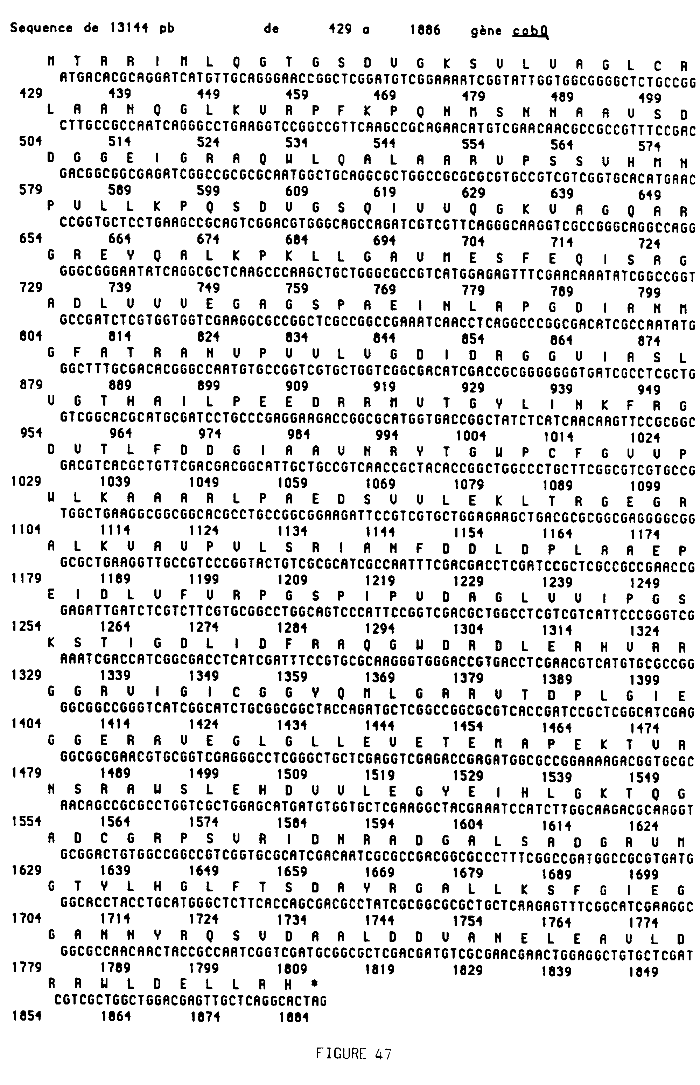

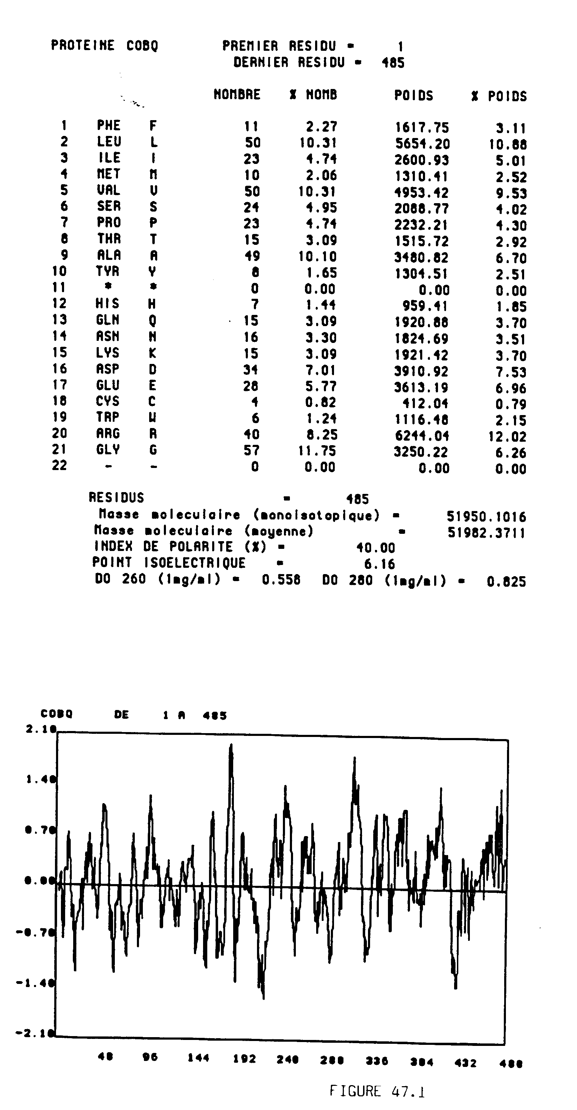

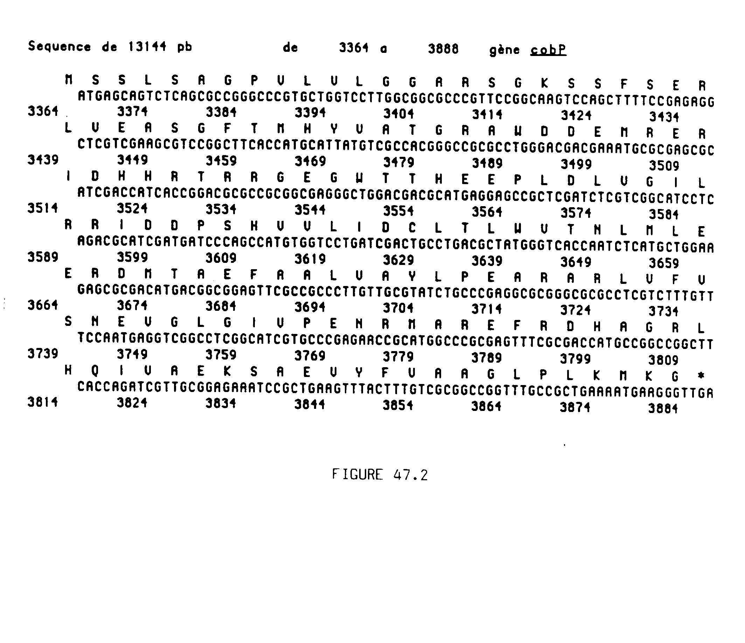

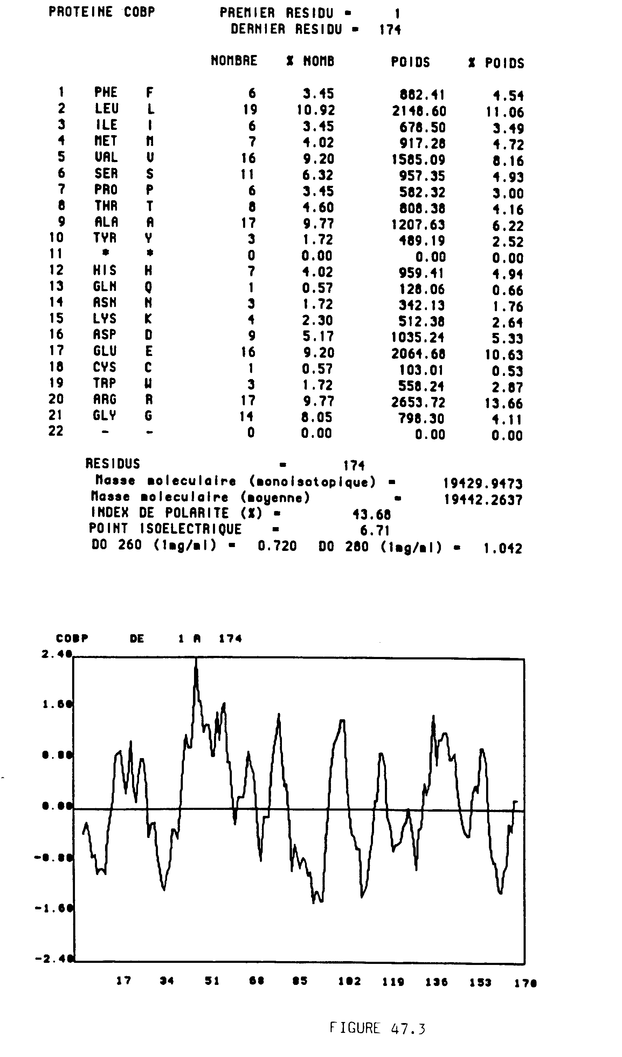

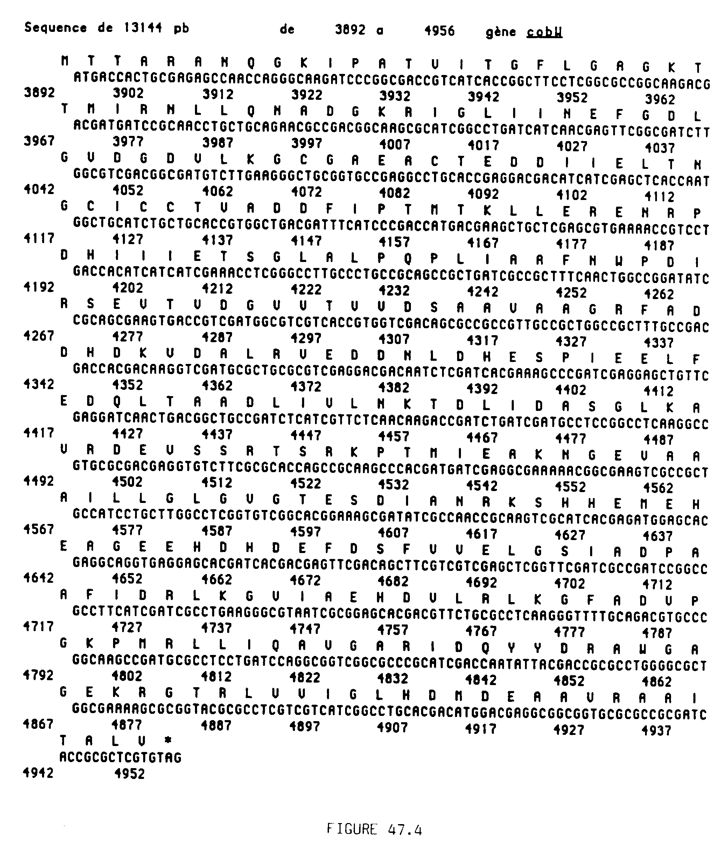

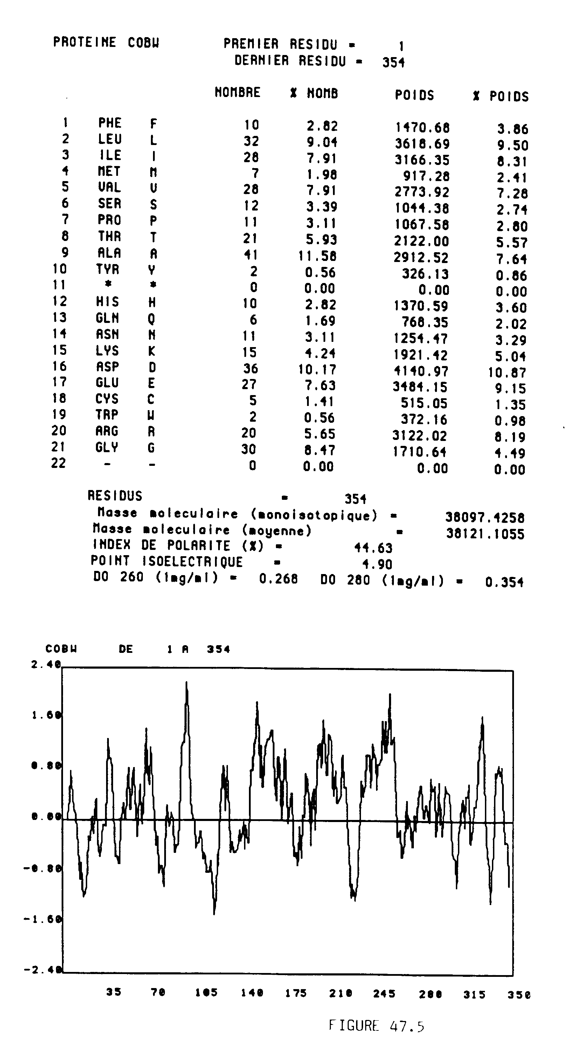

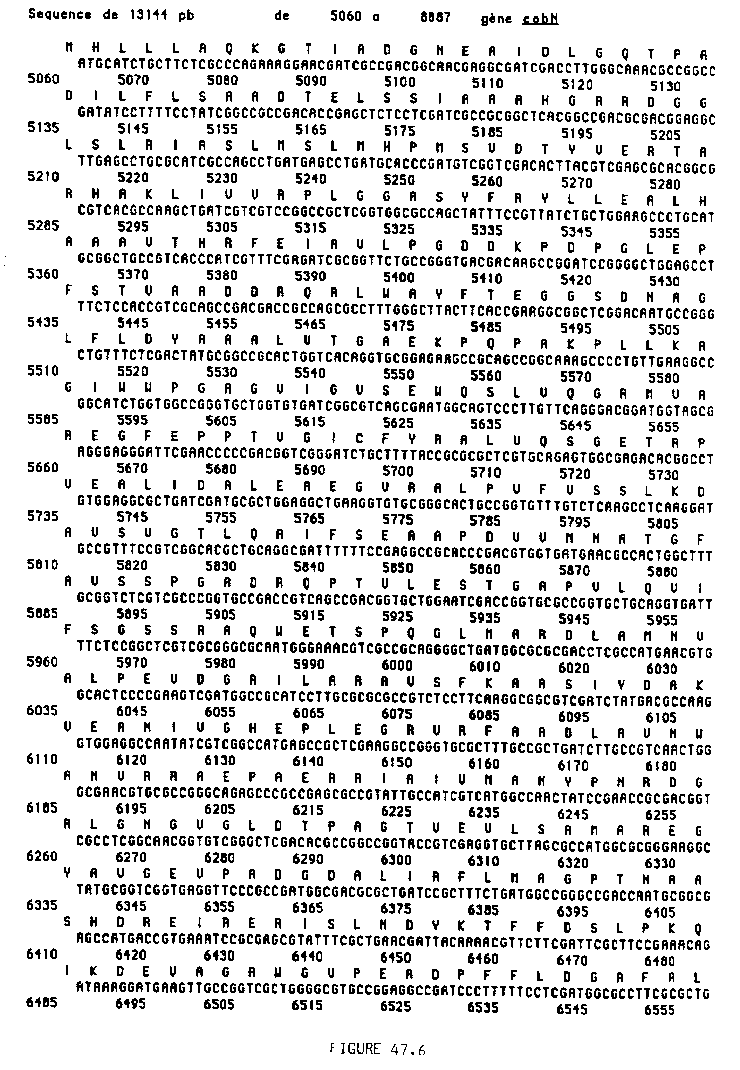

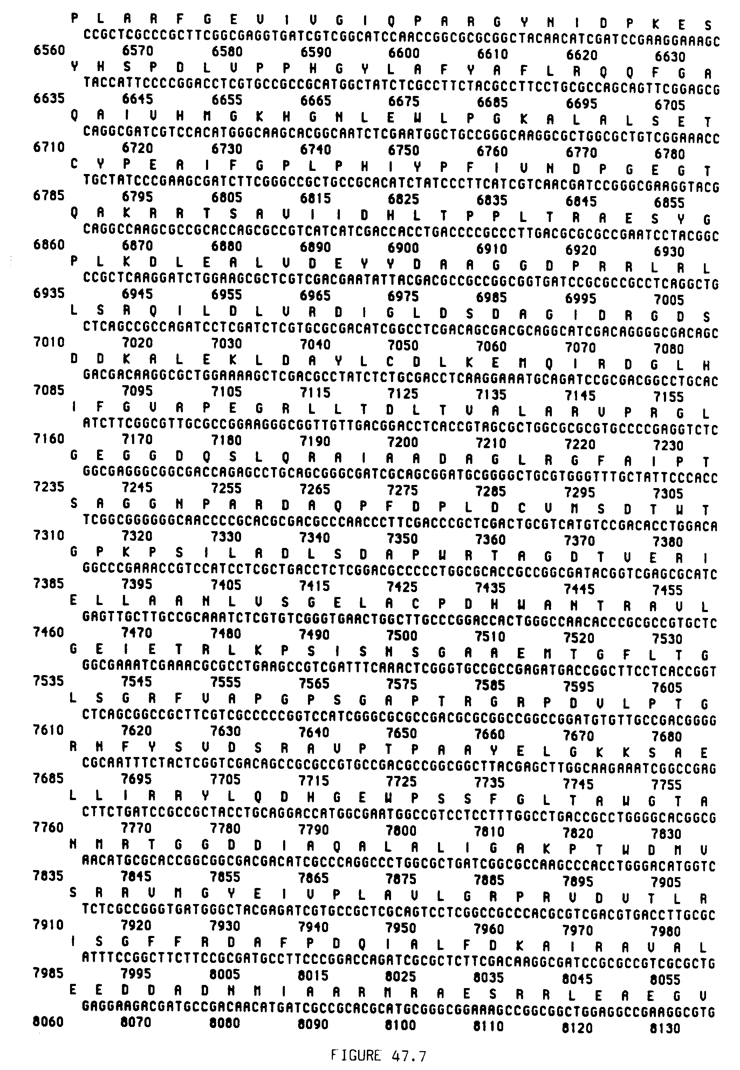

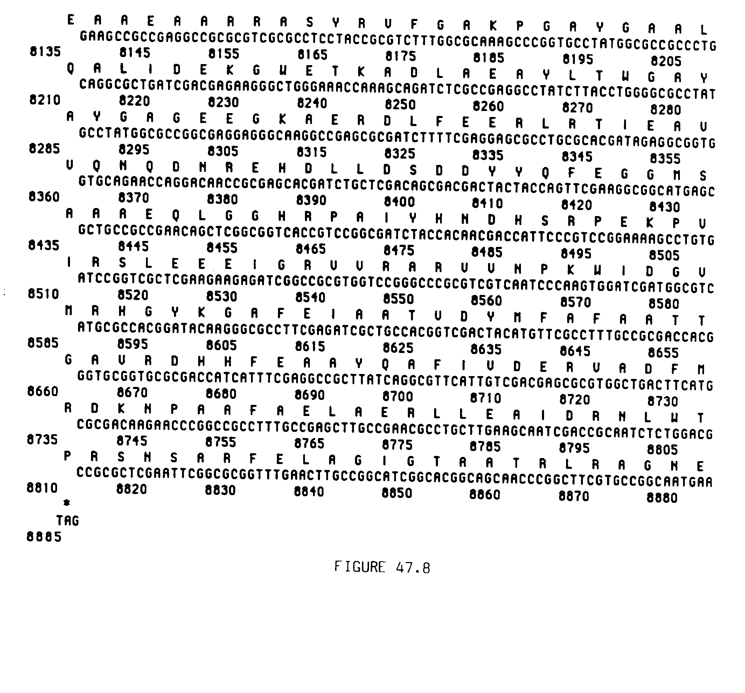

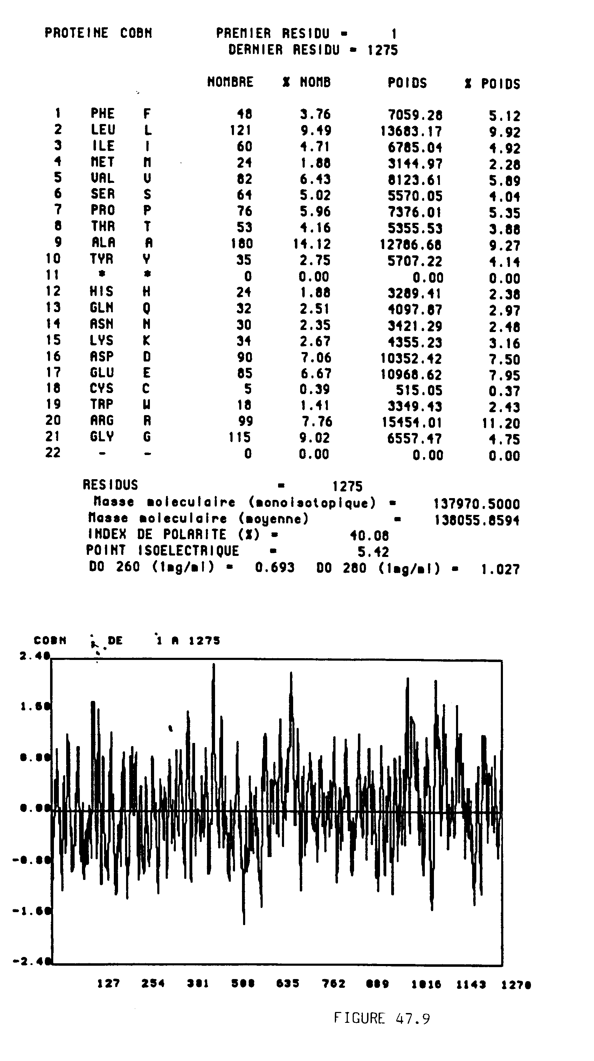

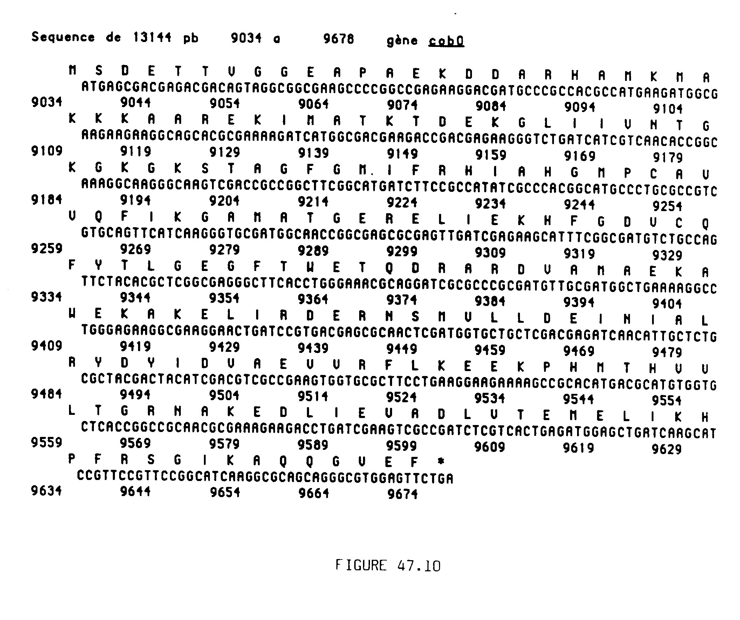

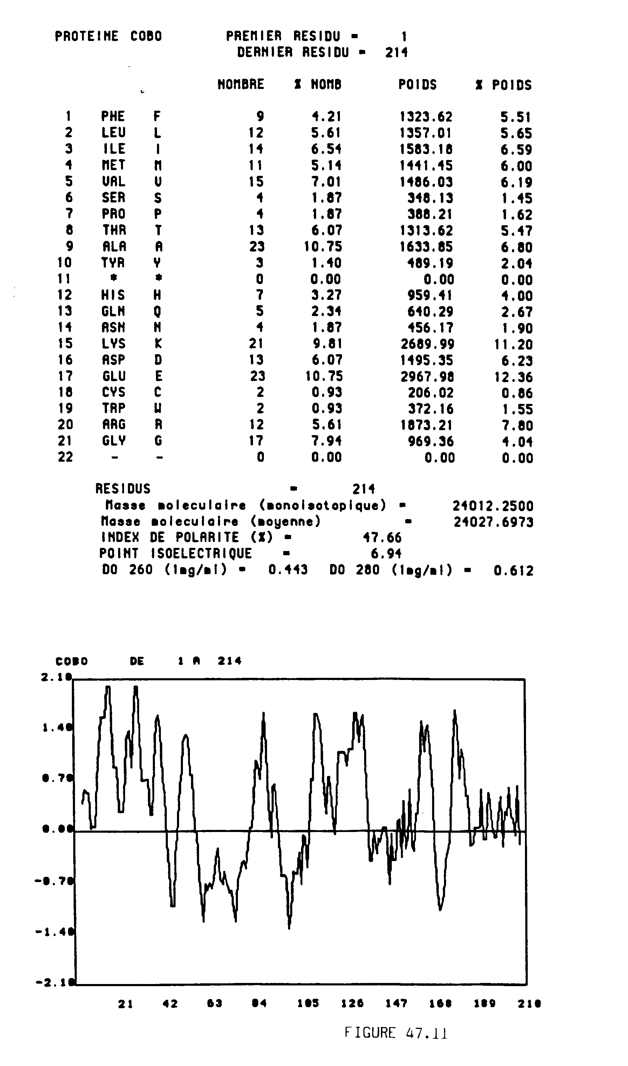

Le très haut degré de pureté de la protéine purifiée ainsi que la constance du ratio des activités d'amidation des acides cobyriniques diamide et pentaamide tout au long du procédé de purification de la protéine (voir tableau çi-dessus) indiquent sans ambiguité qu'une seule et même protéine est responsable des quatre activités d'amidation du noyau corrine aux positions b, d, e, et g.
c) Séquence NH2-terminale de la cobyric acid synthase de Pseudomonas denitrificans et identification du gène de structure de Pseudomonas denitrificans codant pour cette activité.
La séquence NH2-terminale de la cobyric acid synthase de Pseudomonas denitrificans a été déterminée comme décrit précédemment. Seize résidus ont été identifiés: Thr-Arg-Arg-Ile-Met-Leu-Gln-Gly-Thr-Gly-Ser-Asp-Val-Gly-Lys-Ser
La séquence NH2-terminale de la protéine COBQ (figure 47) correspond exactement à cette séquence, si ce n'est que dans la séquence présentée sur la figure 47, une méthionine précède la séquence peptidique déterminée par séquençage direct. Il en résulte que la méthionine aminoterminale est certainement excisée in vivo par la méthionine aminopeptidase (Ben Bassat et Bauer, 1987). Le poids moléculaire de la cobyric acid synthase purifiée est estimé par électrophorèse PAGE-SDS à 57 000. La protéine COBQ a un poids moléculaire déduit de sa séquence de 52 000 (figure 47). Les correspondances entre les séquences NH2-terminales et les poids moléculaires indiquent clairement que la protéine COBQ correspond à la cobyric acid synthase. Le gène cobQ est le gène de structure de la cobyric acid synthase.
**6.1.9. Identification de la protéine COBO codée par le gène cobO.**
a) dosage de l'activité cob(I)alamin adenosyltransferase (EC 2.5.1.17).
Cet exemple illustre le dosage d'une activité enzymatique directement liée à la voie de biosynthèse des cobalamines. Il s'agit de la cob(I)alamin adenosyltransferase (EC 2.5.1.17). Cette enzyme a été mise en évidence dans les cellules bactériennes (Ohta et al., 1976, Brady et al., 1962) et animales (Fenton et al. 1978). Elle a été purifié à partir de Clostridium tetanomorphum (Vitols et al., 1966). Les fractions contenant l'activité cob(I)alamin adenosyltransferase (environ 20 unités) sont incubées de façon anaérobie à 30°C durant 15 min à l'abri de la lumière dans 1 ml de tampon Tris/HCl 0.2 M pH 8.0 en présence de 5 mM DTT, 400 µM [8-14C] ATP (2.5 µCi/µmol) , 800 µM MnCl2, 50 µM hydroxocobalamine ou diaquacobinamide et 3 mg KBH4. La réaction est ensuite stoppée par chauffage à 80°C durant 10 min et, après centrifugation à 15000 x g durant 5 min, 200 µl de surnageant sont analysés par CLHP (Gimsing et al., 1986, Jacobsen et al., 1986).
L'unité d'activité enzymatique est définie comme la quantité d'enzyme nécessaire pour générer 1 nmol d'adénosylcorrinoide par min dans ces conditions.
b) Purification de l'activité cob(I)alamin adenosyltransferase.
A partir du dosage décrit à l'exemple 6.1.9 a), la purification de la cob(I)alamin adenosyltransferase de Pseudomonas denitrificans est réalisée comme décrit çi-dessous.
Dans une expérience typique de purification, 10 g de cellules humides de la souche SC510 Rifr dans laquelle le gène cobO a été amplifié sont suspendues dans 20 ml de tampon Tris-HCl 0.2 M pH 8.0 et soniquées durant 40 min à 4°C. L'extrait brut est ensuite récupéré par centrifugation durant 1 h à 50 000 x g et dessalé sur colonnes PD10 (Pharmacia) équilibrées avec du tampon 50 mM Tris-HCl pH 8.0, 5 mM DTT (tampon A). La solution protéique est ensuite fractionnée (280 mg de protéines à chaque chromatographie) sur une colonne Mono Q HR 10/10 (Pharmacia) à l'aide d'un gradient de 0 à 0.5 M KCL dans le tampon A, puis les fractions contenant l'activité sont poolées, concentrées par ultrafiltration et chromatographiées sur une colonne de Phenyl-Superose HR 10/10 (Pharmacia) dans un gradient linéaire décroissant de sulfate d'ammonium (de 1,7 à 0 M), la colonne étant équilibrée dans le tampon 0.1 M Tris-HCl pH 8.0, 5 mM DTT. Pour parfaire la purification, la protéine est finalement chromatographiée, après concentration par ultrafiltration, sur une colonne Bio-Sil 250 (Bio-Rad) éluée avec le tampon 50 mM Tris-HCl pH 7.5, 0.1 M NaCl, 5 mM DTT.
Après cette étape, l'enzyme est pure à plus de 95%. Elle ne présente aucune protéine contaminante en PAGE-SDS. Son poids moléculaire dans cette technique est de 28 000. Ce degré de pureté est confirmé par l'unicité de la séquence NH2-terminale. Les différentes étapes de purification de la cob(I)alamin adenosyltransferase, avec leur facteur de purification et leur rendement sont portées sur le tableau çi-dessous pour les deux substrats suivants: diaquacobinamide (a) et hydroxocobalamine (b). Ces résultats démontrent l'absence de spécificité de cette enzyme pour la nature du substrat corrinoide. D'autre part, tous les corrinoides de la voie de biosynthèse entre l'acide cobyrinique diamide et la B12 ont été isolés (Blanche et al., résultats non publiés) sous leur forme native, et se sont révélés être sous forme coenzyme. Ceci démontre que le substrat naturel de la cob(I)alamin adenosyltransferase est l'acide cobyrinique a,c-diamide.

**Tableau:**

| Purification de la cob(I)alamin adenosyltransferase. | | | | | |
|---|---|---|---|---|---|
| Etape de purification | vol (ml) | Protéines (mg) | Activité spé. U /mg A^{a} B^{b} | Rendement^{a} | Facteur de purification^{a} |
| Extrait brut ^{c} | 100 | 1400 | 5.4 / 3.4 | - | - |
| MonoQ 10/10 | 90 | 140 | 34.9 / 14.1 | 65 | 6.5 |
| | | | | | |
| Phényl-Superose | 30 | 15.9 | 84.5 / 49.5 | 18 | 16 |
| Bio-Sil 250 | 6.5 | 2.9 | 182.4/ 88.7 | 7.0 | 34 |

| | | | | | |
|---|---|---|---|---|---|
| c/ après dessalage sur PD10 | | | | | |

c) Séquence NH2-terminale de la cob(I)alamin adenosyltransferase de Pseudomonas denitrificans et identification du gène de structure de Pseudomonas denitrificans codant pour cette activité.
La séquence NH2-terminale de la cob(I)alamin adenosyltransferase de Pseudomonas denitrificans purifiée comme décrit à l'exemple 6.1.9 b) a été déterminée comme décrit précédemment. 13 résidus ont été identifiés: Ser-Asp-Glu-Thr-?-Val-Gly-Gly-Glu-Ala-Pro-Ala-Lys-Lys
La séquence NH2-terminale de la protéine COBO (figure 47) correspond exactement à la séquence NH2-terminale de la cob(I)alamin adenosyltransferase, si ce n'est que dans la séquence présentée sur la figure 47, une méthionine précède la séquence peptidique déterminée par séquençage direct. Il en résulte que la méthionine aminoterminale est certainement excisée in vivo par la méthionine aminopeptidase (Ben Bassat et Bauer, 1987). Le poids moléculaire de la cob(I)alamin adenosyltransferase purifiée est estimé par électrophorèse PAGE-SDS à 28 000. La protéine COBO a un poids moléculaire déduit de sa séquence de 24 000 (figure 47). Les correspondances entre les séquences NH2-terminales et les poids moléculaires indiquent clairement que la protéine COBO correspond à la cob(I)alamin adenosyltransferase . Le gène cobO est le gène de structure de la cob(I)alamin adenosyltransferase.
**6.1.10. Identification de la protéine COBN codée par le gène cobN.**
a) Mise en évidence de l'activité de transformation de l'acide hydrogenobyrinique a,c-diamide en acide cobyrinique a,c-diamide.
Cet exemple illustre la mise en évidence d'une activité enzymatique directement liée à la voie de biosynthèse des cobalamines qui n'a jusqu'à ce jour jamais été décrite. Il s'agit de l'activité de transformation de l'acide hydrogenobyrinique a,c-diamide en acide cobyrinique a,c-diamide.
Cette activité est mise en évidence, entre autres, par l'expérience typique suivante. Un extrait brut de la souche SC510 Rifr est obtenu par sonication de 10 g de cellules humides dans 20 ml de tampon Tris/HCl 0.2 M pH 8.0, puis élimination des débris cellulaires par centrifugation durant 1 h à 50 000 x g. 1000 mg de protéines de cet extrait sont incubés 1 h à 30 °C avec de l'acide hydrogénobyrinique diamide marqué au carbone 14 (32 nmol; 50 µCi/µmol) dans 40 ml de tampon Tris/HCl 0.2 M pH 8.0 contenant 7 mM ATP, 200 µM CoCl2 . La réaction est arrêtée par addition de 7.5 ml KH2PO4 1M et 6 ml KCN 0.3 M suivi du chauffage pendant 10 min à 80 °C. Après centrifugation à 15000 x g durant 15 min, l'analyse CLHP du surnageant montre: (1) la formation durant l'incubation de 19.2 nmol d'acide cobyrinique a,c-diamide ayant la même radioactivité spécifique que l'acide hydrogénobyrinique a,c-diamide de départ et (2) la disparition d'une quantité correspondante de ce dernier. Pour confirmer qu'il s'agit bien d'acide cobyrinique a,c-diamide, le produit est purifié par CLHP puis estérifié dans du méthanol contenant 5% d'acide sulfurique (18h, 20°C). L'authenticité de l'ester pentaméthylique de l'acide cobyrinique a,c-diamide produit est démontré par TLC (par rapport à un échantillon de référence) et spectrométrie de masse. Notons que dans des conditions similaires d'incubation où le marquage radioactif est introduit non pas dans l'acide hydrogénobyrinique a,c-diamide mais dans le cobalt (en utilisant le cobalt 57), de l'acide cobyrinique a,c-diamide marqué au cobalt 57 est biosynthétisé et les mêmes conclusions ont pu être tirées. L'acide hydrogénobyrinique a,c-diamide marqué au carbone 14 est obtenu de la manière suivante: l'acide hydrogénobyrinique est biosynthétisé in vitro en utilisant le [methyl-14C]SAM puis transformé en acide hydrogénobyrinique a,c-diamide et purifié par CLHP comme décrit à l'exemple 6.1.2.
Cette étude démontre que l'insertion du cobalt a lieu au niveau de l'acide hydrogénobyrinique a,c-diamide chez P.denitrificans. Dans les conditions décrites, l'acide hydrogénobyrinique n'est pas substrat de la chélation enzymatique par le cobalt.
b) Dosage et purification d'une protéine de la souche SC510 Rifr impliquée dans la transformation de l'acide hydrogenobyrinique a,c-diamide en acide cobyrinique a,c-diamide.
La fraction à doser (0.5 à 2 unités) est incubée pendant 60 min à 30 °C avec 50 µl d'extrait brut de la souche SC510 Rifr obtenu comme décrit ci-dessus, 7 mM ATP, 200 µM CoCl2, 7 µM acide hydrogénobyrinique a,c-diamide marqué au carbone 14 (50 µCi/µmol) dans 400 µl de tampon Tris/HCL 0.1 M pH 8.0. La réaction est arrêtée par addition de 75 pl KH2PO4 1 M et 60 µl KCN 0.3 M, puis chauffage pendant 10 min à 80°C. Après centrifugation à 15000 x g durant 15 min, le surnageant est analysé par CLHP, pour quantifier l'acide cobyrinique a,c-diamide formé (Cf exemple 9). L'unité d'activité enzymatique est définie comme la quantité d'enzyme nécessaire pour générer 1 nmol d'acide cobyrinique a,c-diamide par h dans ces conditions. Dans ces conditions, il apparaît que les extraits de la souche SC510 Rifr dans laquelle on a introduit le plasmide pXL1909 (voir exemple 4.5.2) présentent une activité entre 20 et 50 fois plus élevée que les extraits de la souche SC510 Rifr. C'est sur cette base qu'est purifiée une protéine, seule responsable de cette amplification d'activité.
Dans une expérience typique de purification, 10 g de cellules humides de la souche SC510 Rifr dans laquelle on a introduit le plasmide pXL1909 sont suspendues dans 20 ml de tampon Tris/HCl 0.2 M pH 8.0 et soniquées durant 30 min à 4°C. L'extrait brut est ensuite récupéré par centrifugation durant 1 h à 50 000 x g et dessalé sur colonnes PD10 (Pharmacia) équilibrées avec du tampon 0.1 M Tris/HCl pH 8.0 (tampon A). La solution protéique est ensuite fractionnée (213 mg de protéines à chaque chromatographie) sur une colonne Mono Q HR 10/10 (Pharmacia) à l'aide d'un gradient de 0 à 0.5 M KCL dans le tampon A, puis les fractions contenant l'activité sont poolées, concentrées par ultrafiltration, dessalées sur colonnes PD10 (Pharmacia) équilibrées avec le tampon 0.1 M Tris/HCl pH 7.2 (tampon B), et chromatographiées sur une colonne Mono Q HR 10/10 (Pharmacia) à l'aide d'un gradient de 0 à 0.5 M KCL dans le tampon B. Les fractions contenant l'activité sont poolées, concentrées par ultrafiltration, dessalées sur colonnes PD10 (Pharmacia) équilibrées avec le tampon B, et chromatographiées sur une colonne Mono Q HR 5/5 (Pharmacia) à l'aide d'un gradient de 0 à 0.5 M KCL dans le tampon B. Pour parfaire la purification, la protéine est finalement chromatographiée sur une colonne Bio-Sil 250 (Bio-Rad) éluée avec 20 mM potassium phosphate-50 mM sodium sulfate, pH 6.8. Après cette étape, l'enzyme est pure à plus de 95%. Elle ne présente aucune protéine contaminante en PAGE-SDS. Son poids moléculaire dans cette technique est de 135 000. Ce degré de pureté est confirmé par l'unicité de la séquence NH2-terminale. Les différentes étapes de purification de la protéine de la souche SC510 Rifr impliquée dans la transformation de l'acide hydrogenobyrinique a,c-diamide en acide cobyrinique a,c-diamide, avec leur facteur de purification et leur rendement sont portées sur le tableau çi-dessous.

**Tableau:**

| Purification d'une protéine de la souche SC510 Rifr impliquée dans la transformation de l'acide hydrogenobyrinique a,c-diamide en acide cobyrinique a,c-diamide. | | | | | |
|---|---|---|---|---|---|
| Etape de purification | vol (ml) | Protéines (mg) | Activité spé. (u/mg de protéines) | Rendement | Facteur de purification¹ |
| Extrait brut | 31.5 | 1278 | 0.23 | - | - |
| MonoQ 10/10 | 44 | 79.2 | 2.4 | 64 | 10 |
| MonoQ 10/10 | 21 | 33.6 | 6.8 | 78 | 30 |
| MonoQ 5/5 | 3 | 6.6 | 16.0 | 36 | 70 |
| Bio-Sil 250 | 1.8 | 5.9 | 16.3 | 33 | 71 |

c) Séquence NH2-terminale de la protéine impliquée dans la transformation de l'acide hydrogenobyrinique a,c-diamide en acide cobyrinique a,c-diamide de Pseudomonas denitrificans et identification du gène de structure de Pseudomonas denitrificans codant pour cette activité.
La séquence NH2-terminale de cette protéine purifiée comme décrit à l'exemple 6.1.10b) a été déterminée comme décrit précédemment. Six résidus ont été identifiés: His-Leu-Leu-Leu-Ala-Gln
La séquence NH2-terminale de la protéine COBN (figure 47) correspond exactement à la séquence NH2-terminale de la protéine purifiée, si ce n'est que dans la séquence présentée sur la figure 47, une méthionine précède la séquence peptidique déterminée par séquençage direct. Il en résulte que la méthionine aminoterminale est certainement excisée in vivo par la méthionine aminopeptidase (Ben Bassat et Bauer, 1987). Le poids moléculaire de la protéine purifiée est estimé par électrophorèse PAGE-SDS à 135 000. La protéine COBN a un poids moléculaire déduit de sa séquence de 138 000 (figure 47). Les correspondances entre les séquences NH2-terminales et les poids moléculaires indiquent clairement que la protéine COBN correspond à la protéine impliquée dans la transformation de l'acide hydrogenobyrinique a,c-diamide en acide cobyrinique a,c-diamide. Le gène cobN est donc le gène de structure de cette protéine.
**6.1.11. Identification de la protéine COBP codée par le gène CobP.**
a) Dosage de l'activité cobinamide kinase.
   Cet exemple illustre le dosage d'une activité enzymatique de la voie de biosynthèse des cobalamines qui n'a jusqu'à ce jour jamais été étudiée. Il s'agit de l'activité cobinamide kinase. Elle catalyse la phosphorylation ATP-dépendante du groupement hydroxyl du résidu (R)-1-amino-2-propanol du Ado-cobinamide pour générer le cobinamide phosphate.
   La fraction à doser est incubée à l'obscurité à 30°C pendant 60 min dans 500 µl de tampon 0.1 M Tris-HCl pH 8.8 contenant 1 mM EDTA, 1 mM ATP, 2.5 mM MgCl2, et 16 µM Ado-cobinamide (Blanche et al., 1989). La réaction est stoppée en ajoutant 500 µl d'une solution aqueuse 20 mM de cyanure de potassium. Après chauffage à 80°C durant 10 min et centrifugation à 5000 x g durant 10 min, le cobinamide phosphate formé présent dans le surnageant est dosé par CLHP (Cf exemple 9) en utilisant le gradient linéaire simplifié suivant: de 25% à 30% de B dans A en 15 min, puis 30% à 100% de B en 12 min, et 3 min à 100% de B.
   L'unité d'activité est définie comme la quantité d'enzyme necessaire pour générer 1 nmol de cobinamide phosphate à partir du cobinamide par h dans ces conditions.
b) Dosage de l'activité cobinamide phosphate guanylyltransferase.
   Cet exemple illustre le dosage d'une activité enzymatique de la voie de biosynthèse des cobalamines qui n'a jusqu'à ce jour jamais été étudiée. Il s'agit de l'activité cobinamide phosphate guanylyltransferase. Elle catalyse l'addition de la partie GMP d'une molécule de GTP sur l'Ado-cobinamide phosphate, générant ainsi une molécule de GDP-cobinamide et libérant une molécule de pyrophosphate.
   Cette activité est dosée dans les mêmes conditions que la cobinamide kinase si ce n'est que l'Ado-cobinamide phosphate (16 µM)(Blanche et al., 1989) et le GTP (2 mM) remplacent l'Ado-cobinamide et l'ATP respectivement, durant l'incubation.
   L'unité d'activité est définie comme la quantité d'enzyme necessaire pour générer 1 nmol de GDP-cobinamide à partir du cobinamide phosphate par h dans ces conditions.
c) Purification de la cobinamide kinase.
   A partir du dosage décrit à l'exemple 6.1.11a), la purification de la cobinamide kinase de Pseudomonas denitrificans est réalisée comme décrit çi-dessous.
   Dans une expérience typique de purification, 5 g de cellules humides de SC510 Rifr dans laquelle on a introduit le plasmide pXL623 (voir exemple 4.5.2) sont soniqués dans 20 ml de tampon Tris 0.1 M pH 7.6 (tampon A). Après centrifugation (50 000 x g durant 1 h) et dialyse 4 h contre le tampon A, 4.5 ml du rétentat sont injectés sur Mono Q HR 10/10 (Pharmacia), équilibrée avec le tampon A. Les protéines sont éluées avec un gradient linéaire de 0.4 M NaCl et les fractions actives regroupées sont passées à travers une colonne de PD-10 (Pharmacia) équilibrée dans du Tris-HCl 30 mM-phosphate de potassium 5 mM-chlorure de calcium 5 µM pH 7.6 (tampon B). La solution protéique est fractionnée sur une colonne Bio-Gel HPHT (Bio-Rad), equilibrée dans le tampon B et éluée avec un gradient de 5 à 350 mM de phosphate de potassium. Les fractions actives sont regroupées et amenées à 500 mM en sulfate d'ammonium, puis fractionnées sur une Phényl-Superose HR 5/5 (Pharmacia), éluée avec un gradient décroissant de sulfate d'ammonium. la fraction contenant l'activité est finalement repurifiée sur une colonne Mono Q HR 5/5 dans le Tris-HCl à pH 7.3. Après cette étape, la protéine est pure à plus de 97%. Elle ne présente aucune protéine contaminante en PAGE-SDS. Cette pureté est confirmée par l'unicité de la séquence NH2-terminale. Son poids moléculaire dans cette technique est de 20 000. Les différentes étapes de purification de la cobinamide kinase avec leur facteur de purification et leur rendement sont portées sur le tableau A présenté ci-après.
   Les fractions contenant l'activité cobinamide kinase présentent aussi l'activité cobinamide phosphate guanylyltransferase. D'autre part, comme le montrent les résultats présentés dans le tableau çi-dessus, le ratio de ces deux activités reste constant dans les fractions tout au long de la purification. Enfin, la protéine purifiée présente un très haut degré de pureté dépassant les 97%. L'ensemble de ces résultats indiquent donc sans ambiguité qu'une seule et même protéine est responsable des deux activités successives que sont la cobinamide kinase et la cobinamide phosphate guanylyltransferase de la voie de biosynthèse des cobalamines chez Pseudomonas denitrificans.
d) Séquence NH2-terminale de la cobinamide kinase-cobinamide phosphate guanylyltransferase de Pseudomonas denitrificans et identification du gène de structure de Pseudomonas denitrificans codant pour cette activité.
   La séquence NH2-terminale de la cobinamide kinase-cobinamide phosphate guanylyltransferase de Pseudomonas denitrificans a été déterminée comme décrit précédemment. Dix résidus ont été identifiés: 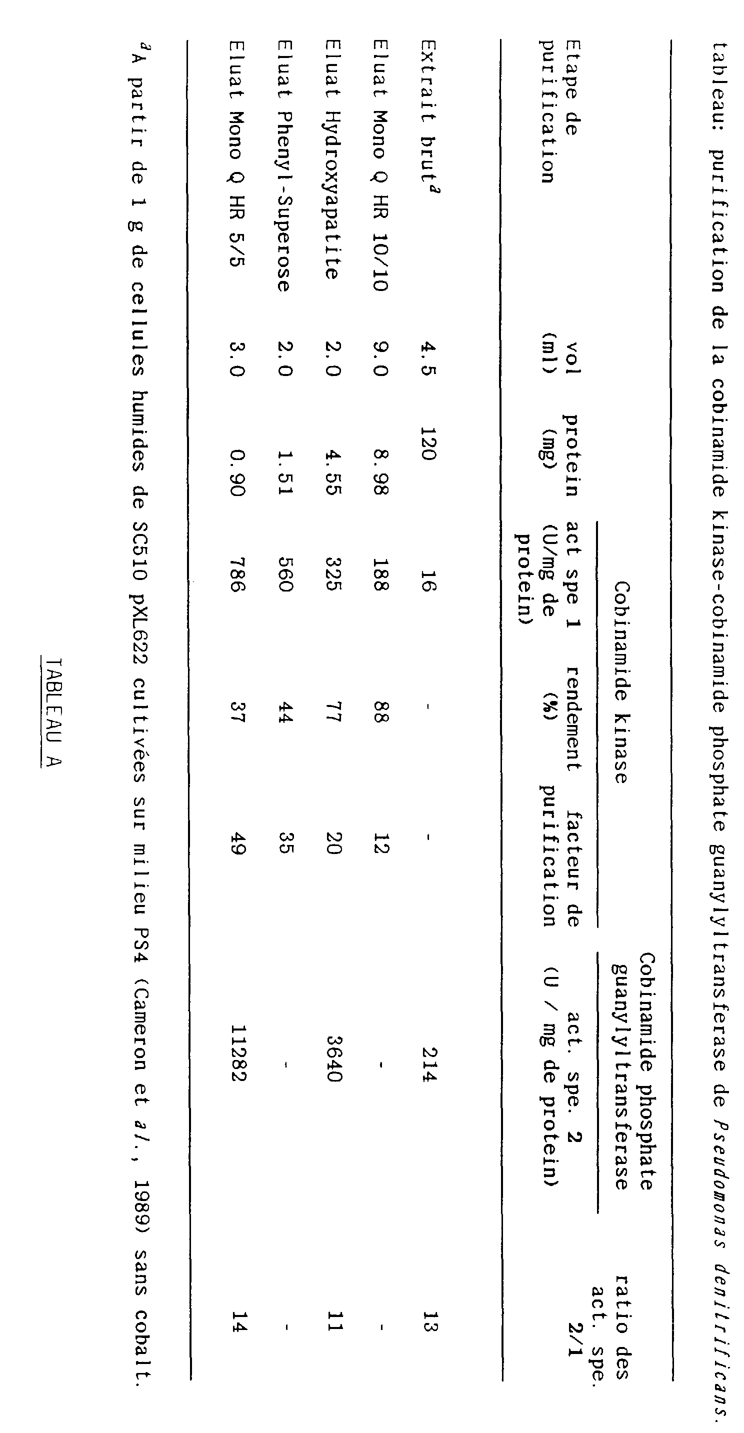 Ser-Ser-Leu-Ser-Ala-Gly-Pro-Val-Leu-Val
   La séquence NH2-terminale de la protéine COBP (figure 47) correspond exactement à cette séquence, si ce n'est que dans la séquence présentée sur la figure 47, une méthionine précède la séquence peptidique déterminée par séquençage direct. Il en résulte que la méthionine aminoterminale est certainement excisée in vivo par la méthionine aminopeptidase (Ben Bassat et Bauer, 1987). Le poids moléculaire de la cobinamide kinase-cobinamide phosphate guanylyltransferase purifiée est estimé par électrophorèse PAGE-SDS à 20 000. La protéine COBP a un poids moléculaire déduit de sa séquence de 19 500 (figure 47). Les correspondances entre les séquences NH2-terminales et les poids moléculaires indiquent clairement que la protéine COBP correspond à la cobinamide kinase-cobinamide phosphate guanylyltransferase. Le gène cobP est le gène de structure de la cobinamide kinase-cobinamide phosphate guanylyltransferase.

**6.2 - Détermination des propriétés de protéines COB par mesures d'intermédiaires de biosynthèse accumulés**
Cet exemple illustre comment il est possible d'attribuer à une protéine COB de Pseudomonas denitrificans une activité enzymatique. Cette activité est attribuée d'après des données obtenues concernant les intermédiaires de biosynthèse accumulés chez le ou les mutants Cob bloqués dans l'étape en question. En effet si un mutant accumule un intermédiaire de biosynthèse, il est très probable que ce mutant est bloqué dans l'étape qui a pour substrat l'intermédiaire en question.
**6.2.1. Propriétés des protéines COBC et COBD**
Les mutants Cob G643 (Agrobacterium tumefaciens) et G572 (Pseudomonas putida) déjà décrits dans les exemples 1 et 4 sont bloqués dans l'étape correspondant à la protéine COBC. En effet ces deux mutants ne sont pas complémentés par les insertions inactivantes de transposons Tn5 qui se trouvent dans le gène cobC. Les deux souches G643 et G572, ainsi que les souches parentes non mutées [C58-C9 Rif^{r} et KT 2440 Rif^{r} (Cameron et al., 1989)] ont été cultivées en milieu PS4' pour les A.tumefaciens, et PS4' pour les P.putida (PS4' et PS4" correspondent à du milieu PS4 contenant respectivement 100 et 1000 fois moins de cobalt que le PS4 décrit précédemment) 3 jours comme cela est décrit ci-dessus. Du ⁵⁷CoCl₂ a été ajouté aux cultures (2,5 µCi/0,1 µm pour une culture de 25 ml). Les corrinoïdes intracellulaires ont été isolés sous leur forme native et identifiés par leur comportement CLHP. Les souches parentes n'accumulent pas de corrinoïdes autres que du coenzyme B₁₂. Les deux mutants, G643 et G572, accumulent de l'acide cobyrique adénosylé dans des proportions respectives de 11 % et 6 %. Ces proportions en % sont calculées par rapport au niveau de coenzyme B₁₂ synthétisé par la souche parente. Outre l'acide cobyrique, le mutant G643 accumule de l'acide cobyrinique pentaamide dans une proportion de 2 %; l'acide cobyrinique pentaamide est l'intermédiaire qui précède l'acide cobyrique. L'étude de ces mutants fait ressortir qu'ils sont bloqués après l'acide cobyrique. Tous ces mutants Cob sont soit bloqués entre l'uro'gen III et le cobinamide, soit entre le cobinamide et les cobalamines. Les mutants G643 et G572 sont bloqués entre l'uro'gen III et le cobinamide. Or si ces mutants sont bloqués avant le cobinamide et accumulent tous les deux de l'acide cobyrique, les protéines pour lesquelles ils codent ne peuvent intervenir que dans l'étape enzymatique (appelée cobinamide synthase) qui catalyse l'amidation de l'acide cobyrique par un résidu aminopropanol pour donner le cobinamide; elles peuvent aussi éventuellement intervenir dans la synthèse du substrat de la réaction qui apporte l'aminopropanol, si ce n'est l'aminopropanol lui-même. Le gène cobC code pour une protéine qui est soit la cobinamide synthase, soit une de ses sous-unités.
Le mutant Cob G634 d'Agrobacterium tumefaciens qui est bloqué dans l'étape correspondant au gène cobD a été analysé de la même manière. Ce mutant n'est pas complémenté par les insertions inactivationnelles dans le gène cobD (exemple 4.1). Le seul corrinoïde intracellulaire trouvé chez ce mutant est de l'acide cobyrique adénosylé. Comme les mutants précédents, ce mutant code pour une protéine intervenant dans la transformation de l'acide cobyrique en cobinamide ou bien éventuellement dans la synthèse de l'autre substrat de la réaction.
Ces deux gènes différents (cobC et cobD) codent pour deux protéines qui interviennent dans la même étape.
**6.2.2. Propriétés des protéines COBF à COBM**
Les mutants d'Agrobacterium tumefaciens déjà décrits dont on connaît, d'après l'étude décrite à l'exemple 4.2, dans quels gènes chacun d'entre eux est bloqué, ont été étudiés. Ce sont les mutants : G612 (cobF), G615 (cobG), G616 (cobH), G613 (cobI), G611 (cobJ), G620 (cobK), G638 (cobL) et G609 (cobM) ; nous avons figuré entre parenthèses le gène de Pseudomonas denitrificans responsable de la complémentation de ces mutants (exemple 5) qui correspond donc au gène muté chez ce mutant. Ces mutants ont été cultivés en milieu PS4 comme cela est décrit précédemment avec du cobalt marqué. Après quatre jours d'incubation, les mutants ont été analysés pour leur contenu intracellulaire en corrinoïdes et descobaltocorrinoïdes (Cf exemples 6.1.2 et 9).

**Tableau :**

| Intermédiaires accumulés par des mutants d'Agrobacterium tumefaciens bloqués dans les gènes du fragment 8,7 kb de Pseudomonas denitrificans | | | | | |
|---|---|---|---|---|---|
| 'p Souches | Descobaltocorrinoïdes intracellulaires en % ¹ | | | Corrinoïdes intracellulaires en % des cobalamines | Gène muté |
| | HAB | HABM | HABD | | |
| C58-C9* | 100 | 100 | 100 | coenzyme B₁₂100 | - |
| G612 | < 5 | < 5 | 64 | cobinamide 2,2 coenzyme B₁₂34 | cobF |
| G615 | < 5 | < 5 | 84 | coenzyme B₁₂17 | cobG |
| G616 | 35 | < 10 | < 10 | coenzyme B₁₂13 | cobH |
| G613 | < 5 | < 5 | 57 | coenzyme B₁₂ <1 | cobI |
| G611 | < 5 | < 5 | 65 | coenzyme B₁₂ <1 | cobJ |
| G620 | 12 | < 5 | < 10 | coenzyme B₁₂<1 | cobK |
| G638 | < 5 | < 5 | 47 | coenzyme B₁₂ <1 | cobL |
| G609 | < 5 | < 5 | 33 | coenzyme B₁₂ <1 | cobM |
| HAB : acide hydrogénobyrinique | | | | | |
| HABM : acide hydrogénobyrinique monoamide | | | | | |
| HABD : acide hydrogénobyrinique diamide | | | | | |

| | | | | | |
|---|---|---|---|---|---|
| * en fait il s'agit de la souche C58-C9 Rif^{r}Nal^{r} déjà décrite (Cameron et al., 1989) | | | | | |
| ¹les valeurs sont exprimées en % des mêmes intermédiaires accumulés chez la souche parente non mutée C58-C9 Rif^{r}Nal^{r}. | | | | | |

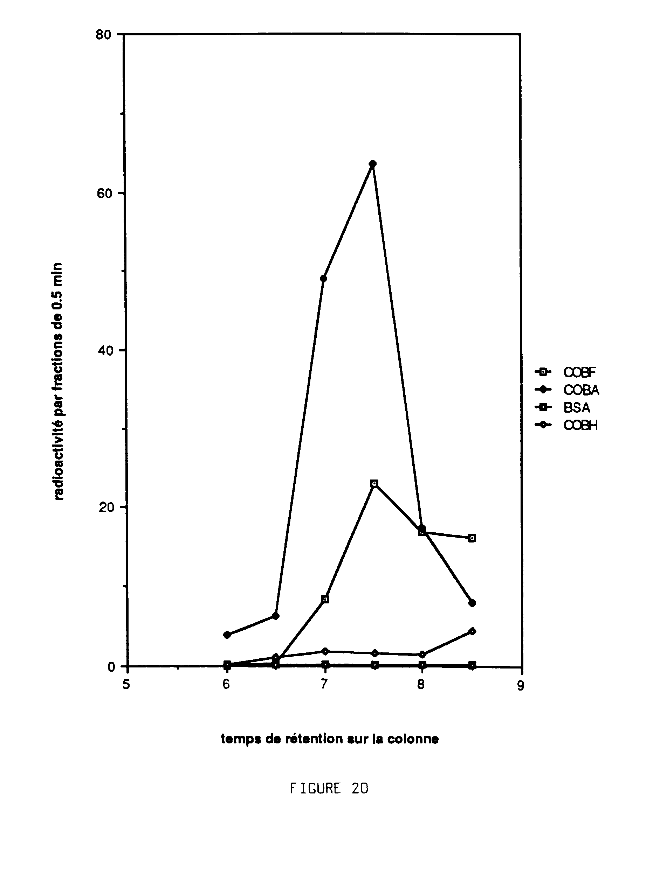

Ces résultats montrent que tous les mutants n'accumulent aucun corrinoïde (à l'exception du mutant inactivé dans le gène cobF, G612, qui lui, accumule du cobinamide mais à un niveau faible équivalent à 2,2 % des cobalamines synthétisées par la souche non mutée). Toutefois, certains mutants (G612, G615 et G616) ont des niveaux de cobalamines qui représentent plus de 10 % du niveau de cobalamines de la souche parente. Il est probable que tous ces mutants sont bloqués au moins avant l'acide cobyrinique diamide. Tous ces mutants accumulent de l'acide hydrogénobyrinique et de l'acide hydrogénobyrinique diamide en quantités inférieures à la souche non mutée; ils sont donc très probablement bloqués avant l'acide hydrogénobyrinique. Nous pouvons conclure que tous les gènes cobF à cobG codent pour des protéines qui interviennent avant l'acide hydrogénobyrinique. On sait que le mutant G613 est muté dans le gène cobI qui code pour la SP₂MT, intervenant bien avant l'acide hydrogénobyrinique. Pour ce mutant, les résultats du présent exemple, concernant l'accumulation d'intermédiaires, sont en parfait accord avec l'étape inactivée chez ce mutant, à savoir que ce mutant n'accumule aucun intermédiaire après l'acide hydrogénobyrinique à un niveau supérieur à ce que l'on observe avec la souche non mutée. Ce résultat est pour les gènes cobF, cobJ, cobL et cobM cohérent avec ceux de l'exemple 6.4 où il est proposé que ces gènes codent pour des protéines qui catalysent des transferts de méthyl SAM dépendants et donc qui interviennent avant l'acide hydrogénobyrinique. A l'exception de cobI qui est le gène de structure de la SP₂MT ces gènes interviennent après le précorrine-3. En effet comme ce ne sont ni les gènes de structure de la SUMT et de la SP₂MT ils interviennent forcément après, c'est-à-dire après le précorrine-3 (tous les gènes cob décrits dans la présente invention interviennent entre l'uro'gen III et les cobalamines). Ces gènes cobF à cobH et cobJ à cobM codent pour des enzymes qui interviennent entre le précorrine-3 et l'acide hydrogénobyrinique.
**6.2.3. Propriétés des protéines COBS et COBT**
Le mutant G2035 décrit dans les exemples 1 et 4.3 est bloqué dans l'étape correspondant à la protéine COBS. Le mutant G2037 décrit dans l'exemple 1 est bloqué dans l'étape correspondant à la protéine COBT. Ces souches ainsi que la souche parente (Agrobacterium tumefaciens C58C9Rifr) sont cultivées en milieu PS4' (il s'agit du milieu PS4 où la concentration en chlorure de cobalt est 100 fois plus faible que dans le milieu PS4) en présence de cobalt radioactif 57CoCl2 pendant 3 jours, leur contenu intracellulaire en descobaltocorrinoïdes est analysé ainsi que le contenu en corrinoïdes, comme cela a déjà été décrit ci-dessus (Cf exemple 6.2.2). Les souches G2035 et G2037 n'accumulent pas de corrinoïdes et de larges concentrations (supérieures à celles observées avec la souche parente) en acide hydrogénobyrinique et acide hydrogénobyrinique mono et diamide ne sont présentes qu'avec la souche G2035. Ce mutant est probablement bloqué dans une étape située après l'acide hydrogénobyrinique diamide et avant l'acide cobyrinique diamide. Par conséquent le gène cobS coderait pour une des enzymes impliquées dans la transformation de l'acide hydrogénobyrinique diamide en acide cobyrinique diamide; cette protéine peut donc intervenir soit dans l'insertion du cobalt, soit dans la réduction du cobalt de l'acide cobyrinique a,c-diamide non adénosylé. Par contre, le mutant G2037 serait bloqué dans une étape située en amont de l'acide hydrogénobyrinique. Le gène cobT coderait pour une protéine impliquée dans une étape enzymatique en amont de l'acide hydrogénobyrinique et en aval du précorrine-3 (d'autres gènes de structure codant pour les enzymes impliqués en aval du précorrine-3 ont déjà été identifiés). Une autre possibilité pour la protéine COBT est qu'elle intervienne, comme cela est proposé à l'exemple 5, comme protéine liant le cobalt, et/ou comme protéine qui interagit avec d'autre(s) protéine(s) par sa partie acide.
**6.2.4. Propriétés de la protéine COBV**
Les mutants G2039 et G2040 décrits dans les exemples 1 et 4.4 sont bloqués dans l'étape correspondant à la protéine COBV. Ces souches ainsi que la souche parente sont cultivées en milieu PS4' pendant 3 jours en présence de cobalt radioactif 57CoCl2, puis leur contenu intracellulaire en descobaltocorrinoïdes est analysé et le contenu en corrinoïdes est déterminé, comme cela est décrit dans l'exemple 9. Les souches G2039 et G2040 accumulent de l'acide cobyrique, du cobinamide, du cobinamide phophate et du GDP-cobinamide. Ces mutants sont probablement bloqués dans une étape enzymatique en aval de la GDP-cobinamide. Le gène cobV coderait pour une enzyme impliquée dans la transformation de la GDP-cobinamide en cobalamine, voir figure 5. Ce résultat est en parfait accord avec l'activité cobalamin (5'-phosphate) synthase de la protéine COBV qui possède l'Ado-GDP-cobinamide comme substrat.
**6.3 - Détermination de l'activité de protéines COB par études d'affinité vis-à-vis du SAM**
Cet exemple illustre comment il est possible, à partir de protéines COB purifiées de Pseudomonas denitrificans, de mettre en évidence in vitro une activité de fixation du SAM. Si une protéine COB possède une telle activité, cela signifie que cette protéine COB est une méthyltransférase de la voie et qu'elle intervient dans un des transferts des huit groupements méthyl qui se produisent entre l'uro'gen III et l'acide cobyrinique.
**6.3.1. Test d'affinité pour le SAM sur une protéine purifiée**
   Le test repose sur le principe suivant lequel les méthyltransférases de la voie de biosynthèse des cobalamines ont certainement un site de fixation du SAM. Ce site doit être mis en évidence par une affinité du SAM plus élevée que pour toute protéine qui ne fixe pas le SAM spécifiquement. Après incubation de la protéine à étudier en présence d'un excès de SAM radioactif, celle-ci est séparée du SAM libre par une chromatographie de perméation de gel. La radioactivité retrouvée dans la fraction du poids moléculaire de la protéine correspond au SAM fixé durant l'incubation. La chromatographie est effectuée à 2°C pour limiter au maximum la libération du SAM lié durant la séparation.
   La protéine (environ 10 µg) est incubée durant 10 minutes à 30°C dans 200 µl de Tris-HCl 0,1 M pH 7,7 avec 5 nmoles de [méthyl-³H]-SAM (1 µCi). Après incubation, 100 µl du mélange sont immédiatement injectés sur une colonne de TSK-125 (Bio-Rad) éluée à 1 ml/minute avec le mélange de sulfate de sodium 50 mM/dihydrogénophosphate de sodium 20 mM pH 6,8 préconisé par le distributeur de cette colonne. Des fractions de 0,5 ml sont collectées et comptées en scintillation liquide. Les temps de rétention de la protéine et du SAM sont obtenus directement d'après l'enregistrement de l'absorbance de l'éluat à 280 nm.
**6.3.2. Etude in vitro de la fixation du SAM sur les protéines COBA et COBF de Pseudomonas denitrificans**
   a) Purification des protéines COBF et COBA
      La protéine COBF de Pseudomonas denitrificans est purifiée comme cela est décrit ci-après. Dans une expérience typique de purification, 5 g de cellules humides de la souche SC150 Rif^{r} dans laquelle a été introduit le plasmide pXL1546 ( voir ex.7.3) obtenues après culture en milieu PS4 sont resuspendues dans 30 ml de Tris-HCl 0,1 M pH 7,7 et soniquées durant 15 minutes à 4°C. L'extrait brut est ensuite récupéré par centrifugation 1 heure à 50 000 g et le surnageant est passé sur une colonne de DEAE-Sephadex (1 ml de gel) pour éliminer les composés tétrapyrroliques présents. 10 mg de protéines (0,7 ml) de cet extrait sont ensuite injectés sur une colonne de MonoQ HR 5/5 équilibrée avec le même tampon. Les protéines sont éluées avec un gradient linéaire de KCl (0 à 0,25 M). La protéine COBF est éluée avec 0,20 M de KCl. Elle est diluée deux fois avec du Tris-HCl 0,1 M pH 7,7 et purifiée une seconde fois sur une MonoQ HR 5/5. L'électrophorèse en PAGE-SDS avec révélation au bleu de Comassie est utilisée pour visualiser la protéine. Cette technique montre d'autre part que COBF est d'une pureté de 95 % environ après cette étape de purification. La séquence NH₂-terminale de la protéine purifiée a été réalisée comme cela est décrit précédemment . Deux séquences NH₂-terminales apparaissent en même temps sur chaque cycle de dégradation; ce sont les séquences suivantes dans les proportions indiquées : 
Séquence 1 (34 % d'abondance)
1 2 3 4 5 6 7 8 9 10 11
Ala Glu Ala Gly Met Arg Lys Ile Leu Ile Ile
 
Séquence 2 (66 % d'abondance)
1 2 3 4 5 6 7 8 9 10 11
Met Arg Lys Ile Leu Ile Ile Gly Ile Gly Ser
      La séquence 1 correspond à la séquence NH₂-terminale de la protéine COBF qui est donnée sur la figure 16, si ce n'est que la méthionine aminoterminale est excisée suivant des règles déjà énoncées (Hirel et al., 1989) par la méthionine aminopeptidase (Ben Bassat et Bauer, 1989). La séquence 2, présente en quantité la plus importante, correspond à la même protéine mais dont l'initiation de traduction se serait faite non pas au codon ATG d'initiation de la traduction que nous avions supposé, mais à celui situé 5 codons en aval sur la phase codante (figure 16). En effet les acides aminés de cette séquence sont exactement ceux que l'on trouve sur la séquence de la protéine COBF à partir de la deuxième méthionine (acide aminé n° 6) de cette séquence (figure 16). Dans ce cas la méthionine aminoterminale n'est pas excisée ce qui confirme les règles déjà énoncées (Hirel et al., 1989). Il y a chez la souche SC510 Rif^{r} portant le plasmide pXL1546, deux initiations de traduction, d'une part celle correspondant au codon méthionine positionné à la bonne distance, sur notre construction, de la séquence de Shine et Delgarno et d'autre part celle qui se fait au deuxième codon méthionine se trouvant sur la séquence du gène cobF présenté à la figure 16. De cela, il ressort que probablement la protéine COBF commence non pas à la méthionine indiquée sur la figure 16 mais à celle se trouvant 5 acides aminés plus loin.
      De toute manière, ce résultat montre qu'il s'agit bien de la protéine COBF qui est exprimée et que celle-ci est exprimée avec une forme allongée de 4 acides aminés. Lors de la purification les deux formes protéiques sont purifiées. Dans cet exemple, nous appellerons protéine COBF purifiée le mélange de ces deux protéines purifiées.
      La protéine COBA de Pseudomonas denitrificans est purifiée comme cela est décrit précédemment (Blanche et al., 1989).
   b) Fixation du SAM
      La fixation du SAM sur ces deux protéines est étudiée comme cela est décrit précédemment à l'exemple 6.3.1.a). La sérum albumine bovine et la protéine COBH purifiée sont utilisées comme contrôles négatifs. Pour les protéines COBA et COBF on observe un pic de radioactivité à la sortie de la colonne de TSK-125 au temps de sortie de ces protéines (figure 20). Dans ce test, la protéine COBI présente la même propriété de fixation du SAM. A l'opposé, il n'y a pas de tels pics de radioactivité avec la BSA et la protéine COBH. Ce test met en évidence la fixation in vitro du SAM sur les protéines COBA, COBI et COBF. Ces résultats montrent que COBA, COBI et COBF sont des SAM-méthyltransférases. Ce résultat est tout à fait en accord avec les activités de COBA et COBI puisqu'il s'agit respectivement de la SUMT et de la SP₂MT de Pseudomonas denitrificans. La protéine COBF est donc probablement une SAM méthyltransférase de la voie de biosynthèse des cobalamines. Ce test confirme que COBF est une méthyltransférase.

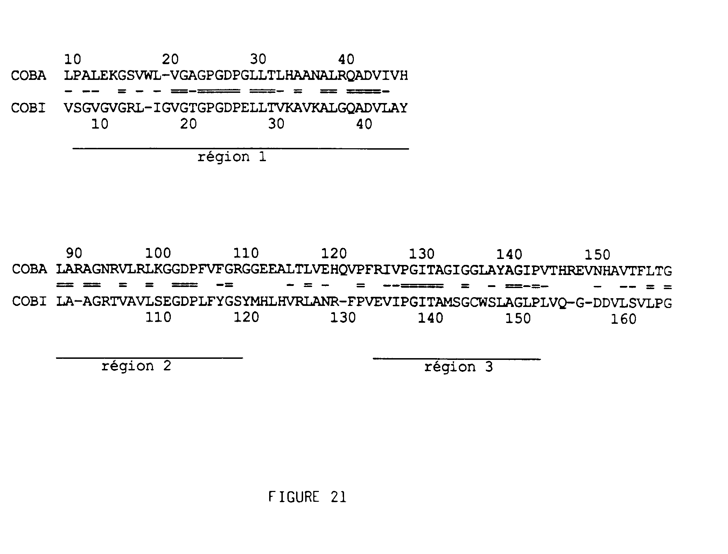

**6.4 - Détermination de l'activité de protéines COB par études d'homologies de séquences**
Cet exemple illustre comment par des comparaisons entre les séquences de diverses protéines COB de Pseudomonas denitrificans il est possible de trouver les protéines COB qui sont des SAM-méthyltransférases de la voie de biosynthèse des cobalamines.
Les protéines COBI et COBA sont toutes les deux des SAM méthyltransférases de la voie de biosynthèse. Ces deux protéines ont été comparées suivant le programme de Kanehisa, 1984. Cette comparaison fait ressortir trois régions de forte homologie (figure 21). Dans chacune de ces régions il y a plus de 45 % d'homologie stricte entre les deux protéines. Trois régions de forte homologie entre COBA et CYSG sont également présentées (figure 22); ce sont les mêmes régions de COBA qui présentent une forte homologie avec COBI. Ces régions de fortes homologies entre COBA, CYSG et COBI présentent de l'homologie avec d'autres protéines COB. Il s'agit des protéines COBF, COBJ, COBL et COBM (figure 23). En ce qui concerne la région 1, les protéines COBF, COBL et COBM présentent des homologies significatives par rapport à toutes les protéines de Genpro, qui est une extraction protéique de Genbank (version 59) augmentée des parties codantes putatives supérieures à 200 acides aminés, suivant le programme de Kanehisa (1984). En ce qui concerne la région 2, les protéines COBJ, COBL et COBM présentent des homologies significatives par rapport à toutes les protéines de Genpro (version 59). En ce qui concerne la troisième région d'homologie, COBJ, COBL et COBM présentent des homologies significatives par rapport à toutes les protéines de Genpro (version 59). Les comparaisons de séquences permettent donc de mettre en évidence que quatre protéines COBF, COBJ, COBL et COBM présentent des homologies significatives avec les régions conservées des séquences de trois types de méthyltransférases COBA, COBI et COBF. Les protéines COBG, COBH et COBK ne présentent pas d'homologies significatives avec les régions conservées des méthylases. La protéine COBF ne présente d'homologie significative avec les autres protéines que dans la région 1. Ces homologies doivent correspondre probablement au fait que toutes ces protéines sont des méthyltransférases. Ce résultat recoupe les données biochimiques décrites sur COBF concernant la capacité qu'a cette protéine à lier le SAM in vitro (exemple 6.3). Ces homologies, d'une part, permettent de confirmer que COBF est une SAM méthyltransférase de la voie de biosynthèse des cobalamines et d'autre part mettent en évidence que COBJ, COBL et COBM pourraient être des SAM méthyltransférases de la voie de biosynthèse des cobalamines. Ces resultats montrent egalement l'homologie qui existe entre les protéines COB de P.denitrificans et les protéines isofonctionnelles d'autres microorganismes.

### EXEMPLE 6(B) - Purification et clonage du gène de structure de la SUMT de Methanobacterium ivanovii.

Cet exemple illustre comment il possible d'obtenir, dans d'autres microorganismes, des enzymes COB et des gènes cob correspondants à ceux identifiés chez P. denitrificans.
**6(B).1. Purification de la SUMT de Methanobacterium ivanovii.**
Cet exemple décrit la purification de la SUMT de Methanobacterium ivanovii et l'étude de ses propriétés catalytiques. La souche de Methanobacterium ivanovii DSM2611 est cultivée comme cela est décrit (Souillard et al., 1988). 12 g de cellules humides sont obtenus. Celles ci sont resuspendues dans 80 ml de tampon Tris/HCl 0.1 M pH 7.6 contenant 5 mM DTT et 1 mM EDTA et soniquées pendant 1h30 à 4 °C puis centrifugées durant 1h à 50 000 g. L'extrait est ensuite débarassé de composés tétrapyrroliques libres par passage à travers une petite colonne de DEAE-Sephadex A25 montée dans le même tampon. Les protéines précipitant entre 55 et 75% de saturation en sulfate d'ammonium sont solubilisées dans un tampon Tris/HCl 0.1M pH 7.5, 0.5 mM DTT, 1.7 M sulfate d'ammonium et injectées sur une colonne de Phényl-Superose HR 10/10 (Pharmacia France/SA), éluée avec un gradient décroissant (de 1.7 M à 0 M en sulfate dammonium). Les fractions actives sont passées sur une colonne de Séphadex G-25 équilibrée avec du tampon Tris/HCl 0.1 M, pH 7.5, 0.5 mM DTT, glycérol 25% (tampon A) puis injectées sur une Mono Q HR 5/5 (Pharmacia France SA) équilibrée avec du tampon A et éluée avec un gradient de KCl de 0 à 0.3 M; cette étape est répétée une seconde fois dans les mêmes conditions. Une chromotographie de perméation de gel sur Bio-Sil TSK-250 (BioRad France SA) de la fraction active de l'étape précédente permet d'obtenir une protéine homogène en PAGE-SDS et en RP-CLHP (C-18 µBondapak). Les différentes étapes de purification avec leur rendement ainsi que leur facteur de purification sont décrites sur le tableau ci-dessous.
Comme il est montré dans ce tableau, le facteur de purification total est de plus de 4 500. Des propriétés de l'enzyme pure ont été étudiées selon des méthodes déjà décrites (Blanche et al., 1989). Cette enzyme a bien une activité SUMT, c'est à dire, qu'elle catalyse bien le transfert de deux groupements méthyls, SAM-dépendant, en C-2 et en C-7 de l'uro'gen III. Le poids moléculaire de l'enzyme estimé par perméation de gel est de 60,000 +/- 1,500 tandis que par PAGE-SDS il est de 29,000 ce qui montre clairement qu'il s'agit d'une enzyme homodimérique. Dans des conditions déjà décrites (Blanche et al.,1989) l'enzyme à un Km pour l'uro'gen III de 52 +/- 8 nM. De plus, cette enzyme ne présente pas d'inhibition par son substrat à des concentrations inférieures à 20 µM, alors que la SUMT de Pseudomonas denitrificans présente une inhibition par l'uro'gen III à une concentration supérieure à 2 µM (Blanche et al., 1989).

**Tableau :**

| Purification de la SUMT de M. ivanovii. | | | | | |
|---|---|---|---|---|---|
| Etape de purification | vol (ml) | Protéines (mg) | Activité spé. (u/mg de protéines) | Rendement | Facteur de purification¹ |
| Extrait brut | 92 | 731 | 0.337 | - | - |
| 55-75% SA | 7.1 | 153 | 1.215 | 76 | 3.6 |
| Phényl-superose | 9.5 | 8.34 | 15.35 | 52 | 46 |
| MonoQ 5/5 | 1.0 | 0.252 | 422 | 43 | 1252 |
| Bio-Sil TSK | 1.0 | 0.061 | 1537 | 38 | 4561 |

| | | | | | |
|---|---|---|---|---|---|
| 1/ calculé d'après le rendement en protéines. | | | | | |

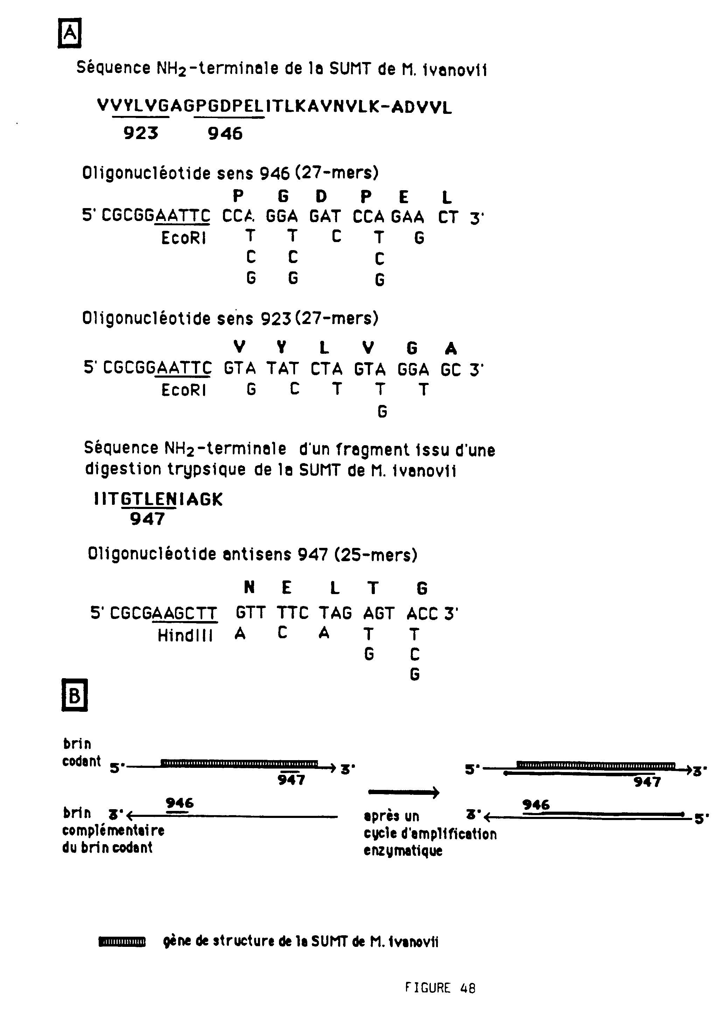

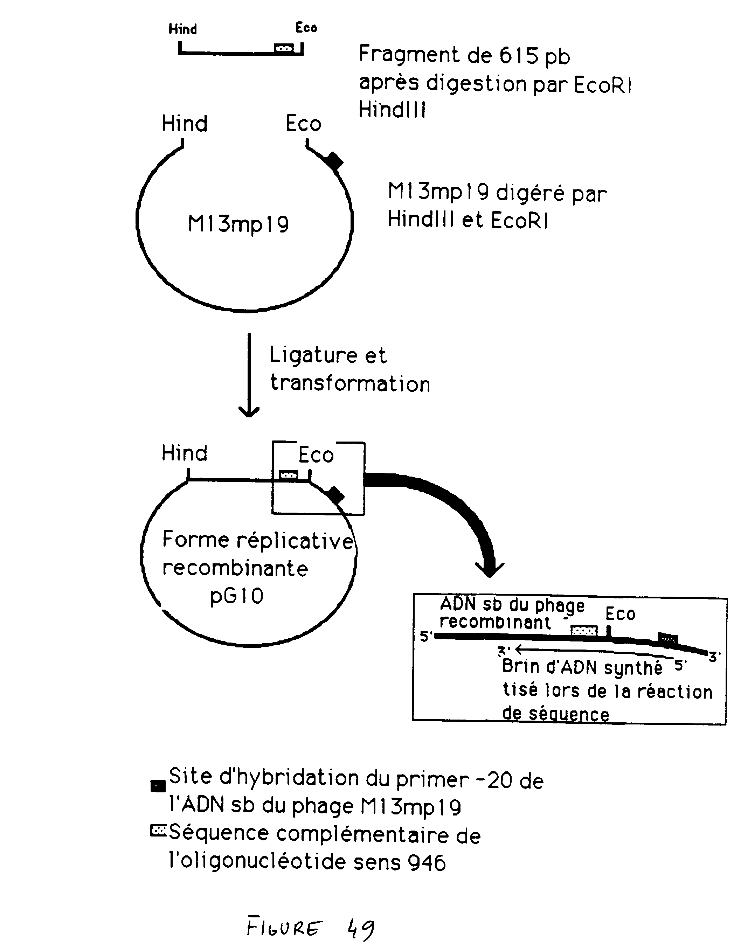

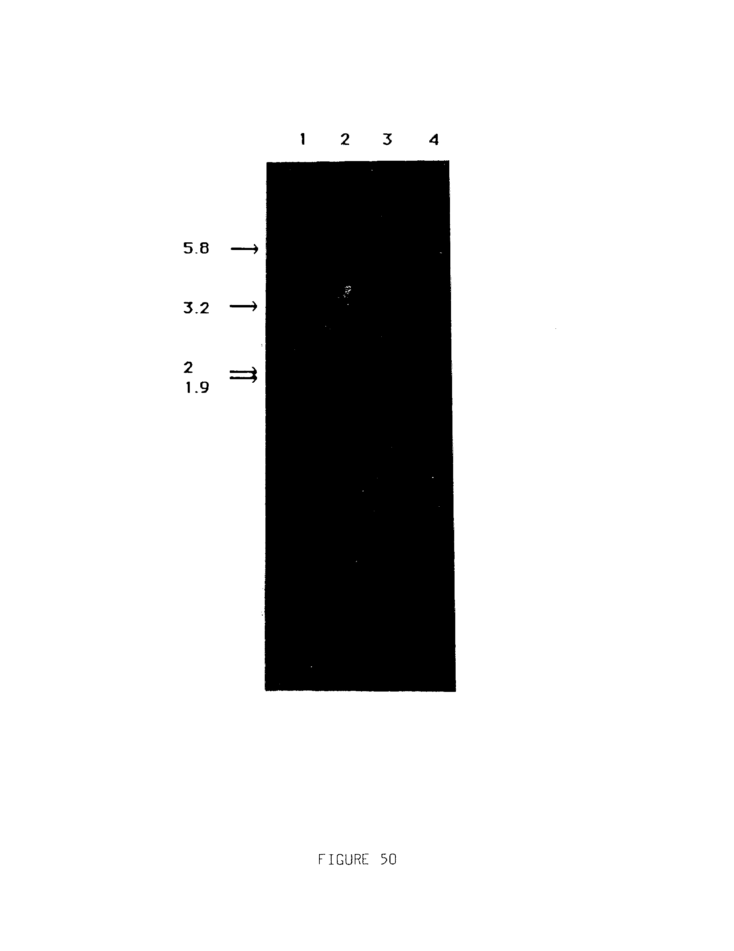

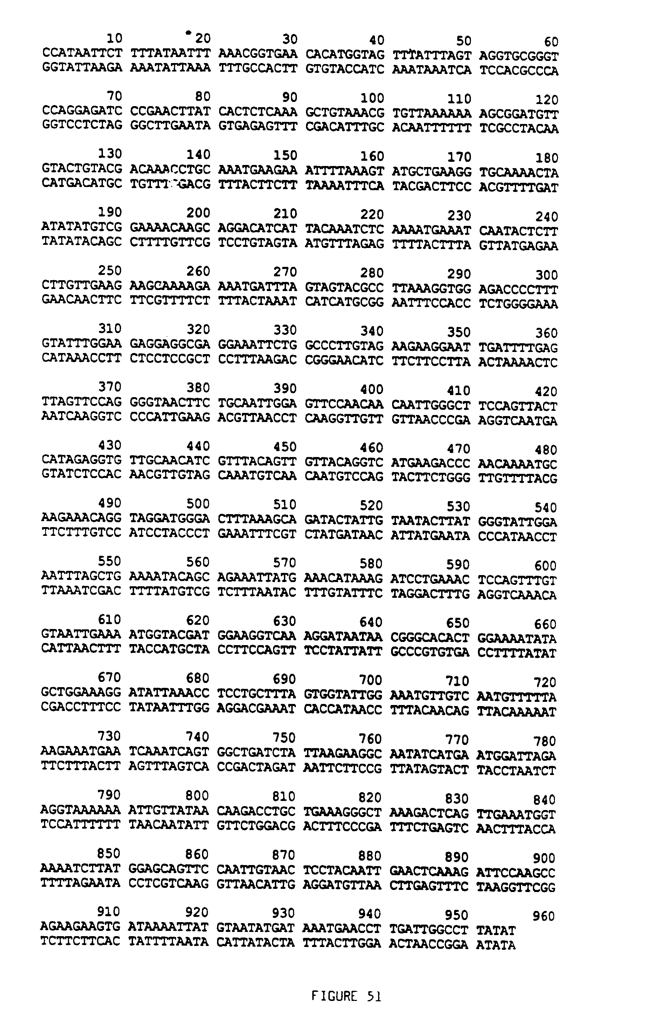

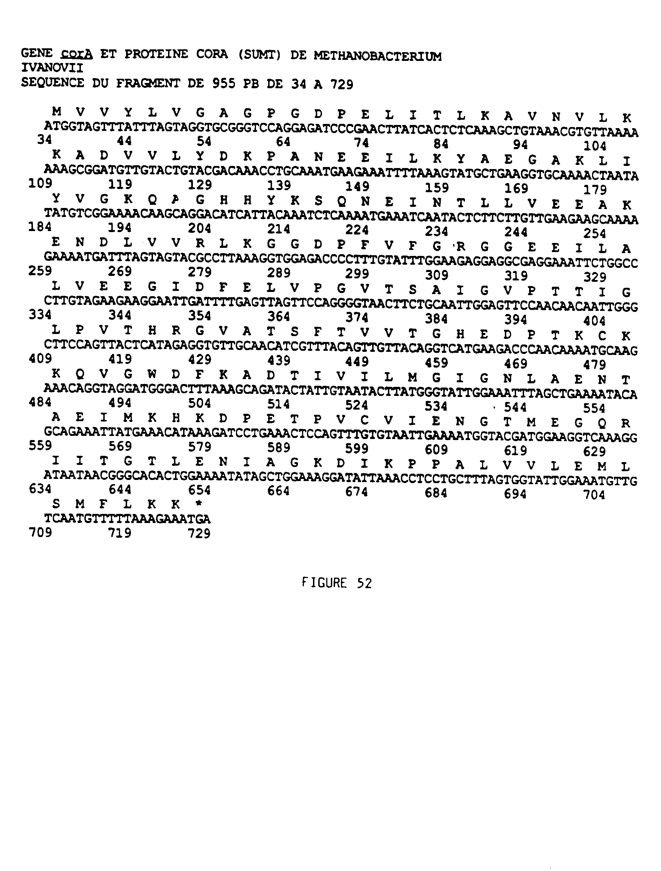

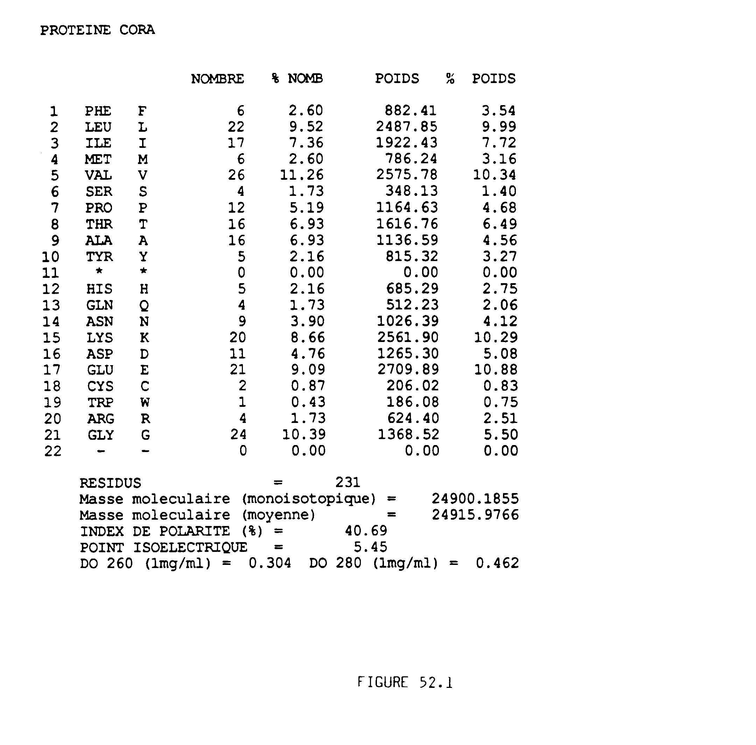

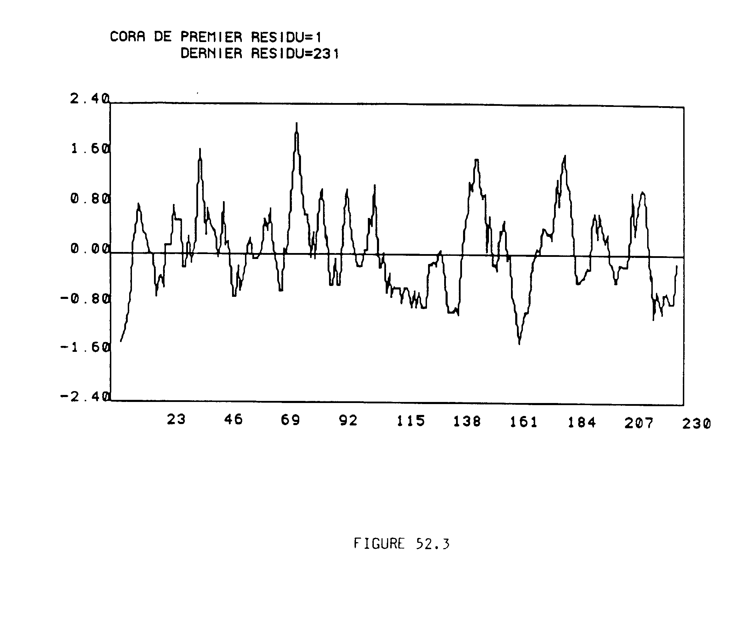

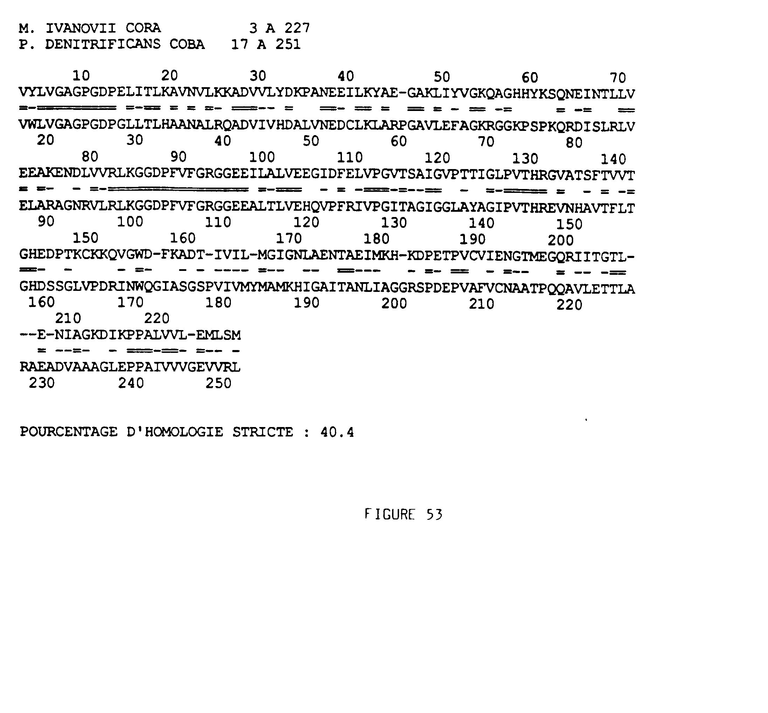

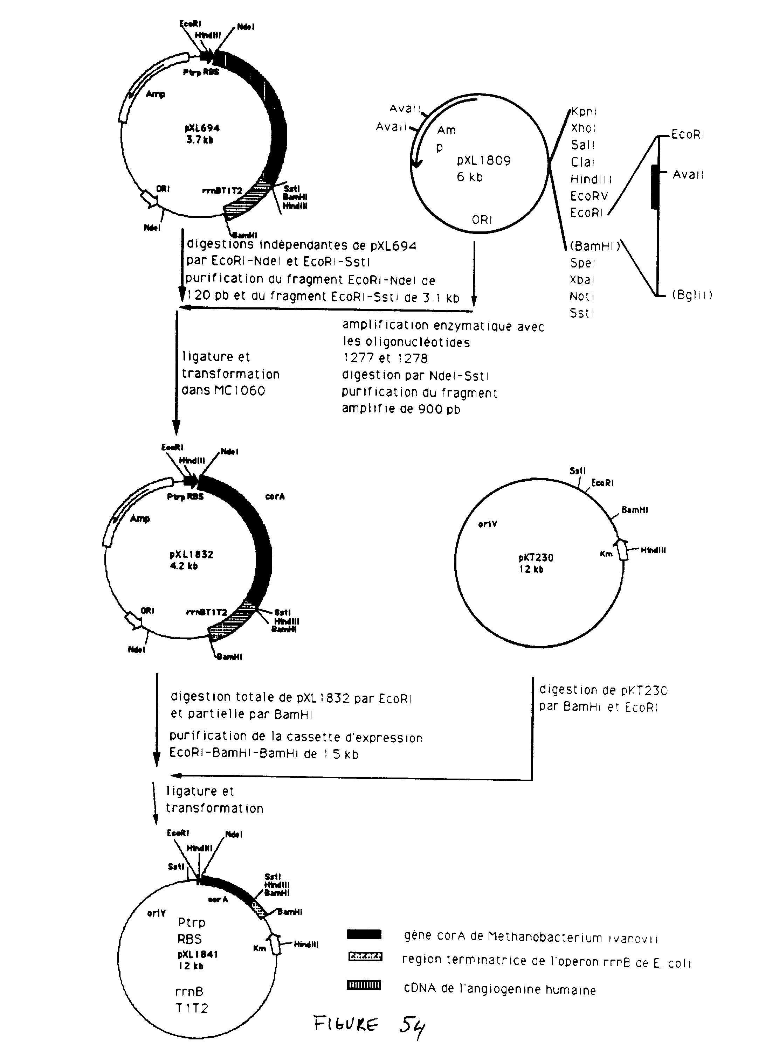

La Vmax de la SUMT de M. ivanovii a été déterminée. Celle-ci est de 1537 U/mg de protéines. Cette valeur est supérieure à celle trouvée pour la SUMT de P. denitrificans dèjà déterminée dans les conditions optimales de la réaction (compte tenu de son inhibition par l'uro'gen III), 489 U/mg de protéines (Blanche et al., 1989).
**6(B).2. Clonage du gène de structure de la SUMT de M. ivanovii chez E. coli.**
**6(B).2.1.** Clonage d'un fragment interne au gène de structure de la SUMT de M. ivanovii. Pour ce faire, on procède de la manière suivante : 200 picomoles de la SUMT de M. ivanovii sont utilisées pour le séquençage NH2-terminal de la protéine comme cela est décrit précédemment. En outre, un fragment peptidique obtenu par une digestion trypsique de la protéine est lui aussi soumis à un séquençage de sa partie NH2-terminale. Les séquences obtenues sont presentées sur la figure 48. Les oligonucléotides sens et antisens respectiment 946, 923 et 947 (voir figure 48) sont synthétisés comme cela est décrit précédemment; ces oligonucléotides contiennent à leur extrémité 5' un site de restriction qui est soit EcoRI pour les oligonucléotides sens soit HindIII pour l'oligonucléotide antisens. Ces oligonucléotides sont utilisés pour une expérience d'amplification enzymatique de l'ADN (Saiki et al., 1988), comme cela est schématisé sur la figure 48.B.
   L'ADN génomique de M. ivanovii est préparé de la manière suivante: 0.4 g de cellules de M. ivanovii (DSM 2611) sont lavées avec une solution 0.15 M NaCl. Les cellules sont ensuite incubées dans 4 ml d'une solution de sucrose 25 %, Tris/HCl 50 mM pH 8, lysozyme 40 mg puis 2 à 3 h à 50°C après addition de 40 mg de protéinase K et de 5 ml d'une solution SDS 0.2 %, EDTA 0.1 M pH 8. L'ADN est ensuite extrait au phénol-chloroforme (50 %-50 %) 2 fois, puis 2 fois au chloroforme et ensuite précipité par l'isopropanol et repris dans 3 ml de TNE (Tris/HCl 10 mM pH 8, EDTA 1 mM, Na-Cl 100 mM).
   L' Amplification enzymatique de l'ADN de M. ivanovii est effectuée suivant le protocole de Saiki et al., 1988, dans un volume de 0.1 ml avec 600 ng d'ADN génomique de M. ivanovii, en utilisant les amorces 946 et 947 (réaction 1) ou 923 et 947 (réaction 2). Le tampon utilisé pour cette réaction est MgCl2 1mM, KCl 50 mM, gélatine 0.001 % et chaque dNTP à une concentration de 0.2 mM; pour chaque réaction d'amplification, 10 mg de chaque oligonucléotide sont utilisés ainsi que 2.5 unités de Taq DNA polymérase (Cetus Corporation). L'amplification est réalisée sur 30 cycles dans le Perkin-Elmer Cetus DNA Amplification system; au cours de chaque cycle, l'ADN est dénaturé 1 min à 95 °C, les amorces oligonucléotidiques sont hybridées à l'ADN simple brin 2 min à 38 °C et les néobrins sont polymérisés pendant 3 min à 72 °C. Les produits d'amplification sont ensuite extraits au chloroforme et subissent ensuite une précipitation éthanolique; ils peuvent ensuite être visualisés après migration sur gel d'acrylamide puis être digérés par les enzymes de restriction telles que EcoRI et HindIII.
   Dans le cas de la réaction 1, deux fragments sont observés : à 615 pb ainsi qu'à 240 pb. En ce qui concerne la réaction 2, deux fragments sont aussi observés : à 630 et 170 pb. La totalité du produit d'une réaction d'amplification enzymatique entre les oligonucléotides 946-947 est séparée par migration sur gel d'acrylamide; le fragment de 615 pb est purifié comme cela est décrit précédemment. Ce fragment est ensuite digéré par EcoRI et HindIII afin de rendre les extrémités du fragment cohésives. Ce fragment est ensuite ligaturé avec de l'ADN de la forme réplicative du phage M13mp19. La ligature est transformée dans E. coli TG1. Six clones recombinants, contenant un insert de 615 pb, sont analysés par séquençage avec le primer universel -20 (Pharmacia SA, France). Comme il est montré sur la figure 49, lorsque l'on séquence l'ADN simple brin des phages recombinants qui contiennent l'insert de 615 pb, il doit être observé, en aval du site EcoRI, une séquence non dégénérée corrrespondant à celle de l'oligonucléotide 946 suivie, dans la même phase, par une séquence qui code pour les acides aminés LITLKAVNVLK?ADVVL (? signifie qu'à cette position, le résidu n'a pas pu être déterminé); cette séquence correspond à celle qui, dans la séquence NH2-terminale de la SUMT, suit les acides aminés correspondant à l'oligonucléotide 946 (voir figure 48). Pour deux clones, il a effectivement été observé, après le site EcoRI, une séquence pouvant coder pour la région NH2-terminale de la SUMT de Methanobacterium ivanovii, celle-ci commençant par l'enchainement Pro-Gly-Asp-Pro-Glu-Leu qui sont les acides aminés codée par une séquence contenant l'oligonucléotide 946. Cette observation montre que ces deux formes réplicatives recombinantes contiennent un insert qui correspond à un fragment interne au gène de structure de la SUMT de Methanobacterium ivanovii. La forme réplicative portant ce fragment interne au gène de structure de M. ivanovii est appelée pG10.
**6(B).2.2.** Clonage du gène de structure de la SUMT de Methanobacterium ivanovii.
   L'ADN génomique de Methanobacterium ivanovii est digéré par plusieurs enzymes de restriction (digestions simples ou doubles). Après digestion, les fragments sont séparés par électrophorèse sur gel d'agarose, puis ils sont transférés sur une membrane de nylon comme cela est décrit précédemment. Après dénaturation des fragments ainsi transférés et préhybridation, on procède à une hybridation avec la forme réplicative pG10 comme sonde marquée au 32P comme cela est décrit précédemment. Il est ainsi trouvé qu'un fragment issu d'une digestion EcoRI-BglII de 3.2 kb de Methanobacterium ivanovii hybride avec la sonde (voir Figure 50). 40 µg d'ADN génomique de M. ivanovii sont ensuite digérés par EcoRI et BglII puis séparés par migration sur gel d'agarose. Les fragments ayant une taille comprise entre 3 et 3.5 kb sont électroélués comme cela est décrit précédemment. Les fragments ainsi purifiés sont mis à ligaturer avec le vecteur pBKS+ (Stratagene Cloning Systems, La Jolla) digéré par BamHI-EcoRI. La ligature est transformée dans E. coli DH5α (Gibco BRL). Les transformants sont sélectionnés sur milieu LB supplémenté avec de l'ampicilline et du X-gal. 800 colonies blanches sont repiquées sur filtre; après croissance puis lyse des bactéries on procède à une hybridation sur colonies selon la technique de Grünstein et Hogness (1975). La sonde utlisée est la forme réplicative pG10 marquée au 32P. Un seul clone positif après ce test d'hybridation avec la sonde est trouvé. L'ADN plasmidique de ce clone est appelé pXL1809 (voir figure 56). Une digestion de cet ADN par EcoRI-XbaI permet de visualiser, comme attendu, un insert de 3.2 kb. Le plasmide pXL1809 est séquencé sur les 2 brins par la technique de Chen et Seeburg (1985). Une séquence de 955 bases est obtenue (figure 51). Une analyse des phases ouvertes nous conduit à identifier une phase ouverte de la base 34 (ATG) à la base 729 (TGA). Cette phase ouverte code pour une protéine dont la séquence est présentée sur la figure 52. Cette protéine à un poids moléculaire de 24 900 (voir figure 53) ce qui est proche du poids moléculaire de la protéine purifiée à partir de M. ivanovii. La séquence NH2-terminale de cette protéine est exactement celle déterminée pour la SUMT de M. ivanovii purifiée (voir figure 48 et figure 52). Ces observations établissent sans ambiguité que le gène cloné et séquencé est bien le gène de structure de la SUMT de M. ivanovii. Comme cette activité est supposée intervenir dans la biosynthèse des corrinoïdes chez toutes les bactéries, ce gène est désigné par gène corA et la protéine codée par ce même gène protéine CORA. Le profil d'hydrophobicité de la protéine CORA de M. ivanovii, réalisé à partir du programme de Hopp et Woods (1981) montre qu'il s'agit, comme cela est attendu, d'une protéine hydrophile, comme cela est présenté sur la figure 54. La protéine CORA de M. ivanovii montre un degré d'homologie stricte de plus de 40 % vis à vis de COBA de P. denitrificans (figure 53). Cette homologie s'étend sur presque la totalité des deux protéines puisqu'elle concerne les résidus 3 à 227 de CORA de M. ivanovii et les résidus 17 à 251 de COBA de P. denitrificans. Cette homologie reflète les homologies structurales qu'il existe entre 5 deux protéines catalysant la même réaction. Ce sont les mêmes régions qui sont les plus conservées entre CORA et COBA de P. denitrificans que celle qui sont conservées entre COBA de P. denitrificans et CYSG d'E. coli (figures 22).

### EXEMPLE 7 - Expression de protéines COB

**7.1 - Expression chez Pseudomonas denitrificans**
Cet exemple illustre que l'amplification d'un gène de structure d'une protéine COB de Pseudomonas denitrificans chez Pseudomonas denitrificans conduit à l'amplification de l'activité de la protéine COB.
7.1.1 - Expression de la protéine COBA
Le plasmide pXL557 correspond au plasmide pXL59 dans lequel le fragment BglII-EcoRV (respectivement aux positions 80 et 2394 sur la sequence de la figure 7) de 2,4 kb du fragment de 5,4 kb a été cloné. Ce fragment contient les gènes cobA et cobE.
Le plasmide pXL545 contient uniquement le gène cobE. Sa construction a été décrite dans l'exemple 4.1.
Ces deux plasmides ont été introduits par transfert conjugatif chez SC510 Rif^{r}. Les souches SC510 Rif^{r}, SC510 Rif^{r} pXL59, SC510 Rif^{r} pXL557 et SC510 Rif^{r} pXL545 ont été cultivées en milieu PS4. A 4 jours, les cultures ont été arrêtées et les activités SUMT ont été dosées suivant un protocole standard déjà décrit (F. Blanche et al., 1989). Les activités sont portées ci-dessous.

**Tableau :**

| Activité SUMT de SC510 Rif^{r} et de quelques unes de ses dérivées | |
|---|---|
| Souche | SUMT dosée nmole/h/mg de protéines |
| SC510 Rif^{r} | 0.05 |
| SC510 Rif^{r} pXL59 | 0.04 |
| SC510 Rif^{r} pXL557 | 2.10 |
| SC510 Rif^{r} pXL545 | 0.05 |

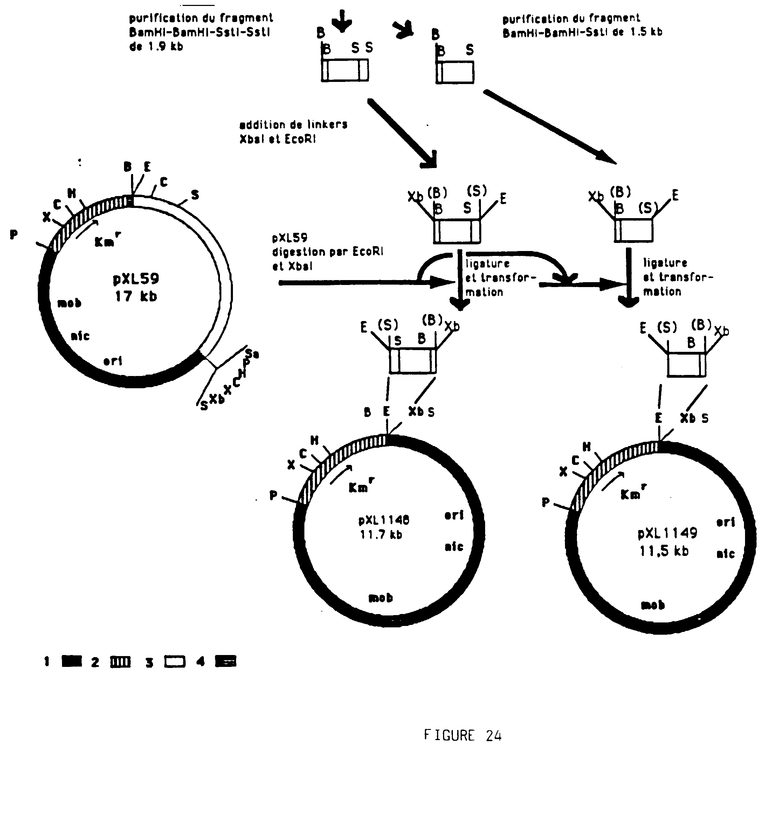

Il ressort clairement de ces résultats que seul le plasmide pXL557 entraîne chez SC510 Rif^{r} une nette augmentation de l'activité SUMT (un facteur 50). Cette augementation résulte de l'amplification de cobA et non de cobE puisque le plasmide pXL545, qui ne permet que l'amplification de cobE, ne provoque pas d'augmentation d'activité SUMT. Ce résultat confirme que cobA est le gène de structure de la SUMT de Pseudomonas denitrificans. Ce résultat montre que l'on peut obtenir une amplification de l'activité SUMT chez Pseudomonas denitrificans par amplification du gène de structure de la SUMT de Pseudomonas denitrificans.
7.1.2 - Expression de la protéine COBI
Un fragment provenant du fragment d'ADN de 8,7 kb, contenant le gène de structure de la SP₂MT (cobI), est cloné sur un plasmide à large spectre d'hôte chez les bactéries gram-négatives, puis ce plasmide est ensuite introduit par conjugaison chez Pseudomonas denitrificans SC510 Rif^{r}. L'activité S-adénosyl-L-méthionine:précorrine-2 méthyltransférase de la souche est ensuite mesurée par rapport à celle de la souche portant le vecteur.
Le fragment BamHI-BamHI-SstI-SstI de 1,9 kb contenant les gènes cobH et cobI est purifié à partir du fragment de 8,7 kb. Des "linkers" XbaI et EcoRI sont placés respectivement aux extrémités BamHI et SstI après que celles-ci aient été remplies avec l'ADN polymérase du bactériophage T4. Le fragment est ensuite inséré entre les sites XbaI et EcoRI du plasmide à large spectre d'hôte pXL59. Il porte la résistance à la kanamycine. Le plasmide ainsi obtenu est nommé le pXL1148 (figure 24).
Par ailleurs un plasmide voisin a été construit : le fragment BamHI-BamHI-SstI de 1,5 kb contenant seulement le gène cobH en entier et la partie 5' du gène cobI a été purifié à partir du fragment de 8,7 kb. Des "linkers" XbaI et EcoRI ont été ajoutés aux sites BamHI et SstI respectivement après que ceux-ci aient été remplis ou digérés avec l'ADN polymérase du phage T4. Ce fragment a ensuite été inséré entre les sites EcoRI et XbaI du pXL59 pour donner le plasmide pXL1149. Les plasmides pXL1148 et pXL1149 ne diffèrent que par la présence sur pXL1148 du fragment SstI-SstI de 0,3 kb qui contient l'extrémité 3' du gène cobI. pXL1148 possède le gène de structure entier de cobI au contraire de pXL1149. Les deux plasmides contiennent le gène cobH.
Ces deux plasmides ont été introduits par conjugaison chez SC510 Rif^{r}. Les souches SC510 Rif^{r}, SC510 Rif^{r} pXL59, SC510 Rif^{r} pXL1148 et SC510 Rif^{r} pXL1149 sont cultivées en milieu PS4. Après 4 jours de culture, les cellules sont récoltées et les activités SP2MT sont dosées comme cela est décrit dans l'exemple 6.1.3 a).
Le résultat de ces dosages est porté ci-dessous avec les activités de SP2MT définies comme à l'exemple 6.1.3 a).

**Tableau :**

| Activités SP₂MT de diverses souches dérivées de Pseudomonas denitrificans | |
|---|---|
| Souche Souche | Activité SP₂MT¹ Activité en % en % |
| SC510 Rif^{r} | < 5 |
| SC510 Rif^{r} pXL59 | < 5 |
| SC510 Rif^{r} pXL1148 | 75 |
| SC510 Rif^{r} pXL1149 | < 5 |

| | |
|---|---|
| ¹pour 500 pg d'extrait brut introduit dans le test. | |

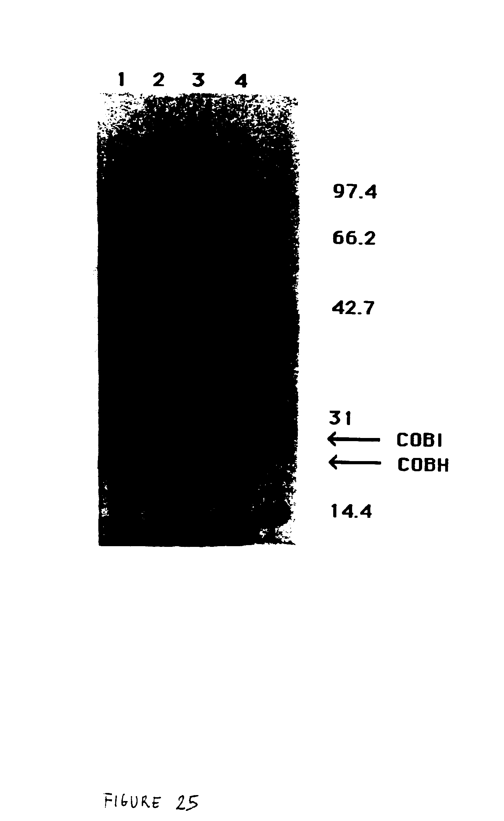

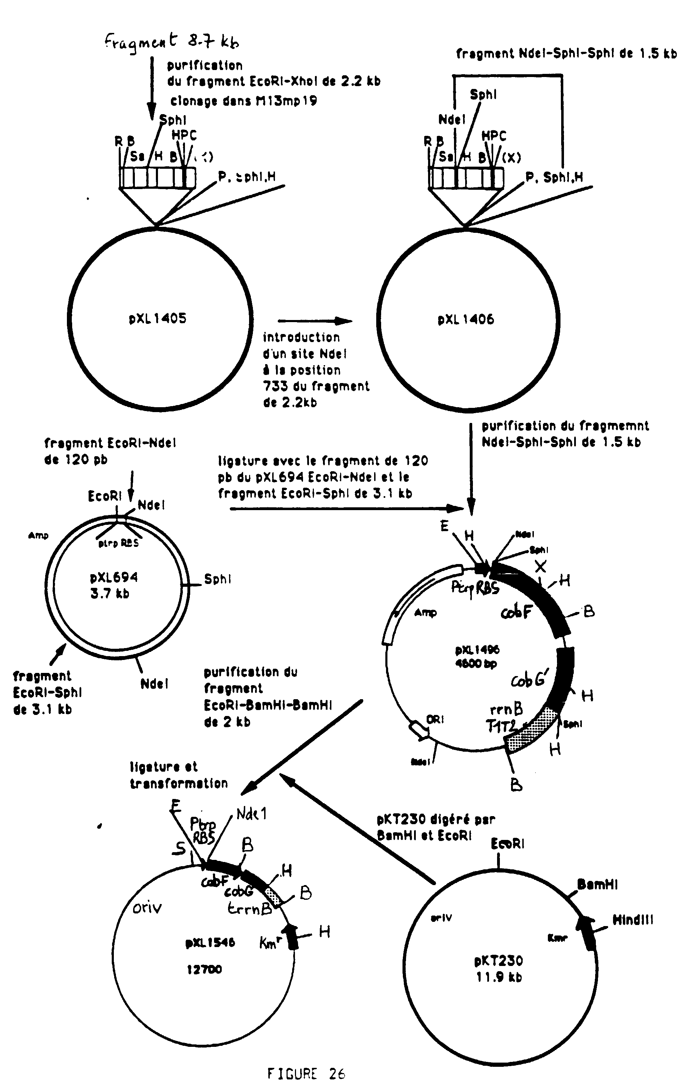

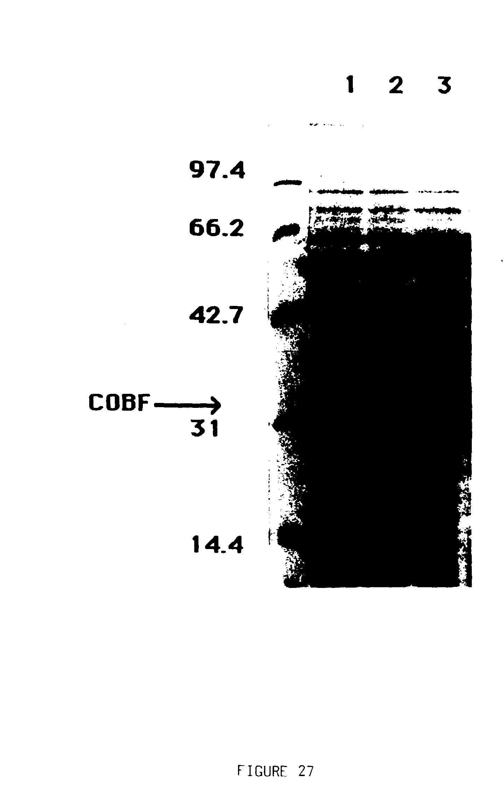

L'activité est exprimée en % comme cela est défini à l'exemple 6.1.3 a).
Seul le plasmide pXL1148 apporte une augmentation sensible de l'activité SP₂MT. Au contraire le plasmide pXL1149 ne donne pas de résultats différents de ceux observés avec les contrôles SC510 Rif^{r} et SC510 Rif^{r} pXL59. Le pXL1148 est le seul plasmide à contenir le gène cobI et il est le seul à amplifier l'activité SP₂MT; ce résultat confirme que le gène de structure de la SP₂MT de Pseudomonas denitrificans est le gène cobI. De plus, si les protéines totales de ces différentes souches sont séparées par électrophorèse en conditions dénaturantes (PAGE-SDS à 10 % d'acrylamide) on observe spécifiquement dans le cas du pXL1148 la présence d'une bande qui correspond à une protéine ayant un poids moléculaire de 25 000 environ (figure 25). Le poids moléculaire de cette protéine correspond à celui de la protéine COBI. Le plasmide pXL1148 permet d'obtenir chez Pseudomonas denitrificans la surproduction de la protéine COBI.
7.1.3 - Expression de COBF
L'expression est obtenue en positionnant en amont du gène cobF le promoteur PtrP de E.coli et le site de fixation des ribosomes du gène cII du bactériophage lambda. L'expression ainsi obtenue est beaucoup plus élevée que celle observée par simple amplification génique grâce au même plasmide multicopie.
Le fragment EcoRI-BamHI-BamHI de 2 kb environ du pXL1496 (exemple 7.2.1) est purifié (figure 26). Ce fragment contient le promoteur PtrP de E.coli et le site de fixation des ribosomes du gène cII du bactériophage lambda en amont du gène cobF. En aval du gène cobF se trouve le terminateur de l'opéron rrnB de E.coli. Ce fragment est cloné aux sites EcoRI-BamHI du plasmide pKT230 pour donner le pXL1546 (figure 26). Le pKT230 est un plasmide du groupe d'incompatibilité Q qui se réplique chez presque toutes les bactéries gram-négatives (Bagdasarian et al., 1981); ce plasmide porte la résistance à la kanamycine. Le plasmide pXL1546 et pKT230 sont introduits, par conjugaison, chez SC510 Rif^{r}. Les souches SC510 Rif^{r}, SC510 Rif^{r} pKT230 et SC510 Rif^{r} pXL1546 sont cultivées en milieu PS4 comme cela est décrit précédemment. Après quatre jours de culture, les protéines totales des différentes souches sont analysées en PAGE-SDS à 10 %. Comme cela est montré à la figure 27, on observe dans l'extrait de SC510 Rif^{r} pXL1546 une protéine d'un poids moléculaire de 32 000 environ qui est surexprimée; cette protéine co-migre avec la protéine qui est surexprimée par E.coli B pXL1496 (exemple 7.2.1). De plus cette protéine est spécifiquement exprimée dans la souche SC510 Rif^{r} contenant le pXL1546 où elle représente au moins 20 % des protéines totales. Par contre on n'observe pas cette protéine dans les protéines totales des souches SC510 Rif^{r} et SC510 Rif^{r} pKT230. Cette protéine surexprimée est donc la protéine COBF.
7.1.4 - Expression de COBH
Cet exemple décrit l'amplification d'un fragment d'ADN de Pseudomoas denitrificans contenant le gène cobH. La protéine qui est codée par ce gène est purifiée; il s'agit de la protéine COBH. Le plasmide pXL1149, décrit à l'exemple 7.1.2, ne contient sur l'insert d'ADN provenant du fragment de 8,7 kb que le gène cobH en entier. Chez SC510 Rif^{r}, ce plasmide, contrairement au vecteur, entraîne la surexpression d'une protéine de poids moléculaire 22 000 (figure 25).
7.1.5 - Expression de COBV
Cet exemple décrit l'amplification de l'activité cobalamin (5'-phosphate) synthase par un plasmide ne portant que cobV (pXL699, voir figure 38) L'activité cobalamin (5'-phosphate) synthase est amplifiée chez SC877Rifr par le plasmide pXL699 d'un facteur 50 par rapport à la même souche avec le vecteur pXL435, pXL1303, pXL1324 ou pKT230. Ce plasmide ne contient sur son insert que cobV en entier plus les parties 5' terminales d'ORF18 et de cobU. Il est certain que dans une telle souche (SC877Rifr pXL699) la protéine COBV est surexprimée; cette surexpression est d'un facteur 50 par rapport à l'expression de la souche SC877Rifr.
7.1.6. Expression de la protéine CORA
Le fragment EcoRI-BamHI-BamHI de 1.5 kb du pXL1832 (voir exemple 7.2.4) contenant le promoteur Ptrp puis le RBS cII du bactériophage λ, le gène de structure de la SUMT de M. ivanovii et la région terminatrice de l'opéron rrnB de E. coli est cloné aux sites EcoRI-BamHI du pKT230 (Bagdasarian et al., 1981). De cette manière, le plasmide pXL1841 est obtenu (voir figure 56). Ce plasmide est mobilisé chez P. denitrificans SC510 Rifr comme cela est décrit précédemment. Un transconjugant est étudié plus en détail. Cette souche est cultivée en milieu PS4 et l'activité SUMT des extraits bactériens est dosée en même temps que celle de la souche contrôle SC510 Rifr pXL435 (Cameron et al., 1989). Les activités de ces souches sont présentées ci-dessous.

| Souche | Activité spécifique SUMT en pmol/h/mg de protéines |
|---|---|
| SC510 RifrpXL435 | 50-100 |
| SC510 RifrpXL1841 | 1700 |

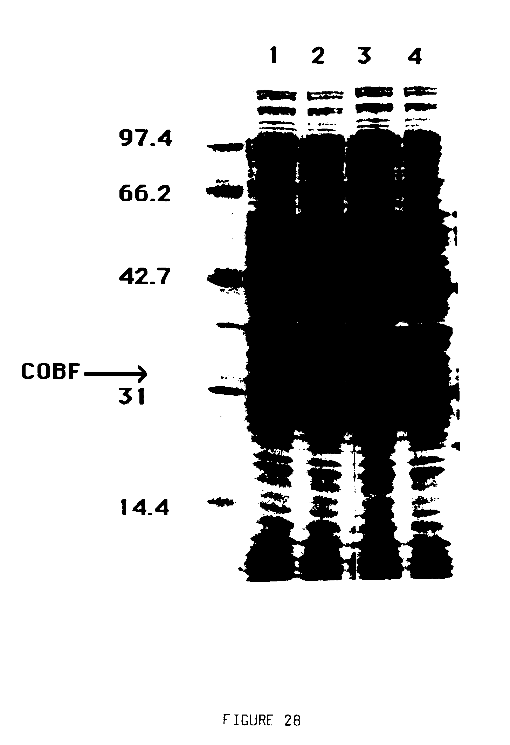

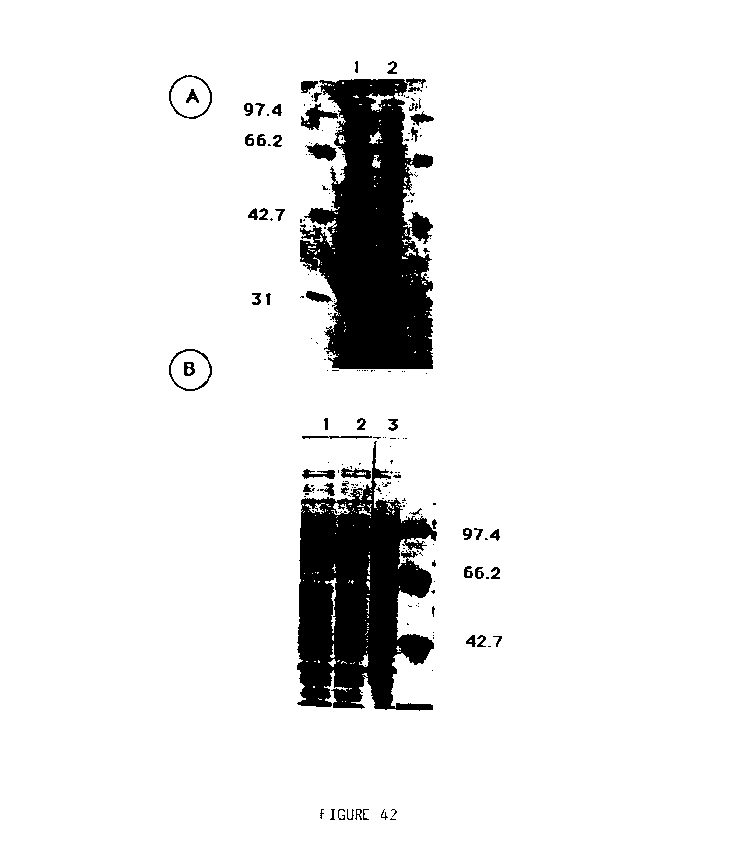

Ce résultat montre clairement qu'il y a expression de l'activité SUMT de M. ivanovii chez P. denitrificans grâce au plasmide pXL1841 puisque l'activité SUMT de la souche SC510 Rifr pXL1841 est nettement supérieure à celle de SC510 Rifr pXL435.
7.2 - Expression chez E.coli
Cet exemple illustre comment une protéine COB de Pseudomonas denitrificans peut être surproduite chez E.coli.
7.2.1 - Expression de COBF
La surproduction est obtenue en positionnant en amont du gène cob le promoteur Ptrp de E.coli et le site de fixation des ribosomes du gène cII du bactériophage lambda. Le fragment EcoRI-XhoI de 2250 pb du fragment EcoRI de 8,7 kb (aux positions respectives 0 et 2250 sur la sequence présentée à la figure 8) a été cloné dans le phage M13mp19 (Norrander et al., 1983) entre les sites EcoRI et SalI. Le plasmide ainsi construit est nommé pXL1405. Un site NdeI a été introduit par mutagénèse dirigée de manière à ce que les trois dernières bases (ATG) de ce site de restriction constituent le site d'initiation de traduction du gène cobF. Cela revient à modifier les trois bases qui précèdent l'ATG du gène cobF GAA (le G est à la position 733 sur la séquence présentée sur la figure 8) en CAT. Le fragment NdeI-SphI-SphI (figure 26) contenant le gène cobF est ensuite purifié; ce fragment de 1,5 kb est ensuite cloné entre les sites NdeI-SphI du plasmide pXL694 (Denèfle et al., 1987). Le plasmide ainsi construit est nommé pXL1496 (figure 26). Sur le fragment EcoRI-NdeI de 120 pb (qui provient du pXL694) qui précède le gène cobF sont présents des signaux de régulation de l'expression génétique de E.coli. Ces signaux sont constitués par la région [-40+1] du promoteur Ptrp de E.coli, puis par 73 pb qui contiennent le site de fixation des ribosomes du gène cII du bactériophage λ (Denèfle et al., 1987). En aval du gène cobF se trouvent les terminateurs de l'opéron rrnB de E.coli (sur le fragment HindIII-BamHI). Le plasmide pXL1496 a été introduit par transformation dans la souche de E.coli (Monod et Wollman, 1947). L'expression du gène cobF a été étudiée comme cela est déjà décrit (Denèfle et al., 1987) dans des conditions où le promoteur PtrP est soit réprimé (présence de tryptophane), soit non réprimé (absence de tryptophane). Le milieu où l'expression a été réalisée est du milieu minimum M9 (Miller, 1972) supplémenté par 0,4 % de glucose, 0,4 % de casaminoacides, 10 mM thiamine et 40 pg/ml de tryptophane dans le cas où l'on veut réprimer le promoteur Ptrp. La souche E.coli B pXL1496 a été cultivée à 37°C dans le milieu décrit ci-dessus avec 100 µg d'ampicilline. Comme il est montré sur la figure 28, l'absence de tryptophane entraîne l'expression d'une protéine d'un poids moléculaire de 32 000. En effet dans l'extrait de protéines totales de E.coli B pXL1496 analysé en PAGE-SDS (figure 28) on observe clairement une protéine d'un poids moléculaire de 32 000 qui représente entre 1 et 4 % des protéines totales. Cette protéine est présente en quantités nettement moins importantes dans l'extrait des protéines totales de E.coli B pXL1496 cultivée dans les mêmes conditions, mais en présence de tryptophane. Le poids moléculaire de la protéine qui est exprimée dans ces conditions est proche du poids moléculaire de la protéine COBF déduit de la séquence en acides aminés de la protéine qui est 28 927 (figure 16). La protéine qui est ainsi exprimée chez E.coli est la protéine COBF.
7.2.2 - Expression de COBT
La surproduction est obtenue en fusionnant à l'extrémité 5' du gène cob le promoteur lac et les trois premiers codons de lacz de E.coli.
Le site EcoRI situé à la position 2624 sur la séquence présentée sur la figure 32 du fragment de 4.8 kb contient le quatrième codon du gène cobT. Le fragment EcoRI-XbaI de 3,5 kb du pXL837 (voir figure 36) est cloné aux sites EcoRI et XbaI du pTZ18R ou pTZ19R (Pharmacia) pour générer les pXL1874 ou pXL1875 respectivement; ces deux plasmides différent par l'orientation du gène cobT tronqué vis à vis du promoteur de l'opéron lactose d'E. coli (Plac). Plac est en amont de cobT sur pXL1874 tandis que c'est le contraire sur le pXL1875. Le clonage aux sites EcoRI-XbaI du pTZ18R du fragment EcoRI-XbaI du pXL837 permet de réaliser une fusion de protéine entre les 4 premiers acides aminés de la β-galactosidase d'E. coli et le gène cobT à partir de son 4ème codon. L'expression de ce gène lacZ' 'cobT est sous le controle des signaux d'expression de lacZ. Les plasmides pXL1874, pXL1875, pTZ18R sont introduits par transformation dans la souche de E.coli BL21. L'expression du gène cobT est étudié comme cela est déjà décrit (Maniatis et al., 1989).
Comme le montre la figure 42B, une protéine d'un poids moléculaire de 72 000 n'est exprimée qu'avec le pXL1874 et représente, dans l'extrait de protéines totales de BL21,pXL1874 analysé en PAGE-SDS, 1 à 4 % des protéines totales. Le poids moléculaire de la protéine qui est exprimée dans ces conditions est proche du poids moléculaire de la protéine COBT déduit de la séquence en acides aminés qui est de 70 335 sur la figure 40. Cette expérience montre clairement qu'à partir du site EcoRI situé sur le quatrième codon du gène cobT une phase ouverte compatible avec celle trouvée pour le gène cobT peut être exprimée.
7.2.3 - Expression d'une protéine COBS tronquée.
Un site BamHI est situé au niveau du 45ème codon du gène COBS. Le fragment BamHI-BamHI de 1.2 kb contenant la partie 3' du gène cobS et des séquences en aval de ce gène est excisé du pXL843 pour être cloné au site BamHI du plasmide pET-3b (Rosenberg et al., 1987) pour générer le pXL1937. Le fragment BamHI est orienté de manière à ce que la partie tronquée du gène cobS soit fusionnée, en phase, avec les 12 premiers codons de la protéine majeure de capside du bactériophage T7 ou gène 10 (Rosenberg et al., -1987). Ce gène hybride est sous le controle du promoteur φ10 du bactériophage T7. Le plasmide pXL1937 ainsi que le pET-3b sont introduits par transformation dans la E. coli BL21 pLysS (W. Studier, communication personnelle). Après réisolement sur milieu sélectif, les 2 souches sont cultivées en milieu liquide L jusqu'à une D0 610 nm de 1; à ce stade là le milieu est ajusté à une concentration en IPTG (isopropyl β-galactoside) de 1 mM afin d'induire l'expression de la polymérase du batériophage T7 (Rosenberg et al., 1987). La culture est ensuite incubée 3 h à 37°C, puis des lysats bactériens sont préparés. Les protéines totales des bactéries ainsi cultivées sont séparées par PAGE en conditions dénaturantes. Comme on le voit sur la figure 42A, il y a spécifiquement surexpression d'une protéine de 33 000 avec la culture BL21 pLysS pXL1937. Ce poids moléculaire est tout à fait compatible avec le poids moléculaire attendu pour la protéine fusion (33 kD). Cette expérience montre clairement qu'à partir du site BamHI situé au niveau du 45ème codon du gène cobS, une phase ouverte compatible avec celle trouvée pour le gène cobS peut être surexprimée.
7.2.4. Expression de la protéine CORA
Les oligonucléotides suivants ont été synthétisés comme cela est décrit précédemment : oligonucléotide 1277 
5' GGC CGA ATT CAT ATG GTA GTT TAT TTA 3'
--------1   2   3   4   5  (1 à 5, 5
EcoRI -------                premiers codons
NdeI        de la SUMT de M.ivanovii)

oligonucléotide 1278
5' GGC CGA GCT CTA TTA CAT AAT T
________================
SstI

(= séquence figurant sur la figure 51, positions 926 à 915 sur le brin
complémentaire du brin codant)
L'oligonucléotide 1277 possède les séquences de reconnaissance des enzymes de restriction EcoRI et NdeI. Les trois dernières bases du site NdeI (ATG), qui correspondent à un codon d'initiation de traduction, sont directement suivies par les codons 2 à 5 du gène de structure de la SUMT de M. ivanovii tels qu'ils figurent dans la séquence présentée sur la figure 52. L'oligonucléotide 1278 contient la séquence de reconnaissance de SstI, suivie directement de la séquence TATTACATAATT qui correspond à une séquence présente sur le fragment de 955 pb contenant le gène corA présenté sur la figure 51; cette séquence se trouve à la position 926 à 915 (voir figure 51) sur le brin complémentaire du brin codant pour la protéine CORA. Les deux oligonucléotides 1277 et 1278 contiennent donc dans leur partie 3' des séquences correspondant respectivement au brin codant du gène corA et au brin complémentaire en aval de ce gène. Ces deux oligonucléotides peuvent être utilisés pour réaliser une expérience d'amplification enzym-atique avec le plasmide pXL1809 comme matrice. Cette expérience permet d'obtenir un fragment de 910 pb contenant le gène corA de M. ivanovii possédant un site NdeI au niveau de l'ATG du gène corA et un site SstI à l'autre extrémité du fragment après la fin du gène corA. L'amplification enzymatique est réalisée comme précédemment décrit pour l'amplification enzymatique faite sur l'ADN génomique de M. ivanovii, si ce n'est que la matrice est constituée par 10 ng d'ADN du plasmide pXL1809; les températures utilisées sont les mêmes, mais seulement 20 cycles d'amplification sont réalisés. Comme cela est précédemment décrit, les produits d'amplification sont digérés par NdeI et SstI avant d'être séparés par migration sur gel d'agarose. Comme attendu, un fragment d'une taille 910 pb est effectivement visualisé. Ce fragment est purifié comme cela est déjà décrit. Ce fragment est cloné aux sites NdeI et SstI du pXL694 (Denèfle el al., 1987). Le plasmide résultant nommé pXL1832 est décrit sur la figure 56. Sur ce plasmide de la même manière que cela est décrit à l'exemple 7.2, le gène de struture de la SUMT de M. ivanovii est précédé par le site de fixation des ribosomes du gène cII du bactériophage λ. En amont de ce RBS se trouve le promoteur Ptrp. Le plasmide pXL1832 est introduit dans E. coli B5548 qui est une souche de E. coli portant la mutation cysG44 (Cossart et Sanzey, 1982) par transformation. Les activités SUMT des souches E. coli B5548 pUC13 et E. coli B5548 pXL1832 sont dosées sur des extraits obtenus à partir de cellules cultivées en milieu LB supplémenté en ampicilline. Le dosage de l'activité SUMT est réalisé comme cela a déjà été décrit (Blanche et al., 1989). Les résultats de ce dosage sont donnés ci-dessous.

| Souche | Activité spécifique SUMT en pmol/h/mg de protéines |
|---|---|
| E. coliB5548 pUC13 | 5.9 |
| E. coli B5548 pXL1832 | 310 |

Les résultats présentés sur le tableau ci-dessus montrent clairement qu'il y a expression d'une activité SUMT chez la souche E. coli B5548 lorsque celle-ci contient une plasmide pXL1832 qui exprime la SUMT de M. ivanovii. La SUMT de M. ivanovii peut donc être exprimée chez E. coli.

### EXEMPLE 8 - Amplification de la production de cobalamines par les techniques d'ADN recombinant

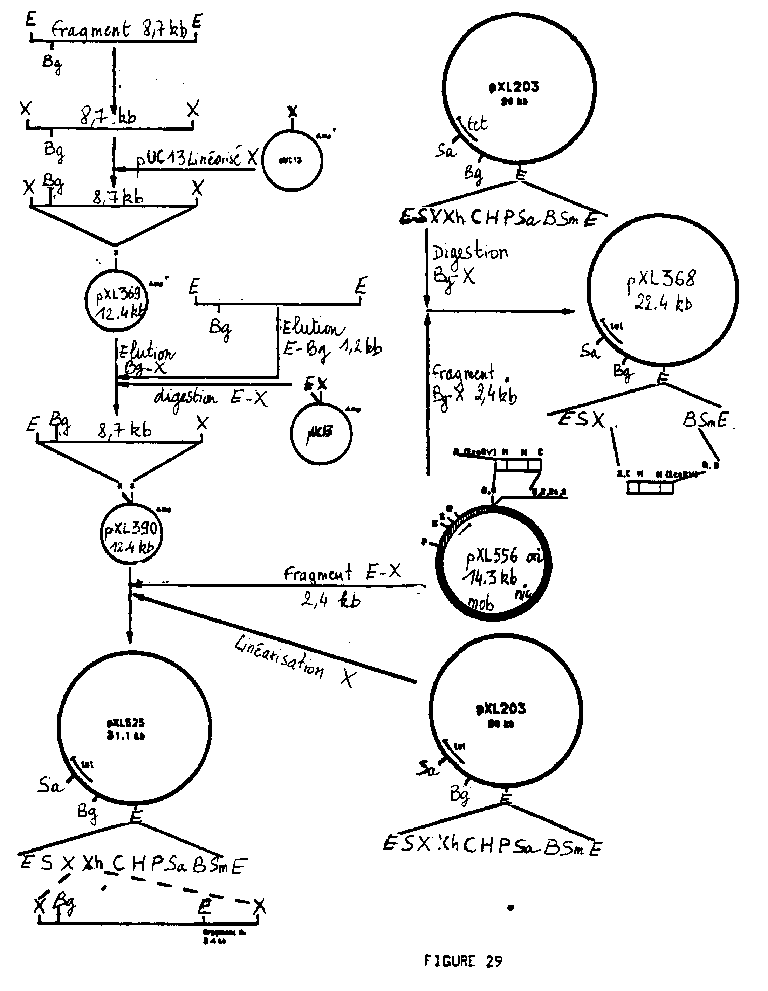

**8.1 - Amplification chez P.denitrificans**
Cet exemple illustre comment une amélioration de la production de cobalamines est obtenue chez Pseudomonas denitrificans SC510 Rif^{r} par amplification de gènes cob de Pseudomonas denitrificans SC510.
8.1.1 Amélioration de la production de cobalamines chez Pseudomonas denitrificans par levée d'une étape limitante dans la biosynthèse des cobalamines
   Cet exemple illustre comment la productivité en cobalamines de souches de Pseudomonas denitrificans peut être améliorée par amplification de gènes cob de Pseudomonas denitrificans. Cette amélioration résulte de la levée d'une étape limitante de la voie de biosynthèse.
   Le plasmide pXL367 est décrit à l'exemple 4.2 (figure 13). Ce plasmide correspond au pRK290 (Ditta et al., 1981) dans lequel le fragment EcoRI de 8,7 kb a été inséré. Ce plasmide pXL367 procure une amélioration de la biosynthèse de cobalamines chez la souche SC510 Rif^{r}. Les souches SC510 Rif^{r}, SC510 Rif^{r} pRK290 et SC510 Rif^{r} pXL367 sont cultivées en erlenmeyer dans du milieu PS4 suivant les conditions décrites dans les protocoles expérimentaux. On observe une amélioration du titre de production due à la présence du plasmide pXL367. En effet la souche SC510 Rif^{r} pXL367 produit 30 % de plus de cobalamines que les souches SC510 Rif^{r} et SC510 Rif^{r} pRK290. Cette amélioration n'est pas due à l'amplification de n'importe quels gènes de Pseudomonas denitrificans mais à l'amplification spécifique de gènes portés par le fragment EcoRI de 8,7 kb. En effet le plasmide pXL723 décrit sur la figure 11 ne donne aucune amélioration et le même titre de production est observé avec ce plasmide qu'avec les souches SC510 Rif^{r} et SC510 Rif^{r} pRK290.
8.1.2 Amélioration de la production de coenzyme B₁₂ chez Pseudomonas denitrificans par levée de deux étapes limitantes dans la biosynthèse des cobalamines
   Cet exemple illustre comment la productivité en cobalamines de souches de Pseudomonas denitrificans peut être améliorée par amplification de gènes cob de Pseudomonas denitrificans. Cette amélioration résulte de la levée de deux étapes limitantes de la voie de biosynthèse.
   Le fragment ClaI-EcoRV de 2,4 kb issu du fragment de 5,4 kb (contenant les gènes cobA et cobE) est cocloné avec le fragment EcoRI de 8,7 kb sur le plasmide à large spectre d'hôte pXL203. Le plasmide ainsi construit est appelé pXL525 (figure 29). Ce plasmide est introduit chez SC510 Rif^{r} par conjugaison. La souche SC510 Rif^{r} pXL525 produit 20 % de cobalamines de plus que SC510 Rif^{r} pXL367. L'amplification des gènes cobA et cobE permet de lever une nouvelle étape limitante chez SC510 Rif^{r} dans la biosynthèse des cobalamines. La souche SC510 Rif^{r} de Pseudomonas denitrificans est améliorée dans le présent exemple par la levée successive de deux étapes limitantes. Cet exemple montre que la levée de deux étapes limitantes dans la biosynthèse des cobalamines peut conduire à des améliorations supplémentaires de production.

**8.2 - Amélioration de la productivité de cobalamines chez Agrobacterium tumefaciens**
Cet exemple illustre l'amélioration de la production de cobalamines d'une souche productrice de cobalamines par l'amplification de gènes cob de Pseudomonas denitrificans SC510.
La souche utilisée est une souche de bactérie gram-négative ; il s'agit d'une souche d'Agrobacterium tumefaciens.
Les plasmides décrits dans les exemples 4.2 et 8.1, pXL367 et pXL525 ainsi que le vecteur pRK290 (Ditta et al., 1981) et le plasmide pXL368 (figure 29), sont introduits par transfert conjugatif chez la souche d'Agrobacterium tumefaciens C58-C9 Rif^{r} (Cameron et al., 1989). Les souches C58-C9 Rif^{r}, C58-C9 Rif^{r} pRK290, C58-C9 Rif^{r} pXL367, C58-C9 Rif^{r} pXL368 et C58-C9 Rif^{r} pXL525 sont cultivées en milieu PS4 à 30°C comme cela est décrit précédemment. Les cobalamines produites sont dosées comme cela est décrit précédemment. Les titres de productions sont portés sur le tableau ci-dessous.

**Tableau :**

| Titres de vitamine B₁₂ produite par différentes souches d'Agrobacterium tumefaciens recombinantes. | |
|---|---|
| Souche | Vitamine B₁₂ en mg/l |
| C58-C9 Rif^{r} | 0.4 |
| C58-C9 Rif^{r} pRK290 | 0.4 |
| C58-C9 Rif^{r} pXL367 | 0.8 |
| C58-C9 Rif^{r} pXL368 | 0.8 |
| C58-C9 Rif^{r} pXL525 | 1.2 |

Comme il apparaît clairement sur le tableau ci-dessus, la production de cobalamines est améliorée chez la souche d'Agrobacterium tumefaciens utilisée. Deux plasmides différents améliorent la production de cobalamines de la souche d'Agrobacterium tumefaciens utilisée: pXL367 et pXL368. Ces plasmides contiennent respectivement le fragment EcoRI de 8,7 kb (gènes cobF à cobM) et le fragment ClaI-EcoRV de 2,4 kb (gène cobE et cobA). Séparément, ils améliorent la production de cobalamines d'Agrobacterium tumefaciens C58-C9 Rif^{r} d'un facteur 2; ce résultat montre qu'il est possible d'améliorer la production de cobalamines d'une souche d'Agrobacterium tumefaciens en amplifiant des fragments portant des gènes cob de Pseudomonas denitrificans. Dans le cas présent on peut parler d'amélioration hétérologue, c'est-à-dire d'amélioration de la production de cobalamines d'une souche moyennant l'amplification de gènes cob d'une autre souche.
Les améliorations de production de cobalamines apportées par les différents fragments de Pseudomonas denitrificans contenant des gènes cob sont cumulables, c'est-à-dire qu'en mettant sur le même plasmide les deux fragments qui sont séparément clonés sur le pXL367 et le pXL368, on observe des améliorations additives. Le plasmide pXL525 apporte chez Agrobacterium tumefaciens C58-C9 Rif^{r} une amélioration de production supérieure à celle apportée par chacun des fragments clonés séparément sur le même vecteur.
**8.3 - Amélioration de la productivité de cobalamines chez Rhizobium meliloti**
Cet exemple décrit l'amélioration de la production de cobalamines d'une autre souche productrice de cobalamines.
Le plasmide décrit à l'exemple 8.2, pXL368, ainsi que le vecteur pRK290 (Ditta et al., 1981) sont introduits par transfert conjugatif chez la souche Rhizobium meliloti 102F34 Rif^{r} (Leong et al., 1982). Les transconjugants: 102F34 Rif^{r}, 102F34 Rif^{r} pRK290 et 102F34 Rif^{r} pXL368 sont cultivés en milieu PS4 à 30°C comme cela est décrit précédemment. Les cobalamines produites sont dosées comme cela est décrit précédemment. Les titres de productions sont portés sur le tableau ci-dessous.

**Tableau :**

| Titres des cobalamines produites par différentes souches de Rhizobium meliloti recombinantes | |
|---|---|
| Souche | Vitamine B₁₂ en mg/l |
| 102F34 Rif^{r} | 0.4 |
| 102F34 Rif^{r} pRK290 | 0.4 |
| 102F34 Rif^{r} pXL368 | 0.8 |

Comme il apparaît clairement sur le tableau ci-dessus, la production de cobalamines est améliorée chez la souche de Rhizobium meliloti utilisée. Le plasmide pXL368 améliore la production de cobalamines de la souche de Rhizobium meliloti utilisée. Ce plasmide contient le fragment ClaI-EcoRV de 2,4 kb (gènes cobA et cobE); il améliore la production de cobalamines de Rhizobium meliloti 102F34 Rif^{r} d'un facteur 2. Ce résultat montre qu'il est possible d'améliorer la production de cobalamines d'une souche de Rhizobium meliloti en amplifiant des fragments portant des gènes cob de Pseudomonas denitrificans. Dans le cas présent on peut parler d'amélioration hétérologue, c'est-à-dire d'amélioration de la production de cobalamines d'une souche moyennant l'amplification de gènes cob d'une autre souche.

### EXEMPLE 9 - Dosage des corrinoïdes et des descobaltocorrinoïdes dans les moûts et les cellules de souches productrices de corrinoïdes

Cet exemple illustre comment il est possible d'identifier et de doser les différents corrinoïdes et descobaltocorrinoïdes produits par différentes souches productrices de cobalamines. Ce dosage permet entre autre de doser le coenzyme B₁₂.

Les moûts (ou les cellules seules) sont cyanurés comme cela a déjà été décrit (Renz, 1971). Après centrifugation, une aliquote du surnageant est passée à travers une colonne de DEAE-Sephadex qui est ensuite lavée extensivement avec du dihydrogenophosphate de potassium 1M. Les fractions recueillies sont regroupées et dessalées sur une cartouche Sep-Päk C-18 (Waters). Après évaporation et resuspension dans l'eau (100 µl à 1 ml suivant la quantité de corrinoïdes présents), les corrinoïdes sont identifiés et dosés par CLHP sur une colonne de Nucléosil C-18 (Macherey-Nagel). La colonne est éluée à 1 ml/mn avec un gradient d'acétonitrile (de 0 % à 100 %) dans du tampon phosphate de potassium 0,1 M contenant 10 mM de KCN.

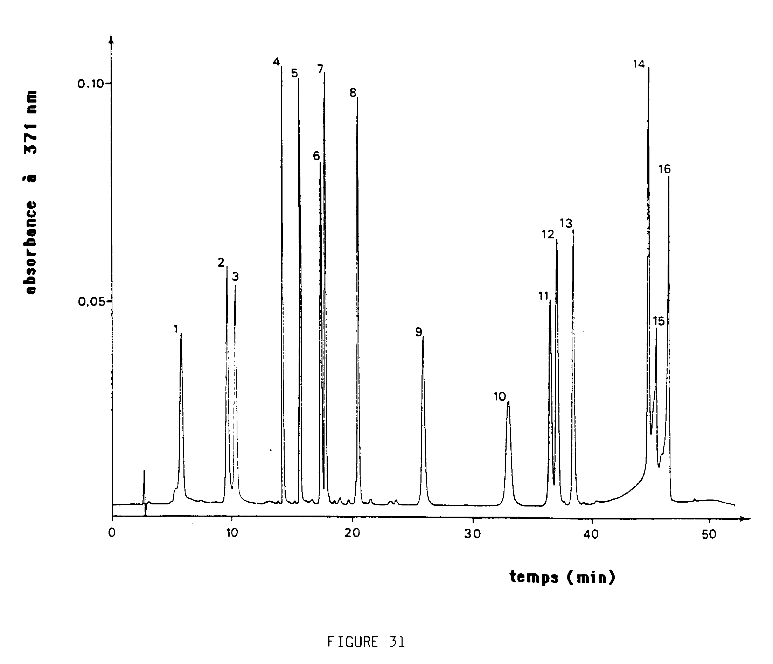

Les corrinoïdes sont visualisés en détection U.V. à 371 nm et/ou par la détection spécifique du ⁵⁷Co (si la culture a été effectuée en présence de ⁵⁷CoCl₂) à l'aide d'un détecteur Berthold LB 505. Ils sont donc identifiés et par comparaison de leur temps de rétention avec des étalons. De même, les descobaltocorrinoïdes (acide hydrogénobyrinique, acide hydrogénobyrinique monoamide et acide hydrogénobyrinique diamide) sont visualisés en détection U.V. à 330 nm. Par cette technique les intermédiaires suivants sont séparés: l'acide cobyrinique, l'acide cobyrinique monoamide, l'acide cobyrinique diamide, l'acide cobyrinique triamide, l'acide cobyrinique tétraamide, l'acide cobyrinique pentaamide, l'acide cobyrique, le cobinamide, le cobinamide phosphate, le GDP-cobinamide, la vitamine B₁₂-phosphate et la vitamine B₁₂. Les formes adénosylées de ces produits sont aussi séparées et dosées par cette technique. Pour ce faire, l'étape initiale de cyanuration est supprimée et la colonne CLHP est éluée avec du tampon dépourvu de KCN. Nous donnons à la figure 31 les temps de rétention de différents étalons séparés par ce système et identifié à la sortie de la colonne par absorbance U.V.

Un échantillon de la souche SC510 Rif^{R} a été déposé le 30 janvier 1990 au Centraal Bureau voor Schimmelcultures à Baarn (Pays-Bas) où il a été enregistré sous la référence CBS 103.90.

### Références bibliographiques.

Ausubel F. M., Brent R., Kinston R. E., Moore D. D.,Smith J. A., Seidman J. G. and K. Struhl. 1987. Current protocols in molecular biology 1987-1988. John Willey and Sons, New York.
Bagdasarian, M., R. Lurz, B. Rückert, F. C. Franklin, M. M. Bagdasarian, J. Frey, and K. Timmis. 1981. Specific-purpose plasmid cloning vectors. II. Broad host range, high copy number, RSF1010-derived vectors, and a host vector system for gene cloning in Pseudomonas. Gene 16:237-247.
Barrère G, Geneste B., Sabatier A., 1981. Fabrication de la vitamine B12: l'amélioration d'un procédé. Pour la Science, 49, 56-64.
Battersby A. R., Fookes C. J. R., Matcham G. W. J., MacDonald E., 1980. Biosynthesis of the pigments of life: formation of the macrocycle. Nature, 285, 17-21.
Battersby, A. R., and E. MacDonald. 1982. Biosynthesis of the corrin macrocycle. p. 107-144. In D. Dolphin (ed.), B12, voi. 1. John Willey & Sons, Inc., New-York.
Beck., W.S. 1982. Biological and medical aspects of vitamin B12. p 1-30. In D. Dolphin (ed.), B12, vol. 1. John Willey & Sons, Inc., New-York.
Ben Bassat A., and K. Bauer. 1987. Amino-terminal processing of proteins. Nature, 326:315.
Blanche F., L. Debussche, D. Thibaut, J. Crouzet and B. Cameron. 1989. Purification and Characterisation of S-Adenosyl-L-Methionine:Uroporphyrinogen III methyltransferase from Pseudomonas denitrificans. J. Bacteriol., 171:4222-4231.
Brey R. N., Banner C. D. B., Wolf J. B., 1986. Cloning of Multiple Genes Involved with Cobalamin (Vitamin B12) Biosynthesis in Bacillus megaterium. J. Bacteriol., 167, 623-630.
Cameron B;, K. Briggs, S; Pridmore, G. Brefort and J. Crouzet, 1989. Cloning and analysis of genes involved in coenzyme B12 biosynthesis in Pseudomonas denitrificans . J. Bacteriol, 171, 547-557.
Casadaban, M. J., A. Martinez-Arias, S. T. Shapira and J. Chou. 1983. β-galactosidase gene fusion for analysing gene expression in Escherichia coli and Yeast. Methods Enzymol. 100, 293-308.
De Bruijn F. J. and J. R. Lupski. 1984. The use of transposon Tn5 mutagenesis in the rapid generation of correlated physical and genetic maps of DNA segments cloned into multicopy plasmids-a review. Gene, 27, 131-149.
De Graff, J., J. H. Crosa, F. Heffron, and S. Falkow. 1978. Replication of the nonconjugative plasmid RSF1010 in Escherichia coli K-12. J. Bacteriol. 146, 117-122.
Denèfle P., S. Kovarik, J.-D. Guiton, T. Cartwright and J.-F. Mayaux. 1987. Chemical synthesis fo a gene coding for human angiogenin, its expression in Escherichia coli and conversion of the product into its active form. Gene, 56, 61-70.
Ditta G., Schmidhauser T., Yakobson E., Lu P., Liang X.-W., Finlay D. R., Guiney D. and D. R. Helinski. 1985. Plasmids related to the broad host range vector pRK290, useful for gene cloning and for monitoring gene expression. Plasmid, 13, 149-154.
Ditta, G., S. Stanfield, D. Corbin, and D. R. Helinski. 1980. Broad host range DNA cloning system for Gram-negative bacteria: Construction of a gene library of Rhizobium melitoti. Proc. Natl. Acad. Sci. USA 77, 7347-7351.
Escalante-Semerena J. C. and J. R. Roth. 1987. Regulation of the cobalamin biosynthetic operons in Salmonella typhimurium. J. Bacteriol, 169, 225-2258.
Florent, J. 1986. Vitamins. p115-158. In H.-J. Rehm and G. Reed (ed.), Biotechnology, vol.4, VCH Verlagsgesellschaft mbH, Weinheim.
Friedmann H. C. and L. M. Cagen. 1970. Microbial biosynthesis of B12-like compounds. Ann. Rev. Microbiol., 24, 159-208.
Friedmann H. C., 1968. Vitamin B12 biosynthesis. J. Biol. Chem., 243, 2065-2075.
Friedmann H. C., 1975. Biosynthesis of corrinoids. In Babior B. M., Cobalamin, 75-110, John Wiley and Sons, New York.
Henikoff S. 1984. Unidirectional digestion with exonuclease III creates targeted breakpoints for DNA sequencing. Gene, 28, 351-359.
Hirel Ph-H, J.-M. Schmitter, P. Dessen and S. Blanquet. 1989. Extent of N-terminal methionine excision within E. coli proteins is governed by the side chain of the penultimate aminoacids. Proc. Natl. Acad. USA, sous presse.
Hopp T. P. and K. R. Woods. 1981. Prediction of protein antigenic determinants from amino acids sequences. Proc. Natl. Acad. Sci. USA, 78-3824-3828.
Huennekens F. M., Vitols K. S., Fujii K., Jacobsen D. W., 1982. Biosynthesis of cobalamin coenzyme. In Dolphin D., B12, vol. 1, 145-167, John Willey & Sons, New York.
Irion R., Ljungdahl L. G., 1965. Isolation of factor IIIm coenzyme and cobyric acid coenzyme plus other B12 factors from Clostridium thermoaceticum. Biochemistry, 4, 2780-2790.
Jeter R. M., Olivera B. M., Roth J. R., 1984. Salmonella typhimurium synthetises cobalamin (vitamin B12) de novo under anaerobic growth conditions. J. Bacteriol., 159, 206-213.
Jeter, R. M. and J. R. Roth. 1987. Cobalamin (Vitamin B12) Biosynthetic Genes of Salmonella typhimurium. J. Bacteriol. 169, 3189-3198.
Jorgensen R. A., Rothstein S. J., Reznikoff W. R., 1979. A restriction enzyme cleavage map of Tn5 and location of a region encoding neomycin resistance. Molec. Gen. Genet., 177, 65-62.
Kanangara C. G., S. P. Gough, P. Bruyant, J. K. Hoober, A. Kahn and D. von Wettstein. 1988. tRNA^{Glu} as a cofactor in d-aminolevulinate biosynthesis: steps that regulate chlorphyll synthesis. Trends in Biochem. Sci., 139-143.
Kanehisa M. 1984. Use of statistical criteria for screening potential homologies in nucleic acids sequences. Nucleic Acids Res., 12:203-215.
Kieny M. P., R. Lathe and J. P. Lecocq. 1983. New versatile cloning vectors based on bacteriophage M13. Gene, 26, 91-99.
Krzycki J. and J. G. Zeikus. Quantification of corrinoids in methanogenic bacteria.1980. Curr. Microbiol., 3, 243-245.
L. Skatrud, A. J. Tietz, T. D. Ingolia, C. A. Cantwell, D. L. Fisher, J. L. Chapman and S. W. Queener. 1989. Use of recombinant DNA to improve production od cephalosporin C by Cephalosporium acremonium. Bio/Technology, 1989, 7, 477-485.
Laemli U. K., 1970. Cleavage of structural proteins during the assembly of the head of bacteriophage T4. Nature, 227, 680-685.
Leong S. A., Ditta G. S., Helinski D. R. , 1982. Heme Biosynthesis in Rhizobium. Identification of a cloned gene coding for d-aminolevulinic acid synthetase from Rhizobium melitoti. J. Biol. Chem. , 257, 8724-8730.
Macdonald H. and J. Cole. Molecular cloning and fuctional analysis of the cysG and nirB genes of E. coli K12, Two closely-linked genes required for NADH-dependant reductase activity. submitted to publication.
Maniatis, T., E. F. Fritsch, and J. Sambrook. 1982. Molecular cloning: a laboratory manual. Cold Spring Harbor Laboratory, Cold Spring Harbor, New York.
Mazumder T. K., N. Nishio, M. Hayashi and S. Nagai. 1987. Production of corrinoids including vitamin by Methanosarcina barkeri. 1986. Biotechnol. Letters, 12, 843:848.
Mazumder T. K., N. Nishio, S. Fukuzaki and S. Nagai. 1987. Production of Extracellular vitamin B12 compounds from methanol by Methanosarcina barkeri. Appl. Mcrobiol. Biotechnol., 26, 511-516.
Miller, J. H. 1972. Experiment in molecular genetics. Cold Spring Harbor Laboratory, Cold Spring Harbor, New-York.
Monod J. and E. Wollman. 1947. Inhibition de la croissance et de l'adaptation enzymatique chez les bactéries infectées par le bactériophage. Ann. Inst Pasteur, 73, 937-956.
Murphy M. J., Siegel L. M, Kamin H., Rosenthal D., 1973. Identification of a new class of heme prosthetic group: an iron-tetrahydroporphyrin (isobacteriochlorin type) with eigth carboxilic acid groups. J. Biol. Chem., 248, 2801-2814.
Murphy M. J., Siegel L. M., 1973. The basis for a new type ot porphyrin-related prosthetic group common to both assimilatory and dissimilatory sulfite reductases. J. Biol. Chem., 248, 6911-6919.
Nexo E., Olesen H., 1982. Intrinsic factor, transcobalamin and haptocorrin. In Dolphin D., B12, 57-85, John Willey & Sons, New York.
Normark S., S. Bergtröm, T. Edlund, T. Grundström, B. Jaurin, F. Lindberg and O. Olsson. 1983. Overlapping genes. Ann. Rev. Genet., 17, 499-525.
Norrander J., T. Kempe and J. Messing. 1983. Construction of improved M13 vectors using oligodeoxynucleotide-directed mutagenesis. Gene 26, 101-106.
Noyes R., 1970. Vitamin B12 manufacture, 145-182, Noyes developpement S.A., Park Ridge, N. J., USA.
Prentki P. and H. M. Krisch. 1984. In vitro insertional mutagenesis with a selectable DNA fragment. Gene, 29, 303-313.
Renz P. 1970. Some intermediates in the biosynthesis of vitamin B₁₂. Methods in Enzymol., 18, 82-92.
Rigby P. W. J., Dieckmann M., Rhodes C., Berg P., 1977. Labeling deoxyribonucleic acid to high specific activity in vitro by nick translation with DNA polymerase I. J. Mol. Biol., 113,237.
Roof D. M. and J. R. Roth. 1988. Ethanolamine utilization in Samonella typhimurium. J. Bacteriol., 170, 3855-3863.
Sanger F., S. Nicklen and A. R. Coulson. 1977. DNA sequencing with chain-terminating inhibitors. Proc. Natl. Acad. Sci., 74, 5463-5468.
Saunders G., Tuite M. F., Holt G., 1986. Fungal cloning vectors. Trends Biotechnol., 4, 93-98.
Scherer P., Höllriegel V., Krug C., Bokel M., Renz P., 1984. On the biosynthesis of 5-hydroxybenziumidazolylcobamide (vitamin B12-factor III) in Methanosarcina barkeri. Arch. Microbiol., 138, 354-359.
Schneider Z., Friedmann H., 1972. Studies on enzymatic dephosphorylation of vitamin B12 5'-phosphate. Arch. Biochem. Biophys., 152, 488-495.
Scott A. I., N. E. Mackenzie, P. J. Santander, P. E. Fagerness, G. Muller, E. Schneider, R. Seldmeier, and G. Worner. 1984. Biosynthesis of vitamin B12-Timing of the methylation steps between uro'gen III and cobyrinic acid. Bioorg. Chem. 12:356-352.
Southern E., 1975. Detection of specific sequences among DNA fragments separated by gel electrophoresis. J. Mol. Biol., 98, 503-517.
Stachel S. E., G. An, C. Flores and E. W. Nester. 1985. A Tn3lacZ transposon for the random generation of b-galactosidase gene fusions: application to the analysis of gene expression in Agrobacterium. Embo J., 4, 891-898.
Staden R. and A. D. McLachlan. 1982. Codon preference and its use in identifying protein coding regions in long DNA sequences. Nucleic Acid Res., 10, 141-156.
Stupperich E., I. Steiner and H. J. Eisinger. 1987. Substitution of Coa-(5-Hydroxybenzimidazolyl)Cobamide (Factor III) by vitamin B12 in Methanobacterium thermoautotrophicum. J. Bacteriol., 169:3076-3081.
Taylor J. W., J. Ott and F. Eckstein. 1985. The rapid generation of oligonucleotide-directed mutations at high frequency using phophorothioate-modified DNA. Nucl. Acid Res., 13, 8764-8765.
Viera J., Messing J., 1982. The pUC plasmids, an M13mp7-derived system for insertion mutagenesis and sequencing with synthetic universal primers. Gene, 19, 259-268.
Wein-Hsiung L., L. Chi-Cheng and W. Chung-I. 1985. Evolution of DNA sequences. p 1-94. In R. J. MacIntyre (ed.), Molecular Evolutionary genentics. Plenum Press, New york and London.
Latta, M., M. Philit, I. Maury, F. Soubrier, P. Denèfle and J.-F. Mayaux. 1990. Tryptophan promoter derivatives on multicopy plasmids : a comparative analysis of the expression potentials en Escherichia coli. DNA Cell Biol., 9, 129-137.
Mayaux, J.-F., E. Cerbelaud, F. Soubrier, D. Faucher and D. Pétré. 1990. Purification, cloning and primary structure of an enantioselective amidase from Brevibacterium sp. R312. Structural evidence for a genetic coupling with nitrile-hydratase. 1990. J. Bacteriol., 172, 6764-6773.
Belyaev, S. S., R. Wolkin, W. R. Kenealy, M. J. De Niro, M. J. Epstein and J. G. Zeikus. 1983. Methanogenic bacteria from Bondyurhskoe oil field: general characterization and analysis of stable-carbon isotopic fractionation. Appl. Environ. Microbiol., 45, 691-697.
Saiki, R. K., D. H. Gelfand, S. Stoffel, S. Scharf, R. Higuchi, G. T. Horn, K. B. Mullis and H. A. Erlich. 1988. Primer-directed enzymatic amplification of DNA with a thermostable DNA polymerase. Science, 239, 487-491.
Souillard, N., M. Magot, O. Possot and L. Sibold. 1988. Nucleotide sequence of regions homologous to NifH (nitrogenase Fe protein) from the nitrogen fixing archaebacteria Methanococcus thermolithotrophicum and Methanobacterium ivanovi : evolutionary implications. J. Mol. Evol., 2, 65-76.
Chen, E. L. and P. H. Seeburg. 1985. Supercoil sequencing : a fast and simple method for sequencing plasmid DNA. DNA, 4, 165-170.
Saiki R. K., D. H. Gelfand, S. Stoffel, S. J. Scharf, R. Higuchi, G. T. Horn, K. B. Mullis and H. Erlich. 1988. Primer-directed enzymatic amplification of DNA with a thermostable DNA polymerase. Science, 239, 487-491.
Grunstein M., Hogness D., 1975. Colony hybridisation: a method for the isolation of cloned DNAs that contains a specific gene. Proc. Natl. Acad. Sci. USA, 72, 3961-3971.
Cossart, P. and B. Gicquel-Sanzey. 1982. Cloning and sequence of the crp gene of Escherichia coli K 12. Nucleic Accid Res., 10, 1363-1378.
Viera, J. and J. Messing. 1987. Production of single stranded plasmid DNA. Meth. Enzymol., 153, 3-11.
Barbieri P. G., Boretti A., Di Marco A., Migliacci A., and Spalla C. 1962. Further observations on the biosynthesis of vitamin B12 in Nocardia rugosa. Biochim. Biophys. Acta., 57, 599-600.
Renz P. 1968. Reaktionfolge der enzymatischen synthese von vitamin B12 aus cobinamid bei Propionibacterium shermanii. Z. Physiol. Chem., 349, 979-981.
Ronzio R. A., and Barker H. A. 1967. Enzimic synthesis of guanosine diphosphate cobinamide by extracts of propionic acid bacteria. Biochemistry, 6, 2344-2354.
Thibaut D., Debussche L., and Blanche F. 1990. Biosynthesis of vitamin B12: Isolation of precorrin-6x, a metal-free precursor of the corrin macrocycle retaining five S-adenosylmethionine-derived peripheral methyl groups. Proc. Natl. Acad. Sci., 87, 8795-8799.
Ohta H., and Beck W. S. 1976. Studies of the ribosome-associated vitamin B12s adenosylating enzyme of Lactobacillus leichmannii. Arch. Biochem. Biophys., 174, 713-725.
Brady R. O., Castanera E. G., and Barker H. A. 1962. The enzymatic synthesis of cobamide coenzymes. J. Biol. Chem., 237, 2325-2332.
Fenton W. A., and Rosenberg L. E. 1978. Mitochondrial metabolism of hydroxocobalamin: synthesis of adenosylcobalamin by intact rat liver mitochondria. Arch. Biochem. Biophys., 189, 441-447.
Vitols E., Walker G. A., and Huennekens F. M. 1966. Enzymatic conversion of vitamin B12s to a cobamide coenzyme, α-(5,6-dimethyl-benzimidazolyl)deoxyadenosylcobamide (Adenosyl-B12). J. Biol. Chem., 241, 1455-1461.
Gimsing P., and Beck W. S. 1986. Determination of cobalamins in biological material. Methods Enzymol., 123, 3-14.
Jacobsen W. J., Green R., and Brown K. L. 1986. Analysis of cobalamin coenzymes and others corrinoids by high-performance liquid chromatography. Methods Enzymol., 123, 14-22.

## Revendications

1. Séquence d'ADN partiellement purifiée caractérisée en ce qu'elle code pour un polypeptide impliqué dans la biosynthèse des cobalamines et/ou des cobamides et en ce qu'elle est choisie parmi
. les gènes cobA, cobB, cobC, cobD, cobE, cobF, cobG, cobH, cobI, cobJ, cobK, cobL, cobM, cobN, cobO, cobP, cobQ, cobS, cobT, cobU, cobV, cobW, cobX, corA, présentés aux figures 15, 16, 40, 41, 47 et 52,
. les homologues de ces séquences résultant de la dégénérescence du code génétique,
. les séquences d'origine naturelle, synthétique ou recombinante, qui hybrident et/ou qui présentent des homologies significatives avec ces séquences d'ADN ou avec des fragments de celles-ci, et qui codent pour des polypeptides impliqués dans la biosynthése des cobalamines et/ou des cobamides.

2. Gène partiellement purifié contenant une séquence d'ADN selon la revendication 1.

3. ADN recombinant caractérisé en ce qu'il contient au moins une séquence d'ADN selon l'une des revendications 1 ou 2.

4. ADN recombinant selon la revendication 3 caractérisé en ce que lesdites séquences d'ADN sont placées sous le contrôle de signaux d'expression.

5. ADN recombinant selon la revendication 4 caractérisé en ce que les signaux d'expression peuvent être homologues ou hétérologues de la séquence d'ADN.

6. ADN recombinant selon l'une des revendications 3 à 5 caractérisé en ce qu'il fait partie d'un plasmide d'expression.

7. Plasmide caractérisé en ce qu'il contient au moins une séquence d'ADN selon la revendication 1, et des séquences permettant leur expression.

8. Plasmide selon la revendication 7 caractérisé en ce qu'il contient
. un ADN recombinant selon l'une des revendications 3 à 6,
. un système de réplication,
. au moins un marqueur de sélection.

9. Plasmide selon la revendication 7 caractérisé en ce qu'il est choisi parmi:
- le plasmide pXL1500, contenant l'insert tel que représenté sur la figure 12 portant les gènes cobA, cobB, cobC et cobE ;
- le plasmide pXL723, contenant les gènes cobC et cobD tel que représenté sur la figure 11 ;
- le plasmide pXL302, contenant l'insert tel que représenté sur la figure 12 portant les gènes cobC et cobD ;
- le plasmide pXL1397, contenant l'insert tel que représenté sur la figure 12 portant les gènes cobB et cobC ;
- le plasmide pXL368, contenant les gènes cobA et cobE tel que représenté dans la figure 29 ;
- le plasmide pXL545, contenant le gène cobE tel que représenté sur la figure 11;
- le plasmide pXL545 Ω, correspondant au plasmide pXL545 dans lequel est inséré un gène de résistance à la spectinomycine ;
- le plasmide pXL253, contenant les gènes cobF, cobG, cobH, cobI, cobJ, cobK, cobL, cobM, tel que représenté sur la figure 13 ;
- le plasmide pXL367, contenant les gènes cobF, cobG, cobH, cobI, cobJ, cobK, cobL, cobM, tel que représenté sur la figure 13 ;
- le plasmide pXL1148, contenant les gènes cobH et cobI, tel que représenté sur la figure 24,
- le plasmide pXL1149, contenant le gène cobH tel que représenté sur la figure 24 ;
- les plasmides pXL1496 et pXL1546 contenant le gène cobF, tels que représentés sur la figure 26 ;
- le plasmide pXL525, contenant les gènes cobA, cobE et cobF à cobM, tel que représenté sur la figure 29 ;
- le plasmide pXL843, contenant les gènes cobX, cobS, et cobT, tel que représenté sur la figure 36 ;
- le plasmide pXL699, contenant le fragment BgIII-XhoI identifé par les positions 251-2234 de la séquence présentée figure 33 portant le gène cobV ;
- le plasmide pXL1324, contenant le gène cobU tel que représenté sur la figure 38 ;
- le plasmide pXL618, contenant l'insert tel que représenté à la figure 46 portant le gène cobQ;
- le plasmide pXL623, contenant l'insert tel que représenté à la figure 46 portant le gène cobP ;
- le plasmide pXL593, contenant les gènes cobP et cobW tel que représenté sur la figure 46, et
- le plasmide pXL1909, contenant les gènes cobP, cobW, cobN, et cobO tel que représenté à la figure 46.

10. Cellule dans laquelle a été introduite une séquence d'ADN selon l'une des revendications 1 à 6 ou un plasmide selon l'une des revendications 7 à 9.

11. Polypeptide codé par une séquence selon la revendication 1 caractérisé en ce qu'il intervient dans la transformation du précorrine-3 en 5'déoxy 5'adénosyl(Ado) acid cobyrinique a,c-diamide.

12. Polypeptide selon la revendication 11 caractérisé en ce qu'il contient tout ou partie des séquences peptidiques COBB, COBF, COBG, COBH, COBJ, COBK, COBL, COBM, COBN, COBS et COBT présentées aux figures 15, 16, 40 et 41.

13. Polypeptide selon la revendication 11 caractérisé en ce qu'il catalyse le transfert d'un groupement méthyl aux positions C1, C5, C11, C15, ou C17 intervenant entre le précorrine-3 et l'acide cobyrinique a, c-diamide.

14. Polypeptide selon la revendication 13 caractérisé en ce qu'il contient tout ou partie des séquences peptidiques COBF, COBJ, COBL et COBM présentées à la figure 16.

15. Polypeptide codé par une séquence selon la revendication 1 caractérisé en ce qu'il intervient dans la transformation de l'acide cobyrique en cobinamide.

16. Polypeptide selon la revendication 15 caractérisé en ce qu'il contient tout ou partie des séquences peptidiques COBC et COBD présentées à la figure 15.

17. Polypeptide codé par une séquence selon la revendication 1 caractérisé en ce qu'il possède une activité S-adénosyl-L-méthionine : précorrine-2 méthyl transférase (SP2MT).

18. Polypeptide selon la revendication 17 caractérisé en ce qu'il contient tout ou partie de la séquence peptidique COBI présentée à la figure 16.

19. Polypeptide codé par une séquence selon la revendication 1 caractérisé en ce qu'il possède une activité acide cobyrinique et/ou hydrogénobyrinique a, c-diamide synthase.

20. Polypeptide selon la revendication 19 caractérisé en ce qu'il contient tout ou partie de la séquence peptidique COBB présentée à la figure 15.

21. Polypeptide codé par une séquence selon la revendication 1 caractérisé en ce qu'il possède une activité précorrin-8x mutase.

22. Polypeptide codé par une séquence selon la revendication 1 selon la revendication 21 caractérisé en ce qu'il contient tout ou partie de la séquence peptidique COBH présentée à la figure 16.

23. Polypeptide codé par une séquence selon la revendication 1 caractérisé en ce qu'il contient tout ou partie de la séquence peptidique COBE présentée à la figure 15.

24. Polypeptide codé par une séquence selon la revendication 1 caractérisé en ce qu'il possède une activité nicotinate-nucléotide: diméthylbenzimidazole phosphoribosyltransferase.

25. Polypeptide selon la revendication 24 caractérisé en ce qu'il contient tout ou partie de la séquence peptidique COBU présentée à la figure 41.

26. Polypeptide codé par une séquence selon la revendication 1 caractérisé en ce qu'il possède une activité cobalamine-(5'-phosphate) synthase.

27. Polypeptide selon la revendication 26 caractérisé en ce qu'il contient tout ou partie de la séquence peptidique COBV présentée à la figure 41.

28. Polypeptide codé par une séquence selon la revendication 1 caractérisé en ce qu'il possède une activité cobyric acid synthase.

29. Polypeptide selon la revendication 28 caractérisé en ce qu'il contient tout ou partie de la séquence peptidique COBQ présentée à la figure 47.

30. Polypeptide codé par une séquence selon la revendication 1 caractérisé en ce qu'il possède une activité précorrin-6x reductase.

31. Polypeptide selon la revendication 30 caractérisé en ce qu'il contient tout ou partie de la séquence peptidique COBK présentée à la figure 16.

32. Polypeptide codé par une séquence selon la revendication 1 caractérisé en ce qu'il intervient dans la transformation du cobinamide en GDP-cobinamide

33. Polypeptide selon la revendication 32 caractérisé en ce qu'il possède une activité cobinamide kinase et cobinamide phosphate guanylyltransferase.

34. Polypeptide selon la revendication 33 caractérisé en ce qu'il contient tout ou partie de la séquence peptidique COBP présentée à la figure 47.

35. Polypeptide codé par une séquence selon la revendication 1 caractérisé en ce qu'il contient tout ou partie des séquences peptidiques COBS, COBT et COBX présentées figure 40.

36. Polypeptide codé par une séquence selon la revendication 1 caractérisé en ce qu'il est choisi parmi les protéines CORA, COBB, COBC, COBD, COBE, COBF, COBG, COBH, COBI, COBJ, COBK, COBL, COBM, COBN, COBP, COBQ, COBS, COBT, COBU, COBV, COBW et COBX présentées aux figures 15, 16, 40, 41, 47 et 52.

37. Procédé de production des polypeptides selon les revendications 11 à 36 caractérisé en ce que
. on introduit dans une cellule hôte une séquence d'ADN selon les revendications 1 à 6, ou un plasmide selon les revendications 7 à 9 contenant une telle séquence,
. on cultive cette cellule recombinante dans des conditions d'expression de ladite séquence, et
. on récupère les polypeptides produits.

38. Procédé selon la revendication 37 caractérisé en ce que la cellule hôte peut être choisie parmi les procaryotes, les eucaryotes, les cellules animales ou végétales.

39. Procédé selon la revendication 38 caractérisé en ce que la cellule hôte est une archaebactérie ou une eubactérie.

40. Procédé selon la revendication 39 caractérisé en ce que la cellule hôte est E. coli, Pseudomonas denitrificans, Rhizobium melitoti, Agrobacterium tumefaciens, ou Salmonella typhimurium.

41. Procédé permettant d'augmenter la production de cobalamines et/ou de cobamides ou de leurs précurseurs caractérisé en ce que
. on introduit dans un microorganisme producteur de ces composés, ou potentiellement producteur de ces composés, une ou plusieurs séquences d'ADN selon la revendication 1,
. on cultive le micororganisme ainsi obtenu dans des conditions de synthèse des cobalamines et/ou des cobamides et d'expression de ladite séquence,
. on récupère les cobalamines et/ou les cobamides ou leurs précurseurs produits.

42. Procédé selon la revendication 41 caractérisé en ce que l'on introduit dans le microorganisme une ou plusieurs séquences d'ADN selon l'une des revendications 3 à 6, ou un plasmide selon les revendications 7 à 9 contenant de telles séquences.

43. Procédé selon la revendication 42 caractérisé en ce que la séquence d'ADN introduite dans le microorganisme code pour un polypeptide catalysant une étape limitante de la biosynthèse des cobalamines et/ou des cobamides.

44. Procédé selon la revendication 43 caractérisé en ce que les séquences d'ADN introduites dans le microorganisme codent pour des polypeptides catalysant des étapes limitantes de la biosynthèse des cobalamines et/ou des cobamides.

45. Procédé selon l'une des revendications 41 à 44 caractérisé en ce que le micoorganisme est choisi parmi P. denitrificans, R. melitoti, A. tumefaciens.

46. Procédé selon la revendication 45 caractérisé en ce que le microorganisme est P. denitrificans.

47. Procédé selon la revendication 46 caractérisé en ce que le microorganisme est P. denitrificans SC510 RifR.

48. Procédé selon l'une des revendications 41 à 47 caractérisé en ce que l'on introduit dans le microorganisme un plasmide selon la revendication 9.

49. Procédé selon les revendications 41 à 48 caractérisé en ce que l'on introduit dans la souche P.denitrificans SC510 RifR le plasmide pXL525 contenant les gènes cobA, cobE et cobF à cobM, tel que représenté sur la figure 29.

50. Procédé selon les revendications 41 à 49 caractérisé en ce que les cobalamines et/ou les cobamides produits sont récupérés par
. solubilisation,
. conversion en forme cyano, et
. purification.

51. Procédé selon les revendications 41 à 50 caractérisé en ce que la cobalamine est le coenzyme B12.

52. Procédé selon les revendications 41 à 51 caractérisé en ce que le précurseur est choisi parmi les descobaltocorrinoïdes et les corrinoïdes.

## Patentansprüche

1. Teilgereinigte DNA-Sequenz, dadurch **gekennzeichnet**, daß sie ein an der Biosynthese von Cobalaminen und/oder Cobamiden beteiligtes Polypeptid codiert und daß sie ausgewählt ist aus
. den Genen cobA, cobB, cobC, cobD, cobE, cobF, cobG, cobH, cobI, cobJ, cobK, cobL, cobM, cobN, cobO, cobP, cobQ, cobS, cobT, cobU, cobV, cobW, cobX, corA, die in den Figuren 15, 16, 40, 41, 47 und 52 dargestellt sind,
. den Homologen dieser Sequenzen als Ergebnis der Degeneriertheit des genetischen Codes,
. den Sequenzen natürlichen, synthetischen oder rekombinanten Ursprungs, die mit diesen DNA-Sequenzen oder den Fragmenten davon hybridisieren und/oder signifikante Homologien mit ihnen zeigen und die an der Biosynthese von Cobalaminen und/oder Cobamiden beteiligte Polypeptide codieren.

2. Teilgereinigtes Gen, enthaltend eine DNA-Sequenz nach Anspruch 1.

3. Rekombinante DNA, dadurch **gekennzeichnet,** daß sie mindestens eine DNA-Sequenz nach einem der Ansprüche 1 oder 2 enthält.

4. Rekombinante DNA nach Anspruch 3, dadurch **gekennzeichnet**, daß die DNA-Sequenzen unter der Kontrolle von Expressionssignalen stehen.

5. Rekombinante DNA nach Anspruch 4, dadurch **gekennzeichnet**, daß die Expressionssignale homolog oder heterolog mit der DNA-Sequenz sein können.

6. Rekombinante DNA nach einem der Ansprüche 3 bis 5, dadurch **gekennzeichnet**, daß sie Teil eines Expressionsplasmids ist.

7. Plasmid, dadurch **gekennzeichnet**, daß es mindestens eine DNA-Sequenz nach Anspruch 1 und Sequenzen, die ihre Expression erlauben, enthält.

8. Plasmid nach Anspruch 7, dadurch **gekennzeichnet,** daß es enthält
. eine rekombinante DNA nach einem der Ansprüche 3 bis 6
. ein Replikationssystem
. mindestens einen Selektionsmarker.

9. Plasmid nach Anspruch 7, dadurch **gekennzeichnet,** daß es ausgewählt ist aus:
- dem Plasmid pXL1500, enthaltend die Insertion, wie in Figur 12 dargestellt, mit den Genen cobA, cobB, cobC und cobE;
- dem Plasmid pXL723, enthaltend die Gene cobC und cobD, wie in Figur 11 dargestellt;
- dem Plasmid pXL302, enthaltend die Insertion, wie in der Figur 12 dargestellt, mit den Genen cobC und cobD;
- dem Plasmid pXL1397, enthaltend die Insertion, wie in Figur 12 dargestellt, mit den Genen cobB und cobC;
- dem Plasmid pXL368, enthaltend die Gene cobA und cobE, wie in Figur 29 dargestellt;
- dem Plasmid pXL545, enthaltend das Gen cobE, wie in Figur 11 dargestellt;
- dem Plasmid pXL545 Ω, entsprechend dem Plasmid pXL545, in das ein Spectinomycinresistenzgen insertiert ist;
- dem Plasmid pXL253, enthaltend die Gene cobF, cobG, cobH, cobI, cobJ, cobK, cobL, cobM, wie in Figur 13 dargestellt;
- dem Plasmid pXL367, enthaltend die Gene cobF, cobG, cobH, cobI, cobJ, cobK, cobL, cobM, wie in Figur 13 dargestellt;
- dem Plasmid pXL1148, enthaltend die Gene cobH und cobI, wie in Figur 24 dargestellt;
- dem Plasmid pXL1149, enthaltend das Gen cobH, wie in Figur 24 dargestellt;
- den Plasmiden pXL1496 und pXL1546, enthaltend das Gen cobF, wie in Figur 26 dargestellt;
- dem Plasmid pXL525, enthaltend die Gene cobA, cobE und cobF bis cobM, wie in Figur 29 dargestellt;
- dem Plasmid pXL843, enthaltend die Gene cobX, cobS und cobT, wie in Figur 36 dargestellt;
- dem Plasmid pXL699, enthaltend das BgIII-XhoI-Fragment, identifiziert durch die Positionen 251-2234 der Sequenz, die in Figur 33 dargestellt ist, mit dem Gen cobV;
- dem Plasmid pXL1324, enthaltend das Gen cobU, wie in Figur 38 dargestellt;
- dem Plasmid pXL618, enthaltend die Insertion, wie in Figur 46 dargestellt, mit dem Gen cobQ,
- dem Plasmid pXL623, enthaltend die Insertion, wie in Figur 46 dargestellt, mit dem Gen cobP;
- dem Plasmid pXL593, enthaltend die Gene cobP und cobW, wie in Figur 46 dargestellt, und
- dem Plasmid pXL1909, enthaltend die Gene cobP, cobW, cobN und cobO, wie in Figur 46 dargestellt.

10. Zelle, in die eine DNA-Sequenz nach einem der Ansprüche 1 bis 6 oder ein Plasmid nach einem der Ansprüche 7 bis 9 eingeschleust wurde.

11. Polypeptid, codiert von einer Sequenz nach Anspruch 1, dadurch **gekennzeichnet,** daß es an der Umwandlung von 3-Präcorrin in 5'-Desoxy-5'-adenosyl-(Ado)-cobyrinsäure-a,c-diamid beteiligt ist.

12. Polypeptid nach Anspruch 11, dadurch **gekennzeichnet**, daß es vollständig oder teilweise die Peptidsequenzen COBB, COBF, COBG, COBH, COBJ, COBK, COBL, COBM, COBN, COBS und COBT, dargestellt in den Figuren 15, 16, 40 und 41, enthält.

13. Polypeptid nach Anspruch 11, dadurch **gekennzeichnet,** daß es die Übertragung einer Methylgruppe in die Positionen C1, C5, C11, C15 oder C17, die zwischen dem 3-Präcorrin und dem Cobyrinsäure-a,c-diamid vorkommen, katalysiert.

14. Polypeptid nach Anspruch 13, dadurch **gekennzeichnet**, daß es vollständig oder teilweise die Peptidsequenzen COBF, COBJ, COBL und COBM, die in Figur 16 dargestellt sind, enthält.

15. Polypeptid, codiert von einer Sequenz nach Anspruch 1, dadurch **gekennzeichnet,** daß es an der Umwandlung von Cobyrinsäure in Cobinamid beteiligt ist.

16. Polypeptid nach Anspruch 15, dadurch **gekennzeichnet,** daß es vollständig oder teilweise die Peptidsequenzen COBC und COBD, dargestellt in Figur 15, enthält.

17. Polypeptid, codiert von einer Sequenz nach Anspruch 1, dadurch **gekennzeichnet**, daß es eine S-Adenosyl-L-methionin- : 2-Präcorrin-methyltransferase(SP2MT)-Aktivität besitzt.

18. Polypeptid nach Anspruch 17, dadurch **gekennzeichnet,** daß es vollständig oder teilweise die Peptidsequenz COBI, dargestellt in Figur 16, enthält.

19. Polypeptid, codiert von einer Sequenz nach Anspruch 1, dadurch **gekennzeichnet,** daß es eine Cobyrinsäure- und/oder Hydrogenobyrin-a,c-diamidsynthase-Aktivität besitzt.

20. Polypeptid nach Anspruch 19, dadurch **gekennzeichnet**, daß es vollständig oder teilweise die Peptidsequenz COBB, dargestellt in Figur 15, enthält.

21. Polypeptid, codiert von einer Sequenz nach Anspruch 1, dadurch **gekennzeichnet**, daß es eine Präcorrin-8x-Mutaseaktivität besitzt.

22. Polypeptid, codiert von einer Sequenz nach Anspruch 1 gemäß Anspruch 21, dadurch **gekennzeichnet**, daß es vollständig oder teilweise die Peptidsequenz COBH, dargestellt in Figur 16, enthält.

23. Polypeptid, codiert von einer Sequenz nach Anspruch 1, dadurch **gekennzeichnet,** daß es vollständig oder teilweise die Peptidsequenz COBE, dargestellt in Figur 15, enthält.

24. Polypeptid, codiert von einer Sequenz nach Anspruch 1, dadurch **gekennzeichnet**, daß es eine Nicotinat-Nukleotid- : Dimethylbenzimidazolphosphorribosyltransferase-Aktivität besitzt.

25. Polypeptid nach Anspruch 24, dadurch **gekennzeichnet**, daß es vollständig oder teilweise die Peptidsequenz COBU, dargestellt in Figur 41, enthält.

26. Polypeptid, codiert von einer Sequenz nach Anspruch 1, dadurch **gekennzeichnet**, daß es eine Cobalamin-(5'-phosphat)synthaseaktivität besitzt.

27. Polypeptid nach Anspruch 26, dadurch **gekennzeichnet**, daß es vollständig oder teilweise die Peptidsequenz COBV, dargestellt in Figur 41, enthält.

28. Polypeptid, codiert von einer Sequenz nach Anspruch 1, dadurch **gekennzeichnet**, daß es eine Cobyrinsäuresynthaseaktivität besitzt.

29. Polypeptid nach Anspruch 28, dadurch **gekennzeichnet,** daß es vollständig oder teilweise die Peptidsequenz COBQ, dargestellt in Figur 47, enthält.

30. Polypeptid, codiert von einer Sequenz nach Anspruch 1, dadurch **gekennzeichnet**, daß es eine Präcorrin-6x-Reduktaseaktivität besitzt.

31. Polypeptid nach Anspruch 30, dadurch **gekennzeichnet**, daß es vollständig oder teilweise die Peptidsequenz COBK, dargestellt in Figur 16, enthält.

32. Polypeptid, codiert von einer Sequenz nach Anspruch 1, dadurch **gekennzeichnet**, daß es an der Umwandlung von Cobinamid in GDP-Cobinamid beteiligt ist.

33. Polypeptid nach Anspruch 32, dadurch **gekennzeichnet**, daß es eine Cobinamidkinase- und Cobinamidphosphatguanylyltransferase-Aktivität besitzt.

34. Polypeptid nach Anspruch 33, dadurch **gekennzeichnet**, daß es vollständig oder teilweise die Peptidsequenz COBP, dargestellt in Figur 47, enthält.

35. Polypeptid, codiert von einer Sequenz nach Anspruch 1, dadurch **gekennzeichnet**, daß es vollständig oder teilweise die Peptidsequenzen COBS, COBT und COBX, dargestellt in Figur 40, enthält.

36. Polypeptid, codiert von einer Sequenz nach Anspruch 1, dadurch **gekennzeichnet**, daß es aus den Proteinen CORA, COBB, COBC, COBD, COBE, COBF, COBG, COBH, COBI, COBJ, COBK, COBL, COBM, COBN, COBP, COBQ, COBS, COBT, COBU, COBV, COBW und COBX, dargestellt in den Figuren 15, 16, 40, 41, 47 und 52, ausgewählt ist.

37. Verfahren zur Produktion von Polypeptiden nach den Ansprüchen 11 bis 36, dadurch **gekennzeichnet**, daß man
. in eine Wirtszelle eine DNA-Sequenz nach den Ansprüchen 1 bis 6 oder ein Plasmid nach den Ansprüchen 7 bis 9, enthaltend eine derartige Sequenz, einschleust,
. diese rekombinante Zelle unter Expressionsbedingungen der Sequenz züchtet und
. die so gebildeten Polypeptide isoliert.

38. Verfahren nach Anspruch 37, dadurch **gekennzeichnet**, daß die Wirtszelle aus Prokaryonten, Eukaryonten, tierischen Zellen oder pflanzlichen Zellen ausgewählt werden kann.

39. Verfahren nach Anspruch 38, dadurch **gekennzeichnet**, daß die Wirtszelle ein Archaebakterium oder ein Eubakterium ist.

40. Verfahren nach Anspruch 39, dadurch **gekennzeichnet,** daß die Wirtszelle, E. coli, Pseudomonas denitrificans, Rhizobium melitoti, Agrobacterium tumefaciens oder Salmonella typhimurium ist.

41. Verfahren, das die Vermehrung der Produktion von Cobalaminen und/oder Cobamiden oder ihren Vorläufern erlaubt, dadurch **gekennzeichnet**, daß man
. in einen Mikroorganismus, der diese Verbindungen produziert oder diese Verbindungen produzieren kann, eine oder mehrere DNA-Sequenz(en) nach Anspruch 1 einschleust,
. den so erhaltenen Mikroorganismus unter Bedingungen der Synthese von Cobalaminen und/oder Cobamiden und der Expression der Sequenz züchtet,
. die Cobalamine und/oder Cobamide oder ihre Vorläuferprodukte isoliert.

42. Verfahren nach Anspruch 41, dadurch **gekennzeichnet**, daß man in den Mikroorganismus eine oder mehrere DNA-Sequenz(en) nach einem der Ansprüche 3 bis 6 oder ein Plasmid nach den Ansprüchen 7 bis 9, das derartige Sequenzen enthält, einschleust.

43. Verfahren nach Anspruch 42, dadurch **gekennzeichnet**, daß die in den Mikroorganismus eingeschleuste DNA-Sequenz ein Polypeptid, das eine begrenzende Stufe der Biosynthese der Cobalamine und/oder Cobamide katalysiert, codiert.

44. Verfahren nach Anspruch 43, dadurch **gekennzeichnet**, daß die in den Mikroorganismus eingeschleusten DNA-Sequenzen Polypeptide, die begrenzende Schritte der Biosynthese von Cobalaminen und/oder Cobamiden katalysieren, codieren.

45. Verfahren nach einem der Ansprüche 41 bis 44, dadurch **gekennzeichnet**, daß der Mikroorganismus aus P. denitrificans, R. melitoti, A. tumefaciens ausgewählt wird.

46. Verfahren nach Anspruch 45, dadurch **gekennzeichnet**, daß der Mikroorganismus P. denitrificans ist.

47. Verfahren nach Anspruch 46, dadurch **gekennzeichnet,** daß der Mikroorganismus P. denitrificans SC510 RifR ist.

48. Verfahren nach einem der Ansprüche 41 bis 47, dadurch **gekennzeichnet**, daß man in den Mikroorganismus ein Plasmid nach Anspruch 9 einschleust.

49. Verfahren nach den Ansprüchen 41 bis 48, dadurch **gekennzeichnet**, daß man in den Stamm P. denitrificans SC510 RifR das Plasmid pXL525, das die Gene cobA, cobE und cobF bis cobM, wie in Figur 29 dargestellt, enthält, einschleust.

50. Verfahren nach den Ansprüchen 41 bis 49, dadurch **gekennzeichnet**, daß die so gebildeten Cobalamine und/oder Cobamide durch
. Solubilisation,
. Umwandlung in die Cyanoform und
. Reinigung
isoliert werden.

51. Verfahren nach den Ansprüchen 41 bis 50, dadurch **gekennzeichnet**, daß das Cobalamin das Coenzym B12 ist.

52. Verfahren nach den Ansprüchen 41 bis 51, dadurch **gekennzeichnet**, daß der Vorläufer aus den Deskobaltocorrinoiden und den Corrinoiden ausgewählt wird.

## Claims

1. Partially purified DNA sequence, characterized in that it codes for a polypeptide involved in the biosynthesis of cobalamins and/or cobamides, and in that it is chosen from
. the cobA, cobB, cobC, cobD, cobE, cobF, cobG, cobH, cobI, cobJ, cobK, cobL, cobM, cobN, cobO, cobQ, cobS, cobT, cobU, cobV, cobW, cobX and corA genes presented in Figures 15, 16, 40, 41, 47 and 52,
. homologues of these sequences resulting from the degeneracy of the genetic code, and
. sequences of natural, synthetic or recombinant origin which hybridize and/or which display significant homologies with these DNA sequences or with fragments of the latter, and which code for polypeptides involved in the biosynthesis of cobalamins and/or cobamides.

2. Partially purified gene containing a DNA sequence according to claim 1.

3. Recombinant DNA, characterized in that it contains at least one DNA sequence according to either of claims 1 and 2.

4. Recombinant DNA according to claim 3, characterized in that the said DNA sequences are placed under the control of expression signals.

5. Recombinant DNA according to claim 4, characterized in that the expression signals may be homologous or heterologous to the DNA sequence.

6. Recombinant DNA according to one of claims 3 to 5, characterized in that it forms part of an expression plasmid.

7. Plasmid, characterized in that it contains at least one DNA sequence according to claim 1, and sequences permitting their expression.

8. Plasmid according to claim 7, characterized in that it contains
. a recombinant DNA according to one of claims 3 to 6
. a replication system, and
. at least one selectable marker.

9. Plasmid according to claim 7, characterized in that it is chosen from:
- the plasmid pXL1500, containing the insert as represented in Figure 12, bearing the genes cobA, cobB, cobC and cobE;
- the plasmid pXL723, containing the genes cobC and cobD as represented in Figure 11;
- the plasmid pXL302, containing the insert as represented in Figure 12, bearing the genes cobC and cobD;
- the plasmid pXL1397, containing the insert as represented in Figure 12, bearing the genes cobB and cobC;
- the plasmid pXL368, containing the genes cobA and cobE as represented in Figure 29;
- the plasmid pXL545, containing the gene cobE as represented in Figure 11;
- the plasmid pXL545 Ω, corresponding to the plasmid pXL545 into which a spectinomycin-resistance gene is inserted;
- the plasmid pXL253, containing the genes cobF, cobG, cobH, cobI, cobJ, cobK, cobL and cobM as represented in Figure 13;
- the plasmid pXL367, containing the genes cobF, cobG, cobH, cobI, cobJ, cobK, cobL, cobM as represented in Figure 13;
- the plasmid pXL1148, containing the genes cobH and cobI as represented in Figure 24;
- the plasmid pXL1149, containing the gene cobH as represented in Figure 24;
- the plasmids pXL1496 and pXL1546 containing the gene cobF as represented in Figure 26;
- the plasmid pXL525, containing the genes cobA, cobE and cobF to cobM, as represented in Figure 29;
- the plasmid pXL843, containing the genes cobX, cobS and cobT, as represented in Figure 36;
- the plasmid pXL699, containing the fragment BgIII-XhoI identified by the positions 251-2234 of the sequence presented in Figure 33, bearing the gene cobV;
- the plasmid pXL1324, containing the gene cobU as represented in Figure 38;
- the plasmid pXL618, containing the insert as represented in Figure 46, bearing the gene cobQ;
- the plasmid pXL623, containing the insert as represented in Figure 46, bearing the gene cobP;
- the plasmid pXL593, containing the genes cobP and cobW as represented in Figure 46, and
- the plasmid pXL1909, containing the genes cobP, cobW, cobN and cobO as represented in Figure 46.

10. Cell into which a DNA sequence according to one of claims 1 to 6 or a plasmid according to one of claims 7 to 9 has been introduced.

11. Polypeptide coded by a sequence according to claim 1, characterized in that it participates in the conversion of precorrin-3 to 5'-deoxy-5' -adenosyl (Ado) cobyrinic acid a,c-diamide.

12. Polypeptide according to claim 11, characterized in that it contains all or part of the COBB, COBF, COBG, COBH, COBJ, COBK, COBL, COBM, COBN, COBS and COBT peptide sequences presented in Figures 15, 16, 40 and 41.

13. Polypeptide according to claim 11, characterized in that it catalyses the transfer of a methyl group to positions C-1, C-5, C-11, C-15 or C-17 occurring between precorrin-3 and cobyrinic acid a,c-diamide.

14. Polypeptide according to claim 13, characterized in that it contains all or part of the COBF, COBJ, COBL and COBM peptide sequences presented in Figure 16.

15. Polypeptide coded by a sequence according to claim 1, characterized in that it participates in the conversion of cobyric acid to cobinamide.

16. Polypeptide according to claim 15, characterized in that it contains all or part of the COBC and COBD peptide sequences presented in Figure 15.

17. Polypeptide coded by a sequence according to claim 1, characterized in that it possesses an S-adenosyl-L-methionine:precorrin-2 methyltransferase (SP₂MT) activity.

18. Polypeptide according to claim 17, characterized in that it contains all or part of the COBI peptide sequence presented in Figure 16.

19. Polypeptide coded by a sequence according to claim 1, characterized in that it possesses a cobyrinic and/or hydrogenobyrinic acid a,c-diamide synthase activity.

20. Polypeptide according to claim 19, characterized in that it contains all or part of the COBB peptide sequence presented in Figure 15.

21. Polypeptide coded by a sequence according to claim 1, characterized in that it possesses a precorrin-8x mutase activity.

22. Polypeptide coded by a sequence according to claim 1 according to claim 21, characterized in that it contains all or part of the COBH peptide sequence presented in Figure 16.

23. Polypeptide coded by a sequence according to claim 1, characterized in that it contains all or part of the COBE peptide sequence presented in Figure 15.

24. Polypeptide coded by a sequence according to claim 1, characterized in that it possesses a nicotinatenucleotide:dimethylbenzimidazole phosphoribosyltransferase activity.

25. Polypeptide according to claim 24, characterized in that it contains all or part of the COBU peptide sequence presented in Figure 41.

26. Polypeptide coded by a sequence according to claim 1, characterized in that it possesses a cobalamin-5'-phosphate synthase activity.

27. Polypeptide according to claim 26, characterized in that it contains all or part of the COBV peptide sequence presented in Figure 41.

28. Polypeptide coded by a sequence according to claim 1, characterized in that it possesses a cobyric acid synthase activity.

29. Polypeptide according to claim 28, characterized in that it contains all or part of the COBQ peptide sequence represented in Figure 47.

30. Polypeptide coded by a sequence according to claim 1, characterized in that it possesses a precorrin-6x reductase activity.

31. Polypeptide according to claim 30, characterized in that it contains all or part of the COBK peptide sequence presented in Figure 16.

32. Polypeptide coded by a sequence according to claim 1, characterized in that it participates in the conversion of cobinamide to GDP-cobinamide.

33. Polypeptide according to claim 32, characterized in that it possesses a cobinamide kinase and cobinamide phosphate guanylyltransferase activity.

34. Polypeptide according to claim 33, characterized in that it contains all or part of the COBP peptide sequence presented in Figure 47.

35. Polypeptide coded by a sequence according to claim 1, characterized in that it contains all or part of the COBS, COBT and COBX peptide sequences presented in Figure 40.

36. Polypeptide coded by a sequence according to claim 1, characterized in that it is chosen from the CORA, COBB, COBC, COBD, COBE, COBF, COBG, COBH, COBI, COBJ, COBK, COBL, COBM, COBN, COBP, COBQ, COBS, COBT, COBU, COBV, COBW and COBX proteins presented in Figures 15, 16, 40, 41, 47 and 52.

37. Method for production of the polypeptides according to claims 11 to 36, characterized in that
. a DNA sequence according to claims 1 to 6, or a plasmid according to claims 7 to 9 containing such a sequence, is introduced into a host cell,
. this recombinant cell is cultured under conditions for expression of the said sequence, and
. the polypeptides produced are recovered.

38. Method according to claim 37, characterized in that the host cell can be chosen from prokaryotes, eukaryotes and animal or plant cells.

39. Method according to claim 38, characterized in that the host cell is an archaebacterium or a eubacterium.

40. Method according to claim 39, characterized in that the host cell is E. coli, Pseudomonas denitrificans, Rhizobium meliloti, Agrobacterium tumefaciens or Salmonella typhimurium.

41. Method enabling the production of cobalamins and/or cobamides or of their precursors to be increased, characterized in that
. one or more DNA sequences according to claim 1 is/are introduced into a microorganism productive of these compounds or potentially productive of these compounds,
. the microorganism thereby obtained is cultured under conditions for synthesis of cobalamins and/or cobamides and for expression of the said sequence, and
. the cobalamins and/or cobamides or their precursors produced are recovered.

42. Method according to claim 41, characterized in that one or more DNA sequences according to one of claims 3 to 6, or a plasmid according to claims 7 to 9 containing such sequences, is/are introduced into the microorganism.

43. Method according to claim 42, characterized in that the DNA sequence introduced into the microorganism codes for a polypeptide catalysing a limiting step of the biosynthesis of cobalamins and/or cobamides.

44. Method according to claim 43, characterized in that the DNA sequences introduced into the microorganism code for polypeptides catalysing limiting steps of the biosynthesis of cobalamins and/or cobamides.

45. Method according to one of claims 41 to 44, characterized in that the microorganism is chosen from P. denitrificans, R. meliloti and A. tumefaciens.

46. Method according to claim 45, characterized in that the microorganism is P. denitrificans.

47. Method according to claim 46, characterized in that the microorganism is P. denitrificans SC510 RifR.

48. Method according to one of claims 41 to 47, characterized in that a plasmid according to claim 9 is introduced into the microorganism.

49. Method according to claims 41 to 48, characterized in that plasmid pXL525 containing the cobA, cobE and cobF to cobM genes as represented in Figure 29, is introduced into P. denitrificans strain SC510 RifR.

50. Method according to claims 41 to 49, characterized in that the cobalamins and/or cobamides produced are recovered by
. solubilisation,
. conversion to a cyano form, and
. purification.

51. Method according to claims 41 to 50, characterized in that the cobalamin is coenzyme B₁₂.

52. Method according to claims 41 to 51, characterized in that the precursor is chosen from decobaltocorrinoids and corrinoids.
